# EUROPEAN PATENT APPLICATION

(11) **EP 4 818 320 A1**
(43) Date of publication of application: **07.10.2026**
(21) Application number: 24896677.2
(22) Date of filing: 28.11.2024
(51) Int. Cl.: C12N 15/113, A61K 31/713, A61P 1/16

(54) **RNA INHIBITOR FOR INHIBITING GENE EXPRESSION OF COMPLEMENT SYSTEM AND USE THEREOF**

(30) Priority: 01.12.2023 CN 202311644327; 12.08.2024 CN 202411102595
(71) Applicant: Kylonova (Xiamen) Biopharma Co., Ltd., Xiamen, Fujian 361022 (CN)
(72) Inventor: CUI, Kunyuan, Xiamen, Fujian 361022 (CN); LU, Xueqin, Xiamen, Fujian 361022 (CN)
(74) Representative: Ter Meer und Partner mbB
(86) International application number: PCT/CN2024/135408
(87) International publication number: WO 2025/113592

(57) **Abstract**

The present invention belongs to the field of biomedicine, and specifically relates to an RNA inhibitor inhibiting the gene expression of the complement system or a pharmaceutically acceptable salt thereof. The structure of the RNA inhibitor further contains a carrier structure 5'MVIP and 3'MVIP. The RNA inhibitor provided by the present application interferes with the translation template function of the mRNA of the complement system, continuously and efficiently inhibits the gene expression of the complement system, and continuously reduces the levels of CFB, C5 and C3 proteins of the complement system in blood, which can be used for the treatment and/or prevention of diseases associated with elevated levels of the complement system.

## Description

### Technical Field

The present invention belongs to the field of biomedicine, and specifically relates to an RNA inhibitor inhibiting the gene expression of the complement system and use thereof.

### Background Art

### RNAi

RNAi (RNA interference) was discovered by Andrew Z. Fire et al. in antisense RNA inhibition experiments in Caenorhabditis elegans in 1998, and this process was named RNA interference. This discovery was rated as one of the top ten scientific advances of 2001 by Science magazine, and ranked first among the top ten scientific advances of 2002. Since then, siRNA with RNA interference as the mechanism of action has attracted extensive attention as a potential gene therapy drug. In 2006, Andrew Fire and Craig C. Mello won the Nobel Prize in Physiology or Medicine for their contributions to the research on the mechanism of RNA interference. RNAi can be triggered by double-stranded RNA (dsRNA) in many organisms, including animals, plants and fungi. During RNA inhibition, a nuclease called "Dicer" cleaves or "dices" long dsRNA into small fragments of 21-25 nucleotides in length. These small fragments, called small interfering RNA (siRNA), of which the antisense strand (Guide strand) is loaded onto Argonaute protein (AGO2). AGO2 loading occurs in the RISC-loading complex, a ternary complex composed of Argonaute protein, Dicer and dsRNA-binding protein (referred to as TRBP). During loading, the sense strand (Passenger strand) is cleaved and expelled by AGO2. Then, AGO2 uses the antisense strand to bind to mRNA containing a fully complementary sequence, and then catalyzes the cleavage of these mRNA, resulting in the loss of the mRNA's function as a translation template, thereby preventing the synthesis of related proteins. After cleavage, the cleaved mRNA is released, and the RISC-loading complex loaded with the antisense strand is recycled for another round of cleavage.

According to statistics, among disease-related proteins in the human body, more than about 80% of proteins cannot be targeted by conventional small molecule drugs and biological macromolecular preparations, and are undruggable proteins. Gene therapy aimed at treating diseases through functions such as gene expression and silencing is considered by the industry as the third generation of therapeutic drugs after chemical small molecule drugs and biological macromolecular drugs. This therapy realizes the treatment of diseases at the gene level and is not restricted by undruggable proteins. As the mainstream type of gene therapy, RNAi technology treats diseases at the mRNA level, and has higher efficiency than chemical small molecule drugs and biological macromolecular drugs at the protein level. Using RNAi technology, sense and antisense strand sequences of siRNA with high specificity and good inhibitory effect can be designed according to specific gene sequences. These single-strand sequences are synthesized by solid phase, then the sense strand and antisense strand are paired into siRNA according to the base pairing principle in a specific annealing buffer, and finally delivered to the corresponding target in vivo through a carrier system to degrade the target mRNA, destroy the function of the target mRNA as a translation template, thereby preventing the synthesis of related proteins.

### siRNA Delivery System

siRNA is unstable in blood and tissues and is easily degraded by nucleases. To improve the stability of siRNA, the sense strand and/or antisense strand of siRNA can be modified, but these chemical modifications only provide limited protection against nuclease degradation and may ultimately affect the activity of siRNA. Therefore, a corresponding delivery system is also required to ensure that siRNA safely and efficiently crosses the cell membrane. Due to the large molecular weight, a large number of negative charges, and high water solubility, siRNA cannot smoothly cross the cell membrane to reach the intracellular space by itself.

The basic structure of liposomes is composed of a hydrophilic core and a phospholipid bilayer, which has a phospholipid bilayer similar to biological membranes and high biocompatibility, so liposomes were once the most popular and widely used siRNA carriers. Liposome-mediated siRNA delivery mainly encapsulates siRNA into liposomes to protect siRNA from nuclease degradation, improve the efficiency of siRNA crossing cell membrane barriers, and thus promote cellular uptake. For example, anionic liposomes, pH-sensitive liposomes, immunoliposomes, fusogenic liposomes, cationic liposomes, etc. Although certain progress has been made, liposomes themselves are prone to induce inflammatory reactions, and a variety of antihistamines and hormones such as cetirizine and dexamethasone must be used before administration to reduce possible acute inflammatory reactions. Therefore, they are not suitable for all therapeutic fields in actual clinical application, especially in the treatment of some chronic diseases. The cumulative toxicity that may occur after long-term use is a potential safety hazard, so a safer and more effective carrier system is needed for siRNA delivery.

Asialoglycoprotein receptor (ASGPR) in the liver is a receptor specifically expressed in hepatocytes and a highly efficient endocytic receptor. Under physiological conditions in vivo, after various glycoproteins hydrolyze sialic acid by enzymes or acids, the exposed sub-terminal is a galactose residue, so the sugars specifically bound by ASGPR are galactosyl groups, so it is also called galactose-specific receptor. Monosaccharides and polysaccharide molecules such as galactose, galactosamine, and N-acetylgalactosamine have high affinity for ASGPR. The main physiological function of ASGPR is to mediate the clearance of asialoglycoproteins, lipoproteins and other substances in the blood, and is closely related to the occurrence and development of liver diseases such as viral hepatitis, liver cirrhosis and liver cancer. The discovery of this characteristic of ASGPR plays an important role in the diagnosis and treatment of liver-derived diseases (Ashwell G, Harford J, Carbohydrate specific Receptors of the Liver, Ann Rev Biochem 1982 51:531-554). Liver-derived disease therapeutic drugs containing galactose or galactosamine and derivatives thereof can specifically bind to ASGPR with affinity, thus having active liver targeting without the need for other carrier systems for delivery.

### Complement System

The activation of the complement system includes three pathways: Classical Pathway, Lectin Pathway and Alternative Pathway, which are three common pathways for complement system activation. The main difference between these three pathways lies in the initiation process. After initiation, C3 convertase is formed and further cleaves C3. The C3b fragment binds to the previous complex to form C5 convertase, which cleaves C5b and C5a, causing the assembly of MAC, which lyses cells and stimulates inflammatory responses to eliminate foreign substances.

The Classical Pathway (CP) is usually initiated by the attachment of C1q to immune complexes (such as IgM or IgG). Then C1q activates C1r, thereby changing the conformation of the C1r2-C1s2 structure. C1r releases C1s, and C1s has serine protease (SP) activity to cleave C4 and C2 to form C4b2a (C3 convertase). C3 convertase cleaves C3 and subsequently binds to C3b to form C5 convertase. C4a, C3a and C5a stimulate inflammatory responses. C5b forms C5b-9 MAC with the help of C6, C7, C8 and C9 to attack host cells or pathogens.

The activation of the Lectin Pathway (LP) is the same as that of the Classical Pathway (CP), except that the initiator of LP is mannose-binding lectin (MBL), which forms a multimeric lectin complex by binding to ficolin. This binding leads to the activation of mbl-associated serine proteases (MASPs), thereby triggering the complement system. MASP-1 and MASP-2 are similar to C1r and C1s, respectively. MASP-1 and MASP-2 fully activate the complement system by cleaving C4 and C2 to form C3 convertase.

The Alternative Pathway (AP) differs from the Classical Pathway (CP) in that activation directly activates C3 by bypassing the three components C1, C4 and C2. When pathogen invasion is detected, a large number of C3 thioester domains (TEDs) undergo metastable state, exposing C3 to the factor b (Fb) binding site, and C3b is recognized by factor B to form the complex C3bB. The C3bB complex is in turn cleaved by factor D to produce the active form of C3 convertase (C3bBb). C3 convertase (C3bBb) decomposes C3 into C3a and C3b, and C3b, as an active fragment, binds to pathogens or target cells. In response to C3 convertase, C3b generates C5 convertase, which cleaves C5 to recruit the membrane attack complex (MAC) to lyse pathogens. Both types of C3 convertase (C3bB\C3bBb) cleave C3 to form C3b. C3b then binds more factor B to enhance complement activation through the AP. Alternatively, C3b leads to the formation of active C5 convertase (C3bBbC3b or C4bC2bC3b), which cleaves C5 and triggers the later events leading to the formation of the membrane attack complex (MAC) (C5b-9).

### Diseases Caused by Inappropriate Activation of the Complement System

Inhibitors inhibiting the expression of complement C3 and C5 genes can be used to treat diseases such as paroxysmal nocturnal hemoglobinuria (PNH), atypical hemolytic uremic syndrome, rheumatoid arthritis, Alzheimer's disease (AD), amyotrophic lateral sclerosis (ALS), schizophrenia, Parkinson's disease (PD), prion diseases, ocular diseases related to complement component C3, ischemia-reperfusion injury and neurodegenerative diseases.

Inhibitors inhibiting the expression of complement factor B gene can be used to treat subjects with complement factor B-related disorders such as C3 glomerulopathy, systemic lupus erythematosus (SLE) such as lupus nephritis, IgA nephropathy, diabetic nephropathy and polycystic kidney disease.

### Drug Combination

Existing complement inhibitors on the market include: Soliris, Ultomiris, Empaveli, Eculizumab, Ravulizumab or Iptacopan (LNP023). These polypeptide, monoclonal antibody or small molecule drugs can be used in combination with RNAi inhibitors inhibiting complement system expression, thereby improving the ability to systemically inhibit the complement system. However, most current complement inhibitors are clinically evaluated to be administered once a week to two weeks, with relatively high cost, and can also cause low-level hemolysis in PNH subjects. Therefore, patients with complement system diseases still need a new alternative drug or combination drug.

### Summary of the Invention

In one aspect, the present invention provides an RNA inhibitor inhibiting the gene expression of the complement system or a pharmaceutically acceptable salt thereof.

An RNA inhibitor inhibiting the gene expression of the complement system or a pharmaceutically acceptable salt thereof, wherein the RNA inhibitor is formed by base pairing of a sense strand and an antisense strand with a chain length of 15-30, the chain length is preferably 19-23, wherein the antisense strand comprises a region complementary to the mRNA encoding the complement system, and wherein the complementary region comprises at least 15 consecutive nucleotides differing by 0, 1, 2 or 3 nucleotides from any antisense strand in any one of Table 1-1, Table 1-2 and Table 1-3.

The aforementioned RNA inhibitor inhibiting the gene expression of the complement system or a pharmaceutically acceptable salt thereof, wherein the RNA inhibitor is an RNA inhibitor inhibiting the expression of the CFB gene, wherein the antisense strand comprises a region complementary to a target sequence, and the target sequence is 5' gucuagucaacuuaauugaga 3' SEQ ID NO: 25, starting at position 1157 in NM_001710.5, and there is at least 85% base complementarity between the sense strand and the antisense strand.

The aforementioned RNA inhibitor inhibiting the gene expression of the complement system or a pharmaceutically acceptable salt thereof, wherein the RNA inhibitor is an RNA inhibitor inhibiting the expression of the C5 gene, wherein the antisense strand comprises a region complementary to a target sequence, and the target sequence is 5' uugucccaguauucuauguuu 3' SEQ ID NO: 826, starting at position 3073 in NM_001735.3, and there is at least 85% base complementarity between the sense strand and the antisense strand.

The aforementioned RNA inhibitor inhibiting the gene expression of the complement system or a pharmaceutically acceptable salt thereof, wherein the RNA inhibitor is an RNA inhibitor inhibiting the expression of the C3 gene, wherein the antisense strand comprises a region complementary to a target sequence, and the target sequences are:5' gguguugacagauacaucu 3' SEQ ID NO: 1952, starting at position 4329 in NM_000064.4; 5' ggagccuacagagaaauucua 3' SEQ ID NO: 2039, starting at position 771 in NM_000064.4; the target sequence is 5' agaaauucuacuacaucuaua 3' SEQ ID NO: 2048, starting at position 782 in NM_000064.4; the target sequence is 5' gcugaggagaauugcuucaua 3'SEQ ID NO : 2240, starting at position 4603 in NM_000064.4; the target sequence is 5' ggagaauugcuucauacaaaa 3' SEQ ID NO: 2245, starting at position 4608 in NM_000064.4; and there is at least 85% base complementarity between the sense strand and the antisense strand.

The aforementioned CFB RNA inhibitor or a pharmaceutically acceptable salt thereof, wherein the antisense strand is selected from the following sequence:

| | |
|---|---|
| 5' ucucaauuaaguugacuagacac 3' | SEQ ID NO: 293; |

or a sequence having at least 15 consecutive nucleotides identical to the antisense strand, or a sequence differing by one, two or three nucleotides from the antisense strand, and the sense strand can be any sequence containing at least 85% base complementarity to the antisense strand;
wherein g = guanosine, a = adenosine, u = uridine, c = cytidine, t = thymidine deoxyribonucleotide.

The aforementioned CFB RNA inhibitor or a pharmaceutically acceptable salt thereof, wherein the sense strand is selected from the following sequence:

| | |
|---|---|
| 5' gucuagucaacuuaauugaga 3' | SEQ ID NO: 25; |

or a sequence having at least 15 consecutive nucleotides identical to the sense strand, or a sequence differing by one, two or three nucleotides from the sense strand, and the sense strand can be any sequence containing at least 85% base complementarity to the antisense strand;
wherein g = guanosine, a = adenosine, u = uridine, c = cytidine.

The aforementioned C5 RNA inhibitor or a pharmaceutically acceptable salt thereof, wherein the antisense strand is selected from the following sequence:

| | |
|---|---|
| 5' aaacauagaauacugggacaacg 3' | SEQ ID NO: 1278; |

or a sequence having at least 15 consecutive nucleotides identical to the antisense strand, or a sequence differing by one, two or three nucleotides from the antisense strand, and the sense strand can be any sequence containing at least 85% base complementarity to the antisense strand;
wherein g = guanosine, a = adenosine, u = uridine, c = cytidine, t = thymidine deoxyribonucleotide.

The aforementioned C5 RNA inhibitor or a pharmaceutically acceptable salt thereof, wherein the sense strand is selected from the following sequence:

| | |
|---|---|
| 5' uugucccaguauucuauguuu 3' | SEQ ID NO: 826; |

or a sequence having at least 15 consecutive nucleotides identical to the sense strand, or a sequence differing by one, two or three nucleotides from the sense strand, and the antisense strand can be any sequence containing at least 85% base complementarity to the sense strand;
wherein g = guanosine, a = adenosine, u = uridine, c = cytidine.

The aforementioned C3 RNA inhibitor or a pharmaceutically acceptable salt thereof, wherein the antisense strand is selected from the following sequences:

| | |
|---|---|
| 5' agauguaucugucaacaccau 3' | SEQ ID NO. 2543; |
| 5' uagaauuucucuguaggcuccac 3' | SEQ ID NO. 2630; |
| 5' uauagauguaguagaauuucucu 3' | SEQ ID NO. 2639; |
| 5' uaugaagcaauucuccucagcac 3' | SEQ ID NO. 2831; |
| 5' uuuuguaugaagcaauucuccuc 3' | SEQ ID NO. 2590; |

or a sequence having at least 15 consecutive nucleotides identical to any of the above sequences, or a sequence differing by one, two or three nucleotides from any of the above sequences,
wherein g = guanosine, a = adenosine, u = uridine, c = cytidine, t = thymidine deoxyribonucleotide; and the sense strand can be any sequence containing at least 85% base complementarity to the antisense strand.

The aforementioned C3 RNA inhibitor or a pharmaceutically acceptable salt thereof, wherein the sense strand is selected from the following sequences:

| | |
|---|---|
| 5' gguguugacagauacaucu 3' | SEQ ID NO. 1952; |
| 5' ggagccuacagagaaauucua 3' | SEQ ID NO. 2039; |
| 5' agaaauucuacuacaucuaua 3' | SEQ ID NO. 2048; |
| 5' gcugaggagaauugcuucaua 3' | SEQ ID NO. 2240; |
| 5' ggagaauugcuucauacaaaa 3' | SEQ ID NO. 2245; |

or a sequence having at least 15 consecutive nucleotides identical to any of the above sequences, or a sequence differing by one, two or three nucleotides from any of the above sequences,
wherein g = guanosine, a = adenosine, u = uridine, c = cytidine; and the antisense strand can be any sequence containing at least 85% base complementarity to the sense strand.

The aforementioned CFB RNA inhibitor or a pharmaceutically acceptable salt thereof, wherein the sense strand is SEQ ID NO:25 or a sequence having at least 15 consecutive nucleotides identical thereto, or a sequence differing by one, two or three nucleotides therefrom; and the antisense strand is SEQ ID NO:293 or a sequence having at least 15 consecutive nucleotides identical thereto, or a sequence differing by one, two or three nucleotides therefrom:

| | |
|---|---|
| Sense strand: 5' gucuagucaacuuaauugaga 3' | SEQ ID NO: 25; |
| Antisense strand: 5' ucucaauuaaguugacuagacac 3' | SEQ ID NO: 293; |

wherein g = guanosine, a = adenosine, u = uridine, c = cytidine, t = thymidine deoxyribonucleotide.

The aforementioned C5 RNA inhibitor or a pharmaceutically acceptable salt thereof, wherein the sense strand is SEQ ID NO:826 or a sequence having at least 15 consecutive nucleotides identical thereto, or a sequence differing by one, two or three nucleotides therefrom; and the antisense strand is SEQ ID NO:1278 or a sequence having at least 15 consecutive nucleotides identical thereto, or a sequence differing by one, two or three nucleotides therefrom:

| | |
|---|---|
| Sense strand: 5' uugucccaguauucuauguuu 3' | SEQ ID NO: 826; |
| Antisense strand: 5' aaacauagaauacugggacaacg 3' | SEQ ID NO: 1278; |

wherein g = guanosine, a = adenosine, u = uridine, c = cytidine, t = thymidine deoxyribonucleotide.

The aforementioned C3 RNA inhibitor or a pharmaceutically acceptable salt thereof, wherein the sense strand is SEQ ID NO:2048 or a sequence having at least 15 consecutive nucleotides identical thereto, or a sequence differing by one, two or three nucleotides therefrom; and the antisense strand is SEQ ID NO:2639 or a sequence having at least 15 consecutive nucleotides identical thereto, or a sequence differing by one, two or three nucleotides therefrom:

| | |
|---|---|
| Sense strand: 5' agaaauucuacuacaucuaua 3' | SEQ ID NO: 2048; |
| Antisense strand: 5' uauagauguaguagaauuucucu 3' | SEQ ID NO: 2639; |

wherein g = guanosine, a = adenosine, u = uridine, c = cytidine, t = thymidine deoxyribonucleotide.

The aforementioned C3 RNA inhibitor or a pharmaceutically acceptable salt thereof, wherein the sense strand is SEQ ID NO:2639 or a sequence having at least 15 consecutive nucleotides identical thereto, or a sequence differing by one, two or three nucleotides therefrom; and the antisense strand is SEQ ID NO:2831 or a sequence having at least 15 consecutive nucleotides identical thereto, or a sequence differing by one, two or three nucleotides therefrom:

| | |
|---|---|
| Sense strand: 5' gcugaggagaauugcuucaua 3' | SEQ ID NO: 2240; |
| Antisense strand: 5' uaugaagcaauucuccucagcac 3' | SEQ ID NO: 2831; |

wherein g = guanosine, a = adenosine, u = uridine, c = cytidine, t = thymidine deoxyribonucleotide.

The aforementioned RNA inhibitor or a pharmaceutically acceptable salt thereof, wherein at least one nucleotide of the RNA inhibitor is modified.

The aforementioned RNA inhibitor or a pharmaceutically acceptable salt thereof, wherein the modification comprises: one or a combination of 2'-fluoro modification, 2'-methoxy modification, phosphorothioate modification, invAb modification, glycerol nucleotide, 3'-terminal deoxythymidine (dT) nucleotide, locked nucleotide, unlocked nucleotide, conformationally restricted nucleotide, constrained ethyl nucleotide, 2'-amino modified nucleotide, 2'-O-allyl modified nucleotide, 2'-C-alkyl modified nucleotide, 2'-hydroxy modified nucleotide, 2'-methoxyethyl modified nucleotide, 2'-O-alkyl modified nucleotide, 2'-phosphate modification or 2-O-(N-methylacetamide) modification, morpholino nucleotide, phosphoramidate, abasic nucleotide, abasic deoxynucleotide, nucleotide containing non-natural base, tetrahydropyran modified nucleotide, 1,5-anhydrohexitol modified nucleotide, cyclohexenyl modified nucleotide, methylphosphonate modification, 5'-phosphate modification, 5'-phosphate analog modification, thermolabile nucleotide, and nucleotide analog.

The aforementioned CFB RNA inhibitor or a pharmaceutically acceptable salt thereof, wherein the antisense strand is selected from the following sequence:

| | |
|---|---|
| 5' UsdCsUCdAATUAAGTUfGACUAGACsAsC 3' | SEQ ID NO: 588; |

wherein G = 2'-O-methylguanosine, A = 2'-O-methyladenosine, U = 2'-O-methyluridine, C = 2'-O-methylcytidine; Gs = 2'-O-methyl-3'-phosphorothioguanosine, As = 2'-O-methyl-3'-phosphorothioadenosine, Cs = 2'-O-methyl-3'-phosphorothiocytidine; fG = 2'-fluoroguanosine, fA = 2'-fluoroadenosine, fU = 2'-fluorouridine, fC = 2'-fluorocytidine; fGs = 2'-fluoro-3'-phosphorothioguanosine, T = 2'-deoxythymidine, dA = 2'-deoxyadenosine, dG = 2'-deoxyguanosine; and the sense strand can be any sequence containing at least 85% base complementarity to the antisense strand, with no restriction on the modification mode.

The aforementioned C5 RNA inhibitor or a pharmaceutically acceptable salt thereof, wherein the antisense strand is selected from the following sequence:

| | |
|---|---|
| 5' AsdAsACdAUdAGAAUdACfUGGGACAAsCsG 3' | SEQ ID NO: 1598; |

wherein G = 2'-O-methylguanosine, A = 2'-O-methyladenosine, U = 2'-O-methyluridine, C = 2'-O-methylcytidine; Gs = 2'-O-methyl-3'-phosphorothioguanosine, As = 2'-O-methyl-3'-phosphorothioadenosine, Cs = 2'-O-methyl-3'-phosphorothiocytidine; fG = 2'-fluoroguanosine, fA = 2'-fluoroadenosine, fU = 2'-fluorouridine, fC = 2'-fluorocytidine; fGs = 2'-fluoro-3'-phosphorothioguanosine, T = 2'-deoxythymidine, dA = 2'-deoxyadenosine, dG = 2'-deoxyguanosine; and the sense strand can be any sequence containing at least 85% base complementarity to the antisense strand, with no restriction on the modification mode.

The aforementioned C3 RNA inhibitor or a pharmaceutically acceptable salt thereof, wherein the antisense strand is selected from the following sequences:

| | |
|---|---|
| 5' UsdAsUAdGATGUAGTAfGAAUUUCUsCsU 3' | SEQ ID NO: 1665; |
| 5' UsfAsUGAfAGfCAAUUCfUCfCUCAGCsAsC 3' | SEQ ID NO: 1671; |

wherein G = 2'-O-methylguanosine, A = 2'-O-methyladenosine, U = 2'-O-methyluridine, C = 2'-O-methylcytidine; Gs = 2'-O-methyl-3'-phosphorothioguanosine, As = 2'-O-methyl-3'-phosphorothioadenosine, Us = 2'-O-methyl-3'-phosphorothiouridine, Cs = 2'-O-methyl-3'-phosphorothiocytidine; fG = 2'-fluoroguanosine, fA = 2'-fluoroadenosine, fU = 2'-fluorouridine, fC = 2'-fluorocytidine; fGs = 2'-fluoro-3'-phosphorothioguanosine, fAs = 2'-fluoro-3'-phosphorothioadenosine, fUs = 2'-fluoro-3'-phosphorothiouridine, fCs = 2'-fluoro-3'-phosphorothiocytidine, T = 2'-deoxythymidine, dG = 2'-deoxyguanosine, dAs = 2'-deoxy-3'-phosphorothioadenosine; and the sense strand can be any sequence containing at least 85% base complementarity to the antisense strand, with no restriction on the modification mode.

The aforementioned CFB RNA inhibitor or a pharmaceutically acceptable salt thereof, wherein the sense strand is selected from the following sequence:

| | |
|---|---|
| 5' GsUsCUAGfUCfAfAfCUUAAUUGAsGsA 3' | SEQ ID NO: 559; |

wherein G = 2'-O-methylguanosine, A = 2'-O-methyladenosine, U = 2'-O-methyluridine, C = 2'-O-methylcytidine; Gs = 2'-O-methyl-3'-phosphorothioguanosine, As = 2'-O-methyl-3'-phosphorothioadenosine, Us = 2'-O-methyl-3'-phosphorothiouridine, Cs = 2'-O-methyl-3'-phosphorothiocytidine; fG = 2'-fluoroguanosine, fA = 2'-fluoroadenosine, fU = 2'-fluorouridine, fC = 2'-fluorocytidine; and the antisense strand can be any sequence containing at least 85% base complementarity to the sense strand, with no restriction on the modification mode.

The aforementioned C5 RNA inhibitor or a pharmaceutically acceptable salt thereof, wherein the sense strand is selected from the following sequence:

| | |
|---|---|
| 5' UsUsGUCCfCAfGfUfAUUCUAUGUsUsU 3' | SEQ ID NO: 1537; |

wherein G = 2'-O-methylguanosine, A = 2'-O-methyladenosine, U = 2'-O-methyluridine, C = 2'-O-methylcytidine; Gs = 2'-O-methyl-3'-phosphorothioguanosine, As = 2'-O-methyl-3'-phosphorothioadenosine, Us = 2'-O-methyl-3'-phosphorothiouridine, Cs = 2'-O-methyl-3'-phosphorothiocytidine; fG = 2'-fluoroguanosine, fA = 2'-fluoroadenosine, fU = 2'-fluorouridine, fC = 2'-fluorocytidine; and the antisense strand can be any sequence containing at least 85% base complementarity to the sense strand, with no restriction on the modification mode.

The aforementioned C3 RNA inhibitor or a pharmaceutically acceptable salt thereof, wherein the sense strand is selected from the following sequences:

| | |
|---|---|
| 5' AsGsAAAUfUCfUfAfCUACAUCUAsUsA 3' | SEQ ID NO: 1645; |
| 5' GsCsUGAGfGAfGfAfAUUGCUUCAsUsA 3' | SEQ ID NO: 1651; |

wherein G = 2'-O-methylguanosine, A = 2'-O-methyladenosine, U = 2'-O-methyluridine, C = 2'-O-methylcytidine; Gs = 2'-O-methyl-3'-phosphorothioguanosine, As = 2'-O-methyl-3'-phosphorothioadenosine, Us = 2'-O-methyl-3'-phosphorothiouridine, Cs = 2'-O-methyl-3'-phosphorothiocytidine; fG = 2'-fluoroguanosine, fA = 2'-fluoroadenosine, fU = 2'-fluorouridine, fC = 2'-fluorocytidine; the antisense strand may be any sequence having at least 85% base complementarity with the sense strand, and the modification manner is not limited.

Preferably, the aforementioned CFB RNA inhibitor or a pharmaceutically acceptable salt thereof, wherein the sense strand is SEQ ID NO:559 or a sequence differing by one, two or three nucleotides therefrom, and the antisense strand is SEQ ID NO:588 or a sequence differing by one, two or three nucleotides therefrom:
Sense strand: 5' GsUsCUAGfUCfAfAfCUUAAUUGAsGsA 3' SEQ ID NO: 559;
Antisense strand: 5' UsdCsUCdAATUAAGTUfGACUAGACsAsC 3' SEQ ID NO: 588;
wherein G = 2'-O-methylguanosine, A = 2'-O-methyladenosine, U = 2'-O-methyluridine, C = 2'-O-methylcytidine; Gs = 2'-O-methyl-3'-phosphorothioguanosine, As = 2'-O-methyl-3'-phosphorothioadenosine, Us = 2'-O-methyl-3'-phosphorothiouridine, Cs = 2'-O-methyl-3'-phosphorothiocytidine; fG = 2'-fluoroguanosine, fA = 2'-fluoroadenosine, fU = 2'-fluorouridine, fC = 2'-fluorocytidine; fGs = 2'-fluoro-3'-phosphorothioguanosine, fAs = 2'-fluoro-3'-phosphorothioadenosine, fUs = 2'-fluoro-3'-phosphorothiouridine, fCs = 2'-fluoro-3'-phosphorothiocytidine, T = 2'-deoxythymidine, Ts = 2'-deoxy-3'-phosphorothiothymidine, dA = 2'-deoxyadenosine.

Preferably, the aforementioned C5 RNA inhibitor or a pharmaceutically acceptable salt thereof, wherein the sense strand is SEQ ID NO:1537 or a sequence differing by one, two or three nucleotides therefrom, and the antisense strand is SEQ ID NO:1598 or a sequence differing by one, two or three nucleotides therefrom:
Sense strand: 5' UsUsGUCCfCAfGfUfAUUCUAUGUsUsU 3' SEQ ID NO: 1537;
Antisense strand: 5' AsdAsACdAUdAGAAUdACfUGGGACAAsCsG 3' SEQ ID NO: 1598;
wherein G = 2'-O-methylguanosine, A = 2'-O-methyladenosine, U = 2'-O-methyluridine, C = 2'-O-methylcytidine; Gs = 2'-O-methyl-3'-phosphorothioguanosine, As = 2'-O-methyl-3'-phosphorothioadenosine, Us = 2'-O-methyl-3'-phosphorothiouridine, Cs = 2'-O-methyl-3'-phosphorothiocytidine; fG = 2'-fluoroguanosine, fA = 2'-fluoroadenosine, fU = 2'-fluorouridine, fC = 2'-fluorocytidine; fGs = 2'-fluoro-3'-phosphorothioguanosine, fAs = 2'-fluoro-3'-phosphorothioadenosine, fUs = 2'-fluoro-3'-phosphorothiouridine, fCs = 2'-fluoro-3'-phosphorothiocytidine, T = 2'-deoxythymidine, Ts = 2'-deoxy-3'-phosphorothiothymidine, dA = 2'-deoxyadenosine.

Preferably, the aforementioned C3 RNA inhibitor or a pharmaceutically acceptable salt thereof, wherein the sense strand is SEQ ID NO:1651 or a sequence differing by one, two or three nucleotides therefrom, and the antisense strand is SEQ ID NO:1671 or a sequence differing by one, two or three nucleotides therefrom:
Sense strand: 5' AsGsAAAUfUCfUfAfCUACAUCUAsUsA 3' SEQ ID NO: 1645;
Antisense strand: 5' UsdAsUAdGATGUAGTAfGAAUUUCUsCsU 3' SEQ ID NO: 1665;

Alternatively, the sense strand is SEQ ID NO:1645 or a sequence differing by one, two or three nucleotides therefrom, and the antisense strand is SEQ ID NO:1665 or a sequence differing by one, two or three nucleotides therefrom:

| | |
|---|---|
| Sense strand: 5' GsCsUGAGfGAfGfAfAUUGCUUCAsUsA 3' | SEQ ID NO: 1651; |
| Antisense strand: 5' UsfAsUGAfAGfCAAUUCfUCfCUCAGCsAsC 3' | SEQ ID NO: 1671; |

wherein G = 2'-O-methylguanosine, A = 2'-O-methyladenosine, U = 2'-O-methyluridine, C = 2'-O-methylcytidine; Gs = 2'-O-methyl-3'-phosphorothioguanosine, As = 2'-O-methyl-3'-phosphorothioadenosine, Us = 2'-O-methyl-3'-phosphorothiouridine, Cs = 2'-O-methyl-3'-phosphorothiocytidine; fG = 2'-fluoroguanosine, fA = 2'-fluoroadenosine, fU = 2'-fluorouridine, fC = 2'-fluorocytidine; fGs = 2'-fluoro-3'-phosphorothioguanosine, fAs = 2'-fluoro-3'-phosphorothioadenosine, fUs = 2'-fluoro-3'-phosphorothiouridine, fCs = 2'-fluoro-3'-phosphorothiocytidine, T = 2'-deoxythymidine, dG = 2'-deoxyguanosine, dAs = 2'-deoxy-3'-phosphorothioadenosine.

In some embodiments, mismatches may be tolerated in the sense strand or antisense strand of the RNA inhibitor of the present invention, and the mismatch positions may occur at the 5'-end, 3'-end or within the sequence. Preferably, the number of mismatches is no more than 3 nucleotides, for example, 0, 1, 2 or 3 mismatched nucleotides.

In some technical embodiments, preferably, the RNA inhibitor or a pharmaceutically acceptable salt thereof of the present invention further comprises carrier structures 5'MVIP and 3'MVIP, and the structure of the RNA inhibitor is as shown in Formula Ia, Ib or Ic: 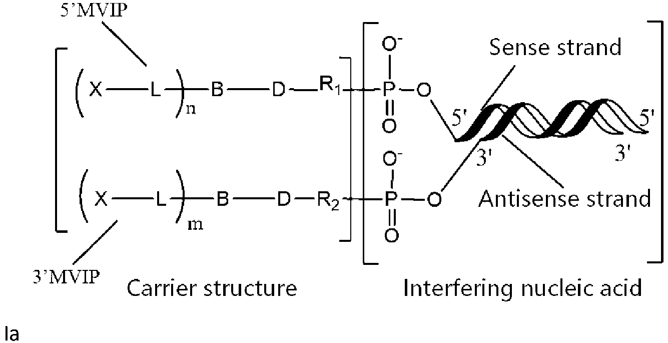 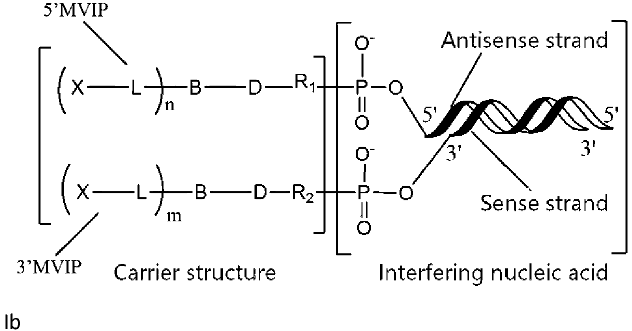 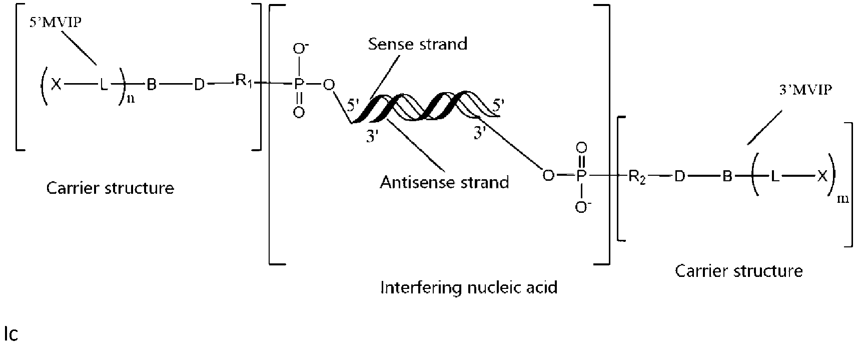 wherein
the carrier structures include 5'MVIP (5' MultiValent Import Platform) and 3'MVIP (3' MultiValent Import Platform);
5'MVIP is composed of a junction point R₁, a linking chain D, a linker B, a branched chain L and a liver-targeting specific ligand X, and is connected to the 5'-end of the sense strand or the 5'-end of the antisense strand through the junction point R₁, with the structure shown in General Formula I:

   **(X-L)ₙ-B-D-R₁-** I
3'MVIP is composed of a junction point R₂, a linking chain D, a linker B, a branched chain L and a liver-targeting specific ligand X, and is connected to the 3'-end of the sense strand or the 3'-end of the antisense strand through the junction point R₂, with the structure shown in General Formula II:

   **(X-L)ₘ-B-D-R₂-** II
wherein
n and m are each independently any integer from 0 to 4, each independently preferably an integer from 1 to 3, and n+m is an integer from 2 to 6, preferably n+m = 2, 3 or 4, more preferably 4;
the junction point R₁ is a heterocyclic or carbocyclic structure containing N, S or O as shown below:

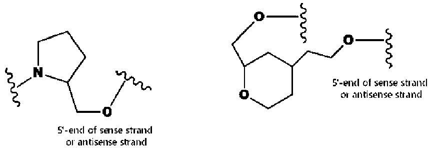

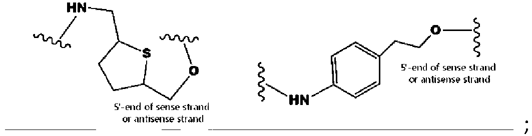

Alternatively, R₁ is -NH(CH₂)ₓCH₂O-, wherein x is any integer from 3 to 12, preferably any integer from 4 to 6;
the junction point R₂ is a heterocyclic or carbocyclic structure containing N, S or O as shown below:

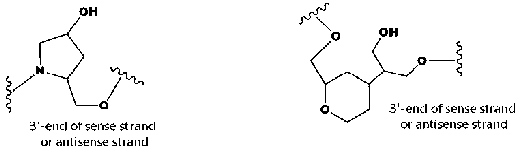

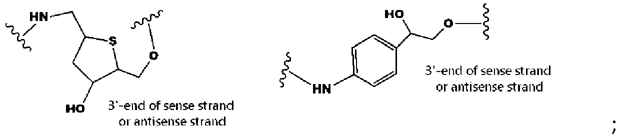

Alternatively, the junction point R₂ is -NH(CH₂)ₓ₁CH(OH)(CH₂)ₓ₂CH₂O-, wherein x1 is any integer from 1 to 4, and x2 is any integer from 0 to 4;
the liver-targeting specific ligand X is selected from structures for enhancing the uptake of RNA inhibitors by hepatocytes, which may be the same or different within 5'MVIP and 3'MVIP or between 5'MVIP and 3'MVIP, and is selected from monosaccharides and derivatives thereof, preferably N-acetylgalactosamine and derivatives thereof, more preferably selected from the following structures: 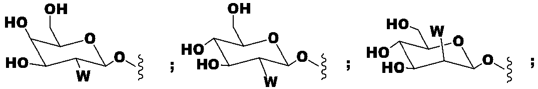 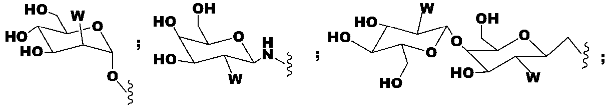 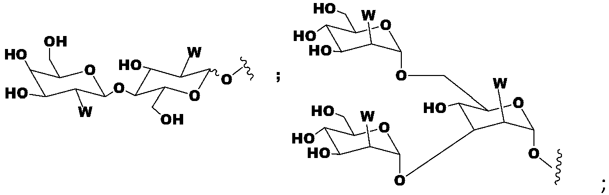
wherein W is selected from one or two of -OH, -NHCOOH and -NHCO(CH₂)_{q}CH₃, wherein q is an integer from 0 to 4;
the branched chain L is the same or different within 5'MVIP and 3'MVIP or between 5'MVIP and 3'MVIP, and is selected from one or more of the following structures: 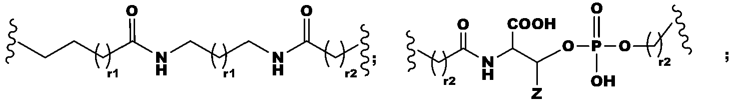 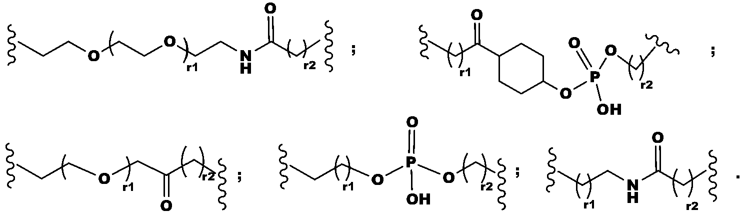 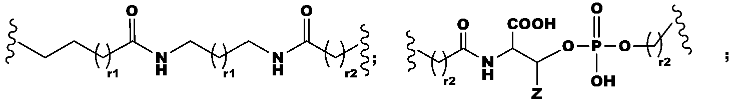 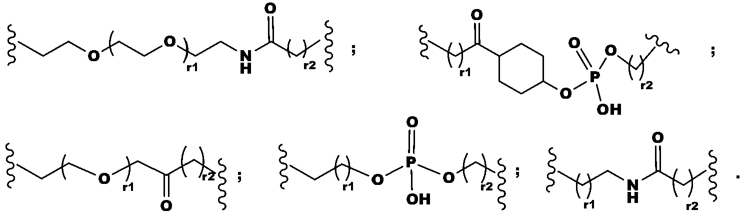
wherein r1 is any integer from 1 to 12, r2 is any integer from 0 to 20, Z is H, alkyl or amido group, and the alkyl is such as C₁-C₅ alkyl;
the linker B is the same or different within 5'MVIP and 3'MVIP or between 5'MVIP and 3'MVIP, and is selected from the following structures: 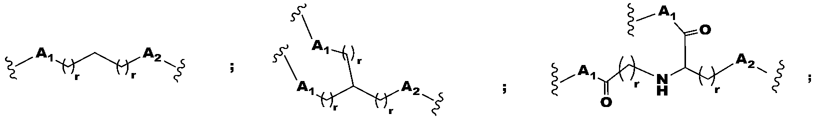 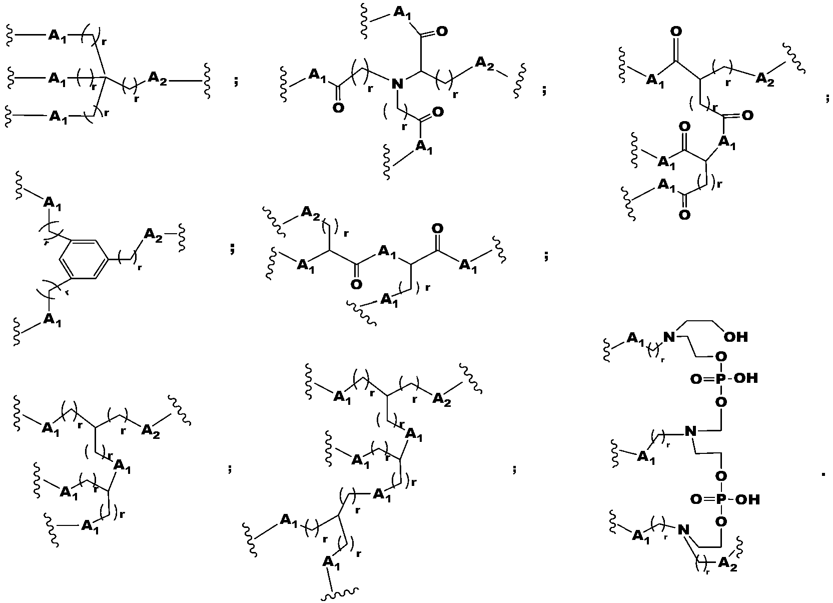
wherein A₁ and A₂ are each independently C, O, S, -NH-, carbonyl, amido group, phosphoryl group or thiophosphoryl group, and r is any integer from 0 to 4;
the linking chain D is the same or different within 5'MVIP and 3'MVIP or between 5'MVIP and 3'MVIP, and is selected from the following structures: 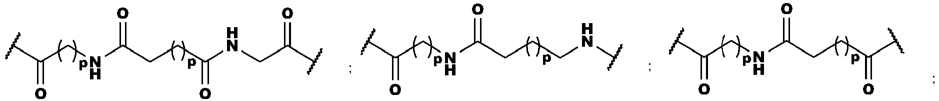 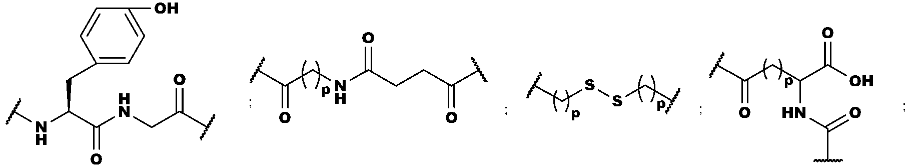 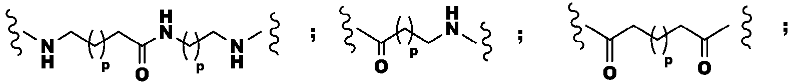 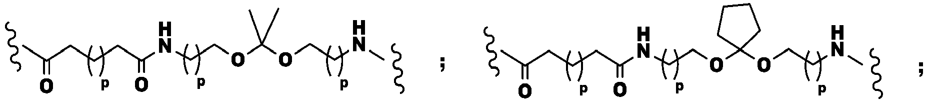 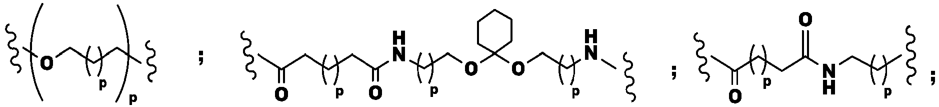 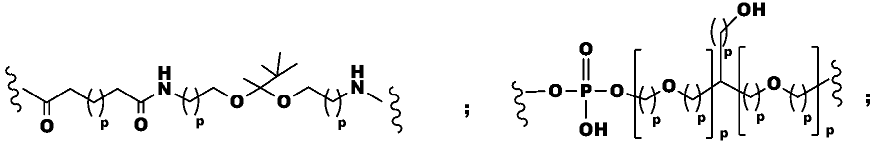 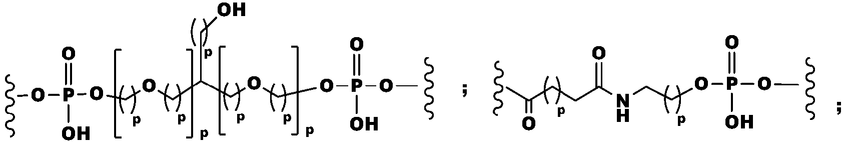 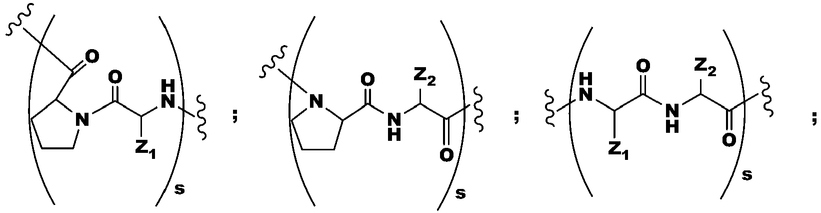 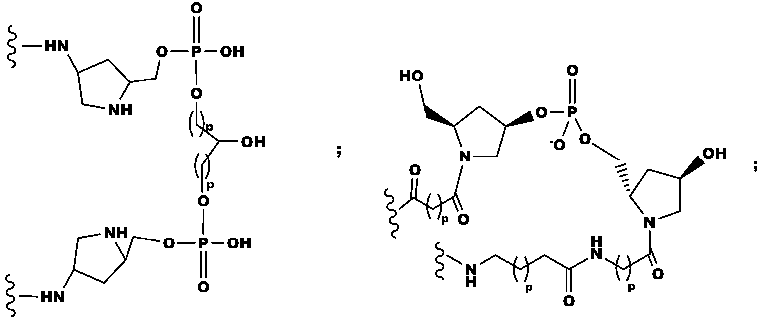 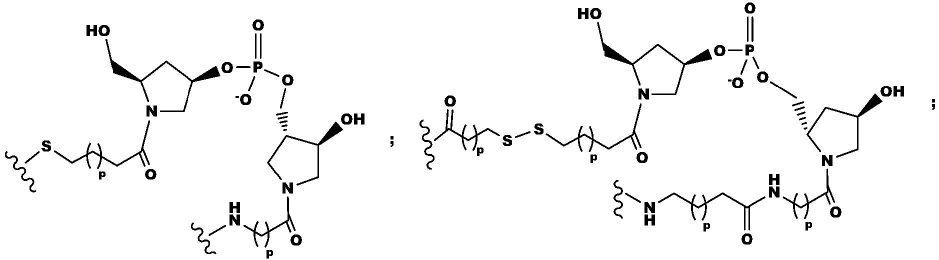 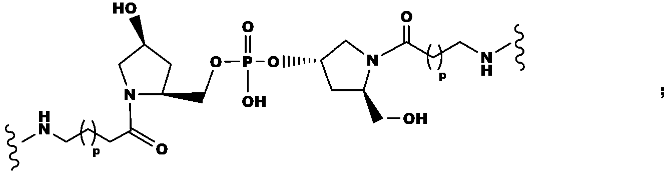 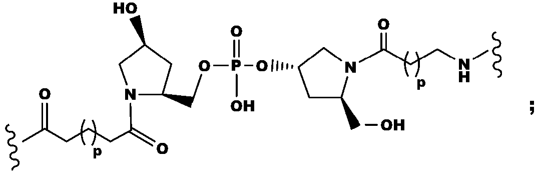 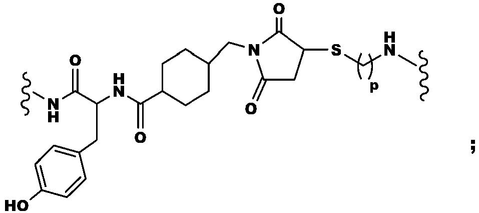
wherein each p is independently any integer from 1 to 20; s is any integer from 2 to 13; Z₁ and Z₂ are the same or different substituent groups, such as C₃-C₁₀ alkyl.

In some embodiments, 5'MVIP is selected from any one of 5'MVIP01 to 5'MVIP22 in Table 11.

In some embodiments, 3'MVIP is selected from any one of 3'MVIP01 to 3'MVIP27 in Table 12.

In some embodiments, the RNA inhibitor or a pharmaceutically acceptable salt thereof of the present invention, wherein 5'MVIP is 5'MVIP01 or 5'MVIP09 as shown below, and 3'MVIP is 3'MVIP01, 3'MVIP09 or 3'MVIP17 as shown below:

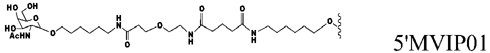

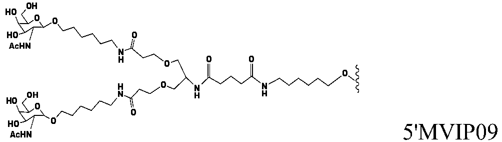

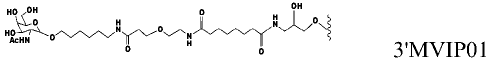

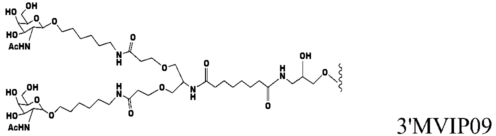

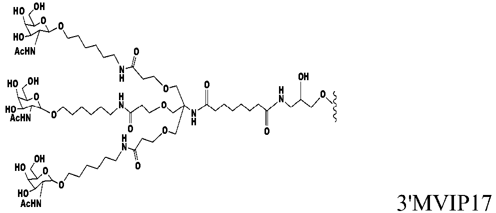

In some embodiments, the RNA inhibitor or a pharmaceutically acceptable salt thereof of the present invention, wherein the combination of 5'MVIP of the sense strand and 3'MVIP of the antisense strand is 5'MVIP01/3'MVIP01, 5'MVIP01/3'MVIP17 or 5'MVIP09/3'MVIP09, or the combination of 5'MVIP of the sense strand and 3'MVIP of the sense strand is 5'MVIP01/3'MVIP09 or 5'MVIP09/3'MVIP01.

In another aspect, the CFB RNA inhibitor is selected from Kylo-17-DS2911.

In another aspect, the C5 RNA inhibitor is selected from Kylo-19-DS7881.

In another aspect, the C3 RNA inhibitor is selected from Kylo-27-DS8201, Kylo-27-DS8141.

In another aspect, use of the RNA inhibitor or a pharmaceutically acceptable salt thereof of the present invention in the preparation of a medicament for the treatment and/or prevention of diseases associated with elevated levels of the complement system, wherein the diseases include but are not limited to lipid metabolism disorders.

In another aspect, a pharmaceutical composition comprising the aforementioned RNA inhibitor or another therapeutic agent for the treatment or prevention of complement system-related diseases.

In another aspect, a pharmaceutical composition comprising the aforementioned RNA inhibitor or a pharmaceutically acceptable salt thereof and pharmaceutically acceptable excipients, wherein the dosage form is an oral preparation, an intravenous injection, or a subcutaneous or intramuscular injection, preferably a subcutaneous injection.

Those skilled in the art can easily perceive other aspects and advantages of the present application from the detailed description below. Only exemplary embodiments of the present application are shown and described in the detailed description below. As those skilled in the art will recognize, the content of the present application enables those skilled in the art to make modifications to the disclosed specific embodiments without departing from the spirit and scope of the invention involved in the present application. Accordingly, the drawings and descriptions in the specification of the present application are merely exemplary rather than restrictive.

### Brief Description of the Drawings

The specific features of the invention involved in the present application are shown in the appended claims. The features and advantages of the invention involved in the present application can be better understood by referring to the exemplary embodiments described in detail below and the accompanying drawings.

The drawings are briefly described as follows:
Figure 1: High-resolution mass spectrum of ERCd-01-c2 synthesized in 3.1.15 of Example 3 of the present application;
Figure 2: High-resolution mass spectrum of 3'MVIP17-c1 synthesized in 3.1.2.6 of Example 3 of the present application;
Figure 3: High-resolution mass spectrum of 5'MVIP09-ERCd-PFP-c2 synthesized in 3.2.1.2 of Example 3 of the present application;
Figure 4: Schematic diagram of the effect of administration of the present invention in hCFB mice on the inhibition rate of hCFB protein in mouse serum;
Figure 5: Schematic diagram of the effect of administration of Example 7-2 of the present invention in hC5 mice on the inhibition rate of hC5 protein in mouse serum;
Figure 6: Schematic diagram of the effect of administration of Example 7-3 of the present invention in hC5 mice on the inhibition rate of hC5 protein in mouse serum;
Figure 7: Schematic diagram of the effect of administration of the present invention in hC3 mice on the inhibition rate of hC3 protein in mouse serum;
Figure 8: Schematic diagram of the effect of CFB RNA inhibitor of the present invention on the inhibition rate of CFB protein in NHP cynomolgus monkeys;
Figure 9: Schematic diagram of the effect of CFB RNA inhibitor of the present invention on CFB mRNA level in liver tissue of cynomolgus monkeys;
Figure 10: Schematic diagram of the effect of C5 RNA inhibitor of the present invention on the inhibition rate of C5 protein in NHP cynomolgus monkeys;
Figure 11: Schematic diagram of the effect of C5 RNA inhibitor of the present invention on the retention level of CH50 activity in NHP cynomolgus monkeys;
Figure 12: Schematic diagram of the effect of C3 RNA inhibitor of the present invention on the inhibition rate of C3 protein in NHP cynomolgus monkeys.

### Detailed Description of the Embodiments

The embodiments of the present invention are illustrated by specific examples below, and those skilled in the art can easily understand other advantages and effects of the invention of the present application from the content disclosed in this specification.

### Definition of Terms

In the present application, examples of mRNA sequences of the complement system can be easily obtained from publicly available databases, such as GenBank, UniProt, OMIM and the Macaca genome project website.

The term "CFB" refers to complement factor B, and its mRNA sequence can be found in, for example, GenBank NM_001710.5; 001710; cynomolgus monkey NC_041757.1, mouse NM_001142706 and NM_008198; rat NM_212466.3.

The term "C3" refers to complement C3, and its mRNA sequence can be found in, for example, GenBank NM_000064.4; cynomolgus monkey NC_000019.10; mouse NM_009778; rat NM_016994.

The term "C5" refers to complement C5, and its mRNA sequence can be found in, for example, GenBank NM_001735.3; mouse NM_013485; rat NM_057146.

The judgment indicators for "inhibition of CFB expression" include: inhibition of mRNA level of CFB gene, inhibition of protein level of CFB gene, and any other level of inhibition, and may also be: CH50 activity as a measure of total hemolytic complement, AH50 for measuring hemolytic activity of the alternative complement pathway, and/or lactate dehydrogenase (LDH) level as a measure of intravascular hemolysis, and/or hemoglobin level; the expression level of CFB can also be evaluated by measuring the levels of C3, C9, C5, C5a, C5b and soluble C5b-9 complex.

The judgment indicators for "inhibition of C3 expression" include: C3 assay, total complement assay, immune complex assay, C3 plasma concentration assay, and C3 fixation antibody test; C3 assay can be inhibition of mRNA level of C3 gene, inhibition of protein level of C3 gene, or any other level of inhibition; C3 assay may be performed together with other complement components to assess the function of the entire complement system, which usually includes measuring C4 concentration and indicators such as CH50; immune complex assay: immune complexes are structures composed of antibodies and antigens, formed in certain autoimmune diseases; serum immune complex assay can be used to detect these immune complexes and assess whether they cause activation of the immune system leading to decreased C3; C3 plasma concentration assay: the function of the complement system is assessed by measuring the concentration of C3 in plasma; under normal conditions, the concentration of C3 should be within the normal range; C3 fixation antibody method: this method is used to measure the ability of C3 to bind to specific antigens to assess the function of the complement system. It can also be measurement of indicators related to C3 gene level, such as: CH50 activity as a measure of total hemolytic complement, AH50 for measuring hemolytic activity of the alternative complement pathway, and/or lactate dehydrogenase (LDH) level as a measure of intravascular hemolysis, and/or hemoglobin level; the expression of C3 can also be evaluated by measuring the levels of CFB, C9, C5, C5a, C5b and soluble C5b-9 complex.

The judgment indicators for "inhibition of C5 expression" include: inhibition of mRNA level of C5 gene, inhibition of protein level of C5 gene, and any other level of inhibition, and may also be: CH50 activity as a measure of total hemolytic complement, AH50 for measuring hemolytic activity of the alternative complement pathway, and/or lactate dehydrogenase (LDH) level as a measure of intravascular hemolysis, and/or hemoglobin level; the expression level of CFB can also be evaluated by measuring the levels of C3, C9, C5, C5a, C5b and soluble C5b-9 complex.

In the present application, "target sequence" refers to a continuous part of the nucleotide sequence of an mRNA molecule formed during transcription of a complement system gene, including mRNA that is a product of RNA processing of a primary transcription product. In some embodiments, the target portion of the sequence will be sufficiently long to serve as a substrate for RNA inhibitor-directed degradation at or near said portion of the nucleotide sequence of the mRNA molecule formed during transcription of a complement system gene. The length of a "target sequence" is generally about 15-30 nucleotides.

In the present application, the term "RNA inhibitor" generally refers to an agent comprising RNA as defined in the terms of the present invention, which can mediate targeted cleavage of RNA transcripts via the RNA-induced silencing complex (RISC) pathway, and directs sequence-specific degradation of mRNA through a process called RNA inhibition to regulate (e.g., inhibit) the expression of complement system genes in cells (e.g., cells in a subject such as a mammalian subject).

In some embodiments, the RNA inhibitor may be a single-stranded siRNA (ssRNA inhibitor) introduced into a cell or organism to inhibit a target mRNA (i.e., a complement system gene). A single-stranded RNA inhibitor binds to the RISC endonuclease Argonaute 2, which then cleaves the target mRNA. Single-stranded siRNAs are generally 15 to 30 nucleotides in length and chemically modified.

In some embodiments, the "RNA inhibitor" used in the present application is double-stranded RNA, and is referred to as "double-stranded RNA inhibitor", "double-stranded RNA (dsRNA, DS) molecule", "dsRNA agent" or "dsRNA" in the present invention. The term "dsRNA" refers to a complex of ribonucleic acid molecules having a duplex structure comprising two anti-parallel and substantially complementary nucleic acid strands, referred to as having "sense" and "antisense" orientations relative to a target mRNA. In some embodiments of the present application, double-stranded RNA (dsRNA) triggers degradation of target mRNA through a post-transcriptional gene silencing mechanism (referred to as RNA inhibition or RNA interference in the present invention).

The duplex structure can be any length that triggers specific degradation of complement system mRNA via the RISC pathway, and can range in length from about 15 to 36 base pairs, for example, about 15-30 base pairs in length, such as about 15, 16, 17, 18, 19, 20, 21, 22, 23, 24, 25, 26, 27, 28, 29, 30 or 36 base pairs in length. In certain embodiments, the RNA inhibitor of the present application is a dsRNA of 15-30 nucleotides that interacts with a target sequence to direct cleavage of complement system mRNA.

Generally, most nucleotides of the sense strand and antisense strand of a dsRNA molecule are ribonucleotides, but as described in detail in the present invention, may also include one or more non-ribonucleotides, such as deoxyribonucleotides or modified nucleotides. In addition, the RNA inhibitor involved in the present specification may include ribonucleotides with chemical modifications, and modified nucleotides may be present in multiple regions. The term "modified nucleotide" as used in the present invention means a nucleotide independently having a modified sugar moiety, a modified internucleotide linkage, a modified nucleobase, or any combination thereof. Thus, the term "modified nucleotide" encompasses the substitution, addition or removal of, for example, functional groups or atoms on internucleotide linkages, sugar moieties or nucleobases. Modifications suitable for the RNA inhibitor of the present application include all types of modifications disclosed in the present invention or known in the art.

In the present application, the term "nucleotide sequence" generally refers to a string or sequence of nucleotides, whether modified or unmodified, described by a string of letters using standard nucleotide nomenclature and the symbol table for modified nucleotides described in the present application. The nucleotide sequences described in the present application are polymers linked by phosphodiester bonds (or related structural variants or synthetic analogs thereof), including naturally occurring nucleotide polymers, but it is understood that the scope of the term also includes various analogs, including but not limited to: peptide nucleic acids (PNA), phosphoramidates, phosphorothioates, methylphosphonates, 2'-O-methylribonucleic acids, etc. They are usually about 15-30 nucleotides in length, but the term can also refer to molecules of any length.

In some embodiments, a nucleotide sequence comprises one or more unmodified ribonucleosides (RNA) and/or unmodified deoxyribonucleosides (DNA) and/or one or more modified nucleotides. The term "modified nucleotide sequence" generally refers to a string or sequence of nucleotides comprising at least one modification and/or at least one modified internucleotide linkage.

In the present application, the term "modified nucleotide" generally means a nucleotide comprising at least one chemical modification compared to a naturally occurring RNA or DNA nucleotide. For example, 2'-deoxythymidine, 2'-O-methyl modified nucleotide, 2'-fluoro modified nucleotide, 2'-deoxy modified nucleotide, locked nucleotide, abasic nucleotide, 2'-amino modified nucleotide, 2'-O-long chain alkyl modified nucleotide (e.g., hexadecyl), morpholino nucleotide, phosphoramidate nucleotide, non-natural nucleobase nucleotide, 5'-phosphorothioate nucleotide, and nucleotide linked to a cholesterol derivative or a didodecanamide dodecanoate group.

Modified nucleotides comprise modified sugar groups and/or modified nucleobases.

In the present application, the term "nucleobase" or "base" generally means a heterocyclic pyrimidine or purine compound that is a component of all nucleic acids and includes adenine, guanine, cytosine, thymine and uracil. Nucleotides may include modified nucleotides or nucleoside mimetics, abasic (Abasics) or alternative substitution moieties. The term "unmodified nucleobase" or "naturally occurring nucleobase" generally means the naturally occurring heterocyclic nucleobases of RNA or DNA: the purine bases adenine and guanine; and the pyrimidine bases thymine, cytosine and uracil. "Modified nucleobase" generally means any nucleobase that is not a naturally occurring nucleobase.

In the present application, the term "sugar group" generally means a naturally occurring sugar group or a modified sugar group of a nucleotide. The term "naturally occurring sugar group" generally means a ribofuranosyl group as found in naturally occurring RNA or a deoxyribofuranosyl group as found in naturally occurring DNA. "Modified sugar group" means a substituted sugar group or a sugar surrogate, such as a sugar group with a fluoro or methoxy substitution at the 2'-position.

In the present application, the term "internucleotide linkage" generally means a covalent linkage between adjacent nucleotides in a nucleotide sequence. "Naturally occurring internucleotide linkage" means a 3' to 5' phosphodiester linkage. "Modified internucleotide linkage" means any internucleotide linkage other than a naturally occurring internucleotide linkage.

In the present application, the term "antisense strand" (AS) generally refers to the strand of an RNA inhibitor (e.g., dsRNA) comprising a region substantially complementary to a target sequence. As used in the present invention, the term "complementary region" generally refers to a region on the antisense strand that is substantially complementary to a sequence defined in the present application (e.g., a target sequence).

In the present application, the term "sense strand" (SS) generally refers to the strand of an RNA inhibitor (e.g., dsRNA) that comprises a region substantially complementary to a region of the "antisense strand" (AS). The "sense" strand is sometimes referred to as the "sense" strand, "passenger" strand or "anti-guide" strand. By virtue of the sequence of the sense strand, the antisense strand targets the desired mRNA, while the sense strand may target a different target or be degraded. Thus, if the antisense strand is incorporated into RISC, the correct target is targeted. Incorporation of the sense strand can lead to off-target effects. These off-target effects can be limited by using modifications on the sense strand or using a 5'-end cap.

Loading of the siRNA duplex into the ago protein must be initiated by recognition of the 5'-end of the antisense strand, which is a prerequisite for placing the rest of the duplex into the nucleic acid binding channel of the AGO protein. The MID domain of the AGO protein recognizes the nucleotide at the 5'-end, and upon recognition, the siRNA duplex is loaded into the nucleic acid binding channel of the AGO protein to form pre-RISC. After loading the siRNA duplex into the nucleic acid binding channel, pre-RISC expels the sense strand (passenger strand) to form RISC with the remaining antisense strand (guide strand).

In the present application, the term "complementary" refers to the ability of two nucleotide sequences to hybridize under certain conditions to form base-pair hydrogen bonds and form a duplex or double helix structure. For example, the RNA inhibitor antisense strand hybridizes with the RNA inhibitor sense strand or complement system mRNA to form Watson-Crick base pairs or non-Watson-Crick base pairs, including natural or modified nucleotides or nucleotide mimetics. "Complementary" does not require nucleobase complementarity on every nucleoside. Rather, some mismatches can be tolerated.

In the present application, the term "mismatch", when the complementary region is not completely complementary to the target sequence, the mismatch may be in the core region or the terminal region. Generally, the most tolerated mismatches are in the terminal regions, e.g., within 5, 4, 3 or 2 nucleotides at the 5'-end and/or 3'-end, and no more than 3 mismatches.

As shown in the study by Gu S, Jin L, Zhang F, Huang Y, Grimm D, Rossi JJ, Kay MA. Thermodynamic stability of small hairpin RNAs highly influences the loading process of different mammalian Argonautes. Proc Natl Acad Sci USA 2011, 108:9208 - 9213, any factor affecting the thermodynamic stability of the duplex, such as mismatches and non-Watson-Crick base pairs, facilitates the expulsion of the sense strand by pre-RISC to form RISC.

In the present application, the term "ligand" generally refers to any compound or molecule capable of covalently or otherwise chemically binding to a biologically active substance (e.g., dsRNA). In some embodiments, a ligand is capable of directly or indirectly interacting with another compound, such as a receptor, which may be present on the cell surface, or alternatively may be an intracellular and/or intercellular receptor, and the interaction of the ligand with the receptor may result in a biochemical reaction, or may be merely a physical interaction or binding.

In the present application, the term "pharmaceutically acceptable" generally means one or more non-toxic substances that do not inhibit the effectiveness of the biological activity of the active ingredient. Such preparations may usually contain salts, excipients, buffers, preservatives, compatible carriers and optionally other therapeutic agents. Such pharmaceutically acceptable preparations may also usually include compatible solid or liquid fillers, diluents or encapsulating materials suitable for administration to humans. For pharmaceutical use, the salts should be pharmaceutically acceptable salts, but non-pharmaceutically acceptable salts may be conveniently used to prepare pharmaceutically acceptable salts, and they are not excluded from the scope of the present application. Such pharmacologically and pharmaceutically acceptable salts include, but are not limited to, salts prepared from the following acids: hydrochloric acid, hydrobromic acid, sulfuric acid, nitric acid, phosphoric acid, maleic acid, acetic acid, salicylic acid, citric acid, boric acid, formic acid, malonic acid, succinic acid, etc. Pharmaceutically acceptable salts can also be prepared as alkali metal or alkaline earth metal salts, such as sodium, potassium or calcium salts.

In the present application, the term "lipid nanoparticle" or "LNP" generally refers to a vesicle comprising a lipid layer encapsulating a pharmacologically active molecule (e.g., dsRNA). LNPs are described in, for example, Chinese Patent No. CN103189057B, the entire content of which is incorporated herein by reference.

### Detailed Description of the Invention

In one aspect, the present invention provides an RNA inhibitor inhibiting the gene expression of the complement system or a pharmaceutically acceptable salt thereof.

### siRNA Design

A set of siRNAs targeting human complement system genes were screened using independently developed sequence screening software: human CFB: GenBank NM_001710.5; human C3: GenBank NM_000064.4; human C5: GenBank NM_001735.3.

The primary sequences selected for CFB are shown in Table 1-1, and the primary sequences selected for C5 are shown in Table 1-2:
The modified secondary sequences selected by CFB are shown in Table 2-1, those selected by C5 in Table 2-2, and those selected by C3 in Table 2-3:
siRNA are synthesized and annealed using conventional methods known in the art.

In certain embodiments, the duplex structure (complementary region) formed by the antisense strand and the sense strand comprises at least 12, 13, 14, 15, 16, 17, 18, 19, 20 or 21 consecutive nucleotides.

**Table 1-1 Primary Sequences of CFB**

| siRNA inhibitor number | SEQ ID NO: | **Sense Strand (5'-3')** | SEQ ID NO: | **Antisense Strand (5'-3')** |
|---|---|---|---|---|
| Kylo-17-DS01 | 1 | auuccugaauuuuaugacuau | 269 | auagucauaaaauucaggaauuc |
| Kylo-17-DS02 | 2 | auuccugaauuuuaugacuau | 270 | auagucauaaaauucaggaau |
| Kylo-17-DS03 | 3 | ccugaucaagcucaagaauaa | 271 | uuauucuugagcuugaucagggc |
| Kylo-17-DS04 | 4 | ccugaucaagcucaagaauaa | 272 | uuauucuugagcuugaucagg |
| Kylo-17-DS05 | 5 | gaagcaggaauuccugaauuu | 273 | aaauucaggaauuccugcuucuu |
| Kylo-17-DS06 | 6 | gaagcaggaauuccugaauuu | 274 | aaauucaggaauuccugcuuc |
| Kylo-17-DS07 | 7 | agcaacauguguucaaaguca | 275 | ugacuuugaacacauguugcuca |
| Kylo-17-DS08 | 8 | agcaacauguguucaaaguca | 276 | ugacuuugaacacauguugcu |
| Kylo-17-DS09 | 9 | gcuguggugucugaguacuuu | 277 | aaaguacucagacaccacagccc |
| Kylo-17-DS10 | 10 | gcuguggugucugaguacuuu | 278 | aaaguacucagacaccacagc |
| Kylo-17-DS11 | 11 | aagugucuagucaacuuaauu | 279 | aauuaaguugacuagacacuuuu |
| Kylo-17-DS12 | 12 | aagugucuagucaacuuaauu | 280 | aauuaaguugacuagacacuu |
| Kylo-17-DS13 | 13 | agcugugagagagaugcucaa | 281 | uugagcaucucucucacagcugc |
| Kylo-17-DS14 | 14 | agcugugagagagaugcucaa | 282 | uugagcaucucucucacagcu |
| Kylo-17-DS15 | 15 | agccaaaaagugucuagucaa | 283 | uugacuagacacuuuuuggcucc |
| Kylo-17-DS16 | 16 | agccaaaaagugucuagucaa | 284 | uugacuagacacuuuuuggcu |
| Kylo-17-DS17 | 17 | ugugagugaugagaucucuuu | 285 | aaagagaucucaucacucacauu |
| Kylo-17-DS18 | 18 | ugugagugaugagaucucuuu | 286 | aaagagaucucaucacucaca |
| Kylo-17-DS19 | 19 | aauugagaagguggcaaguua | 287 | uaacuugccaccuucucaauuaa |
| Kylo-17-DS20 | 20 | aauugagaagguggcaaguua | 288 | uaacuugccaccuucucaauu |
| Kylo-17-DS21 | 21 | caacauguguucaaagucaag | 289 | cuugacuuugaacacauguugcu |
| Kylo-17-DS22 | 22 | caacauguguucaaagucaag | 290 | cuugacuuugaacacauguug |
| Kylo-17-DS23 | 23 | ugugagagagaugcucaauau | 291 | auauugagcaucucucucacagc |
| Kylo-17-DS24 | 24 | ugugagagagaugcucaauau | 292 | auauugagcaucucucucaca |
| Kylo-17-DS25 | 25 | gucuagucaacuuaauugaga | 293 | ucucaauuaaguugacuagacac |
| Kylo-17-DS26 | 26 | gucuagucaacuuaauugaga | 294 | ucucaauuaaguugacuagac |
| Kylo-17-DS27 | 27 | uccaagaaagacaaugagcaa | 295 | uugcucauugucuuucuuggaag |
| Kylo-17-DS28 | 28 | uccaagaaagacaaugagcaa | 296 | uugcucauugucuuucuugga |
| Kylo-17-DS29 | 29 | cccuacuacaaugugagugau | 297 | aucacucacauuguaguagggag |
| Kylo-17-DS30 | 30 | ccuacuacaaugugagugaug | 298 | caucacucacauuguaguaggga |
| Kylo-17-DS31 | 31 | cuacuacaaugugagugauga | 299 | ucaucacucacauuguaguaggg |
| Kylo-17-DS32 | 32 | acuacaaugugagugaugaga | 300 | ucucaucacucacauuguaguag |
| Kylo-17-DS33 | 33 | cuacaaugugagugaugagau | 301 | aucucaucacucacauuguagua |
| Kylo-17-DS34 | 34 | acaaugugagugaugagaucu | 302 | agaucucaucacucacauuguag |
| Kylo-17-DS35 | 35 | gccaaaaagugucuagucaac | 303 | guugacuagacacuuuuuggcuc |
| Kylo-17-DS36 | 36 | ccaaaaagugucuagucaacu | 304 | aguugacuagacacuuuuuggcu |
| Kylo-17-DS37 | 37 | caaaaagugucuagucaacuu | 305 | aaguugacuagacacuuuuuggc |
| Kylo-17-DS38 | 38 | aaaaagugucuagucaacuua | 306 | uaaguugacuagacacuuuuugg |
| Kylo-17-DS39 | 39 | aaaagugucuagucaacuuaa | 307 | uuaaguugacuagacacuuuuug |
| Kylo-17-DS40 | 40 | aaagugucuagucaacuuaau | 308 | auuaaguugacuagacacuuuuu |
| Kylo-17-DS41 | 41 | agugucuagucaacuuaauug | 309 | caauuaaguugacuagacacuuu |
| Kylo-17-DS42 | 42 | gugucuagucaacuuaauuga | 310 | ucaauuaaguugacuagacacuu |
| Kylo-17-DS43 | 43 | ugucuagucaacuuaauugag | 311 | cucaauuaaguugacuagacacu |
| Kylo-17-DS44 | 44 | ucuagucaacuuaauugagaa | 312 | uucucaauuaaguugacuagaca |
| Kylo-17-DS45 | 45 | cuagucaacuuaauugagaag | 313 | cuucucaauuaaguugacuagac |
| Kylo-17-DS46 | 46 | uagucaacuuaauugagaagg | 314 | ccuucucaauuaaguugacuaga |
| Kylo-17-DS47 | 47 | agucaacuuaauugagaaggu | 315 | accuucucaauuaaguugacuag |
| Kylo-17-DS48 | 48 | gucaacuuaauugagaaggug | 316 | caccuucucaauuaaguugacua |
| Kylo-17-DS49 | 49 | acuuaauugagaagguggcaa | 317 | uugccaccuucucaauuaaguug |
| Kylo-17-DS50 | 50 | aaaucaauuaugaagaccaca | 318 | uguggucuucauaauugauuuca |
| Kylo-17-DS51 | 51 | aaucaauuaugaagaccacaa | 319 | uuguggucuucauaauugauuuc |
| Kylo-17-DS52 | 52 | aucaauuaugaagaccacaag | 320 | cuuguggucuucauaauugauuu |
| Kylo-17-DS53 | 53 | ucaauuaugaagaccacaagu | 321 | acuuguggucuucauaauugauu |
| Kylo-17-DS54 | 54 | caauuaugaagaccacaaguu | 322 | aacuuguggucuucauaauugau |
| Kylo-17-DS55 | 55 | auuaugaagaccacaaguuga | 323 | ucaacuuguggucuucauaauug |
| Kylo-17-DS56 | 56 | uuaugaagaccacaaguugaa | 324 | uucaacuuguggucuucauaauu |
| Kylo-17-DS57 | 57 | ggacccaauuacugucauuga | 325 | ucaaugacaguaauugggucccc |
| Kylo-17-DS58 | 58 | gacccaauuacugucauugau | 326 | aucaaugacaguaauuggguccc |
| Kylo-17-DS59 | 59 | acccaauuacugucauugaug | 327 | caucaaugacaguaauugggucc |
| Kylo-17-DS60 | 60 | cccaauuacugucauugauga | 328 | ucaucaaugacaguaauuggguc |
| Kylo-17-DS61 | 61 | ccaauuacugucauugaugag | 329 | cucaucaaugacaguaauugggu |
| Kylo-17-DS62 | 62 | caauuacugucauugaugaga | 330 | ucucaucaaugacaguaauuggg |
| Kylo-17-DS63 | 63 | ggaggauuaucuggaugucua | 331 | uagacauccagauaauccucccu |
| Kylo-17-DS64 | 64 | gaggauuaucuggaugucuau | 332 | auagacauccagauaauccuccc |
| Kylo-17-DS65 | 65 | aggauuaucuggaugucuaug | 333 | cauagacauccagauaauccucc |
| Kylo-17-DS66 | 66 | ggauuaucuggaugucuaugu | 334 | acauagacauccagauaauccuc |
| Kylo-17-DS67 | 67 | gauuaucuggaugucuaugug | 335 | cacauagacauccagauaauccu |
| Kylo-17-DS68 | 68 | auuaucuggaugucuaugugu | 336 | acacauagacauccagauaaucc |
| Kylo-17-DS69 | 69 | uuaucuggaugucuauguguu | 337 | aacacauagacauccagauaauc |
| Kylo-17-DS70 | 70 | caagugaacaucaaugcuuug | 338 | caaagcauugauguucacuuggu |
| Kylo-17-DS71 | 71 | ugaacaucaaugcuuuggcuu | 339 | aagccaaagcauugauguucacu |
| Kylo-17-DS72 | 72 | ggcuuccaagaaagacaauga | 340 | ucauugucuuucuuggaagccaa |
| Kylo-17-DS73 | 73 | ccaagaaagacaaugagcaac | 341 | guugcucauugucuuucuuggaa |
| Kylo-17-DS74 | 74 | caagaaagacaaugagcaaca | 342 | uguugcucauugucuuucuugga |
| Kylo-17-DS75 | 75 | aagaaagacaaugagcaacau | 343 | auguugcucauugucuuucuugg |
| Kylo-17-DS76 | 76 | agaaagacaaugagcaacaug | 344 | cauguugcucauugucuuucuug |
| Kylo-17-DS77 | 77 | gaaagacaaugagcaacaugu | 345 | acauguugcucauugucuuucuu |
| Kylo-17-DS78 | 78 | agacaaugagcaacauguguu | 346 | aacacauguugcucauugucuuu |
| Kylo-17-DS79 | 79 | caaugagcaacauguguucaa | 347 | uugaacacauguugcucauuguc |
| Kylo-17-DS80 | 80 | aaugagcaacauguguucaaa | 348 | uuugaacacauguugcucauugu |
| Kylo-17-DS81 | 81 | ugagcaacauguguucaaagu | 349 | acuuugaacacauguugcucauu |
| Kylo-17-DS82 | 82 | gcaacauguguucaaagucaa | 350 | uugacuuugaacacauguugcuc |
| Kylo-17-DS83 | 83 | acauguguucaaagucaagga | 351 | uccuugacuuugaacacauguug |
| Kylo-17-DS84 | 84 | cauguguucaaagucaaggau | 352 | auccuugacuuugaacacauguu |
| Kylo-17-DS85 | 85 | auguguucaaagucaaggaua | 353 | uauccuugacuuugaacacaugu |
| Kylo-17-DS86 | 86 | uguguucaaagucaaggauau | 354 | auauccuugacuuugaacacaug |
| Kylo-17-DS87 | 87 | guguucaaagucaaggauaug | 355 | cauauccuugacuuugaacacau |
| Kylo-17-DS88 | 88 | uguucaaagucaaggauaugg | 356 | ccauauccuugacuuugaacaca |
| Kylo-17-DS89 | 89 | guucaaagucaaggauaugga | 357 | uccauauccuugacuuugaacac |
| Kylo-17-DS90 | 90 | uucaaagucaaggauauggaa | 358 | uuccauauccuugacuuugaaca |
| Kylo-17-DS91 | 91 | ucaaagucaaggauauggaaa | 359 | uuuccauauccuugacuuugaac |
| Kylo-17-DS92 | 92 | caaagucaaggauauggaaaa | 360 | uuuuccauauccuugacuuugaa |
| Kylo-17-DS93 | 93 | aaagucaaggauauggaaaac | 361 | guuuuccauauccuugacuuuga |
| Kylo-17-DS94 | 94 | agucaaggauauggaaaaccu | 362 | agguuuuccauauccuugacuuu |
| Kylo-17-DS95 | 95 | ggauauggaaaaccuggaaga | 363 | ucuuccagguuuuccauauccuu |
| Kylo-17-DS96 | 96 | gaaaaaagaagcaggaauucc | 364 | ggaauuccugcuucuuuuuuccc |
| Kylo-17-DS97 | 97 | aaaaaagaagcaggaauuccu | 365 | aggaauuccugcuucuuuuuucc |
| Kylo-17-DS98 | 98 | agaagcaggaauuccugaauu | 366 | aauucaggaauuccugcuucuuu |
| Kylo-17-DS99 | 99 | aagcaggaauuccugaauuuu | 367 | aaaauucaggaauuccugcuucu |
| Kylo-17-DS100 | 100 | agcaggaauuccugaauuuua | 368 | uaaaauucaggaauuccugcuuc |
| Kylo-17-DS101 | 101 | gcaggaauuccugaauuuuau | 369 | auaaaauucaggaauuccugcuu |
| Kylo-17-DS102 | 102 | caggaauuccugaauuuuaug | 370 | cauaaaauucaggaauuccugcu |
| Kylo-17-DS103 | 103 | ggaauuccugaauuuuaugac | 371 | gucauaaaauucaggaauuccug |
| Kylo-17-DS104 | 104 | gaauuccugaauuuuaugacu | 372 | agucauaaaauucaggaauuccu |
| Kylo-17-DS105 | 105 | aauuccugaauuuuaugacua | 373 | uagucauaaaauucaggaauucc |
| Kylo-17-DS106 | 106 | uuccugaauuuuaugacuaug | 374 | cauagucauaaaauucaggaauu |
| Kylo-17-DS107 | 107 | uccugaauuuuaugacuauga | 375 | ucauagucauaaaauucaggaau |
| Kylo-17-DS108 | 108 | ccugaauuuuaugacuaugac | 376 | gucauagucauaaaauucaggaa |
| Kylo-17-DS109 | 109 | cugaauuuuaugacuaugacg | 377 | cgucauagucauaaaauucagga |
| Kylo-17-DS110 | 110 | ugaauuuuaugacuaugacgu | 378 | acgucauagucauaaaauucagg |
| Kylo-17-DS111 | 111 | gaauuuuaugacuaugacguu | 379 | aacgucauagucauaaaauucag |
| Kylo-17-DS112 | 112 | aauuuuaugacuaugacguug | 380 | caacgucauagucauaaaauuca |
| Kylo-17-DS113 | 113 | cccugaucaagcucaagaaua | 381 | uauucuugagcuugaucagggca |
| Kylo-17-DS114 | 114 | cugaucaagcucaagaauaag | 382 | cuuauucuugagcuugaucaggg |
| Kylo-17-DS115 | 115 | acaggauaucaaagcucuguu | 383 | aacagagcuuugauauccugugc |
| Kylo-17-DS116 | 116 | caggauaucaaagcucuguuu | 384 | aaacagagcuuugauauccugug |
| Kylo-17-DS117 | 117 | aggauaucaaagcucuguuug | 385 | caaacagagcuuugauauccugu |
| Kylo-17-DS118 | 118 | ggauaucaaagcucuguuugu | 386 | acaaacagagcuuugauauccug |
| Kylo-17-DS119 | 119 | gauaucaaagcucuguuugug | 387 | cacaaacagagcuuugauauccu |
| Kylo-17-DS120 | 120 | auaucaaagcucuguuugugu | 388 | acacaaacagagcuuugauaucc |
| Kylo-17-DS121 | 121 | ucuacaucaagaauggggaua | 389 | uauccccauucuugauguagacc |
| Kylo-17-DS122 | 122 | cuacaucaagaauggggauaa | 390 | uuauccccauucuugauguagac |
| Kylo-17-DS123 | 123 | acaucaagaauggggauaaga | 391 | ucuuauccccauucuugauguag |
| Kylo-17-DS124 | 124 | caucaagaauggggauaagaa | 392 | uucuuauccccauucuugaugua |
| Kylo-17-DS125 | 125 | aucaagaauggggauaagaaa | 393 | uuucuuauccccauucuugaugu |
| Kylo-17-DS126 | 126 | ucaagaauggggauaagaaag | 394 | cuuucuuauccccauucuugaug |
| Kylo-17-DS127 | 127 | gcuaugacaaagucaaggaca | 395 | uguccuugacuuugucauagccu |
| Kylo-17-DS128 | 128 | cuaugacaaagucaaggacau | 396 | auguccuugacuuugucauagcc |
| Kylo-17-DS129 | 129 | ccaauacuugcagaggugauu | 397 | aaucaccucugcaaguauugggg |
| Kylo-17-DS130 | 130 | cccuugauaguucacaagaga | 398 | ucucuugugaacuaucaaggggc |
| Kylo-17-DS131 | 131 | ccuugauaguucacaagagaa | 399 | uucucuugugaacuaucaagggg |
| Kylo-17-DS132 | 132 | cuugauaguucacaagagaag | 400 | cuucucuugugaacuaucaaggg |
| Kylo-17-DS133 | 133 | aguucacaagagaagucguuu | 401 | aaacgacuucucuugugaacuau |
| Kylo-17-DS134 | 134 | aguaguggaugucugcaaaaa | 402 | uuuuugcagacauccacuacucc |
| Kylo-17-DS135 | 135 | ggaugucugcaaaaaccagaa | 403 | uucugguuuuugcagacauccac |
| Kylo-17-DS136 | 136 | gaaacuccaagaugaggauuu | 404 | aaauccucaucuuggaguuucuc |
| Kylo-17-DS137 | 137 | ccaagaugaggauuuggguuu | 405 | aaacccaaauccucaucuuggag |
| Kylo-17-DS138 | 138 | caagaugaggauuuggguuuu | 406 | aaaacccaaauccucaucuugga |
| Kylo-17-DS139 | 139 | agaugaggauuuggguuuucu | 407 | agaaaacccaaauccucaucuug |
| Kylo-17-DS140 | 140 | gauugaauuaaaacagcugcg | 408 | cgcagcuguuuuaauucaauccc |
| Kylo-17-DS141 | 141 | gaauuaaaacagcugcgacaa | 409 | uugucgcagcuguuuuaauucaa |
| Kylo-17-DS142 | 142 | ccuacuacaaugugagugaua | 410 | uaucacucacauuguaguaggga |
| Kylo-17-DS143 | 143 | uacuacaaugugagugaugaa | 411 | uucaucacucacauuguaguagg |
| Kylo-17-DS144 | 144 | uacaaugugagugaugagauc | 412 | gaucucaucacucacauuguagu |
| Kylo-17-DS145 | 145 | augugagugaugagaucucuu | 413 | aagagaucucaucacucacauug |
| Kylo-17-DS146 | 146 | ugugagugaugagaucucuuu | 414 | aaagagaucucaucacucacauu |
| Kylo-17-DS147 | 147 | gugagugaugagaucucuuuc | 415 | gaaagagaucucaucacucacau |
| Kylo-17-DS148 | 148 | gagugaugagaucucuuucca | 416 | uggaaagagaucucaucacucac |
| Kylo-17-DS149 | 149 | gugaugagaucucuuuccacu | 417 | aguggaaagagaucucaucacuc |
| Kylo-17-DS150 | 150 | ugaugagaucucuuuccacua | 418 | uaguggaaagagaucucaucacu |
| Kylo-17-DS151 | 151 | gagaucucuuuccacugcuau | 419 | auagcaguggaaagagaucucau |
| Kylo-17-DS152 | 152 | agaucucuuuccacugcuaua | 420 | uauagcaguggaaagagaucuca |
| Kylo-17-DS153 | 153 | ccaagacuccuucauguacga | 421 | ucguacaugaaggagucuuggca |
| Kylo-17-DS154 | 154 | gcuagauggaucagacagcau | 422 | augcugucugauccaucuagcac |
| Kylo-17-DS155 | 155 | aggagccaaaaagugucuagu | 423 | acuagacacuuuuuggcuccugu |
| Kylo-17-DS156 | 156 | ggagccaaaaagugucuaguc | 424 | gacuagacacuuuuuggcuccug |
| Kylo-17-DS157 | 157 | gagccaaaaagugucuaguca | 425 | ugacuagacacuuuuuggcuccu |
| Kylo-17-DS158 | 158 | agccaaaaagugucuagucaa | 426 | uugacuagacacuuuuuggcucc |
| Kylo-17-DS159 | 159 | aagugucuagucaacuuaauu | 427 | aauuaaguugacuagacacuuuu |
| Kylo-17-DS160 | 160 | agugucuagucaacuuaauua | 428 | uaauuaaguugacuagacacuuu |
| Kylo-17-DS161 | 161 | ugucuagucaacuuaauugaa | 429 | uucaauuaaguugacuagacacu |
| Kylo-17-DS162 | 162 | uagucaacuuaauugagaaga | 430 | ucuucucaauuaaguugacuaga |
| Kylo-17-DS163 | 163 | aacuuaauugagaagguggca | 431 | ugccaccuucucaauuaaguuga |
| Kylo-17-DS164 | 164 | cuuaauugagaagguggcaaa | 432 | uuugccaccuucucaauuaaguu |
| Kylo-17-DS165 | 165 | uuaauugagaagguggcaagu | 433 | acuugccaccuucucaauuaagu |
| Kylo-17-DS166 | 166 | uaauugagaagguggcaaguu | 434 | aacuugccaccuucucaauuaag |
| Kylo-17-DS167 | 167 | auugagaagguggcaaguuau | 435 | auaacuugccaccuucucaauua |
| Kylo-17-DS168 | 168 | aguuauggugugaagccaaga | 436 | ucuuggcuucacaccauaacuug |
| Kylo-17-DS169 | 169 | guuauggugugaagccaagau | 437 | aucuuggcuucacaccauaacuu |
| Kylo-17-DS170 | 170 | uuauggugugaagccaagaua | 438 | uaucuuggcuucacaccauaacu |
| Kylo-17-DS171 | 171 | uauggugugaagccaagauau | 439 | auaucuuggcuucacaccauaac |
| Kylo-17-DS172 | 172 | aucaauuaugaagaccacaaa | 440 | uuuguggucuucauaauugauuu |
| Kylo-17-DS173 | 173 | uaugaagaccacaaguugaaa | 441 | uuucaacuuguggucuucauaau |
| Kylo-17-DS174 | 174 | ugaagaccacaaguugaaguc | 442 | gacuucaacuuguggucuucaua |
| Kylo-17-DS175 | 175 | gaagaccacaaguugaaguca | 443 | ugacuucaacuuguggucuucau |
| Kylo-17-DS176 | 176 | acaaguugaagucagggacua | 444 | uagucccugacuucaacuugugg |
| Kylo-17-DS177 | 177 | caaguugaagucagggacuaa | 445 | uuagucccugacuucaacuugug |
| Kylo-17-DS178 | 178 | aguugaagucagggacuaaca | 446 | uguuagucccugacuucaacuug |
| Kylo-17-DS179 | 179 | gccaugucaucauccucauga | 447 | ucaugaggaugaugacauggcgg |
| Kylo-17-DS180 | 180 | ccaugucaucauccucaugac | 448 | gucaugaggaugaugacauggcg |
| Kylo-17-DS181 | 181 | ggggacccaauuacugucauu | 449 | aaugacaguaauuggguccccgc |
| Kylo-17-DS182 | 182 | gggacccaauuacugucauua | 450 | uaaugacaguaauuggguccccg |
| Kylo-17-DS183 | 183 | acccaauuacugucauugaua | 451 | uaucaaugacaguaauugggucc |
| Kylo-17-DS184 | 184 | caagugaacaucaaugcuuua | 452 | uaaagcauugauguucacuuggu |
| Kylo-17-DS185 | 185 | aagugaacaucaaugcuuuga | 453 | ucaaagcauugauguucacuugg |
| Kylo-17-DS186 | 186 | gugaacaucaaugcuuuggcu | 454 | agccaaagcauugauguucacuu |
| Kylo-17-DS187 | 187 | gaacaucaaugcuuuggcuuc | 455 | gaagccaaagcauugauguucac |
| Kylo-17-DS188 | 188 | acaucaaugcuuuggcuucca | 456 | uggaagccaaagcauugauguuc |
| Kylo-17-DS189 | 189 | caucaaugcuuuggcuuccaa | 457 | uuggaagccaaagcauugauguu |
| Kylo-17-DS190 | 190 | ucaaugcuuuggcuuccaaga | 458 | ucuuggaagccaaagcauugaug |
| Kylo-17-DS191 | 191 | caaugcuuuggcuuccaagaa | 459 | uucuuggaagccaaagcauugau |
| Kylo-17-DS192 | 192 | ugcuuuggcuuccaagaaaga | 460 | ucuuucuuggaagccaaagcauu |
| Kylo-17-DS193 | 193 | gcuuuggcuuccaagaaagac | 461 | gucuuucuuggaagccaaagcau |
| Kylo-17-DS194 | 194 | uggcuuccaagaaagacaaua | 462 | uauugucuuucuuggaagccaaa |
| Kylo-17-DS195 | 195 | uuccaagaaagacaaugagca | 463 | ugcucauugucuuucuuggaagc |
| Kylo-17-DS196 | 196 | uccaagaaagacaaugagcaa | 464 | uugcucauugucuuucuuggaag |
| Kylo-17-DS197 | 197 | aaagacaaugagcaacaugua | 465 | uacauguugcucauugucuuucu |
| Kylo-17-DS198 | 198 | aagacaaugagcaacaugugu | 466 | acacauguugcucauugucuuuc |
| Kylo-17-DS199 | 199 | gacaaugagcaacauguguuc | 467 | gaacacauguugcucauugucuu |
| Kylo-17-DS200 | 200 | ugagcaacauguguucaaagu | 468 | acuuugaacacauguugcucauu |
| Kylo-17-DS201 | 201 | gagcaacauguguucaaaguc | 469 | gacuuugaacacauguugcucau |
| Kylo-17-DS202 | 202 | agcaacauguguucaaaguca | 470 | ugacuuugaacacauguugcuca |
| Kylo-17-DS203 | 203 | caacauguguucaaagucaaa | 471 | uuugacuuugaacacauguugcu |
| Kylo-17-DS204 | 204 | aacauguguucaaagucaaga | 472 | ucuugacuuugaacacauguugc |
| Kylo-17-DS205 | 205 | ucaaggauauggaaaaccuga | 473 | ucagguuuuccauauccuugacu |
| Kylo-17-DS206 | 206 | caaggauauggaaaaccugga | 474 | uccagguuuuccauauccuugac |
| Kylo-17-DS207 | 207 | gguaccgauuaccacaagcaa | 475 | uugcuugugguaaucgguacccu |
| Kylo-17-DS208 | 208 | cgauuaccacaagcaaccaua | 476 | uaugguugcuugugguaaucggu |
| Kylo-17-DS209 | 209 | gcuguggugucugaguacuuu | 477 | aaaguacucagacaccacagccc |
| Kylo-17-DS210 | 210 | uguggugucugaguacuuugu | 478 | acaaaguacucagacaccacagc |
| Kylo-17-DS211 | 211 | ugucugaguacuuugugcuga | 479 | ucagcacaaaguacucagacacc |
| Kylo-17-DS212 | 212 | ucugaguacuuugugcugaca | 480 | ugucagcacaaaguacucagaca |
| Kylo-17-DS213 | 213 | gcugacagcagcacauuguuu | 481 | aaacaaugugcugcugucagcac |
| Kylo-17-DS214 | 214 | caaggaacacucaaucaaggu | 482 | accuugauugaguguuccuuguc |
| Kylo-17-DS215 | 215 | aggaacacucaaucaagguca | 483 | ugaccuugauugaguguuccuug |
| Kylo-17-DS216 | 216 | aagaagcaggaauuccugaau | 484 | auucaggaauuccugcuucuuuu |
| Kylo-17-DS217 | 217 | gaagcaggaauuccugaauuu | 485 | aaauucaggaauuccugcuucuu |
| Kylo-17-DS218 | 218 | caggaauuccugaauuuuaua | 486 | uauaaaauucaggaauuccugcu |
| Kylo-17-DS219 | 219 | auuccugaauuuuaugacuau | 487 | auagucauaaaauucaggaauuc |
| Kylo-17-DS220 | 220 | uuccugaauuuuaugacuaua | 488 | uauagucauaaaauucaggaauu |
| Kylo-17-DS221 | 221 | cugaauuuuaugacuaugaca | 489 | ugucauagucauaaaauucagga |
| Kylo-17-DS222 | 222 | aauuuuaugacuaugacguua | 490 | uaacgucauagucauaaaauuca |
| Kylo-17-DS223 | 223 | ccugaucaagcucaagaauaa | 491 | uuauucuugagcuugaucagggc |
| Kylo-17-DS224 | 224 | cugaucaagcucaagaauaaa | 492 | uuuauucuugagcuugaucaggg |
| Kylo-17-DS225 | 225 | ccugcacaggauaucaaagcu | 493 | agcuuugauauccugugcaggga |
| Kylo-17-DS226 | 226 | ugcacaggauaucaaagcucu | 494 | agagcuuugauauccugugcagg |
| Kylo-17-DS227 | 227 | gauaucaaagcucuguuugua | 495 | uacaaacagagcuuugauauccu |
| Kylo-17-DS228 | 228 | uaucaaagcucuguuuguguc | 496 | gacacaaacagagcuuugauauc |
| Kylo-17-DS229 | 229 | aucaaagcucuguuugugucu | 497 | agacacaaacagagcuuugauau |
| Kylo-17-DS230 | 230 | ggaaggaggucuacaucaaga | 498 | ucuugauguagaccuccuuccga |
| Kylo-17-DS231 | 231 | gaaggaggucuacaucaagaa | 499 | uucuugauguagaccuccuuccg |
| Kylo-17-DS232 | 232 | aaggaggucuacaucaagaau | 500 | auucuugauguagaccuccuucc |
| Kylo-17-DS233 | 233 | uacaucaagaauggggauaaa | 501 | uuuauccccauucuugauguaga |
| Kylo-17-DS234 | 234 | ucaagaauggggauaagaaaa | 502 | uuuucuuauccccauucuugaug |
| Kylo-17-DS235 | 235 | caagaauggggauaagaaaga | 503 | ucuuucuuauccccauucuugau |
| Kylo-17-DS236 | 236 | agaauggggauaagaaaggca | 504 | ugccuuucuuauccccauucuug |
| Kylo-17-DS237 | 237 | ggauaagaaaggcagcuguga | 505 | ucacagcugccuuucuuaucccc |
| Kylo-17-DS238 | 238 | gcugugagagagaugcucaau | 506 | auugagcaucucucucacagcug |
| Kylo-17-DS239 | 239 | cugugagagagaugcucaaua | 507 | uauugagcaucucucucacagcu |
| Kylo-17-DS240 | 240 | ugugagagagaugcucaauau | 508 | auauugagcaucucucucacagc |
| Kylo-17-DS241 | 241 | gugagagagaugcucaauaua | 509 | uauauugagcaucucucucacag |
| Kylo-17-DS242 | 242 | cccaggcuaugacaaagucaa | 510 | uugacuuugucauagccuggggc |
| Kylo-17-DS243 | 243 | ccaggcuaugacaaagucaaa | 511 | uuugacuuugucauagccugggg |
| Kylo-17-DS244 | 244 | aggcuaugacaaagucaagga | 512 | uccuugacuuugucauagccugg |
| Kylo-17-DS245 | 245 | ggcuaugacaaagucaaggac | 513 | guccuugacuuugucauagccug |
| Kylo-17-DS246 | 246 | uaugacaaagucaaggacauc | 514 | gauguccuugacuuugucauagc |
| Kylo-17-DS247 | 247 | augacaaagucaaggacaucu | 515 | agauguccuugacuuugucauag |
| Kylo-17-DS248 | 248 | ugacaaagucaaggacaucuc | 516 | gagauguccuugacuuugucaua |
| Kylo-17-DS249 | 249 | ccuaugcugaccccaauacuu | 517 | aaguauuggggucagcauaggga |
| Kylo-17-DS250 | 250 | cccaauacuugcagaggugau | 518 | aucaccucugcaaguauuggggu |
| Kylo-17-DS251 | 251 | caauacuugcagaggugauuc | 519 | gaaucaccucugcaaguauuggg |
| Kylo-17-DS252 | 252 | aauacuugcagaggugauucu | 520 | agaaucaccucugcaaguauugg |
| Kylo-17-DS253 | 253 | gccccuugauaguucacaaga | 521 | ucuugugaacuaucaaggggccg |
| Kylo-17-DS254 | 254 | ccccuugauaguucacaagaa | 522 | uucuugugaacuaucaaggggcc |
| Kylo-17-DS255 | 255 | gauaguucacaagagaaguca | 523 | ugacuucucuugugaacuaucaa |
| Kylo-17-DS256 | 256 | auaguucacaagagaagucgu | 524 | acgacuucucuugugaacuauca |
| Kylo-17-DS257 | 257 | uaguucacaagagaagucguu | 525 | aacgacuucucuugugaacuauc |
| Kylo-17-DS258 | 258 | ggaguaguggaugucugcaaa | 526 | uuugcagacauccacuacucccc |
| Kylo-17-DS259 | 259 | gaguaguggaugucugcaaaa | 527 | uuuugcagacauccacuacuccc |
| Kylo-17-DS260 | 260 | guaguggaugucugcaaaaac | 528 | guuuuugcagacauccacuacuc |
| Kylo-17-DS261 | 261 | uggaugucugcaaaaaccaga | 529 | ucugguuuuugcagacauccacu |
| Kylo-17-DS262 | 262 | caaaaaccagaagcggcaaaa | 530 | uuuugccgcuucugguuuuugca |
| Kylo-17-DS263 | 263 | gagaaacuccaagaugaggau | 531 | auccucaucuuggaguuucuccu |
| Kylo-17-DS264 | 264 | aaacuccaagaugaggauuua | 532 | uaaauccucaucuuggaguuucu |
| Kylo-17-DS265 | 265 | uccaagaugaggauuuggguu | 533 | aacccaaauccucaucuuggagu |
| Kylo-17-DS266 | 266 | uugaauuaaaacagcugcgac | 534 | gucgcagcuguuuuaauucaauc |
| Kylo-17-DS267 | 267 | aauuaaaacagcugcgacaac | 535 | guugucgcagcuguuuuaauuca |

**Table 1-2 Primary Sequences of C5**

| siRNA Inhibitor | SEQ ID No: | Sense Strand (5'-3') | SEQ ID No: | Antisense Strand (5'-3') |
|---|---|---|---|---|
| Kylo-19-DS298 | 595 | gaaguuaaagaauaugucuu g | 1047 | agacauauucuuuaacuucaa |
| Kylo-19-DS299 | 596 | gaauaugucuugccacauuu u | 1048 | auguggcaagacauauucuuu |
| Kylo-19-DS300 | 597 | caagaacuuuaagaauuuug a | 1049 | aaaauucuuaaaguucuugua |
| Kylo-19-DS301 | 598 | gaacuuuaagaauuuugaaa u | 1050 | uucaaaauucuuaaaguucuu |
| Kylo-19-DS302 | 599 | cuuuaagaauuuugaaauuc u | 1051 | aauuucaaaauucuuaaaguu |
| Kylo-19-DS303 | 600 | gaauuuugaaauucuauaaa a | 1052 | aauuucaaaauucuuaaaguu |
| Kylo-19-DS304 | 601 | gaaauucuauaaaaagcaag a | 1053 | uugcuuuuuauagaauuucaa |
| Kylo-19-DS305 | 602 | cuauaaaaagcaagauauuu u | 1054 | aauaucuugcuuuuuauagaa |
| Kylo-19-DS306 | 603 | gcaagauauuuuuauaauaa a | 1055 | uauuauaaaaauaucuugcuu |
| Kylo-19-DS307 | 604 | caagauauuuuuauaauaaa g | 1056 | uuauuauaaaaauaucuugcu |
| Kylo-19-DS308 | 605 | gauauuuuuauaauaaagua g | 1057 | acuuuauuauaaaaauaucuu |
| Kylo-19-DS309 | 606 | aaagcaagauauuuuuauaa u | 1058 | uauaaaaauaucuugcuuuuu |
| Kylo-19-DS310 | 607 | caaaauaacucacuauaauu a | 1059 | auuauagugaguuauuuuguc |
| Kylo-19-DS311 | 608 | acaaaauaacucacuauaau u | 1060 | uuauagugaguuauuuuguca |
| Kylo-19-DS312 | 609 | agcaagauauuuuuauaaua a | 1061 | auuauaaaaauaucuugcuuu |
| Kylo-19-DS313 | 610 | caggagcaaacauaugucau u | 1062 | aaugacauauguuugcuccugu c |
| Kylo-19-DS314 | 611 | ggagcaaacauaugucauu | 1063 | aaugacauauguuugcuccug |
| Kylo-19-DS315 | 612 | acacugaagcauuugaugca a | 1064 | uugcaucaaaugcuucagugua u |
| Kylo-19-DS316 | 613 | auccugauaaaaaauuuagu u | 1065 | aacuaaauuuuuuaucaggaua a |
| Kylo-19-DS317 | 614 | gaagaaauugaucauauugg a | 1066 | uccaauaugaucaauuucuucu a |
| Kylo-19-DS318 | 615 | uuuaagaauuuugaaauuac u | 1067 | aguaauuucaaaauucuuaaag u |
| Kylo-19-DS319 | 616 | uaagaauuuugaaauuacu | 1068 | aguaauuucaaaauucuuaaa |
| Kylo-19-DS320 | 617 | aagcaagauauuuuuauaau a | 1069 | uauuauaaaaauaucuugcuuu u |
| Kylo-19-DS321 | 618 | auugcucaagucacauuuga u | 1070 | aucaaaugugacuugagcaauu c |
| Kylo-19-DS322 | 619 | auuuaaacaacaaguaccuu u | 1071 | aaagguacuuguuguuuaaauc u |
| Kylo-19-DS323 | 620 | ucccuacaaacugaauuugg u | 1072 | accaaauucaguuuguagggag a |
| Kylo-19-DS324 | 621 | ugacaaaauaacucacuaua a | 1073 | uuauagugaguuauuuugucaa u |
| Kylo-19-DS325 | 622 | gacaaaauaacucacuauaa u | 1074 | auuauagugaguuauuuuguca a |
| Kylo-19-DS326 | 623 | caaaauaacucacuauaau | 1075 | auuauagugaguuauuuuguc |
| Kylo-19-DS327 | 624 | aauaacucacuauaauuacu u | 1076 | aaguaauuauagugaguuauuu u |
| Kylo-19-DS328 | 625 | ucuuaucaaaguauaaacau u | 1077 | aauguuuauacuuugauaagau g |
| Kylo-19-DS329 | 626 | gcccuuggaaagaguauuuc a | 1078 | ugaaauacucuuuccaagggcu u |
| Kylo-19-DS330 | 627 | cccuuggaaagaguauuuca a | 1079 | uugaaauacucuuuccaagggc u |
| Kylo-19-DS331 | 628 | gaaaaugaugaaccuuguaa a | 1080 | uuuacaagguucaucauuuucu u |
| Kylo-19-DS332 | 629 | aaugaugaaccuuguaaaga a | 1081 | uucuuuacaagguucaucauuu u |
| Kylo-19-DS333 | 630 | agccagaaauucggaguuau u | 1082 | aauaacuccgaauuucuggcuu g |
| Kylo-19-DS334 | 631 | gcaguucuguguuaaaaugu c | 1083 | gacauuuuaacacagaacugcau |
| Kylo-19-DS335 | 632 | aguucuguguuaaaaugucu c | 1084 | gacauuuuaacacagaacugc |
| Kylo-19-DS336 | 633 | cuuccucuggaaauuggccu u | 1085 | aaggccaauuuccagaggaagca |
| Kylo-19-DS337 | 634 | uccucuggaaauuggccuuc a | 1086 | ugaaggccaauuuccagaggaag |
| Kylo-19-DS338 | 635 | uggaaaagaaaucuuaguaa a | 1087 | uuuacuaagauuucuuuuccaa a |
| Kylo-19-DS339 | 636 | aggauuuugaguguaaaagg a | 1088 | uccuuuuacacucaaauccuu u |
| Kylo-19-DS340 | 637 | caggaaaucauuggaacauu u | 1089 | aaauguuccaaugauuuccugu u |
| Kylo-19-DS341 | 638 | ggaaaucauuggaacauuu | 1090 | aaauguuccaaugauuuccug |
| Kylo-19-DS342 | 639 | aucauuggaacauuuuucau u | 1091 | aaugaaaaauguuccaaugauu u |
| Kylo-19-DS343 | 640 | ucccugggagauaaaacucac | 1092 | gugaguuuuaucucccagggaa a |
| Kylo-19-DS344 | 641 | acauguaacaacuguaguuc a | 1093 | ugaacuacaguuguuacaugua c |
| Kylo-19-DS345 | 642 | auguaacaacuguaguuca | 1094 | ugaacuacaguuguuacaugu |
| Kylo-19-DS346 | 643 | aggaaguuugcagcuuuuau u | 1095 | aauaaaagcugcaaacuuccuca |
| Kylo-19-DS347 | 644 | uauucugcaacugaauucga u | 1096 | aucgaauucaguugcagaauaa c |
| Kylo-19-DS348 | 645 | guuccggauauuugaacuuu u | 1097 | aaaaguucaaauauccggaaccg |
| Kylo-19-DS349 | 646 | gagauugcauaugcuuauaa a | 1098 | uuuauaagcauaugcaaucucu g |
| Kylo-19-DS350 | 647 | aaaauguuuuugucaaguac a | 1099 | uguacuugacaaaaacauuuuc u |
| Kylo-19-DS351 | 648 | ugcagaucaaacacaauuuc a | 1100 | ugaaauuguguuugaucugcag a |
| Kylo-19-DS352 | 649 | cagaucaaacacaauuucag u | 1101 | acugaaauuguguuugaucugc a |
| Kylo-19-DS353 | 650 | gcaaacauaugucauuucag u | 1102 | acugaaaugacauauguuugcu c |
| Kylo-19-DS354 | 651 | ucauuucagcaccaaaaaua u | 1103 | auauuuuuggugcugaaaugac a |
| Kylo-19-DS355 | 652 | guuuagaagauuuaaacaac a | 1104 | uguuguuuaaaucuucuaaacu g |
| Kylo-19-DS356 | 653 | ggaaguuugcagcuuuuauu u | 1105 | aaauaaaagcugcaaacuuccuc |
| Kylo-19-DS357 | 654 | agcauuuuuagcuaauuuag a | 1106 | ucuaaauuagcuaaaaaugcuu g |
| Kylo-19-DS358 | 655 | uuguuuuuuaaucuuccugg g | 1107 | cccaggaagauuaaaaaacaaag |
| Kylo-19-DS359 | 656 | acaggagcaaacauauguca u | 1108 | augacauauguuugcuccuguc c |
| Kylo-19-DS360 | 657 | uuucagcaccaaaaauauuc c | 1109 | ggaauauuuuuggugcugaaau g |
| Kylo-19-DS361 | 658 | ucagcaccaaaaauauuccgu | 1110 | acggaauauuuuuggugcugaa a |
| Kylo-19-DS362 | 659 | ugugauucaaguuuauggau a | 1111 | uauccauaaacuugaaucacaa u |
| Kylo-19-DS363 | 660 | gugauucaaguuuauggaua c | 1112 | guauccauaaacuugaaucacaa |
| Kylo-19-DS364 | 661 | ugauucaaguuuauggauac a | 1113 | uguauccauaaacuugaaucac a |
| Kylo-19-DS365 | 662 | auucaaguuuauggauacac u | 1114 | aguguauccauaaacuugaauc a |
| Kylo-19-DS366 | 663 | guuuauggauacacugaagc a | 1115 | ugcuucaguguauccauaaacu u |
| Kylo-19-DS367 | 664 | uauggauacacugaagcauu u | 1116 | aaaugcuucaguguauccauaa a |
| Kylo-19-DS368 | 665 | gauacacugaagcauuugau g | 1117 | caucaaaugcuucaguguaucca |
| Kylo-19-DS369 | 666 | ugaagcauuugaugcaacaa u | 1118 | auuguugcaucaaaugcuucag u |
| Kylo-19-DS370 | 667 | aagcauuugaugcaacaauc u | 1119 | agauuguugcaucaaaugcuuc a |
| Kylo-19-DS371 | 668 | cauuugaugcaacaaucucu a | 1120 | uagagauuguugcaucaaaugc u |
| Kylo-19-DS372 | 669 | auuugaugcaacaaucucua u | 1121 | auagagauuguugcaucaaaug c |
| Kylo-19-DS373 | 670 | uuugaugcaacaaucucuau u | 1122 | aauagagauuguugcaucaaau g |
| Kylo-19-DS374 | 671 | augcaacaaucucuauuaaa a | 1123 | uuuuaauagagauuguugcauc a |
| Kylo-19-DS375 | 672 | ugcaacaaucucuauuaaaa g | 1124 | cuuuuaauagagauuguugcau c |
| Kylo-19-DS376 | 673 | caacaaucucuauuaaaagu u | 1125 | aacuuuuaauagagauuguugc a |
| Kylo-19-DS377 | 674 | aacaaucucuauuaaaaguu a | 1126 | uaacuuuuaauagagauuguug c |
| Kylo-19-DS378 | 675 | acaaucucuauuaaaaguua u | 1127 | auaacuuuuaauagagauuguu g |
| Kylo-19-DS379 | 676 | caaucucuauuaaaaguuau c | 1128 | gauaacuuuuaauagagauugu u |
| Kylo-19-DS380 | 677 | aaucucuauuaaaaguuauc c | 1129 | ggauaacuuuuaauagagauug u |
| Kylo-19-DS381 | 678 | aucucuauuaaaaguuaucc u | 1130 | aggauaacuuuuaauagagauu g |
| Kylo-19-DS382 | 679 | ucucuauuaaaaguuauccu g | 1131 | caggauaacuuuuaauagagau u |
| Kylo-19-DS383 | 680 | uauccugauaaaaaauuuag u | 1132 | acuaaauuuuuuaucaggauaa c |
| Kylo-19-DS384 | 681 | aaaaauuuaguuacuccuca g | 1133 | cugaggaguaacuaaauuuuuu a |
| Kylo-19-DS385 | 682 | aaaauuuaguuacuccucag g | 1134 | ccugaggaguaacuaaauuuuu u |
| Kylo-19-DS386 | 683 | uuauccucagagaauaaauu c | 1135 | gaauuuauucucugaggauaaa u |
| Kylo-19-DS387 | 684 | uauccucagagaauaaauuc c | 1136 | ggaauuuauucucugaggauaa a |
| Kylo-19-DS388 | 685 | auccucagagaauaaauucc a | 1137 | uggaauuuauucucugaggaua a |
| Kylo-19-DS389 | 686 | ucagagaauaaauuccaaaa c | 1138 | guuuuggaauuuauucucugag g |
| Kylo-19-DS390 | 687 | uucuuauguguauuuggaag u | 1139 | acuuccaaauacacauaagaaac |
| Kylo-19-DS391 | 688 | auguguauuuggaaguugua u | 1140 | auacaacuuccaaauacacauaa |
| Kylo-19-DS392 | 689 | aagcauuuuucaaaaucaaa a | 1141 | uuuugauuuugaaaaaugcuu ug |
| Kylo-19-DS393 | 690 | aauaaccuaugacaauggau u | 1142 | aauccauugucauagguuauug g |
| Kylo-19-DS394 | 691 | auaaccuaugacaauggauu u | 1143 | aaauccauugucauagguuauu g |
| Kylo-19-DS395 | 692 | uaaccuaugacaauggauuu c | 1144 | gaaauccauugucauagguuau u |
| Kylo-19-DS396 | 693 | uaugacaauggauuucucuu c | 1145 | gaagagaaauccauugucauag g |
| Kylo-19-DS397 | 694 | augacaauggauuucucuuc a | 1146 | ugaagagaaauccauugucaua g |
| Kylo-19-DS398 | 695 | ugacaauggauuucucuuca u | 1147 | augaagagaaauccauugucau a |
| Kylo-19-DS399 | 696 | gacaauggauuucucuucau u | 1148 | aaugaagagaaauccauuguca u |
| Kylo-19-DS400 | 697 | caauggauuucucuucauuc a | 1149 | ugaaugaagagaaauccauugu c |
| Kylo-19-DS401 | 698 | aauggauuucucuucauuca u | 1150 | augaaugaagagaaauccauug u |
| Kylo-19-DS402 | 699 | auggauuucucuucauucau a | 1151 | uaugaaugaagagaaauccauu g |
| Kylo-19-DS403 | 700 | uggauuucucuucauucaua c | 1152 | guaugaaugaagagaaauccau u |
| Kylo-19-DS404 | 701 | auuucucuucauucauacag a | 1153 | ucuguaugaaugaagagaaauc c |
| Kylo-19-DS405 | 702 | uuucucuucauucauacaga C | 1154 | gucuguaugaaugaagagaaau C |
| Kylo-19-DS406 | 703 | cucuucauucauacagacaa a | 1155 | uuugucuguaugaaugaagaga a |
| Kylo-19-DS407 | 704 | uucauucauacagacaaacc u | 1156 | agguuugucuguaugaaugaag a |
| Kylo-19-DS408 | 705 | ucauacagacaaaccuguuu a | 1157 | uaaacagguuugucuguaugaa u |
| Kylo-19-DS409 | 706 | auacagacaaaccuguuuau a | 1158 | uauaaacagguuugucuguaug a |
| Kylo-19-DS410 | 707 | uacagacaaaccuguuuaua c | 1159 | guauaaacagguuugucuguau g |
| Kylo-19-DS411 | 708 | acagacaaaccuguuuauac u | 1160 | aguauaaacagguuugucugua u |
| Kylo-19-DS412 | 709 | cagacaaaccuguuuauacuc | 1161 | gaguauaaacagguuugucugu a |
| Kylo-19-DS413 | 710 | gacaaaccuguuuauacucca | 1162 | uggaguauaaacagguuugucu g |
| Kylo-19-DS414 | 711 | acaaaccuguuuauacuccag | 1163 | cuggaguauaaacagguuuguc u |
| Kylo-19-DS415 | 712 | uagaguuuauucguugaaug a | 1164 | ucauucaacgaauaaacucuaac |
| Kylo-19-DS416 | 713 | caaaagagaaacugucuuaa c | 1165 | guuaagacaguuucucuuuugg c |
| Kylo-19-DS417 | 714 | aaaagagaaacugucuuaac u | 1166 | aguuaagacaguuucucuuuug g |
| Kylo-19-DS418 | 715 | aaagagaaacugucuuaacu u | 1167 | aaguuaagacaguucucuuuu g |
| Kylo-19-DS419 | 716 | aagagaaacugucuuaacuu u | 1168 | aaaguuaagacaguuucucuuu u |
| Kylo-19-DS420 | 717 | agaaacugucuuaacuuuca u | 1169 | augaaaguuaagacaguuucuc u |
| Kylo-19-DS421 | 718 | aacugucuuaacuuucauag a | 1170 | ucuaugaaaguuaagacaguuu c |
| Kylo-19-DS422 | 719 | gacaugguagaagaaauuga u | 1171 | aucaauuucuucuaccauguca a |
| Kylo-19-DS423 | 720 | acaugguagaagaaauugau c | 1172 | gaucaauuucuucuaccauguc a |
| Kylo-19-DS424 | 721 | caugguagaagaaauugauc a | 1173 | ugaucaauuucuucuaccaugu c |
| Kylo-19-DS425 | 722 | aucauauuggaauuaucucu u | 1174 | aagagauaauuccaauaugauc a |
| Kylo-19-DS426 | 723 | cauauuggaauuaucucuuu u | 1175 | aaaagagauaauuccaauauga u |
| Kylo-19-DS427 | 724 | ucucuuuuccugacuucaag a | 1176 | ucuugaagucaggaaaagagau a |
| Kylo-19-DS428 | 725 | uaaauauaaagaggacuuuu c | 1177 | gaaaaguccucuuuauauuuag c |
| Kylo-19-DS429 | 726 | aaauauaaagaggacuuuuc a | 1178 | ugaaaaguccucuuuauauuua g |
| Kylo-19-DS430 | 727 | aaagaggacuuuucaacaac u | 1179 | aguuguugaaaaguccucuuua u |
| Kylo-19-DS431 | 728 | ggacuuuucaacaacuggaac | 1180 | guuccaguuguugaaaaguccu c |
| Kylo-19-DS432 | 729 | guuaaagaauaugucuugcc a | 1181 | uggcaagacauauucuuuaacu u |
| Kylo-19-DS433 | 730 | uuaaagaauaugucuugcca c | 1182 | guggcaagacauauucuuuaac u |
| Kylo-19-DS434 | 731 | uaaagaauaugucuugccac a | 1183 | ugugcaagacauauucuuuaa c |
| Kylo-19-DS435 | 732 | aaagaauaugucuugccaca u | 1184 | auguggcaagacauauucuuua a |
| Kylo-19-DS436 | 733 | agaauaugucuugccacauu u | 1185 | aaauguggcaagacauauucuu u |
| Kylo-19-DS437 | 734 | gaacuuuaagaauuuugaaa u | 1186 | auuucaaaauucuuaaaguucu u |
| Kylo-19-DS438 | 735 | aacuuuaagaauuuugaaau u | 1187 | aauuucaaaauucuuaaaguuc u |
| Kylo-19-DS439 | 736 | acuuuaagaauuuugaaauu a | 1188 | uaauuucaaaauucuuaaaguu c |
| Kylo-19-DS440 | 737 | cuuuaagaauuuugaaauua c | 1189 | guaauuucaaaauucuuaaagu u |
| Kylo-19-DS441 | 738 | uuugaaauuacuauaaaagc a | 1190 | ugcuuuuauaguaauuucaaaa u |
| Kylo-19-DS442 | 739 | gaaauuacuauaaaagcaag a | 1191 | ucuugcuuuuauaguaauuuca a |
| Kylo-19-DS443 | 740 | aaauuacuauaaaagcaaga u | 1192 | aucuugcuuuuauaguaauuuc a |
| Kylo-19-DS444 | 741 | aagcaagauauuuuuauaau a | 1193 | uauuauaaaaauaucuugcuuu u |
| Kylo-19-DS445 | 742 | agcaagauauuuuuauaaua a | 1194 | uuauuauaaaaauaucuugcuu u |
| Kylo-19-DS446 | 743 | auauuuuuauaauaaaguag u | 1195 | acuacuuuauuauaaaaauauc u |
| Kylo-19-DS447 | 744 | uuauaucacauuuggaauaa g | 1196 | cuuauuccaaaugugauauaaa c |
| Kylo-19-DS448 | 745 | uauaucacauuuggaauaag a | 1197 | ucuuauuccaaaugugauauaa a |
| Kylo-19-DS449 | 746 | auaucacauuuggaauaaga g | 1198 | cucuuauuccaaaugugauaua a |
| Kylo-19-DS450 | 747 | uuuggaauaagagaagacuu a | 1199 | uaagucuucucuuauuccaaau g |
| Kylo-19-DS451 | 748 | gagaagacuuaaaagaugau c | 1200 | gaucaucuuuuaagucuucucu u |
| Kylo-19-DS452 | 749 | agaugaucaaaagaaauga u | 1201 | aucauuucuuuuugaucaucuu u |
| Kylo-19-DS453 | 750 | gaugaucaaaaagaaaugau g | 1202 | caucauuucuuuuugaucaucu u |
| Kylo-19-DS454 | 751 | ugaucaaaaagaaaugaugc a | 1203 | ugcaucauuucuuuuugaucau c |
| Kylo-19-DS455 | 752 | gaaaugaugcaaacagcaau g | 1204 | caauugcuguuugcaucauuucu u |
| Kylo-19-DS456 | 753 | gcaaacagcaaugcaaaacac | 1205 | guguuuugcauugcuguuugca u |
| Kylo-19-DS457 | 754 | cagcaaugcaaaacacaaugu | 1206 | acauuguguuuugcauugcugu u |
| Kylo-19-DS458 | 755 | agcaaugcaaaacacaaugu u | 1207 | aacauuguguuuugcauugcug u |
| Kylo-19-DS459 | 756 | gcaaugcaaaacacaauguu g | 1208 | caacauuguguuuugcauugcu g |
| Kylo-19-DS460 | 757 | cacaauguugauaaauggaa u | 1209 | auuccauuuaucaacauugugu u |
| Kylo-19-DS461 | 758 | auguugauaaauggaauugc u | 1210 | agcaauuccauuuaucaacauu g |
| Kylo-19-DS462 | 759 | acauuugauucugaaacagc a | 1211 | ugcuguuucagaaucaaaugug a |
| Kylo-19-DS463 | 760 | auacuacaguuuagaagauu u | 1212 | aaaucuucuaaacuguaguaug a |
| Kylo-19-DS464 | 761 | caguuuagaagauuuaaaca a | 1213 | uuguuuaaaucuucuaaacugu a |
| Kylo-19-DS465 | 762 | uagaagauuuaaacaacaag u | 1214 | acuuguuguuuaaaucuucuaa a |
| Kylo-19-DS466 | 763 | aagauuuaaacaacaaguac c | 1215 | gguacuuguuguuuaaaucuuc u |
| Kylo-19-DS467 | 764 | agauuuaaacaacaaguacc u | 1216 | agguacuuguuguuuaaaucuu c |
| Kylo-19-DS468 | 765 | uaaacaacaaguaccuuuau a | 1217 | uauaaagguacuuguuguuuaa a |
| Kylo-19-DS469 | 766 | aacaacaaguaccuuuauau u | 1218 | aauauaaagguacuuguuguuu a |
| Kylo-19-DS470 | 767 | ccuuuauauugcuguaacag u | 1219 | acuguuacagcaauauaaaggu a |
| Kylo-19-DS471 | 768 | cuuuauauugcuguaacagu c | 1220 | gacuguuacagcaauauaaagg u |
| Kylo-19-DS472 | 769 | uuuauauugcuguaacaguc a | 1221 | ugacuguuacagcaauauaaag g |
| Kylo-19-DS473 | 770 | auauugcuguaacagucaua g | 1222 | cuaugacuguuacagcaauaua a |
| Kylo-19-DS474 | 771 | uauugcuguaacagucauag a | 1223 | ucuaugacuguuacagcaauau a |
| Kylo-19-DS475 | 772 | uguaacagucauagagucua c | 1224 | guagacucuaugacuguuacag c |
| Kylo-19-DS476 | 773 | guaacagucauagagucuac a | 1225 | uguagacucuaugacuguuaca g |
| Kylo-19-DS477 | 774 | cccuacaaacugaauuuggu u | 1226 | aaccaaauucaguuuguaggga g |
| Kylo-19-DS478 | 775 | uacaaacugaauuugguugc u | 1227 | agcaaccaaauucaguuuguag g |
| Kylo-19-DS479 | 776 | guaacacugaaugcacaaaca | 1228 | uguuugugcauucaguguuacu g |
| Kylo-19-DS480 | 777 | uaacacugaaugcacaaacaa | 1229 | uuguuugugcauucaguguuac u |
| Kylo-19-DS481 | 778 | aguguaacacguguugauga u | 1230 | aucaucaacacguguuacacuu u |
| Kylo-19-DS482 | 779 | gcuggaguuuaaugucaaaa c | 1231 | guuuugacauuaaacuccagcac |
| Kylo-19-DS483 | 780 | cuggaguuuaaugucaaaac u | 1232 | aguuuugacauuaaacuccagc a |
| Kylo-19-DS484 | 781 | uggaguuuaaugucaaaacu g | 1233 | caguuuugacauuaaacuccagc |
| Kylo-19-DS485 | 782 | gaguuuaaugucaaaacuga u | 1234 | aucaguuuugacauuaaacucc a |
| Kylo-19-DS486 | 783 | agauuuaaugucaaaacugau g | 1235 | caucaguuuugacauuaaacuc c |
| Kylo-19-DS487 | 784 | aagcccauauauugacaaaa u | 1236 | auuuugucaauauaugggcuuu u |
| Kylo-19-DS488 | 785 | agcccauauauugacaaaau a | 1237 | uauuuugucaauauaugggcuu u |
| Kylo-19-DS489 | 786 | uauugacaaaauaacucacu a | 1238 | uagugaguuauuuugucaauau a |
| Kylo-19-DS490 | 787 | uugacaaaauaacucacuau a | 1239 | uauagugaguuauuuugucaau a |
| Kylo-19-DS491 | 788 | uaacucacuauaauuacuug a | 1240 | ucaaguaauuauagugaguuau u |
| Kylo-19-DS492 | 789 | uuuauccaagggcaaaauua u | 1241 | auaauuuugcccuuggauaaaa u |
| Kylo-19-DS493 | 790 | uuauccaagggcaaaauuau c | 1242 | gauaauuuugcccuuggauaaa a |
| Kylo-19-DS494 | 791 | augcaucuuaucaaaguaua a | 1243 | uuauacuuugauaagaugcauc u |
| Kylo-19-DS495 | 792 | aaaguauaaacauuccagua a | 1244 | uuacuggaauguuuauacuuug a |
| Kylo-19-DS496 | 793 | ggccaaacugugucucuuaa u | 1245 | auuaagagacacaguuuggccu g |
| Kylo-19-DS497 | 794 | ccuuggaaagaguauuucaa u | 1246 | auugaaauacucuuuccaaggg c |
| Kylo-19-DS498 | 795 | uuggaaagaguauuucaauu c | 1247 | gaauugaaauacucuuuccaag g |
| Kylo-19-DS499 | 796 | uggaaagaguauuucaauuc u | 1248 | agaauugaaauacucuuuccaa g |
| Kylo-19-DS500 | 797 | aagaguauuucaauucuuag a | 1249 | ucuaagaauugaaauacucuuu c |
| Kylo-19-DS501 | 798 | agaguauuucaauucuuaga g | 1250 | cucuaagaauugaaauacucuu u |
| Kylo-19-DS502 | 799 | aauucuuagagaagagugau c | 1251 | gaucacucuucucuaagaauug a |
| Kylo-19-DS503 | 800 | cucacuaaugcaaaugcagau | 1252 | aucugcauuugcauuagugagg a |
| Kylo-19-DS504 | 801 | ucacuaaugcaaaugcagau g | 1253 | caucugcauuugcauuagugag g |
| Kylo-19-DS505 | 802 | ugacucccaagaaaaugaug a | 1254 | ucaucauuuucuugggagucau c |
| Kylo-19-DS506 | 803 | acucccaagaaaaugaugaac | 1255 | guucaucauuuucuugggaguc a |
| Kylo-19-DS507 | 804 | ucccaagaaaaugaugaaccu | 1256 | agguucaucauuuucuugggag u |
| Kylo-19-DS508 | 805 | cccaagaaaaugaugaaccuu | 1257 | aaggguucaucauuuucuuggga g |
| Kylo-19-DS509 | 806 | caagaaaaugaugaaccuug u | 1258 | acaagguucaucauuuucuugg g |
| Kylo-19-DS510 | 807 | aagaaaaugaugaaccuugu a | 1259 | uacaagguucaucauuuucuug g |
| Kylo-19-DS511 | 808 | augaugaaccuuguaaagaa a | 1260 | uuucuuuacaagguucaucauu u |
| Kylo-19-DS512 | 809 | aagauagaagaaauagcugc u | 1261 | agcagcuauuucuucuaucuuc u |
| Kylo-19-DS513 | 810 | uagcugcuaaauauaaacau u | 1262 | aauguuuauauuuagcagcuau u |
| Kylo-19-DS514 | 811 | uagugaagaaauguuguuac g | 1263 | cguaacaacauuucuucacuacu |
| Kylo-19-DS515 | 812 | agugaagaaauguuguuacg a | 1264 | ucguaacaacauuucuucacuac |
| Kylo-19-DS516 | 813 | ugaagaaauguuguuacgau g | 1265 | caucguaacaacauuucuucacu |
| Kylo-19-DS517 | 814 | uguucccagaagaaaacagu u | 1266 | aacuguuuucuucugggaacaa g |
| Kylo-19-DS518 | 815 | gaugucuuccuggaaaugaa u | 1267 | auucauuuccaggaagacaucu u |
| Kylo-19-DS519 | 816 | acuguuuacaacuauaggac u | 1268 | aguccuauaguuguaaacaguu c |
| Kylo-19-DS520 | 817 | cuguuuacaacuauaggacu u | 1269 | aaguccuauaguuguaaacagu u |
| Kylo-19-DS521 | 818 | caguucuguguuaaaauguc u | 1270 | agacauuuuaacacagaacugca |
| Kylo-19-DS522 | 819 | uucuguguuaaaaugucugc u | 1271 | agcagacauuuuaacacagaacu |
| Kylo-19-DS523 | 820 | ucuguguuaaaaugucugcu g | 1272 | cagcagacauuuuaacacagaac |
| Kylo-19-DS524 | 821 | ugugguuaaaaugucugcugu g | 1273 | cacagcagacauuuuaacacaga |
| Kylo-19-DS525 | 822 | uguuaaaaugucugcugugg a | 1274 | uccacagcagacauuuuaacaca |
| Kylo-19-DS526 | 823 | aaaucaaaaggauuuugagu g | 1275 | cacucaaaauccuuuugauuuc u |
| Kylo-19-DS527 | 824 | aaucaaaaggauuuugagug u | 1276 | acacucaaaauccuuuugauuu c |
| Kylo-19-DS528 | 825 | guugucccaguauucuaugu u | 1277 | aacauagaauacugggacaacgc |
| Kylo-19-DS529 | 826 | uugucccaguauucuauguu u | 1278 | aaacauagaauacugggacaacg |
| Kylo-19-DS530 | 827 | ugucccaguauucuauguuu u | 1279 | aaaacauagaauacugggacaac |
| Kylo-19-DS531 | 828 | cccaguauucuauguuuuuc a | 1280 | ugaaaaacauagaauacugggac |
| Kylo-19-DS532 | 829 | uauucuauguuuucacuac c | 1281 | gguagugaaaaacauagaauac u |
| Kylo-19-DS533 | 830 | gaaacaggaaaucauuggaa c | 1282 | guuccaaugauuuccuguuucc a |
| Kylo-19-DS534 | 831 | acaggaaaucauuggaacau u | 1283 | aauguuccaaugauuuccuguu u |
| Kylo-19-DS535 | 832 | ugcuuuaagaguacuuggac a | 1284 | uguccaaguacucuuaaagcaaa |
| Kylo-19-DS536 | 833 | agagcagaaccaaaauucaau | 1285 | auugaauuuugguucugcucua c |
| Kylo-19-DS537 | 834 | ggaaaauucacaguaucaacc | 1286 | gguugauacugugaauuuuccu u |
| Kylo-19-DS538 | 835 | gaaaauucacaguaucaacca | 1287 | ugguugauacugugaauuuucc u |
| Kylo-19-DS539 | 836 | aaaauucacaguaucaaccaa | 1288 | uuggguugauacugugaauuuuc c |
| Kylo-19-DS540 | 837 | aaauucacaguaucaaccaa u | 1289 | auugguugauacugugaauuuu c |
| Kylo-19-DS541 | 838 | aguaucaaccaauaaaauua c | 1290 | guaauuuuauugguugauacug u |
| Kylo-19-DS542 | 839 | ucaaccaauaaaauuacagg g | 1291 | cccuguaauuuuauugguugau a |
| Kylo-19-DS543 | 840 | caaccaauaaaauuacaggg u | 1292 | acccuguaauuuuauugguuga u |
| Kylo-19-DS544 | 841 | accaauaaaauuacagggua c | 1293 | guacccuguaauuuuauugguu g |
| Kylo-19-DS545 | 842 | ccaauaaaauuacaggguacc | 1294 | ggguacccuguaauuuuauuggu u |
| Kylo-19-DS546 | 843 | caauaaaauuacaggguacc u | 1295 | agguacccuguaauuuuauugg u |
| Kylo-19-DS547 | 844 | auaaaauuacaggguaccuu g | 1296 | caagguacccuguaauuuuauu g |
| Kylo-19-DS548 | 845 | ccgagagaacagcuuauaucu | 1297 | agauauaagcuguucucucggg c |
| Kylo-19-DS549 | 846 | agagaacagcuuauaucuua c | 1298 | guaagauauaagcuguucucuc g |
| Kylo-19-DS550 | 847 | gagaacagcuuauaucuuac a | 1299 | uguaagauauaagcuguucucu c |
| Kylo-19-DS551 | 848 | agaacagcuuauaucuuaca g | 1300 | cuguaagauauaagcuguucuc u |
| Kylo-19-DS552 | 849 | uguuucagcuuugaagagag a | 1301 | ucucucuucaaagcugaaacaau |
| Kylo-19-DS553 | 850 | gguaauccacccauuuaucg u | 1302 | acgauaaauggguggauuaccu u |
| Kylo-19-DS554 | 851 | guaauccacccauuuaucgu u | 1303 | aacgauaaauggguggauuacc u |
| Kylo-19-DS555 | 852 | auccacccauuuaucguuuu u | 1304 | aaaaacgauaaauggguggauu a |
| Kylo-19-DS556 | 853 | uccacccauuuaucguuuuu g | 1305 | caaaaacgauaaauggguggau u |
| Kylo-19-DS557 | 854 | ccacccauuuaucguuuuug g | 1306 | ccaaaaacgauaaauggguggau |
| Kylo-19-DS558 | 855 | cacccauuuaucguuuuugg a | 1307 | uccaaaaacgauaaaugggugga |
| Kylo-19-DS559 | 856 | uuuaucguuuuuggaaagac a | 1308 | ugucuuuccaaaaacgauaaau g |
| Kylo-19-DS560 | 857 | agaaacaacugccuaugcuu u | 1309 | aaagcauaggcaguuguuucua c |
| Kylo-19-DS561 | 858 | gaaacaacugccuaugcuuu a | 1310 | uaaagcauaggcaguuguuucu a |
| Kylo-19-DS562 | 859 | auauucacuccugguuaaac a | 1311 | uguuuaaccaggagugaauauu c |
| Kylo-19-DS563 | 860 | uacauaauuauaaaaugaca g | 1312 | cugucauuuuauaauuauguaa g |
| Kylo-19-DS564 | 861 | cauaauuauaaaaugacaga c | 1313 | gucugucauuuuauaauuaugu a |
| Kylo-19-DS565 | 862 | aaaaugacagacaagaauuu c | 1314 | gaaauucuugucugucauuuua u |
| Kylo-19-DS566 | 863 | augacagacaagaauuuccu u | 1315 | aaggaaauucuugucugucauu u |
| Kylo-19-DS567 | 864 | caacuguaguucacaaaacca | 1316 | ugguuuugugaacuacaguugu u |
| Kylo-19-DS568 | 865 | uguaguucacaaaaccaguac | 1317 | guacugguuuugugaacuacag u |
| Kylo-19-DS569 | 866 | aaguuugcagcuuuuauuug a | 1318 | ucaaauaaaagcugcaaacuucc |
| Kylo-19-DS570 | 867 | gaaacucugauuacaaacgca | 1319 | ugcguuuguaaucagaguuucc g |
| Kylo-19-DS571 | 868 | aacucugauuacaaacgcau a | 1320 | uaugcguuuguaaucagaguuu c |
| Kylo-19-DS572 | 869 | acucugauuacaaacgcauag | 1321 | cuaugcguuuguaaucagaguu u |
| Kylo-19-DS573 | 870 | cucugauuacaaacgcauag u | 1322 | acuaugcguuuguaaucagagu u |
| Kylo-19-DS574 | 871 | gauggacauguuauucugca a | 1323 | uugcagaauaacauguccaucu u |
| Kylo-19-DS575 | 872 | acauguuauucugcaacuga a | 1324 | uucaguugcagaauaacauguc c |
| Kylo-19-DS576 | 873 | guuauucugcaacugaauuc g | 1325 | cgaauucaguugcagaauaaca u |
| Kylo-19-DS577 | 874 | uuauucugcaacugaauucg a | 1326 | ucgaauucaguugcagaauaac a |
| Kylo-19-DS578 | 875 | ccagugauuuccuuugugua c | 1327 | guacacaaaggaaaucacuggag |
| Kylo-19-DS579 | 876 | agugauuuccuuuguguacg a | 1328 | ucguacacaaaggaaaucacugg |
| Kylo-19-DS580 | 877 | ugauuuccuuuguguacgau u | 1329 | aaucguacacaaaggaaaucacu |
| Kylo-19-DS581 | 878 | ugaacucuuugaaguugggu u | 1330 | aacccaacuucaaagaguucaaa |
| Kylo-19-DS582 | 879 | gaacucuuugaaguugggguu u | 1331 | aaacccaacuucaaagaguucaa |
| Kylo-19-DS583 | 880 | acagaccagauaaacagugua | 1332 | uacacuguuuaucuggucugug g |
| Kylo-19-DS584 | 881 | accagauaaacaguguaccau | 1333 | augguacacuguuuaucugguc u |
| Kylo-19-DS585 | 882 | gauaaacaguguaccauguu u | 1334 | aaacaugguacacuguuuaucu g |
| Kylo-19-DS586 | 883 | aguguaccauguuuuauagc a | 1335 | ugcuauaaaacaugguacacug u |
| Kylo-19-DS587 | 884 | guguaccauguuuuauagca c | 1336 | gugcuauaaaacaugguacacu g |
| Kylo-19-DS588 | 885 | caugguuuuauagcacuucca a | 1337 | uuggaagugcuauaaaacaugg u |
| Kylo-19-DS589 | 886 | augguuuuauagcacuuccaa u | 1338 | auuggaagugcuauaaaacaug g |
| Kylo-19-DS590 | 887 | uguuuuauagcacuuccaau a | 1339 | uauuggaagugcuauaaaacau g |
| Kylo-19-DS591 | 888 | uuuuauagcacuuccaauau c | 1340 | gauauuggaagugcuauaaaac a |
| Kylo-19-DS592 | 889 | uuuauagcacuuccaauauc a | 1341 | ugauauuggaagugcuauaaaa c |
| Kylo-19-DS593 | 890 | uauagcacuuccaauaucaa a | 1342 | uuugauauuggaagugcuauaa a |
| Kylo-19-DS594 | 891 | uagcacuuccaauaucaaaa u | 1343 | auuuugauauuggaagugcuau a |
| Kylo-19-DS595 | 892 | gcacuuccaauaucaaaauu c | 1344 | gaauuuugauauuggaagugcu a |
| Kylo-19-DS596 | 893 | aucaaaauucagaaagucug u | 1345 | acagacuuucugaauuuugaua u |
| Kylo-19-DS597 | 894 | cccuucuggauaucuacaaaa | 1346 | uuuuguagauauccagaagggu u |
| Kylo-19-DS598 | 895 | uucuggauaucuacaaaacu g | 1347 | caguuuuguagauauccagaag g |
| Kylo-19-DS599 | 896 | ucuggauaucuacaaaacug g | 1348 | ccaguuuuguagauauccagaa g |
| Kylo-19-DS600 | 897 | gaagacaguacuuaauuaug g | 1349 | ccauaauuaaguacugucuucc u |
| Kylo-19-DS601 | 898 | aagacaguacuuaauuaugg g | 1350 | cccauaauuaaguacugucuucc |
| Kylo-19-DS602 | 899 | uggucuuauuuguaaagcuc a | 1351 | ugagcuuuacaaauaagaccagc |
| Kylo-19-DS603 | 900 | ggucuuauuuguaaagcuca c | 1352 | gugagcuuuacaaauaagaccag |
| Kylo-19-DS604 | 901 | uaaagcucacuuuacuuaga a | 1353 | uucuaaguaaagugagcuuuac a |
| Kylo-19-DS605 | 902 | cuuuacuuagaauuaguggc a | 1354 | ugccacuaauucuaaguaaagu g |
| Kylo-19-DS606 | 903 | uuuacuuagaauuaguggca c | 1355 | gugccacuaauucuaaguaaag u |
| Kylo-19-DS607 | 904 | ggcacuugcuuuuauuagag a | 1356 | ucucuaauaaaagcaagugccac |
| Kylo-19-DS608 | 905 | ugcuuuuauuagagaaugau u | 1357 | aaucauucucuaauaaaagcaa g |
| Kylo-19-DS609 | 906 | uguaacuuucugaaauaaca u | 1358 | auguuauuucagaaaguuacag c |
| Kylo-19-DS610 | 907 | uggacaccggaaacaauaaau | 1359 | auuuauuguuuccgguguccaa u |
| Kylo-19-DS611 | 908 | ggacaccggaaacaauaaauu | 1360 | aauuuauuguuuccggugucca a |
| Kylo-19-DS612 | 909 | cuuugcuugaaagaaaauac c | 1361 | |
| Kylo-19-DS613 | 910 | gaaacugaucauuaaagccu g | 1362 | caggcuuuaaugaucaguuucc u |
| Kylo-19-DS614 | 911 | aaacugaucauuaaagccug a | 1363 | ucaggcuuuaaugaucaguuuc c |
| Kylo-19-DS615 | 912 | cugaucauuaaagccugagu u | 1364 | aacucaggcuuuaaugaucagu u |
| Kylo-19-DS616 | 913 | ugaucauuaaagccugaguu u | 1365 | aaacucaggcuuuaaugaucag u |
| Kylo-19-DS617 | 914 | ucauuaaagccugaguuugc u | 1366 | agcaaacucaggcuuuaaugauc |
| Kylo-19-DS618 | 915 | cauuaaagccugaguuugcu u | 1367 | aagcaaacucaggcuuuaauga u |
| Kylo-19-DS619 | 916 | cauuucagcaccaaaaauau u | 1368 | aauauuuuuggugcugaaauga c |
| Kylo-19-DS620 | 917 | auauugugauucaaguuuau g | 1369 | cauaaacuugaaucacaauauu u |
| Kylo-19-DS621 | 918 | gcauuugaugcaacaaucuc u | 1370 | agagauuguugcaucaaaugcu u |
| Kylo-19-DS622 | 919 | gcaacaaucucuauuaaaag u | 1371 | acuuuuaauagagauuguugca u |
| Kylo-19-DS623 | 920 | uaaaaguuauccugauaaaa a | 1372 | uuuuuaucaggauaacuuuuaa u |
| Kylo-19-DS624 | 921 | aaaaguuauccugauaaaaa u | 1373 | auuuuuaucaggauaacuuuua a |
| Kylo-19-DS625 | 922 | ccugauaaaaaauuuaguua c | 1374 | guaacuaaauuuuuuaucagga u |
| Kylo-19-DS626 | 923 | cugauaaaaaauuuaguuac u | 1375 | aguaacuaaauuuuuuaucagg a |
| Kylo-19-DS627 | 924 | gauaaaaaauuuaguuacuc a | 1376 | ugaguaacuaaauuuuuuauca g |
| Kylo-19-DS628 | 925 | auuuauccucagagaauaaa u | 1377 | auuuauucucugaggauaaaug a |
| Kylo-19-DS629 | 926 | gaauaaauuccaaaacucug c | 1378 | gcagaguuuuggaauuuauucu c |
| Kylo-19-DS630 | 927 | ccaaaacucugcaaucuuaac | 1379 | guuaagauugcagaguuuugga a |
| Kylo-19-DS631 | 928 | caauacaaccaaaacaauugc | 1380 | gcaauuguuuugguuguauugu u |
| Kylo-19-DS632 | 929 | gacaaaacccaguuucuuau g | 1381 | cauaagaaacuggguuuugucc u |
| Kylo-19-DS633 | 930 | guaaaaguuagaguuuauuc g | 1382 | cgaauaaacucuaacuuuuacu g |
| Kylo-19-DS634 | 931 | gaguuuauucguugaaugac g | 1383 | cgucauucaacgaauaaacucua |
| Kylo-19-DS635 | 932 | agaagaaauugaucauauug g | 1384 | ccaauaugaucaauuucuucua c |
| Kylo-19-DS636 | 933 | agaaauugaucauauuggaa u | 1385 | auuccaauaugaucaauuucuu c |
| Kylo-19-DS637 | 934 | gaucauauuggaauuaucuc u | 1386 | agagauaauuccaauaugauca a |
| Kylo-19-DS638 | 935 | auauaaagaggacuuuucaa c | 1387 | guugaaaaguccucuuuauauu u |
| Kylo-19-DS639 | 936 | cgcauauuuugaaguuaaag a | 1388 | ucuuuaacuucaaaauaugcgg u |
| Kylo-19-DS640 | 937 | gaaguuaaagaauaugucuu g | 1389 | caagacauauucuuuaacuuca a |
| Kylo-19-DS641 | 938 | gguuacaagaacuuuaagaa u | 1390 | auucuuaaaguucuuguaacca a |
| Kylo-19-DS642 | 939 | guuacaagaacuuuaagaau u | 1391 | aauucuuaaaguucuuguaacc a |
| Kylo-19-DS643 | 940 | agaacuuuaagaauuuugaa a | 1392 | uuucaaaauucuuaaaguucuu g |
| Kylo-19-DS644 | 941 | ugaaauuacuauaaaagcaa g | 1393 | cuugcuuuuauaguaauuucaa a |
| Kylo-19-DS645 | 942 | acuauaaaagcaagauauuu u | 1394 | aaaauaucuugcuuuuauagua a |
| Kylo-19-DS646 | 943 | cuauaaaagcaagauauuuu u | 1395 | aaaaauaucuugcuuuuauagu a |
| Kylo-19-DS647 | 944 | gaauaagagaagacuuaaaa g | 1396 | cuuuuaagucuucucuuauucc a |
| Kylo-19-DS648 | 945 | aauaagagaagacuuaaaag a | 1397 | ucuuuuaagucuucucuuauuc c |
| Kylo-19-DS649 | 946 | acuuaaaagaugaucaaaaa g | 1398 | cuuuuugaucaucuuuuaaguc u |
| Kylo-19-DS650 | 947 | cuuaaaagaugaucaaaag a | 1399 | ucuuuuugaucaucuuuuaagu c |
| Kylo-19-DS651 | 948 | caaaacacaauguugauaaa u | 1400 | auuuaucaacauuguguuuugc a |
| Kylo-19-DS652 | 949 | cauacuacaguuuagaagau u | 1401 | aaucuucuaaacuguaguauga c |
| Kylo-19-DS653 | 950 | acaguuuagaagauuuaaac a | 1402 | uguuuaaaucuucuaaacugua g |
| Kylo-19-DS654 | 951 | acaacaaguaccuuuauauu g | 1403 | caauauaaagguacuuguuguu u |
| Kylo-19-DS655 | 952 | gaaauaccuggcaucaaaua u | 1404 | auauuugaugccagguauuucu g |
| Kylo-19-DS656 | 953 | ccuacaaacugaauuugguu g | 1405 | caaccaaauucaguuuguaggg a |
| Kylo-19-DS657 | 954 | ggaguuuaaugucaaaacug a | 1406 | ucaguuuugacauuaaacucca g |
| Kylo-19-DS658 | 955 | ccaaaguuaccuuuauauug a | 1407 | ucaauauaaagguaacuuuggc u |
| Kylo-19-DS659 | 956 | aaaguuaccuuuauauugau u | 1408 | aaucaauauaaagguaacuuug g |
| Kylo-19-DS660 | 957 | cccauauauugacaaaauaa c | 1409 | guuauuuugucaauauaugggc u |
| Kylo-19-DS661 | 958 | ccauauauugacaaaauaac u | 1410 | agguuauuuugucaauauauggg c |
| Kylo-19-DS662 | 959 | acaaaauaacucacuauaau u | 1411 | aauuauagugaguuauuuuguc a |
| Kylo-19-DS663 | 960 | caaaauaacucacuauaauu a | 1412 | uaauuauagugaguuauuuug uc |
| Kylo-19-DS664 | 961 | aaaauaacucacuauaauua c | 1413 | guaauuauagugaguuauuuug u |
| Kylo-19-DS665 | 962 | aaauaacucacuauaauuac u | 1414 | aguaauuauagugaguuauuu ug |
| Kylo-19-DS666 | 963 | gcaucuuaucaaaguauaaa c | 1415 | guuuauacuuugauaagaugca u |
| Kylo-19-DS667 | 964 | caucuuaucaaaguauaaac a | 1416 | uguuuauacuuugauaagaugc a |
| Kylo-19-DS668 | 965 | cuuaucaaaguauaaacauu c | 1417 | gaauguuuauacuuugauaaga u |
| Kylo-19-DS669 | 966 | uuaucaaaguauaaacauuc c | 1418 | ggaauguuuauacuuugauaag a |
| Kylo-19-DS670 | 967 | ccaaacugugucucuuaaua u | 1419 | auauuaagagacacaguuuggc c |
| Kylo-19-DS671 | 968 | gaguauuucaauucuuagag a | 1420 | ucucuaagaauugaaauacucu u |
| Kylo-19-DS672 | 969 | guauuucaauucuuagagaa g | 1421 | cuucucuaagaauugaaauacu c |
| Kylo-19-DS673 | 970 | aaaugaugaaccuuguaaag a | 1422 | ucuuuacaagguucaucauuuu c |
| Kylo-19-DS674 | 971 | ccuuguaaagaaauucucag a | 1423 | ucugagaauuucuuuacaaggu u |
| Kylo-19-DS675 | 972 | agaaauagcugcuaaauaua a | 1424 | uuauauuuagcagcuauuucuu c |
| Kylo-19-DS676 | 973 | ugcuaaauauaaacauucag u | 1425 | acugaauguuuauauuuagcag c |
| Kylo-19-DS677 | 974 | gcguuaauaaugaugaaacc u | 1426 | agguuucaucauuauuaacgca g |
| Kylo-19-DS678 | 975 | ugcuaauaucucucauaaag a | 1427 | ucuuuaugagagauauuagcac g |
| Kylo-19-DS679 | 976 | cuaauaucucucauaaagac a | 1428 | ugucuuuaugagagauauuagc a |
| Kylo-19-DS680 | 977 | ccagaaauucggaguuauuu u | 1429 | aaaauaacuccgaauuucuggc u |
| Kylo-19-DS681 | 978 | cagaaauucggaguuauuuu c | 1430 | gaaaauaacuccgaauuucuggc |
| Kylo-19-DS682 | 979 | agaaauucggaguuauuuuc c | 1431 | ggaaaauaacuccgaauuucug g |
| Kylo-19-DS683 | 980 | agaaaucuuaguaaaaacau u | 1432 | aauguuuuuacuaagauuucuu u |
| Kylo-19-DS684 | 981 | cuuaguaaaaacauuacgag u | 1433 | acucguaauguuuuuacuaaga u |
| Kylo-19-DS685 | 982 | gcuauucugguguuacuuug g | 1434 | ccaaaguaacaccagaauagcuu |
| Kylo-19-DS686 | 983 | gauuuugaguguaaaaggac u | 1435 | aguccuuuuacacucaaaauccu |
| Kylo-19-DS687 | 984 | ccaguauucuauguuuuuca c | 1436 | gugaaaaacauagaauacuggg a |
| Kylo-19-DS688 | 985 | caguauucuauguuuuucac u | 1437 | agugaaaaacauagaauacugg g |
| Kylo-19-DS689 | 986 | guauucuauguuuuucacua c | 1438 | guagugaaaaacauagaauacu g |
| Kylo-19-DS690 | 987 | ggaaaucauuggaacauuuu u | 1439 | aaaaauguuccaaugauuuccu g |
| Kylo-19-DS691 | 988 | gaaaucauuggaacauuuuu c | 1440 | gaaaaauguuccaaugauuucc u |
| Kylo-19-DS692 | 989 | aaaucauuggaacauuuuuc a | 1441 | ugaaaaauguuccaaugauuuc c |
| Kylo-19-DS693 | 990 | cagcuuuugcuuuaagagua c | 1442 | guacucuuaaagcaaaagcugu u |
| Kylo-19-DS694 | 991 | acaaguaaauaaauacguag a | 1443 | ucuacguauuuauuuacuuguc c |
| Kylo-19-DS695 | 992 | accaaaauucaauuuguaau u | 1444 | aauuacaaauugaauuuugguu c |
| Kylo-19-DS696 | 993 | cuuuauuguggcuaguugag a | 1445 | ucucaacuagccacaauaaagaa |
| Kylo-19-DS697 | 994 | gcuaguugagaauuaucaau u | 1446 | aauugauaauucucaacuagcc a |
| Kylo-19-DS698 | 995 | ugagaauuaucaauuagaua a | 1447 | uuaucuaauugauaauucucaa c |
| Kylo-19-DS699 | 996 | gagaauuaucaauuagauaa u | 1448 | auuaucuaauugauaauucuca a |
| Kylo-19-DS700 | 997 | agaauuaucaauuagauaau g | 1449 | cauuaucuaauugauaauucuc a |
| Kylo-19-DS701 | 998 | gaauuaucaauuagauaaug u | 1450 | acauuaucuaauugauaauucu c |
| Kylo-19-DS702 | 999 | uuagauaauggaucuuucaa g | 1451 | cuugaaagauccauuaucuaau u |
| Kylo-19-DS703 | 1000 | cuuucaaggaaaauucacag u | 1452 | acugugaauuuuccuugaaaga u |
| Kylo-19-DS704 | 1001 | guaucaaccaauaaaauuac a | 1453 | uguaauuuuauugguugauacu g |
| Kylo-19-DS705 | 1002 | acaacuuucugcuugaaaau a | 1454 | uauuuucaagcagaaaguuguc a |
| Kylo-19-DS706 | 1003 | caacuuucugcuugaaaaua C | 1455 | guauuuucaagcagaaaguugu C |
| Kylo-19-DS707 | 1004 | guuucguucaauuguuucag c | 1456 | gcugaaacaauugaacgaaacug |
| Kylo-19-DS708 | 1005 | cccauuuaucguuuuuggaa a | 1457 | uuuccaaaaacgauaaaugggu g |
| Kylo-19-DS709 | 1006 | acuugaaagauauaaauuau g | 1458 | cauaauuuauaucuuucaaguu c |
| Kylo-19-DS710 | 1007 | cuugaaagauauaaauuaug u | 1459 | acauaauuuauaucuuucaagu u |
| Kylo-19-DS711 | 1008 | cauaaaggugccuuacauaa u | 1460 | auuauguaaggcaccuuuaugc u |
| Kylo-19-DS712 | 1009 | ccuuacauaauuauaaaaug a | 1461 | ucauuuuauaauuauguaaggc a |
| Kylo-19-DS713 | 1010 | acauaauuauaaaaugacag a | 1462 | ucugucauuuuauaauuaugua a |
| Kylo-19-DS714 | 1011 | gaaguuugcagcuuuuauuu g | 1463 | caaauaaaagcugcaaacuuccu |
| Kylo-19-DS715 | 1012 | gcuuuuauuugaaaaucgau a | 1464 | uaucgauuuucaaauaaaagcu g |
| Kylo-19-DS716 | 1013 | cuuuuauuugaaaaucgaua c | 1465 | guaucgauuuucaaauaaaagc u |
| Kylo-19-DS717 | 1014 | cugauuaccaaaucaaagau g | 1466 | caucuuugauuugguaaucagu g |
| Kylo-19-DS718 | 1015 | caaagauggacauguuauuc u | 1467 | agaauaacauguccaucuuuga u |
| Kylo-19-DS719 | 1016 | ggauauuugaacucuuugaa g | 1468 | cuucaaagaguucaaauauccgg |
| Kylo-19-DS720 | 1017 | ugagauuaccuucauuaaaa a | 1469 | uuuuuaaugaagguaaucucag a |
| Kylo-19-DS721 | 1018 | gagauuaccuucauuaaaaa g | 1470 | cuuuuuaaugaagguaaucuca g |
| Kylo-19-DS722 | 1019 | cuuaauuauggguaaagaag u | 1471 | acuucuuuacccauaauuaagu a |
| Kylo-19-DS723 | 1020 | cagauaaaauacaauuucag u | 1472 | acugaaauuguauuuuaucugg a |
| Kylo-19-DS724 | 1021 | cauuuuuagcuaauuuagau g | 1473 | caucuaaauuagcuaaaaaugc u |
| Kylo-19-DS725 | 1022 | gaauuugccgaagauaucuu u | 1474 | aaagauaucuucggcaaauuca u |
| Kylo-19-DS726 | 1023 | auucauagcuggucuuauuu g | 1475 | caaauaagaccagcuaugaaugu |
| Kylo-19-DS727 | 1024 | cacuuuacuuagaauuagug g | 1476 | ccacuaauucuaaguaaaguga g |
| Kylo-19-DS728 | 1025 | guaacuuucugaaauaacau g | 1477 | caugguuauuucagaaaguuaca g |
| Kylo-19-DS729 | 1026 | ccauugaaagggaguauuac a | 1478 | uguaauacucccuuucaaugga c |
| Kylo-19-DS730 | 1027 | gguuaaauauugaagaaaaa u | 1479 | auuuuucuucaauauuuaacca g |
| Kylo-19-DS731 | 1028 | gcaagauauuuuuauaauaa a | 1480 | uuuauuauaaaaauaucuugcu u |
| Kylo-19-DS732 | 1029 | cgaucaaggcuaaauauaaa g | 1481 | cuuuauauuuagccuugaucgu c |
| Kylo-19-DS733 | 1030 | cgcauauuuugaaguuaaag a | 1482 | ucuuuaacuucaaaauaugcgg u |
| Kylo-19-DS734 | 1031 | gcauaugcuuauaaaguuag u | 1483 | acuaacuuuauaagcauaugca a |
| Kylo-19-DS735 | 1032 | ggaacuguuuacaacuauag u | 1484 | acuauaguuguaaacaguuccu u |
| Kylo-19-DS736 | 1033 | gcaucuuaucaaaguauaaa c | 1485 | guuuauacuuugauaagaugca u |
| Kylo-19-DS737 | 1034 | ccauauauugacaaaauaac u | 1486 | agguuauuuugucaauauauggg c |
| Kylo-19-DS738 | 1035 | ccgcauauuuugaaguuaaa g | 1487 | cuuuaacuucaaaauaugcggu u |
| Kylo-19-DS739 | 1036 | ggaaggcaucaauauccuaac | 1488 | guuaggauauugaugccuuccu g |
| Kylo-19-DS740 | 1037 | gcuuuuauuugaaaaucgau a | 1489 | uaucgauuuucaaauaaaagcu g |
| Kylo-19-DS741 | 1038 | gaauuuugaaauuacuauaa a | 1490 | uuuauaguaauuucaaaauucu u |
| Kylo-19-DS742 | 1039 | gaaacugaagaaaaaauuaa a | 1491 | uuuaauuuuuucuucaguuuc ug |
| Kylo-19-DS743 | 1040 | ggaagacaguacuuaauuaa u | 1492 | auuaauuaaguacugucuuccu u |
| Kylo-19-DS744 | 1041 | gcagagacaagaaaacaaaca | 1493 | uguuuguuuucuugucucugca g |
| Kylo-19-DS745 | 1042 | gggaugcaguucuguguuaa a | 1494 | uuuaacacagaacugcaucccag |
| Kylo-19-DS746 | 1043 | gcaaaagaagauagaagaaa u | 1495 | auuucuucuaucuucuuuugca g |
| Kylo-19-DS747 | 1044 | caaaagaagauagaagaaau a | 1496 | uauuucuucuaucuucuuuugc a |
| Kylo-19-DS748 | 1045 | gcuaguugagaauuaucaau u | 1497 | aauugauaauucucaacuagcc a |
| Kylo-19-DS749 | 1046 | gcauaugcuuauaaaguuag a | 1498 | ucuaacuuuauaagcauaugca a |
| Kylo-19-DS821 | 3355 | ggaaguuugcagcuuuuauu u | 3356 | aaauaaaagcugcaaacuuccuc |

**Table 1-3 Primary Sequences of C3**

| RNA inhibitor | Sense sequence 5 'to 3' | SEQ NO ID: | Start position in NM_000064 .4 | Antisense sequence 5 'to 3' | SEQ NO ID: |
|---|---|---|---|---|---|
| Kylo-27-DS01 | gagucccauguacucuauc au | 3357 | 125 | gauagaguacaugggacuccc | 2272 |
| Kylo-27-DS02 | gucccauguacucuauca uca | 3358 | 127 | augauagaguacaugggacu c | 2273 |
| Kylo-27-DS03 | uguuccagucacuguuac ugu | 3359 | 215 | aguaacagugacuggaacauc | 2274 |
| Kylo-27-DS04 | acuguuacuguccacgacu uc | 3360 | 225 | agucguggacaguaacaguga | 2275 |
| Kylo-27-DS05 | cuguuacuguccacgacu ucc | 3361 | 226 | aagucguggacaguaacagug | 2276 |
| Kylo-27-DS06 | uagugcuguccagugaga aga | 3362 | 259 | uucucacuggacagcacuagu | 2277 |
| Kylo-27-DS07 | cagggaguucaagucagaa aa | 1687 | 338 | uucugacuugaacucccugu u | 2278 |
| Kylo-27-DS08 | ggaguucaagucagaaaa ggg | 1688 | 341 | cuuuucugacuugaacuccc u | 2279 |
| Kylo-27-DS09 | gacagacaagaccaucuac ac | 1689 | 458 | guagauggucuugucugucu g | 2280 |
| Kylo-27-DS10 | auccagacagacaagacca uu | 1690 | 474 | aauggucuugucugucugga u | 2281 |
| Kylo-27-DS11 | auccagacagacaagacca uu | 1691 | 474 | aauggucuugucugucugga uga | 2282 |
| Kylo-27-DS12 | uccagacagacaagaccau cu | 1692 | 475 | agauggucuugucugucugg a | 2283 |
| Kylo-27-DS13 | uccagacagacaagaccau cu | 1693 | 475 | agauggucuugucugucugg aug | 2284 |
| Kylo-27-DS14 | gacagacaagaccaucuac aa | 1694 | 479 | uuguagauggucuugucugu c | 2285 |
| Kylo-27-DS15 | gacagacaagaccaucuac aa | 1695 | 479 | uuguagauggucuugucugu cug | 2286 |
| Kylo-27-DS16 | uccacaguucucuaucgga uc | 1696 | 486 | uccgauagagaacuguggagc | 2287 |
| Kylo-27-DS17 | ccacaguucucuaucggau cu | 1697 | 487 | auccgauagagaacuguggag | 2288 |
| Kylo-27-DS1 8 | acaguucucuaucggauc uuc | 169 8 | 489 | agauccgauagagaacugugg | 228 9 |
| Kylo-27-DS1 9 | caguucucuaucggaucu uca | 169 9 | 490 | aagauccgauagagaacugu g | 229 0 |
| Kylo-27-DS2 0 | guucucuaucggaucuuc acc | 170 0 | 492 | ugaagauccgauagagaacu g | 229 1 |
| Kylo-27-DS2 1 | uucucuaucggaucuuca ccg | 170 1 | 493 | gugaagauccgauagagaac u | 229 2 |
| Kylo-27-DS2 2 | aucggaucuucaccgucaa cc | 170 2 | 499 | uugacggugaagauccgaua g | 229 3 |
| Kylo-27-DS2 3 | ggucauggucaacauuga gaa | 170 3 | 545 | cucaauguugaccaugaccgu | 229 4 |
| Kylo-27-DS2 4 | gacuccuugucuucucag aac | 170 4 | 591 | ucugagaagacaaggaguccu | 229 5 |
| Kylo-27-DS2 5 | acuccuugucuucucaga acc | 170 5 | 592 | uucugagaagacaaggagucc | 229 6 |
| Kylo-27-DS2 6 | cuccuugucuucucagaac ca | 170 6 | 593 | guucugagaagacaaggaguc | 229 7 |
| Kylo-27-DS2 7 | ugucuucucagaaccagcu ug | 170 7 | 598 | agcugguucugagaagacaag | 229 8 |
| Kylo-27-DS2 8 | auuccggaacucgucaaca ug | 170 8 | 642 | uguugacgaguuccggaaug u | 229 9 |
| Kylo-27-DS2 9 | uuccggaacucgucaacau gg | 170 9 | 643 | auguugacgaguuccggaau g | 230 0 |
| Kylo-27-DS3 0 | gaagauccgagccuacuau ga | 171 0 | 671 | auaguaggcucggaucuucca | 230 1 |
| Kylo-27-DS3 1 | agauccgagccuacuauga aa | 171 1 | 673 | ucauaguaggcucggaucuu c | 230 2 |
| Kylo-27-DS3 2 | gauccgagccuacuaugaa aa | 171 2 | 674 | uucauaguaggcucggaucu u | 230 3 |
| Kylo-27-DS3 3 | auccgagccuacuaugaaa ac | 171 3 | 675 | uuucauaguaggcucggauc u | 230 4 |
| Kylo-27-DS3 4 | uccgagccuacuaugaaaa cu | 171 4 | 676 | uuuucauaguaggcucggau c | 230 5 |
| Kylo-27-DS3 5 | ccgagccuacuaugaaaac uc | 171 5 | 677 | guuuucauaguaggcucgga u | 230 6 |
| Kylo-27-DS3 6 | cgagccuacuaugaaaacu ca | 171 6 | 678 | aguuuucauaguaggcucgg a | 230 7 |
| Kylo-27-DS3 7 | gagccuacuaugaaaacuc ac | 171 7 | 679 | gaguuuucauaguaggcucg g | 230 8 |
| Kylo-27-DS3 8 | agccuacuaugaaaacuca cc | 171 8 | 680 | ugaguuuucauaguaggcuc g | 230 9 |
| Kylo-27-DS3 9 | gccuacuaugaaaacucac ca | 171 9 | 681 | gugaguuuucauaguaggcu C | 231 0 |
| Kylo-27-DS40 | ccaguuucgaggucauag ugg | 1720 | 748 | acuaugaccucgaaacugggc | 2311 |
| Kylo-27-DS41 | ggagccuacagagaaauuc ua | 1721 | 767 | gaauuucucuguaggcuccac | 2312 |
| Kylo-27-DS42 | gagccuacagagaaauucu ac | 1722 | 768 | agaauuucucuguaggcucc a | 2313 |
| Kylo-27-DS43 | agccuacagagaaauucua cu | 1723 | 769 | uagaauuucucuguaggcuc c | 2314 |
| Kylo-27-DS44 | cagagaaauucuacuaca ucu | 1724 | 775 | auguaguagaauuucucugu a | 2315 |
| Kylo-27-DS45 | gaaauucuacuacaucua uaa | 1725 | 779 | auagauguaguagaauuucu c | 2316 |
| Kylo-27-DS46 | aaauucuacuacaucuau aac | 1726 | 780 | uauagauguaguagaauuuc u | 2317 |
| Kylo-27-DS47 | aauucuacuacaucuaua acg | 1727 | 781 | uuauagauguaguagaauuu c | 2318 |
| Kylo-27-DS48 | ucuacuacaucuauaacg aga | 1728 | 784 | ucguuauagauguaguagaa u | 2319 |
| Kylo-27-DS49 | acuacaucuauaacgagaa gg | 1729 | 787 | uucucguuauagauguagua g | 2320 |
| Kylo-27-DS50 | aggauuucccugccugaa ucc | 1730 | 900 | auucaggcagggaaauccucu | 2321 |
| Kylo-27-DS51 | ucaagcgcauuccgauuga gg | 1731 | 922 | ucaaucggaaugcgcuugagg | 2322 |
| Kylo-27-DS52 | gauccacuucaccaagaca cc | 1732 | 1121 | ugucuuggugaaguggaucu g | 2323 |
| Kylo-27-DS53 | uucaccaagacacccaagu ac | 1733 | 1128 | acuugggugucuuggugaag u | 2324 |
| Kylo-27-DS54 | ucaccaagacacccaagua cu | 1734 | 1129 | uacuugggugucuuggugaa g | 2325 |
| Kylo-27-DS55 | ccaagacacccaaguacuu ca | 1735 | 1132 | aaguacuugggugucuuggu g | 2326 |
| Kylo-27-DS56 | gacacccaaguacuucaaa cc | 1736 | 1136 | uuugaaguacuugggugucu u | 2327 |
| Kylo-27-DS57 | acacccaaguacuucaaac ca | 1737 | 1137 | guuugaaguacuugggugucu u | 2328 |
| Kylo-27-DS58 | cacccaaguacuucaaacc ag | 1738 | 1138 | gguuugaaguacuugggugu c | 2329 |
| Kylo-27-DS59 | aaaccaggaaugcccuuug ac | 1739 | 1152 | caaagggcauuccugguuug a | 2330 |
| Kylo-27-DS60 | aaccaggaaugcccuuuga cc | 1740 | 1153 | ucaaagggcauuccugguuu g | 2331 |
| Kylo-27-DS6 1 | uugaccucaugguguucg uga | 174 1 | 1168 | acgaacaccaugaggucaaag | 233 2 |
| Kylo-27-DS6 2 | ucauggguguucgugacga acc | 174 2 | 1174 | uucgucacgaacaccaugagg | 233 3 |
| Kylo-27-DS6 3 | gccaaacucagcaucaaca ca | 174 3 | 1278 | uguugaugcugaguuuggcc a | 233 4 |
| Kylo-27-DS6 4 | ggcaacuccaacaauuacc ug | 174 4 | 1407 | gguaauuguuggaguugccc a | 233 5 |
| Kylo-27-DS6 5 | gcaacuccaacaauuaccu gc | 174 5 | 1408 | agguaauuguuggaguugcc c | 233 6 |
| Kylo-27-DS6 6 | ucucucagugcuacguaca ga | 174 6 | 1430 | uguacguagcacugagagau g | 233 7 |
| Kylo-27-DS6 7 | ucaacuuccuccugcgaau gg | 174 7 | 1477 | auucgcaggaggaaguugacg | 233 8 |
| Kylo-27-DS6 8 | acuacaccuaccugaucau ga | 174 8 | 1525 | augaucagguagguguagua g | 233 9 |
| Kylo-27-DS6 9 | ccuaccugaucaugaacaa gg | 174 9 | 1531 | uuguucaugaucagguaggu g | 234 0 |
| Kylo-27-DS7 0 | cuaccugaucaugaacaag gg | 175 0 | 1532 | cuuguucaugaucagguagg u | 234 1 |
| Kylo-27-DS7 1 | uaccugaucaugaacaagg gc | 175 1 | 1533 | ccuuguucaugaucagguag g | 234 2 |
| Kylo-27-DS7 2 | gaugacccugaagauagag gg | 175 2 | 1799 | cucuaucuucagggucaucu g | 234 3 |
| Kylo-27-DS7 3 | cguguucgugcugaauaa gaa | 175 3 | 1862 | cuuauucagcacgaacacgcc | 234 4 |
| Kylo-27-DS7 4 | guguucgugcugaauaag aag | 175 4 | 1863 | ucuuauucagcacgaacacgc | 234 5 |
| Kylo-27-DS7 5 | uguucgugcugaauaaga aga | 175 5 | 1864 | uucuuauucagcacgaacacg | 234 6 |
| Kylo-27-DS7 6 | guucgugcugaauaagaa gaa | 175 6 | 1865 | cuucuuauucagcacgaacac | 234 7 |
| Kylo-27-DS7 7 | uucgugcugaauaagaag aac | 175 7 | 1866 | ucuucuuauucagcacgaac a | 234 8 |
| Kylo-27-DS7 8 | ucgugcugaauaagaaga aca | 175 8 | 1867 | uucuucuuauucagcacgaa c | 234 9 |
| Kylo-27-DS7 9 | ugcugaauaagaagaaca aac | 175 9 | 1870 | uuguucuucuuauucagcac g | 235 0 |
| Kylo-27-DS8 0 | gcugaauaagaagaacaaa cu | 176 0 | 1871 | uuuguucuucuuauucagca c | 235 1 |
| Kylo-27-DS8 1 | aacugacgcagaguaagau cu | 176 1 | 1888 | aucuuacucugcgucaguuu g | 235 2 |
| Kylo-27-DS8 2 | acugacgcagaguaagauc ug | 176 2 | 1889 | gaucuuacucugcgucaguu u | 235 3 |
| Kylo-27-DS8 3 | cugacgcagaguaagaucu gg | 176 3 | 1890 | agaucuuacucugcgucagu u | 235 4 |
| Kylo-27-DS8 4 | ugacgcagaguaagaucu ggg | 176 4 | 1891 | cagaucuuacucugcgucagu | 235 5 |
| Kylo-27-DS8 5 | auggacaaagucggcaagu ac | 176 5 | 2097 | acuugccgacuuuguccauuc | 235 6 |
| Kylo-27-DS8 6 | uggacaaagucggcaagua cc | 176 6 | 2098 | uacuugccgacuuuguccau u | 235 7 |
| Kylo-27-DS8 7 | uggacugcugcaacuacau ca | 176 7 | 2233 | auguaguugcagcaguccagg | 235 8 |
| Kylo-27-DS8 8 | ugcugcaacuacaucacag ag | 176 8 | 2238 | cugugauguaguugcagcag u | 235 9 |
| Kylo-27-DS8 9 | gcugcaacuacaucacaga gc | 176 9 | 2239 | ucugugauguaguugcagca g | 236 0 |
| Kylo-27-DS9 0 | gcaacuacaucacagagcu gc | 177 0 | 2242 | agcucugugauguaguugca g | 236 1 |
| Kylo-27-DS9 1 | guaaccuggaugaggaca uca | 177 1 | 2305 | auguccucauccagguuacuc | 236 2 |
| Kylo-27-DS9 2 | uugcagaagagaacaucg uuu | 177 2 | 2326 | acgauguucucuucugcaau g | 236 3 |
| Kylo-27-DS9 3 | ugcagaagagaacaucgu uuc | 177 3 | 2327 | aacgauguucucuucugcaa u | 236 4 |
| Kylo-27-DS9 4 | gcagaagagaacaucguu ucc | 177 4 | 2328 | aaacgauguucucuucugca a | 236 5 |
| Kylo-27-DS9 5 | gaacaucguuucccgaagu ga | 177 5 | 2336 | acuucgggaaacgauguucuc | 236 6 |
| Kylo-27-DS9 6 | aucguuucccgaagugag uuc | 177 6 | 2340 | acucacuucgggaaacgaugu | 236 7 |
| Kylo-27-DS9 7 | ucguuucccgaagugagu ucc | 177 7 | 2341 | aacucacuucgggaaacgaug | 236 8 |
| Kylo-27-DS9 8 | uggaacguugaggacuug aaa | 177 8 | 2376 | ucaaguccucaacguuccaca | 236 9 |
| Kylo-27-DS9 9 | ggaacguugaggacuuga aag | 177 9 | 2377 | uucaaguccucaacguuccac | 237 0 |
| Kylo-27-DS1 00 | gaacguugaggacuugaa aga | 178 0 | 2378 | uuucaaguccucaacguucca | 237 1 |
| Kylo-27-DS1 01 | aacguugaggacuugaaa gag | 178 1 | 2379 | cuuucaaguccucaacguucc | 237 2 |
| Kylo-27-DS1 02 | acguugaggacuugaaag agc | 178 2 | 2380 | ucuuucaaguccucaacguu c | 237 3 |
| Kylo-27-DS1 03 | cuugaaagagccaccgaaa aa | 178 3 | 2390 | uuucgguggcucuuucaagu c | 237 4 |
| Kylo-27-DS1 04 | uugaaagagccaccgaaaa au | 178 4 | 2391 | uuuucgguggcucuuucaag u | 237 5 |
| Kylo-27-DS1 05 | ugaaagagccaccgaaaaa ug | 178 5 | 2392 | uuuuucgguggcucuuucaa g | 237 6 |
| Kylo-27-DS1 06 | gaaagagccaccgaaaaau gg | 178 6 | 2393 | auuuuucgguggcucuuuca a | 237 7 |
| Kylo-27-DS1 07 | aaagagccaccgaaaaaug ga | 178 7 | 2394 | cauuuuucgguggcucuuuc a | 237 8 |
| Kylo-27-DS1 08 | gagccaccgaaaaauggaa uc | 178 8 | 2397 | uuccauuuuucgguggcucu u | 237 9 |
| Kylo-27-DS1 09 | agccaccgaaaaauggaau cu | 178 9 | 2398 | auuccauuuuucgguggcuc u | 238 0 |
| Kylo-27-DS1 10 | ccgaaaaauggaaucucua cg | 179 0 | 2403 | uagagauuccauuuuucggu g | 238 1 |
| Kylo-27-DS1 11 | cgaaaaauggaaucucuac ga | 179 1 | 2404 | guagagauuccauuuuucgg u | 238 2 |
| Kylo-27-DS1 12 | auggaaucucuacgaagc uca | 179 2 | 2410 | agcuucguagagauuccauu u | 238 3 |
| Kylo-27-DS1 13 | ucucuacgaagcucaugaa ua | 179 3 | 2416 | uucaugagcuucguagagau u | 238 4 |
| Kylo-27-DS1 14 | ucuacgaagcucaugaau aua | 179 4 | 2418 | uauucaugagcuucguagag a | 238 5 |
| Kylo-27-DS1 15 | cuacgaagcucaugaaua uau | 179 5 | 2419 | auauucaugagcuucguaga g | 238 6 |
| Kylo-27-DS1 16 | gagcaugucggacaagaaa gg | 179 6 | 2480 | uuucuuguccgacaugcucac | 238 7 |
| Kylo-27-DS1 17 | agcaugucggacaagaaag gg | 179 7 | 2481 | cuuucuuguccgacaugcuca | 238 8 |
| Kylo-27-DS1 18 | ucggacaagaaagggaucu gu | 179 8 | 2487 | agaucccuuucuuguccgaca | 238 9 |
| Kylo-27-DS1 19 | cuucgaggucacaguaau gca | 179 9 | 2519 | cauuacugugaccucgaaggg | 239 0 |
| Kylo-27-DS1 20 | ucgaggucacaguaaugca gg | 180 0 | 2521 | ugcauuacugugaccucgaag | 239 1 |
| Kylo-27-DS1 21 | guaaugcaggacuucuuc auc | 180 1 | 2532 | ugaagaaguccugcauuacu g | 239 2 |
| Kylo-27-DS1 22 | aggacuucuucaucgaccu gc | 180 2 | 2539 | aggucgaugaagaaguccugc | 239 3 |
| Kylo-27-DS1 23 | gcuacccuacucuguugu ucg | 180 3 | 2561 | aacaacagaguaggguagccg | 239 4 |
| Kylo-27-DS1 24 | cuacccuacucuguuguu cga | 180 4 | 2562 | gaacaacagaguaggguagcc | 239 5 |
| Kylo-27-DS1 25 | uacccuacucuguuguuc gaa | 180 5 | 2563 | cgaacaacagaguaggguagc | 239 6 |
| Kylo-27-DS1 26 | cccuacucuguuguucga aac | 180 6 | 2565 | uucgaacaacagaguagggu a | 239 7 |
| Kylo-27-DS1 27 | ccuacucuguuguucgaa acg | 180 7 | 2566 | uuucgaacaacagaguaggg u | 239 8 |
| Kylo-27-DS1 28 | cuacucuguuguucgaaa cga | 180 8 | 2567 | guuucgaacaacagaguaggg | 239 9 |
| Kylo-27-DS1 29 | uacucuguuguucgaaac gag | 180 9 | 2568 | cguuucgaacaacagaguagg | 240 0 |
| Kylo-27-DS1 30 | acucuguuguucgaaacg agc | 181 0 | 2569 | ucguuucgaacaacagagua g | 240 1 |
| Kylo-27-DS1 31 | gaaacgagcagguggaaau cc | 181 1 | 2581 | auuuccaccugcucguuucga | 240 2 |
| Kylo-27-DS1 32 | aaacgagcagguggaaauc cg | 181 2 | 2582 | gauuuccaccugcucguuucg | 240 3 |
| Kylo-27-DS1 33 | cgagccguucucuacaauu ac | 181 3 | 2601 | aauuguagagaacggcucgga | 240 4 |
| Kylo-27-DS1 34 | gagccguucucuacaauu acc | 181 4 | 2602 | uaauuguagagaacggcucg g | 240 5 |
| Kylo-27-DS1 35 | agccguucucuacaauuac cg | 181 5 | 2603 | guaauuguagagaacggcuc g | 240 6 |
| Kylo-27-DS1 36 | gccguucucuacaauuacc gg | 181 6 | 2604 | gguaaauuguagagaacggcu c | 240 7 |
| Kylo-27-DS1 37 | ucuacaauuaccggcagaa cc | 181 7 | 2611 | uucugccgguaauuguagag a | 240 8 |
| Kylo-27-DS1 38 | cuacaauuaccggcagaac ca | 181 8 | 2612 | guucugccgguaauuguaga g | 240 9 |
| Kylo-27-DS1 39 | ggugggaacuacuccacaau cc | 181 9 | 2648 | auuguggaguaguuccaccc u | 241 0 |
| Kylo-27-DS1 40 | guggaacuacuccacaauc ca | 182 0 | 2649 | gauuguggaguaguuccaccc | 241 1 |
| Kylo-27-DS1 41 | uggaacuacuccacaaucc ag | 182 1 | 2650 | ggauuguggaguaguuccac c | 241 2 |
| Kylo-27-DS1 42 | ggaacuacuccacaaucca gc | 182 2 | 2651 | uggauuguggaguaguucca c | 241 3 |
| Kylo-27-DS1 43 | cucguuguccguuccaua ugu | 182 3 | 2735 | auauggaacggacaacgagga | 241 4 |
| Kylo-27-DS1 44 | ucguuguccguuccauau guc | 182 4 | 2736 | cauauggaacggacaacgagg | 241 5 |
| Kylo-27-DS1 45 | cguuguccguuccauaug uca | 182 5 | 2737 | acauauggaacggacaacgag | 241 6 |
| Kylo-27-DS1 46 | ugucgguuccauauguca ucg | 182 6 | 2740 | augacauauggaacggacaac | 241 7 |
| Kylo-27-DS1 47 | guccguuccauaugucau cgu | 182 7 | 2741 | gaugacauauggaacggacaa | 241 8 |
| Kylo-27-DS1 48 | uccguuccauaugucauc gug | 182 8 | 2742 | cgaugacauauggaacggaca | 241 9 |
| Kylo-27-DS1 49 | ccguuccauaugucaucg ugc | 182 9 | 2743 | acgaugacauauggaacggac | 242 0 |
| Kylo-27-DS1 50 | augucaucgugccgcuaaa ga | 183 0 | 2752 | uuuagcggcacgaugacaua u | 242 1 |
| Kylo-27-DS1 51 | ugcaggaaguggaaguca agg | 183 1 | 2779 | uugacuuccacuuccugcagg | 242 2 |
| Kylo-27-DS1 52 | ugcugucuaccaucauuu cau | 183 2 | 2801 | gaaaugaugguagacagcagc | 242 3 |
| Kylo-27-DS1 53 | ugucuaccaucauuucau cag | 183 3 | 2804 | gaugaaaugaugguagacag c | 242 4 |
| Kylo-27-DS1 54 | gucuaccaucauuucauc agu | 183 4 | 2805 | ugaugaaaugaugguagaca g | 242 5 |
| Kylo-27-DS1 55 | uaccaucauuucaucagu gac | 183 5 | 2808 | cacugaugaaaugaugguag a | 242 6 |
| Kylo-27-DS1 56 | accaucauuucaucagug acg | 183 6 | 2809 | ucacugaugaaaugauggua g | 242 7 |
| Kylo-27-DS1 57 | ccggaaggaaucagaauga ac | 183 7 | 2856 | ucauucugauuccuuccggca | 242 8 |
| Kylo-27-DS1 58 | cggaaggaaucagaaugaa ca | 183 8 | 2857 | uucauucugauuccuuccgg c | 242 9 |
| Kylo-27-DS1 59 | aggaaucagaaugaacaaa ac | 183 9 | 2861 | uuuguucauucugauuccuu c | 243 0 |
| Kylo-27-DS1 60 | ggaaucagaaugaacaaaa cu | 184 0 | 2862 | uuuuguucauucugauuccu u | 243 1 |
| Kylo-27-DS1 61 | ucagaaugaacaaaacug ugg | 184 1 | 2866 | acaguuuuguucauucugau u | 243 2 |
| Kylo-27-DS1 62 | gaacaaaacuguggcugu ucg | 184 2 | 2873 | aacagccacaguuuuguuca u | 243 3 |
| Kylo-27-DS1 63 | augaacaaaacuguggcu guu | 184 3 | 2892 | aacagccacaguuuuguuca u | 243 4 |
| Kylo-27-DS1 64 | augaacaaaacuguggcu guu | 184 4 | 2892 | aacagccacaguuuuguuca uuc | 243 5 |
| Kylo-27-DS1 65 | gaccagaauucuccugcaa gg | 184 5 | 2990 | uugcaggagaauucuggucu c | 243 6 |
| Kylo-27-DS1 66 | ugcauuaccuggaugaaa cgg | 184 6 | 3133 | guuucauccagguaaugcac a | 243 7 |
| Kylo-27-DS1 67 | gcauuaccuggaugaaacg ga | 184 7 | 3134 | cguuucauccagguaaugcac | 243 8 |
| Kylo-27-DS1 68 | ccuuggagcucaucaagaa gg | 184 8 | 3193 | uucuugaugagcuccaaggcc | 2439 |
| Kylo-27-DS1 69 | cuuggagcucaucaagaag gg | 184 9 | 3194 | cuucuugaugagcuccaaggc | 244 0 |
| Kylo-27-DS1 70 | uggucaaggucuucucuc ugg | 185 0 | 3304 | agagagaagaccuugaccacg | 244 1 |
| Kylo-27-DS1 71 | cguggucaaggucuucuc ucu | 185 1 | 3313 | agagagaagaccuugaccacg | 244 2 |
| Kylo-27-DS1 72 | cguggucaaggucuucuc ucu | 185 2 | 3313 | agagagaagaccuugaccacg ua | 244 3 |
| Kylo-27-DS1 73 | cgugauacaccaagaaaug au | 185 3 | 3428 | cauuucuugguguaucacgg g | 244 4 |
| Kylo-27-DS1 74 | gugauacaccaagaaauga uu | 185 4 | 3429 | ucauuucuugguguaucacg g | 244 5 |
| Kylo-27-DS1 75 | ugauacaccaagaaauga uug | 185 5 | 3430 | aucauuucuugguguaucac g | 244 6 |
| Kylo-27-DS1 76 | gauacaccaagaaaugau ugg | 185 6 | 3431 | aaucauuucuugguguauca c | 244 7 |
| Kylo-27-DS1 77 | uuggugguauuacggaaca aca | 185 7 | 3448 | uuguuccguaauccaccaauc | 244 8 |
| Kylo-27-DS1 78 | uggugggauuacggaacaa caa | 185 8 | 3449 | guuguuccguaauccaccaa u | 244 9 |
| Kylo-27-DS1 79 | ggugggauuacggaacaaca ac | 185 9 | 3450 | uguuguuccguaauccacca a | 245 0 |
| Kylo-27-DS1 80 | guggauuacggaacaacaa cg | 186 0 | 3451 | uuguuguuccguaauccacc a | 245 1 |
| Kylo-27-DS1 81 | uggauuacggaacaacaac ga | 186 1 | 3452 | guuguuguuccguaauccac c | 245 2 |
| Kylo-27-DS1 82 | ggauuacggaacaacaacg ag | 186 2 | 3453 | cguuguuguuccguaaucca c | 245 3 |
| Kylo-27-DS1 83 | gauuacggaacaacaacga ga | 186 3 | 3454 | ucguuguuguuccguaaucc a | 245 4 |
| Kylo-27-DS1 84 | uacggaacaacaacgagaa ag | 186 4 | 3457 | uucucguuguuguuccguaa u | 245 5 |
| Kylo-27-DS1 85 | acggaacaacaacgagaaa ga | 186 5 | 3458 | uuucucguuguuguuccgua a | 245 6 |
| Kylo-27-DS1 86 | ucacggccuuuguucuca ucu | 186 6 | 3487 | augagaacaaaggccgugagg | 245 7 |
| Kylo-27-DS1 87 | acggccuuuguucucauc ucg | 186 7 | 3489 | agaugagaacaaaggccguga | 245 8 |
| Kylo-27-DS1 88 | gaggcuaaagauauuugc gag | 186 8 | 3516 | cgcaaauaucuuuagccuccu | 245 9 |
| Kylo-27-DS1 89 | aggcuaaagauauuugcg agg | 186 9 | 3517 | ucgcaaauaucuuuagccucc | 246 0 |
| Kylo-27-DS1 90 | ggcuaaagauauuugcga gga | 187 0 | 3518 | cucgcaaauaucuuuagccuc | 246 1 |
| Kylo-27-DS1 91 | uuccuugaagccaacuaca ug | 187 1 | 3582 | uguaguuggcuucaaggaag u | 246 2 |
| Kylo-27-DS1 92 | uccuugaagccaacuacau ga | 187 2 | 3583 | auguaguuggcuucaaggaa g | 246 3 |
| Kylo-27-DS1 93 | ccuugaagccaacuacaug aa | 187 3 | 3584 | cauguaguuggcuucaagga a | 246 4 |
| Kylo-27-DS1 94 | cuugaagccaacuacauga ac | 187 4 | 3585 | ucauguaguuggcuucaagg a | 246 5 |
| Kylo-27-DS1 95 | uugaagccaacuacaugaa cc | 187 5 | 3586 | uucauguaguuggcuucaag g | 246 6 |
| Kylo-27-DS1 96 | ugaagccaacuacaugaac cu | 187 6 | 3587 | guucauguaguuggcuucaa g | 246 7 |
| Kylo-27-DS1 97 | gaagccaacuacaugaacc ua | 187 7 | 3588 | gguucauguaguuggcuuca a | 246 8 |
| Kylo-27-DS1 98 | aagccaacuacaugaaccu ac | 187 8 | 3589 | agguucauguaguuggcuuc a | 246 9 |
| Kylo-27-DS1 99 | ccaacuacaugaaccuaca ga | 187 9 | 3592 | uguagguucauguaguuggc u | 247 0 |
| Kylo-27-DS2 00 | gaaccuacagagauccuac ac | 188 0 | 3602 | guaggaucucuguagguuca u | 247 1 |
| Kylo-27-DS2 01 | aaccuacagagauccuaca cu | 188 1 | 3603 | uguaggaucucuguagguuc a | 247 2 |
| Kylo-27-DS2 02 | accuacagagauccuacac ug | 188 2 | 3604 | guguaggaucucuguagguu c | 247 3 |
| Kylo-27-DS2 03 | cucuucuuaacaaauuuc uga | 188 3 | 3673 | agaaauuuguuaagaagagg c | 247 4 |
| Kylo-27-DS2 04 | cuucuuaacaaauuucug acc | 188 4 | 3675 | ucagaaauuuguuaagaaga g | 247 5 |
| Kylo-27-DS2 05 | ugaccacagccaaagauaa ga | 188 5 | 3691 | uuaucuuuggcuguggucag a | 247 6 |
| Kylo-27-DS2 06 | gaccacagccaaagauaag aa | 188 6 | 3692 | cuuaucuuuggcuguggguca g | 247 7 |
| Kylo-27-DS2 07 | accacagccaaagauaaga ac | 188 7 | 3693 | ucuuaucuuuggcuguggguc a | 247 8 |
| Kylo-27-DS2 08 | ccacagccaaagauaagaa cc | 188 8 | 3694 | uucuuaucuuuggcuguggu c | 247 9 |
| Kylo-27-DS2 09 | cagccaaagauaagaaccg cu | 188 9 | 3697 | cgguucuuaucuuuggcugu g | 248 0 |
| Kylo-27-DS2 10 | acugcagcuaaaagacuu uga | 189 0 | 3779 | aaagucuuuuagcugcagua g | 248 1 |
| Kylo-27-DS2 11 | cagcuaaaagacuuugac uuu | 189 1 | 3783 | agucaaagucuuuuagcugc a | 248 2 |
| Kylo-27-DS2 12 | agcuaaaagacuuugacu uug | 189 2 | 3784 | aagucaaagucuuuuagcug c | 248 3 |
| Kylo-27-DS2 13 | gcuaaaagacuuugacuu ugu | 189 3 | 3785 | aaagucaaagucuuuuagcu g | 248 4 |
| Kylo-27-DS2 14 | uaaaagacuuugacuuug ugc | 189 4 | 3787 | acaaagucaaagucuuuuag c | 248 5 |
| Kylo-27-DS2 15 | gcagcuaaaagacuuugac uu | 189 5 | 3803 | aagucaaagucuuuuagcug c | 248 6 |
| Kylo-27-DS2 16 | gcagcuaaaagacuuugac uu | 189 6 | 3803 | aagucaaagucuuuuagcug cag | 248 7 |
| Kylo-27-DS2 17 | ugcguuggcucaaugaac aga | 189 7 | 3817 | uguucauugagccaacgcacg | 248 8 |
| Kylo-27-DS2 18 | guuggcucaaugaacaga gau | 189 8 | 3820 | cucuguucauugagccaacgc | 248 9 |
| Kylo-27-DS2 19 | uuggcucaaugaacagag aua | 189 9 | 3821 | ucucuguucauugagccaacg | 249 0 |
| Kylo-27-DS2 20 | uggcucaaugaacagaga uac | 190 0 | 3822 | aucucuguucauugagccaa c | 249 1 |
| Kylo-27-DS2 21 | ggcucaaugaacagagaua cu | 190 1 | 3823 | uaucucuguucauugagcca a | 249 2 |
| Kylo-27-DS2 22 | gcucaaugaacagagauac ua | 190 2 | 3824 | guaucucuguucauugagcc a | 249 3 |
| Kylo-27-DS2 23 | cucaaugaacagagauacu ac | 190 3 | 3825 | aguaucucuguucauugagc c | 249 4 |
| Kylo-27-DS2 24 | uuccaagccuuggcucaau ac | 190 4 | 3885 | auugagccaaggcuuggaaca | 249 5 |
| Kylo-27-DS2 25 | uccaagccuuggcucaaua cc | 190 5 | 3886 | uauugagccaaggcuuggaac | 249 6 |
| Kylo-27-DS2 26 | agccuuggcucaauaccaa aa | 190 6 | 3890 | uugguauugagccaaggcuu g | 249 7 |
| Kylo-27-DS2 27 | cuuggcucaauaccaaaag ga | 190 7 | 3893 | cuuuugguauugagccaagg c | 249 8 |
| Kylo-27-DS2 28 | uuggcucaauaccaaaagg ac | 190 8 | 3894 | ccuuuugguauugagccaag g | 249 9 |
| Kylo-27-DS2 29 | uggcucaauaccaaaagga cg | 190 9 | 3895 | uccuuuugguauugagccaa g | 250 0 |
| Kylo-27-DS2 30 | ggcucaauaccaaaaggac gc | 191 0 | 3896 | guccuuuugguauugagcca a | 250 1 |
| Kylo-27-DS2 31 | gcucaauaccaaaaggacg cc | 191 1 | 3897 | cguccuuuugguauugagcc a | 250 2 |
| Kylo-27-DS2 32 | ugcgaucagaagagaccaa gg | 191 2 | 4015 | uuggucucuucugaucgcag g | 250 3 |
| Kylo-27-DS2 33 | uggugacaauguaccaug cua | 191 3 | 4090 | gcaugguacauugucaccacc | 250 4 |
| Kylo-27-DS2 34 | ggugacaauguaccaugc uaa | 191 4 | 4091 | agcaugguacauugucaccac | 250 5 |
| Kylo-27-DS2 35 | gugacaauguaccaugcu aag | 191 5 | 4092 | uagcaugguacauugucacc a | 250 6 |
| Kylo-27-DS2 36 | ugacaauguaccaugcua agg | 191 6 | 4093 | uuagcaugguacauugucac c | 250 7 |
| Kylo-27-DS2 37 | caugcuaaggccaaagauc aa | 191 7 | 4104 | gaucuuuggccuuagcaugg u | 250 8 |
| Kylo-27-DS2 38 | aaggccaaagaucaacuca cc | 191 8 | 4110 | ugaguugaucuuuggccuua g | 250 9 |
| Kylo-27-DS2 39 | gaucaacucaccuguaaua aa | 191 9 | 4119 | uauuacaggugaguugaucu u | 251 0 |
| Kylo-27-DS2 40 | caacucaccuguaauaaau uc | 192 0 | 4122 | auuuauuacaggugaguuga u | 251 1 |
| Kylo-27-DS2 41 | aacucaccuguaauaaau ucg | 192 1 | 4123 | aauuuauuacaggugaguug a | 251 2 |
| Kylo-27-DS2 42 | ucaccuguaauaaauucg acc | 192 2 | 4126 | ucgaauuuauuacaggugag u | 251 3 |
| Kylo-27-DS2 43 | caccuguaauaaauucgac cu | 192 3 | 4127 | gucgaauuuauuacagguga g | 251 4 |
| Kylo-27-DS2 44 | ccuguaauaaauucgacc uca | 192 4 | 4129 | aggucgaauuuauuacaggu g | 251 5 |
| Kylo-27-DS2 45 | accucaaggucaccauaaa ac | 192 5 | 4144 | uuuauggugaccuugagguc g | 251 6 |
| Kylo-27-DS2 46 | ccucaaggucaccauaaaa cc | 192 6 | 4145 | uuuuauggugaccuugaggu c | 251 7 |
| Kylo-27-DS2 47 | cucaaggucaccauaaaac ca | 192 7 | 4146 | guuuuauggugaccuugagg u | 251 8 |
| Kylo-27-DS2 48 | caaggucaccauaaaacca gc | 192 8 | 4148 | ugguuuuauggugaccuuga g | 251 9 |
| Kylo-27-DS2 49 | ggucaccauaaaaccagca cc | 192 9 | 4151 | ugcugguuuuauggugaccu u | 252 0 |
| Kylo-27-DS2 50 | agcaccggaaacagaaaag ag | 193 0 | 4166 | cuuuucuguuuccggugcug g | 252 1 |
| Kylo-27-DS2 51 | gcaccggaaacagaaaaga gg | 193 1 | 4167 | ucuuuucuguuuccggugcu g | 252 2 |
| Kylo-27-DS2 52 | caccggaaacagaaaagag gc | 193 2 | 4168 | cucuuuucuguuuccggugc u | 252 3 |
| Kylo-27-DS2 53 | accggaaacagaaaagagg cc | 193 3 | 4169 | ccucuuuucuguuuccggug c | 252 4 |
| Kylo-27-DS2 54 | aggaugccaagaacacuau ga | 193 4 | 4192 | auaguguucuuggcauccug a | 252 5 |
| Kylo-27-DS2 55 | ggaugccaagaacacuaug au | 193 5 | 4193 | cauaguguucuuggcauccu g | 252 6 |
| Kylo-27-DS2 56 | gaugccaagaacacuauga uc | 193 6 | 4194 | ucauaguguucuuggcaucc u | 252 7 |
| Kylo-27-DS2 57 | augccaagaacacuaugau cc | 193 7 | 4195 | aucauaguguucuuggcauc c | 252 8 |
| Kylo-27-DS2 58 | ugccaagaacacuaugauc cu | 193 8 | 4196 | gaucauaguguucuuggcau c | 252 9 |
| Kylo-27-DS2 59 | acacuaugauccuugaga ucu | 193 9 | 4204 | aucucaaggaucauaguguu c | 253 0 |
| Kylo-27-DS2 60 | gauccuugagaucuguac cag | 194 0 | 4211 | gguacagaucucaaggauca u | 253 1 |
| Kylo-27-DS2 61 | ggaugccacuaugucuau auu | 194 1 | 4247 | uauagacauaguggcauccu g | 253 2 |
| Kylo-27-DS2 62 | gaugccacuaugucuaua uug | 194 2 | 4248 | auauagacauaguggcaucc u | 253 3 |
| Kylo-27-DS2 63 | augccacuaugucuauau ugg | 194 3 | 4249 | aauauagacauaguggcauc c | 253 4 |
| Kylo-27-DS2 64 | ugccacuaugucuauauu gga | 194 4 | 4250 | caauauagacauaguggcau c | 253 5 |
| Kylo-27-DS2 65 | ccacuaugucuauauugg aca | 194 5 | 4252 | uccaauauagacauaguggc a | 253 6 |
| Kylo-27-DS2 66 | ugucuauauuggacauau cca | 194 6 | 4258 | gauauguccaauauagacau a | 253 7 |
| Kylo-27-DS2 67 | gucuauauuggacauauc cau | 194 7 | 4259 | ggauauguccaauauagaca u | 253 8 |
| Kylo-27-DS2 68 | cuauauuggacauaucca uga | 194 8 | 4261 | auggauauguccaauauaga c | 253 9 |
| Kylo-27-DS2 69 | ggacauauccaugaugac ugg | 194 9 | 4268 | agucaucauggauaugucca a | 254 0 |
| Kylo-27-DS2 70 | caugaugacuggcuuugc ucc | 195 0 | 4277 | agcaaagccagucaucaugga | 254 1 |
| Kylo-27-DS2 71 | aauggguguugacagauac auc | 195 1 | 4326 | uguaucugucaacaccauug g | 254 2 |
| Kylo-27-DS2 72 | ggugguugacagauacauc u | 195 2 | 4329 | agauguaucugucaacacca u | 254 3 |
| Kylo-27-DS2 73 | ugacagauacaucuccaag ua | 195 3 | 4334 | cuuggagauguaucugucaa c | 254 4 |
| Kylo-27-DS2 74 | gacagauacaucuccaagu au | 195 4 | 4335 | acuuggagauguaucuguca a | 254 5 |
| Kylo-27-DS2 75 | gauacaucuccaaguaug agc | 195 5 | 4339 | ucauacuuggagauguaucu g | 254 6 |
| Kylo-27-DS2 76 | gacaaagccuucuccgaua gg | 195 6 | 4362 | uaucggagaaggcuuugucc a | 254 7 |
| Kylo-27-DS2 77 | aagccuucuccgauaggaa ca | 195 7 | 4366 | uuccuaucggagaaggcuuu g | 254 8 |
| Kylo-27-DS2 78 | gauaggaacacccucauca uc | 195 8 | 4377 | ugaugaggguguuccuaucg g | 254 9 |
| Kylo-27-DS2 79 | auaggaacacccucaucau cu | 195 9 | 4378 | augaugaggguguuccuauc g | 255 0 |
| Kylo-27-DS2 80 | uaggaacacccucaucauc ua | 196 0 | 4379 | gaugaugaggguguuccuau c | 255 1 |
| Kylo-27-DS2 81 | aggaacacccucaucaucu ac | 196 1 | 4380 | agaugaugaggguguuccua u | 255 2 |
| Kylo-27-DS2 82 | acacccucaucaucuaccu gg | 196 2 | 4384 | agguagaugaugaggguguu c | 255 3 |
| Kylo-27-DS2 83 | aggaugacugucuagcuu uca | 196 3 | 4423 | aaagcuagacagucauccuca | 255 4 |
| Kylo-27-DS2 84 | ggaugacugucuagcuuu caa | 196 4 | 4424 | gaaagcuagacagucauccuc | 255 5 |
| Kylo-27-DS2 85 | cugucuagcuuucaaagu uca | 196 5 | 4430 | aacuuugaaagcuagacagu c | 255 6 |
| Kylo-27-DS2 86 | ucuagcuuucaaaguuca cca | 196 6 | 4433 | gugaacuuugaaagcuagac a | 255 7 |
| Kylo-27-DS2 87 | agcuuucaaaguucaccaa ua | 196 7 | 4436 | uuggugaacuuugaaagcua g | 255 8 |
| Kylo-27-DS2 88 | gcuuucaaaguucaccaa uac | 196 8 | 4437 | auuggugaacuuugaaagcu a | 255 9 |
| Kylo-27-DS2 89 | caaaguucaccaauacuu uaa | 196 9 | 4442 | aaaguauuggugaacuuuga a | 256 0 |
| Kylo-27-DS2 90 | aaaguucaccaauacuuu aau | 197 0 | 4443 | uaaaguauuggugaacuuug a | 256 1 |
| Kylo-27-DS2 91 | aaguucaccaauacuuua aug | 197 1 | 4444 | uuaaaguauuggugaacuuu g | 256 2 |
| Kylo-27-DS2 92 | aguucaccaauacuuuaa ugu | 197 2 | 4445 | auuaaaguauuggugaacuu u | 256 3 |
| Kylo-27-DS2 93 | guucaccaauacuuuaau gua | 197 3 | 4446 | cauuaaaguauuggugaacu u | 256 4 |
| Kylo-27-DS2 94 | uucaccaauacuuuaaug uag | 197 4 | 4447 | acauuaaaguauuggugaac u | 256 5 |
| Kylo-27-DS2 95 | ucaccaauacuuuaaugu aga | 197 5 | 4448 | uacauuaaaguauuggugaa c | 256 6 |
| Kylo-27-DS2 96 | caccaauacuuuaaugua gag | 197 6 | 4449 | cuacauuaaaguauugguga a | 256 7 |
| Kylo-27-DS2 97 | accaauacuuuaauguag agc | 197 7 | 4450 | ucuacauuaaaguauuggug a | 256 8 |
| Kylo-27-DS2 98 | ccaauacuuuaauguaga gcu | 197 8 | 4451 | cucuacauuaaaguauuggu g | 256 9 |
| Kylo-27-DS2 99 | agucaaggucuacgccuau ua | 197 9 | 4487 | auaggcguagaccuugacugc | 257 0 |
| Kylo-27-DS3 00 | gucaaggucuacgccuau uac | 198 0 | 4488 | aauaggcguagaccuugacug | 257 1 |
| Kylo-27-DS3 01 | ucaaggucuacgccuauu aca | 198 1 | 4489 | uaauaggcguagaccuugac u | 257 2 |
| Kylo-27-DS3 02 | caaggucuacgccuauuac aa | 198 2 | 4490 | guaauaggcguagaccuugac | 257 3 |
| Kylo-27-DS3 03 | aaggucuacgccuauuaca ac | 198 3 | 4491 | uguaauaggcguagaccuug a | 257 4 |
| Kylo-27-DS3 04 | aggucuacgccuauuacaa cc | 198 4 | 4492 | uuguaauaggcguagaccuu g | 257 5 |
| Kylo-27-DS3 05 | ggucuacgccuauuacaac cu | 198 5 | 4493 | guuguaauaggcguagaccu u | 257 6 |
| Kylo-27-DS3 06 | acgccuauuacaaccugga gg | 198 6 | 4498 | uccagguuguaauaggcgua g | 257 7 |
| Kylo-27-DS3 07 | aaagcuguacccgguucua cc | 198 7 | 4519 | uagaaccggguacagcuuucc | 257 8 |
| Kylo-27-DS3 08 | gguucuaccauccggaaaa gg | 198 8 | 4531 | uuuuccggaugguagaaccg g | 257 9 |
| Kylo-27-DS3 09 | guucuaccauccggaaaag ga | 198 9 | 4532 | cuuuuccggaugguagaaccg | 258 0 |
| Kylo-27-DS3 10 | aggauggaaagcugaacaa gc | 199 0 | 4552 | uuguucagcuuuccauccucc | 258 1 |
| Kylo-27-DS3 11 | ugugcugaggagaauugc uuc | 199 1 | 4596 | agcaauucuccucagcacagc | 258 2 |
| Kylo-27-DS3 12 | gugcugaggagaauugcu uca | 199 2 | 4597 | aagcaauucuccucagcacag | 258 3 |
| Kylo-27-DS3 13 | gaggagaauugcuucaua caa | 199 3 | 4602 | guaugaagcaauucuccuca g | 258 4 |
| Kylo-27-DS3 14 | ggagaauugcuucauaca aaa | 199 4 | 4604 | uuguaugaagcaauucuccu c | 258 5 |
| Kylo-27-DS3 15 | gagaauugcuucauacaa aag | 199 5 | 4605 | uuuguaugaagcaauucucc u | 258 6 |
| Kylo-27-DS3 16 | gcugaggagaauugcuuc aua | 199 6 | 4620 | uaugaagcaauucuccucagc | 258 7 |
| Kylo-27-DS3 17 | gcugaggagaauugcuuc aua | 199 7 | 4620 | uaugaagcaauucucccucagc ac | 258 8 |
| Kylo-27-DS3 18 | ggagaauugcuucauaca aaa | 199 8 | 4625 | uuuuguaugaagcaauucuc c | 258 9 |
| Kylo-27-DS3 19 | ggagaauugcuucauaca aaa | 199 9 | 4625 | uuuuguaugaagcaauucuc cuc | 259 0 |
| Kylo-27-DS3 20 | aggaguggacuaugugua caa | 200 0 | 4676 | guacacauaguccacuccugg | 259 1 |
| Kylo-27-DS3 21 | ggaguggacuauguguac aag | 200 1 | 4677 | uguacacauaguccacuccug | 259 2 |
| Kylo-27-DS3 22 | gaguggacuauguguaca aga | 200 2 | 4678 | uuguacacauaguccacuccu | 259 3 |
| Kylo-27-DS3 23 | agugggacuauguguacaa gac | 200 3 | 4679 | cuuguacacauaguccacucc | 259 4 |
| Kylo-27-DS3 24 | guggacuauguguacaag acc | 200 4 | 4680 | ucuuguacacauaguccacuc | 259 5 |
| Kylo-27-DS3 25 | uggacuauguguacaaga ccc | 200 5 | 4681 | gucuuguacacauaguccac u | 259 6 |
| Kylo-27-DS3 26 | ggacuauguguacaagacc cg | 200 6 | 4682 | ggucuuguacacauaguccac | 259 7 |
| Kylo-27-DS3 27 | agguucagcuguccaaug acu | 200 7 | 4711 | ucauuggacagcugaaccuug | 259 8 |
| Kylo-27-DS3 28 | uucagcuguccaaugacu uug | 200 8 | 4714 | aagucauuggacagcugaacc | 259 9 |
| Kylo-27-DS3 29 | guccaaugacuuugacga gua | 200 9 | 4721 | cucgucaaagucauuggacag | 260 0 |
| Kylo-27-DS3 30 | gacuuugacgaguacauc aug | 201 0 | 4728 | ugauguacucgucaaaguca u | 260 1 |
| Kylo-27-DS3 31 | guacaucauggccauuga gca | 201 1 | 4739 | cucaauggccaugauguacuc | 260 2 |
| Kylo-27-DS3 32 | ugagcagaccaucaaguca gg | 201 2 | 4754 | ugacuugauggucugcucaa u | 260 3 |
| Kylo-27-DS3 33 | cuggaggagaagaaacacu ac | 201 3 | 4845 | aguguuucuucuccuccagc u | 260 4 |
| Kylo-27-DS3 34 | uggaggagaagaaacacua cc | 201 4 | 4846 | uaguguuucuucuccuccag c | 260 5 |
| Kylo-27-DS3 35 | ggaggagaagaaacacuac cu | 201 5 | 4847 | guaguguuucuucuccucca g | 260 6 |
| Kylo-27-DS3 36 | aggagaagaaacacuaccu ca | 201 6 | 4849 | agguaguguuucuucuccuc c | 260 7 |
| Kylo-27-DS3 37 | ucagcuacaucaucgggaa gg | 201 7 | 4912 | uucccgaugauguagcugag g | 260 8 |
| Kylo-27-DS3 38 | augccaagacgaagagaac ca | 201 8 | 4967 | guucucuucgucuuggcauu c | 260 9 |
| Kylo-27-DS3 39 | gaagagaaccagaaacaau gc | 201 9 | 4977 | auuguuucugguucucuucg u | 261 0 |
| Kylo-27-DS3 40 | cgagagcaugguugucuu ugg | 202 0 | 5018 | aaagacaaccaugcucucggu | 261 1 |
| Kylo-27-DS3 41 | ccagauaaagcuucaguu aua | 202 1 | 5073 | uaacugaagcuuuaucugga g | 261 2 |
| Kylo-27-DS3 42 | ucaguuauaucucacgug ucu | 202 2 | 5085 | acacgugagauauaacugaa g | 261 3 |
| Kylo-27-DS3 43 | aucucacgugucuggagu ucu | 202 3 | 5093 | aacuccagacacgugagauau | 261 4 |
| Kylo-27-DS3 44 | ucacgugucuggaguucu uug | 202 4 | 5096 | aagaacuccagacacgugaga | 261 5 |
| Kylo-27-DS3 45 | cacgugucuggaguucuu ugc | 202 5 | 5097 | aaagaacuccagacacgugag | 261 6 |
| Kylo-27-DS3 46 | acgugucuggaguucuuu gcc | 202 6 | 5098 | caaagaacuccagacacguga | 261 7 |
| Kylo-27-DS3 47 | gucuggaguucuuugcca aga | 202 7 | 5102 | uuggcaaagaacuccagacac | 261 8 |
| Kylo-27-DS3 48 | cagcuccauccuacuugaa ac | 202 8 | 5161 | uucaaguaggauggagcuga g | 261 9 |
| Kylo-27-DS3 49 | cuccauccuacuugaaacc uc | 202 9 | 5164 | gguuucaaguaggauggagc u | 262 0 |
| Kylo-27-DS3 50 | cauccuacuugaaaccuca cc | 203 0 | 5167 | ugagguuucaaguaggaugg a | 262 1 |
| Kylo-27-DS3 51 | gagccguucucuacaauu acu | 203 1 | 136 | aguaauuguagagaacggcu cgg | 262 2 |
| Kylo-27-DS3 52 | ccagacagacaagaccauc ua | 203 2 | 459 | uagauggucuugucugucug gau | 262 3 |
| Kylo-27-DS3 53 | agacagacaagaccaucua ca | 203 3 | 461 | uguagauggucuugucuguc ugg | 262 4 |
| Kylo-27-DS3 54 | ggacuccuugucuucuca gaa | 203 4 | 594 | uucugagaagacaaggagucc ug | 262 5 |
| Kylo-27-DS3 55 | agauccgagccuacuauga aa | 203 5 | 677 | uuucauaguaggcucggauc uuc | 262 6 |
| Kylo-27-DS3 56 | gauccgagccuacuaugaa aa | 203 6 | 678 | uuuucauaguaggcucggau cuu | 262 7 |
| Kylo-27-DS3 57 | ugaguuugaggugaagga gua | 203 7 | 723 | uacuccuucaccucaaacuca gu | 262 8 |
| Kylo-27-DS3 58 | uggagccuacagagaaau ucu | 203 8 | 770 | agaauuucucuguaggcucc acu | 262 9 |
| Kylo-27-DS3 59 | ggagccuacagagaaauuc ua | 203 9 | 771 | uagaauuucucuguaggcuc cac | 263 0 |
| Kylo-27-DS3 60 | gagccuacagagaaauucu ac | 204 0 | 772 | guagaauuucucuguaggcu cca | 263 1 |
| Kylo-27-DS3 61 | agccuacagagaaauucua cu | 204 1 | 773 | aguagaauuucucuguaggc ucc | 263 2 |
| Kylo-27-DS3 62 | gccuacagagaaauucuac ua | 204 2 | 774 | uaguagaauuucucuguagg cuc | 263 3 |
| Kylo-27-DS3 63 | ccuacagagaaauucuacu ac | 204 3 | 775 | guaguagaauuucucuguag gcu | 263 4 |
| Kylo-27-DS3 64 | cuacagagaaauucuacu aca | 204 4 | 776 | uguaguagaauuucucugua ggc | 263 5 |
| Kylo-27-DS3 65 | cagagaaauucuacuaca ucu | 204 5 | 779 | agauguaguagaauuucucu gua | 263 6 |
| Kylo-27-DS3 66 | agagaaauucuacuacau cua | 204 6 | 780 | uagauguaguagaauuucuc ugu | 263 7 |
| Kylo-27-DS3 67 | gagaaauucuacuacuacauc uau | 204 7 | 781 | auagauguaguagaauuucu cug | 263 8 |
| Kylo-27-DS3 68 | agaaauucuacuacaucu aua | 204 8 | 782 | uauagauguaguagaauuuc ucu | 263 9 |
| Kylo-27-DS3 69 | gaaauucuacuacaucua uaa | 204 9 | 783 | uuauagauguaguagaauuu cuc | 264 0 |
| Kylo-27-DS3 70 | aaauucuacuacaucuau aac | 205 0 | 784 | guuauagauguaguagaauu ucu | 264 1 |
| Kylo-27-DS3 71 | gggaacugccuuugucau cuu | 205 1 | 861 | aagaugacaaaggcaguuccc uc | 264 2 |
| Kylo-27-DS3 72 | caaguacuucaaaccagga au | 205 2 | 1146 | auuccugguuugaaguacuu ggg | 264 3 |
| Kylo-27-DS3 73 | aaguacuucaaaccaggaa ug | 205 3 | 1147 | cauuccugguuugaaguacu ugg | 264 4 |
| Kylo-27-DS3 74 | gccaaacucagcaucaaca ca | 205 4 | 1282 | | 264 5 |
| Kylo-27-DS3 75 | ccaaacucagcaucaacac ac | 205 5 | 1283 | guguguugaugcugaguuug gcc | 264 6 |
| Kylo-27-DS3 76 | caaacucagcaucaacaca ca | 205 6 | 1284 | ugugugguugaugcugaguuu ggc | 264 7 |
| Kylo-27-DS3 77 | cgugggcaacuccaacaau ua | 205 7 | 1407 | uaauuguuggaguugcccac ggu | 264 8 |
| Kylo-27-DS3 78 | gggcaacuccaacaauuac cu | 205 8 | 1410 | agguaauuguuggaguugcc cac | 264 9 |
| Kylo-27-DS3 79 | caacuccaacaauuaccug ca | 205 9 | 1413 | ugcagguaauuguuggaguu gcc | 265 0 |
| Kylo-27-DS3 80 | aacuccaacaauuaccugc au | 206 0 | 1414 | augcagguaauuguuggagu ugc | 265 1 |
| Kylo-27-DS3 81 | acuccaacaauuaccugca uc | 206 1 | 1415 | gaugcagguaauuguuggag uug | 265 2 |
| Kylo-27-DS3 82 | cuccaacaauuaccugcau cu | 206 2 | 1416 | agaugcagguaauuguugga guu | 265 3 |
| Kylo-27-DS3 83 | uccaacaauuaccugcauc uc | 206 3 | 1417 | gagaugcagguaauuguugg agu | 265 4 |
| Kylo-27-DS3 84 | ccaacaauuaccugcaucu cu | 206 4 | 1418 | agagaugcagguaauuguug gag | 265 5 |
| Kylo-27-DS3 85 | caacaauuaccugcaucuc uc | 206 5 | 1419 | gagagaugcagguaauuguu gga | 265 6 |
| Kylo-27-DS3 86 | caauuaccugcaucucuca gu | 206 6 | 1422 | acugagagaugcagguaauu guu | 265 7 |
| Kylo-27-DS3 87 | gcagaugacccugaagaua ga | 206 7 | 1800 | ucuaucuucagggucaucug cug | 265 8 |
| Kylo-27-DS3 88 | gaagcucaugaauauauu uuu | 206 8 | 2427 | aaaaauauauucaugagcuu cgu | 265 9 |
| Kylo-27-DS3 89 | aagcucaugaauauauuu uug | 206 9 | 2428 | caaaaauauauucaugagcu ucg | 266 0 |
| Kylo-27-DS3 90 | agcucaugaauauauuuu uga | 207 0 | 2429 | ucaaaaauauauucaugagc uuc | 266 1 |
| Kylo-27-DS3 91 | gcucaugaauauauuuuu gaa | 207 1 | 2430 | uucaaaaauauauucaugag cuu | 266 2 |
| Kylo-27-DS3 92 | cucaugaauauauuuuug aaa | 207 2 | 2431 | uuucaaaaauauauucauga gcu | 266 3 |
| Kylo-27-DS3 93 | ucaugaauauauuuuuga aag | 207 3 | 2432 | cuuucaaaaauauauucaug agc | 266 4 |
| Kylo-27-DS3 94 | augaauauauuuuugaaa gac | 207 4 | 2434 | gucuuucaaaauauauuca uga | 266 5 |
| Kylo-27-DS3 95 | ugaauauauuuuugaaag acu | 207 5 | 2435 | agucuuucaaaauauauuc aug | 266 6 |
| Kylo-27-DS3 96 | gaauauauuuuugaaaga cuc | 207 6 | 2436 | gagucuuucaaaaauauauu cau | 266 7 |
| Kylo-27-DS3 97 | aauauauuuuugaaagac ucc | 207 7 | 2437 | ggagucuuucaaaaauauau uca | 266 8 |
| Kylo-27-DS3 98 | uauauuuuugaaagacuc cau | 207 8 | 2439 | auggagucuuucaaaaauau auu | 266 9 |
| Kylo-27-DS3 99 | auauuuuugaaagacucc auc | 207 9 | 2440 | gauggagucuuucaaaaaua uau | 267 0 |
| Kylo-27-DS4 00 | uauuuuugaaagacucca uca | 208 0 | 2441 | ugauggagucuuucaaaaau aua | 267 1 |
| Kylo-27-DS4 01 | auuuuugaaagacuccau cac | 208 1 | 2442 | gugauggagucuuucaaaaa uau | 267 2 |
| Kylo-27-DS4 02 | uuuuugaaagacuccauc acc | 208 2 | 2443 | ggugauggagucuuucaaaa aua | 267 3 |
| Kylo-27-DS4 03 | uuuugaaagacuccauca cca | 208 3 | 2444 | uggugauggagucuuucaaa aau | 267 4 |
| Kylo-27-DS4 04 | ggucacaguaaugcaggac uu | 208 4 | 2529 | aaguccugcauuacugugacc uc | 267 5 |
| Kylo-27-DS4 05 | ucacaguaaugcaggacu ucu | 208 5 | 2531 | agaaguccugcauuacugug acc | 267 6 |
| Kylo-27-DS4 06 | cacaguaaugcaggacuuc uu | 208 6 | 2532 | aagaaguccugcauuacugu gac | 267 7 |
| Kylo-27-DS4 07 | acaguaaugcaggacuuc uuc | 208 7 | 2533 | gaagaaguccugcauuacug uga | 267 8 |
| Kylo-27-DS4 08 | caguaaugcaggacuucu uca | 208 8 | 2534 | ugaagaaguccugcauuacu gug | 267 9 |
| Kylo-27-DS4 09 | aguaaugcaggacuucuu cau | 208 9 | 2535 | augaagaaguccugcauuac ugu | 268 0 |
| Kylo-27-DS4 10 | guaaugcaggacuucuuc auc | 209 0 | 2536 | gaugaagaaguccugcauua cug | 268 1 |
| Kylo-27-DS4 11 | acccuacucuguuguucg aaa | 209 1 | 2568 | uuucgaacaacagaguaggg uag | 268 2 |
| Kylo-27-DS4 12 | cccuacucuguuguucga aac | 209 2 | 2569 | guuucgaacaacagaguaggg ua | 268 3 |
| Kylo-27-DS4 13 | ccuacucuguuguucgaa acg | 209 3 | 2570 | cguuucgaacaacagaguagg gu | 268 4 |
| Kylo-27-DS4 14 | cuacucuguuguucgaaa cga | 209 4 | 2571 | ucguuucgaacaacagagua ggg | 268 5 |
| Kylo-27-DS4 15 | gccggaaggaaucagaaug aa | 209 5 | 2859 | uucauucugauuccuuccgg cac | 268 6 |
| Kylo-27-DS4 16 | ccggaaggaaucagaauga ac | 209 6 | 2860 | guucauucugauuccuuccg gca | 268 7 |
| Kylo-27-DS4 17 | cggaaggaaucagaaugaa ca | 209 7 | 2861 | uguucauucugauuccuucc ggc | 268 8 |
| Kylo-27-DS4 18 | ggaaggaaucagaaugaac aa | 209 8 | 2862 | uuguucauucugauuccuuc cgg | 268 9 |
| Kylo-27-DS4 19 | gaaggaaucagaaugaaca aa | 209 9 | 2863 | uuuguucauucugauuccuu ccg | 269 0 |
| Kylo-27-DS4 20 | aaggaaucagaaugaacaa aa | 210 0 | 2864 | uuuuguucauucugauuccu ucc | 269 1 |
| Kylo-27-DS4 21 | aggaaucagaaugaacaaa ac | 210 1 | 2865 | guuuuguucauucugauucc uuc | 269 2 |
| Kylo-27-DS4 22 | ggaaucagaaugaacaaaa cu | 210 2 | 2866 | aguuuuguucauucugauuc cuu | 269 3 |
| Kylo-27-DS4 23 | gaaucagaaugaacaaaac ug | 210 3 | 2867 | cagguuuuguucauucugauu ccu | 269 4 |
| Kylo-27-DS4 24 | aaucagaaugaacaaaac ugu | 210 4 | 2868 | acaguuuuguucauucugau ucc | 269 5 |
| Kylo-27-DS4 25 | aucagaaugaacaaaacug ug | 210 5 | 2869 | cacaguuuuguucauucuga uuc | 269 6 |
| Kylo-27-DS4 26 | ucagaaugaacaaaacug ugg | 210 6 | 2870 | ccacaguuuuguucauucug auu | 269 7 |
| Kylo-27-DS4 27 | agaaugaacaaaacugug gcu | 210 7 | 2872 | agccacaguuuuguucauuc uga | 269 8 |
| Kylo-27-DS4 28 | gaaugaacaaaacuguggc ug | 210 8 | 2873 | cagccacaguuuuguucauu cug | 269 9 |
| Kylo-27-DS4 29 | aaugaacaaaacuguggc ugu | 210 9 | 2874 | acagccacaguuuuguucau ucu | 270 0 |
| Kylo-27-DS4 30 | augaacaaaacuguggcu guu | 211 0 | 2875 | aacagccacaguuuuguuca uuc | 270 1 |
| Kylo-27-DS4 31 | ugaacaaaacuguggcug uuc | 211 1 | 2876 | gaacagccacaguuuuguuc auu | 270 2 |
| Kylo-27-DS4 32 | cgagucugagaccagaauu cu | 211 2 | 2985 | agaauucuggucucagacucg gu | 270 3 |
| Kylo-27-DS4 33 | gcugugcauuaccuggau gaa | 211 3 | 3133 | uucauccagguaaugcacagc ga | 270 4 |
| Kylo-27-DS4 34 | cugugcauuaccuggaug aaa | 211 4 | 3134 | uuucauccagguaaugcaca gcg | 270 5 |
| Kylo-27-DS4 35 | ugugcauuaccuggauga aac | 211 5 | 3135 | guuucauccagguaaugcac agc | 270 6 |
| Kylo-27-DS4 36 | gcauuaccuggaugaaacg ga | 211 6 | 3138 | uccguuucauccagguaaugc ac | 270 7 |
| Kylo-27-DS4 37 | ggcuguuaaauggcugau ccu | 211 7 | 3369 | aggaucagccauuuaacagcc cc | 270 8 |
| Kylo-27-DS4 38 | gcuguuaaauggcugauc cug | 211 8 | 3370 | caggaucagccauuuaacagc cc | 270 9 |
| Kylo-27-DS4 39 | uguuaaauggcugauccu gga | 211 9 | 3372 | uccaggaucagccauuuaaca gc | 271 0 |
| Kylo-27-DS4 40 | gcaucacuaaagcaggaga cu | 212 0 | 3566 | agucuccugcuuuagugaug cug | 271 1 |
| Kylo-27-DS4 41 | caucacuaaagcaggagac uu | 212 1 | 3567 | aagucuccugcuuuagugau gcu | 271 2 |
| Kylo-27-DS4 42 | acuaaagcaggagacuucc uu | 212 2 | 3571 | aaggaagucuccugcuuuag uga | 271 3 |
| Kylo-27-DS4 43 | acuuccuugaagccaacua ca | 212 3 | 3584 | uguaguuggcuucaaggaag ucu | 271 4 |
| Kylo-27-DS4 44 | cuuccuugaagccaacuac au | 212 4 | 3585 | auguaguuggcuucaaggaa guc | 271 5 |
| Kylo-27-DS4 45 | uccuugaagccaacuacau ga | 212 5 | 3587 | ucauguaguuggcuucaagg aag | 271 6 |
| Kylo-27-DS4 46 | ccuugaagccaacuacaug aa | 212 6 | 3588 | uucauguaguuggcuucaag gaa | 271 7 |
| Kylo-27-DS4 47 | ugaagccaacuacaugaac cu | 212 7 | 3591 | agguucauguaguuggcuuc aag | 271 8 |
| Kylo-27-DS4 48 | gaagccaacuacaugaacc ua | 212 8 | 3592 | uagguucauguaguuggcuu caa | 271 9 |
| Kylo-27-DS4 49 | agccaacuacaugaaccua ca | 212 9 | 3594 | uguagguucauguaguuggc uuc | 272 0 |
| Kylo-27-DS4 50 | gccaacuacaugaaccuac ag | 213 0 | 3595 | cuguagguucauguaguugg cuu | 272 1 |
| Kylo-27-DS4 51 | ccaacuacaugaaccuaca ga | 213 1 | 3596 | ucuguagguucauguaguug gcu | 272 2 |
| Kylo-27-DS4 52 | caacuacaugaaccuacag ag | 213 2 | 3597 | cucuguagguucauguaguu ggc | 272 3 |
| Kylo-27-DS4 53 | aacuacaugaaccuacaga ga | 213 3 | 3598 | ucucuguagguucauguagu ugg | 272 4 |
| Kylo-27-DS4 54 | acuacaugaaccuacagag au | 213 4 | 3599 | aucucuguagguucauguag uug | 272 5 |
| Kylo-27-DS4 55 | cuacaugaaccuacagaga uc | 213 5 | 3600 | gaucucuguagguucaugua guu | 272 6 |
| Kylo-27-DS4 56 | caugaaccuacagagaucc ua | 213 6 | 3603 | uaggaucucuguagguucau gua | 272 7 |
| Kylo-27-DS4 57 | ugaaccuacagagauccua ca | 213 7 | 3605 | uguaggaucucuguagguuc aug | 272 8 |
| Kylo-27-DS4 58 | ccuacagagauccuacacu gu | 213 8 | 3609 | acaguguaggaucucuguag guu | 272 9 |
| Kylo-27-DS4 59 | aaauuucugaccacagcca aa | 213 9 | 3688 | uuuggcuguggucagaaauu ugu | 273 0 |
| Kylo-27-DS4 60 | ucugaccacagccaaagau aa | 214 0 | 3693 | uuaucuuuggcuguggucag aaa | 273 1 |
| Kylo-27-DS4 61 | ugaccacagccaaagauaa ga | 214 1 | 3695 | ucuuaucuuuggcugugguc aga | 273 2 |
| Kylo-27-DS4 62 | gaccacagccaaagauaag aa | 214 2 | 3696 | uucuuaucuuuggcuguggu cag | 273 3 |
| Kylo-27-DS4 63 | ccacagccaaagauaagaa cc | 214 3 | 3698 | gguucuuaucuuuggcugug guc | 273 4 |
| Kylo-27-DS4 64 | cccuacugcagcuaaaaga cu | 214 4 | 3779 | agucuuuuagcugcaguagg gcc | 273 5 |
| Kylo-27-DS4 65 | ccuacugcagcuaaaagac uu | 214 5 | 3780 | aagucuuuuagcugcaguag ggc | 273 6 |
| Kylo-27-DS4 66 | cuacugcagcuaaaagacu uu | 214 6 | 3781 | aaagucuuuuagcugcagua ggg | 273 7 |
| Kylo-27-DS4 67 | uacugcagcuaaaagacu uug | 214 7 | 3782 | caaagucuuuuagcugcagu agg | 273 8 |
| Kylo-27-DS4 68 | acugcagcuaaaagacuu uga | 214 8 | 3783 | ucaaagucuuuuagcugcag uag | 273 9 |
| Kylo-27-DS4 69 | cugcagcuaaaagacuuu gac | 214 9 | 3784 | gucaaagucuuuuagcugca gua | 274 0 |
| Kylo-27-DS4 70 | ugcagcuaaaagacuuug acu | 215 0 | 3785 | agucaaagucuuuuagcugc agu | 274 1 |
| Kylo-27-DS4 71 | gcagcuaaaagacuuugac uu | 215 1 | 3786 | aagucaaagucuuuuagcug cag | 274 2 |
| Kylo-27-DS4 72 | cagcuaaaagacuuugac uuu | 215 2 | 3787 | aaagucaaagucuuuuagcu gca | 274 3 |
| Kylo-27-DS4 73 | agcuaaaagacuuugacu uug | 215 3 | 3788 | caaagucaaagucuuuuagc ugc | 274 4 |
| Kylo-27-DS4 74 | gcuaaaagacuuugacuu ugu | 215 4 | 3789 | acaaagucaaagucuuuuag cug | 274 5 |
| Kylo-27-DS4 75 | cuaaaagacuuugacuuu gug | 215 5 | 3790 | cacaaagucaaagucuuuua gcu | 274 6 |
| Kylo-27-DS4 76 | aaaagacuuugacuuugu gcc | 215 6 | 3792 | ggcacaaagucaaagucuuu uag | 274 7 |
| Kylo-27-DS4 77 | aaagacuuugacuuugug ccu | 215 7 | 3793 | aggcacaaagucaaagucuu uua | 274 8 |
| Kylo-27-DS4 78 | cguuggcucaaugaacaga ga | 215 8 | 3823 | ucucuguucauugagccaacg ca | 274 9 |
| Kylo-27-DS4 79 | guuggcucaaugaacaga gau | 215 9 | 3824 | aucucuguucauugagccaa cgc | 275 0 |
| Kylo-27-DS4 80 | uuggcucaaugaacagag aua | 216 0 | 3825 | uaucucuguucauugagcca acg | 275 1 |
| Kylo-27-DS4 81 | uggcucaaugaacagaga uac | 216 1 | 3826 | guaucucuguucauugagcc aac | 275 2 |
| Kylo-27-DS4 82 | ggcucaaugaacagagaua cu | 216 2 | 3827 | aguaucucuguucauugagc caa | 275 3 |
| Kylo-27-DS4 83 | gcucaaugaacagagauac ua | 216 3 | 3828 | uaguaucucuguucauugag cca | 275 4 |
| Kylo-27-DS4 84 | cucaaugaacagagauacu ac | 216 4 | 3829 | guaguaucucuguucauuga gcc | 275 5 |
| Kylo-27-DS4 85 | ucaaugaacagagauacu acg | 216 5 | 3830 | cguaguaucucuguucauug agc | 275 6 |
| Kylo-27-DS4 86 | caaugaacagagauacuac gg | 216 6 | 3831 | ccguaguaucucuguucauu gag | 275 7 |
| Kylo-27-DS4 87 | aaugaacagagauacuacg gu | 216 7 | 3832 | accguaguaucucuguucau uga | 275 8 |
| Kylo-27-DS4 88 | agccuuggcucaauaccaa aa | 216 8 | 3894 | uuuugguauugagccaaggc uug | 275 9 |
| Kylo-27-DS4 89 | gccuuggcucaauaccaaa ag | 216 9 | 3895 | cuuuugguauugagccaagg cuu | 276 0 |
| Kylo-27-DS4 90 | ccuuggcucaauaccaaaa gg | 217 0 | 3896 | ccuuuugguauugagccaag gcu | 276 1 |
| Kylo-27-DS4 91 | cuuggcucaauaccaaaag ga | 217 1 | 3897 | uccuuuugguauugagccaa ggc | 276 2 |
| Kylo-27-DS4 92 | gaucagaagagaccaagga aa | 217 2 | 4022 | uuuccuuggucucuucugau cgc | 276 3 |
| Kylo-27-DS4 93 | aucagaagagaccaaggaa aa | 217 3 | 4023 | uuuuccuuggucucuucuga ucg | 276 4 |
| Kylo-27-DS4 94 | agaagagaccaaggaaaau ga | 217 4 | 4026 | ucauuuuccuuggucucuuc uga | 276 5 |
| Kylo-27-DS4 95 | agaccaaggaaaaugaggg uu | 217 5 | 4031 | aacccucauuuuccuuggguc ucu | 276 6 |
| Kylo-27-DS4 96 | gaccaaggaaaaugagggu uu | 217 6 | 4032 | aaacccucauuuuccuuggu cuc | 276 7 |
| Kylo-27-DS4 97 | accaaggaaaaugagggu uuc | 217 7 | 4033 | gaaacccucauuuuccuugg ucu | 276 8 |
| Kylo-27-DS4 98 | ccaaggaaaaugaggguu uca | 217 8 | 4034 | ugaaacccucauuuuccuug guc | 276 9 |
| Kylo-27-DS4 99 | caaggaaaaugaggguuu cac | 217 9 | 4035 | gugaaacccucauuuuccuu ggu | 277 0 |
| Kylo-27-DS5 00 | aaggaaaaugaggguuuc aca | 218 0 | 4036 | ugugaaacccucauuuuccu ugg | 277 1 |
| Kylo-27-DS5 01 | agggaaaaugaggguuuca cag | 218 1 | 4037 | cugugaaacccucauuuucc uug | 277 2 |
| Kylo-27-DS5 02 | ggaaaaugaggguuucac agu | 218 2 | 4038 | acugugaaacccucauuuucc uu | 277 3 |
| Kylo-27-DS5 03 | gaaaaugaggguuucaca guc | 218 3 | 4039 | gacugugaaacccucauuuu ccu | 277 4 |
| Kylo-27-DS5 04 | aacucaccuguaauaaau ucg | 218 4 | 4127 | cgaauuuauuacaggugagu uga | 277 5 |
| Kylo-27-DS5 05 | acucaccuguaauaaauu cga | 218 5 | 4128 | ucgaauuuauuacaggugag uug | 277 6 |
| Kylo-27-DS5 06 | cucaccuguaauaaauuc gac | 218 6 | 4129 | gucgaauuuauuacagguga guu | 277 7 |
| Kylo-27-DS5 07 | ucaccuguaauaaauucg acc | 218 7 | 4130 | ggucgaauuuauuacaggug agu | 277 8 |
| Kylo-27-DS5 08 | caccuguaauaaauucgac cu | 218 8 | 4131 | aggucgaauuuauuacaggu gag | 277 9 |
| Kylo-27-DS5 09 | accuguaauaaauucgacc uc | 218 9 | 4132 | gaggucgaauuuauuacagg uga | 278 0 |
| Kylo-27-DS5 10 | ccuguaauaaauucgacc uca | 219 0 | 4133 | ugaggucgaauuuauuacag gug | 278 1 |
| Kylo-27-DS5 11 | cuguaauaaauucgaccu caa | 219 1 | 4134 | uugaggucgaauuuauuaca ggu | 278 2 |
| Kylo-27-DS5 12 | uguaauaaauucgaccuc aag | 219 2 | 4135 | cuugaggucgaauuuauuac agg | 278 3 |
| Kylo-27-DS5 13 | guaauaaauucgaccuca agg | 219 3 | 4136 | ccuugaggucgaauuuauua cag | 278 4 |
| Kylo-27-DS5 14 | uaauaaauucgaccucaa ggu | 219 4 | 4137 | accuugaggucgaauuuauu aca | 278 5 |
| Kylo-27-DS5 15 | aauaaauucgaccucaagg uc | 219 5 | 4138 | gaccuugaggucgaauuuau uac | 278 6 |
| Kylo-27-DS5 16 | cgaccucaaggucaccaua aa | 219 6 | 4146 | uuuauggugaccuugagguc gaa | 278 7 |
| Kylo-27-DS5 17 | gaccucaaggucaccauaa aa | 219 7 | 4147 | uuuuauggugaccuugaggu cga | 278 8 |
| Kylo-27-DS5 18 | accucaaggucaccauaaa ac | 219 8 | 4148 | guuuuauggugaccuugagg ucg | 278 9 |
| Kylo-27-DS5 19 | ccucaaggucaccauaaaa cc | 219 9 | 4149 | ggguuuuauggugaccuugag guc | 279 0 |
| Kylo-27-DS5 20 | cucaaggucaccauaaaac ca | 220 0 | 4150 | ugguuuuauggugaccuuga ggu | 279 1 |
| Kylo-27-DS5 21 | ucaaggucaccauaaaacc ag | 220 1 | 4151 | cugguuuuauggugaccuug agg | 279 2 |
| Kylo-27-DS5 22 | ccauaaaaccagcaccgga aa | 220 2 | 4160 | uuuccggugcugguuuuaug gug | 279 3 |
| Kylo-27-DS5 23 | ucaggaugccaagaacacu au | 220 3 | 4194 | auaguguucuuggcauccug agg | 279 4 |
| Kylo-27-DS5 24 | aggaugccaagaacacuau ga | 220 4 | 4196 | ucauaguguucuuggcaucc uga | 279 5 |
| Kylo-27-DS5 25 | ggaugccaagaacacuaug au | 220 5 | 4197 | aucauaguguucuuggcauc cug | 279 6 |
| Kylo-27-DS5 26 | gaugccaagaacacuauga uc | 220 6 | 4198 | gaucauaguguucuuggcau ccu | 279 7 |
| Kylo-27-DS5 27 | ugccaagaacacuaugauc cu | 220 7 | 4200 | aggaucauaguguucuuggc auc | 279 8 |
| Kylo-27-DS5 28 | gccaagaacacuaugaucc uu | 220 8 | 4201 | aaggaucauaguguucuugg cau | 279 9 |
| Kylo-27-DS5 29 | ccaagaacacuaugauccu ug | 220 9 | 4202 | caaggaucauaguguucuug gca | 280 0 |
| Kylo-27-DS5 30 | caagaacacuaugauccuu ga | 221 0 | 4203 | ucaaggaucauaguguucuu ggc | 280 1 |
| Kylo-27-DS5 31 | aagaacacuaugauccuu gag | 221 1 | 4204 | cucaaggaucauaguguucu ugg | 280 2 |
| Kylo-27-DS5 32 | agaacacuaugauccuug aga | 221 2 | 4205 | ucucaaggaucauaguguuc uug | 280 3 |
| Kylo-27-DS5 33 | gaacacuaugauccuuga gau | 221 3 | 4206 | aucucaaggaucauaguguu cuu | 280 4 |
| Kylo-27-DS5 34 | aacacuaugauccuugag auc | 221 4 | 4207 | gaucucaaggaucauagugu ucu | 280 5 |
| Kylo-27-DS5 35 | acacuaugauccuugaga ucu | 221 5 | 4208 | agaucucaaggaucauagug uuc | 280 6 |
| Kylo-27-DS5 36 | cacuaugauccuugagau cug | 221 6 | 4209 | cagaucucaaggaucauagu guu | 280 7 |
| Kylo-27-DS5 37 | uaugauccuugagaucug uac | 221 7 | 4212 | guacagaucucaaggaucau agu | 280 8 |
| Kylo-27-DS5 38 | ugauccuugagaucugua cca | 221 8 | 4214 | ugguacagaucucaaggauc aua | 280 9 |
| Kylo-27-DS5 39 | ccuugagaucuguaccagg ua | 221 9 | 4218 | uaccugguacagaucucaagg au | 281 0 |
| Kylo-27-DS5 40 | ccaggaugccacuaugucu au | 222 0 | 4248 | auagacauaguggcauccugg uc | 281 1 |
| Kylo-27-DS5 41 | acauauccaugaugacug gcu | 222 1 | 4274 | agccagucaucauggauaug ucc | 281 2 |
| Kylo-27-DS5 42 | cauauccaugaugacuggc uu | 222 2 | 4275 | aagccagucaucauggauau guc | 281 3 |
| Kylo-27-DS5 43 | ugacagauacaucuccaag ua | 222 3 | 4338 | uacuuggagauguaucuguc aac | 281 4 |
| Kylo-27-DS5 44 | gacagauacaucuccaagu au | 222 4 | 4339 | auacuuggagauguaucugu caa | 281 5 |
| Kylo-27-DS5 45 | acagauacaucuccaagua ug | 222 5 | 4340 | cauacuuggagauguaucug uca | 281 6 |
| Kylo-27-DS5 46 | cagauacaucuccaaguau ga | 222 6 | 4341 | ucauacuuggagauguaucu guc | 281 7 |
| Kylo-27-DS5 47 | agauacaucuccaaguau gag | 222 7 | 4342 | cucauacuuggagauguauc ugu | 281 8 |
| Kylo-27-DS5 48 | auacaucuccaaguauga gcu | 222 8 | 4344 | agcucauacuuggagaugua ucu | 281 9 |
| Kylo-27-DS5 49 | uccaaguaugagcuggaca aa | 222 9 | 4351 | uuuguccagcucauacuugg aga | 282 0 |
| Kylo-27-DS5 50 | ccaaguaugagcuggacaa ag | 223 0 | 4352 | cuuuguccagcucauacuug gag | 282 1 |
| Kylo-27-DS5 51 | cgauaggaacacccucauc au | 223 1 | 4380 | augaugaggguguuccuauc gga | 282 2 |
| Kylo-27-DS5 52 | ccucaucaucuaccuggac aa | 223 2 | 4392 | uuguccagguagaugaugag ggu | 282 3 |
| Kylo-27-DS5 53 | agucaaggucuacgccuau ua | 223 3 | 4491 | uaauaggcguagaccuugac ugc | 282 4 |
| Kylo-27-DS5 54 | ucaaggucuacgccuauu aca | 223 4 | 4493 | uguaauaggcguagaccuug acu | 282 5 |
| Kylo-27-DS5 55 | caaggucuacgccuauuac aa | 223 6 | 4494 | uuguaauaggcguagaccuu gac | 282 6 |
| Kylo-27-DS5 56 | ggucuacgccuauuacaac cu | 223 6 | 4497 | agguuguaauaggcguagac cuu | 282 7 |
| Kylo-27-DS5 57 | ggaggauggaaagcugaac aa | 223 7 | 4554 | uuguucagcuuuccauccucc uu | 282 8 |
| Kylo-27-DS5 58 | ggauggaaagcugaacaag cu | 223 8 | 4557 | agcuuguucagcuuuccaucc uc | 282 9 |
| Kylo-27-DS5 59 | ugcugaggagaauugcuu cau | 223 9 | 4602 | augaagcaauucuccucagca ca | 283 0 |
| Kylo-27-DS5 60 | gcugaggagaauugcuuc aua | 224 0 | 4603 | uaugaagcaauucucccucagc ac | 283 1 |
| Kylo-27-DS5 61 | cugaggagaauugcuuca uac | 224 1 | 4604 | guaugaagcaauucuccuca gca | 283 2 |
| Kylo-27-DS5 62 | ugaggagaauugcuucau aca | 224 2 | 4605 | uguaugaagcaauucuccuc agc | 283 3 |
| Kylo-27-DS5 63 | gaggagaauugcuucaua caa | 224 3 | 4606 | uuguaugaagcaauucuccu cag | 283 4 |
| Kylo-27-DS5 64 | aggagaauugcuucauac aaa | 224 4 | 4607 | uuuguaugaagcaauucucc uca | 283 5 |
| Kylo-27-DS5 65 | ggagaauugcuucauaca aaa | 224 5 | 4608 | uuuuguaugaagcaauucuc cuc | 283 6 |
| Kylo-27-DS5 66 | gagaauugcuucauacaa aag | 224 6 | 4609 | cuuuuguaugaagcaauucu ccu | 283 7 |
| Kylo-27-DS5 67 | agaauugcuucauacaaa agu | 224 7 | 4610 | acuuuuguaugaagcaauuc ucc | 283 8 |
| Kylo-27-DS5 68 | aggaguggacuaugugua caa | 224 8 | 4680 | uuguacacauaguccacuccu gg | 283 9 |
| Kylo-27-DS5 69 | ggaguggacuauguguac aag | 224 9 | 4681 | cuuguacacauaguccacucc ug | 284 0 |
| Kylo-27-DS5 70 | gaguggacuauguguaca aga | 225 0 | 4682 | ucuuguacacauaguccacuc cu | 284 1 |
| Kylo-27-DS5 71 | agugggacuauguguacaa gac | 225 1 | 4683 | gucuuguacacauaguccac ucc | 284 2 |
| Kylo-27-DS5 72 | gcuguccaaugacuuuga cga | 225 2 | 4722 | ucgucaaagucauuggacagc ug | 284 3 |
| Kylo-27-DS5 73 | uguccaaugacuuugacg agu | 225 3 | 4724 | acucgucaaagucauuggaca gc | 284 4 |
| Kylo-27-DS5 74 | guccaaugacuuugacga gua | 225 4 | 4725 | uacucgucaaagucauugga cag | 284 5 |
| Kylo-27-DS5 75 | uccaaugacuuugacgag uac | 225 5 | 4726 | guacucgucaaagucauugg aca | 284 6 |
| Kylo-27-DS5 76 | ccaaugacuuugacgagu aca | 225 6 | 4727 | uguacucgucaaagucauug gac | 284 7 |
| Kylo-27-DS5 77 | caaugacuuugacgagua cau | 225 7 | 4728 | auguacucgucaaagucauu gga | 284 8 |
| Kylo-27-DS5 78 | aaugacuuugacgaguac auc | 225 8 | 4729 | gauguacucgucaaagucau ugg | 284 9 |
| Kylo-27-DS5 79 | augacuuugacgaguaca uca | 225 9 | 4730 | ugauguacucgucaaaguca uug | 285 0 |
| Kylo-27-DS5 80 | ugacuuugacgaguacau cau | 226 0 | 4731 | augauguacucgucaaaguc auu | 285 1 |
| Kylo-27-DS5 81 | gacuuugacgaguacauc aug | 226 1 | 4732 | caugauguacucgucaaagu cau | 285 2 |
| Kylo-27-DS5 82 | acuuugacgaguacauca ugg | 226 2 | 4733 | ccaugauguacucgucaaag uca | 285 3 |
| Kylo-27-DS5 83 | cgaguacaucauggccauu ga | 226 3 | 4740 | ucaauggccaugauguacucg uc | 285 4 |
| Kylo-27-DS5 84 | agagaaccagaaacaaugc ca | 226 4 | 4983 | uggcauuguuucugguucuc uuc | 285 5 |
| Kylo-27-DS5 85 | gacuugcauccuggaaau aua | 226 5 | Neg. | uauuuccaggaugaaagucc a | 285 6 |
| Kylo-27-DS5 86 | aacaagaagaacaaacuca ca | 226 6 | Mou. | ugugaguuuguucuucuugu uca | 285 7 |
| Kylo-27-DS5 87 | gcugaacaagaagaacaaa cu | 226 7 | Mou. | aguuuguucuucuuguucag cac | 285 8 |
| Kylo-27-DS5 88 | gcugaauaagaagaacaaa cu | 226 8 | 1871 | aguuuguucuucuuauucag cac | 285 9 |
| Kylo-27-DS5 89 | aauaagaagaacaaacug aca | 226 9 | 1877 | ugucaguuuguucuucuuau uca | 286 0 |
| Kylo-27-DS5 90 | aagaagaacaaacucacac aa | 227 0 | Mou. | uugugugaguuuguucuucu ugu | 286 1 |
| Kylo-27-DS5 91 | aagaagaacaaacugacgc aa | 227 1 | 1880 | uugcgucaguuuguucuucu uau | 286 2 |

wherein n is a, u, g or c; g = guanosine, a = adenosine, u = uridine, c = cytidine, t = thymidine deoxyribonucleotide.

In some screening embodiments, the sense strand and antisense strand of the RNAi agent are selected from the sequences in Table 1, or sequences differing from each sequence in Table 1 by one, two or three nucleotides.

In some embodiments, the sense strand in Table 1 and the corresponding antisense strand in Table 1 form a dsRNA through base-pair complementation, which may be partially complementary or fully complementary. The partial complementarity may be at least 85% base pairing.

In some embodiments, the combination of the sense strand and the antisense strand is not limited to the double-strand combination in Table 1; any one sense strand in Table 1 may complementarily pair with any one antisense strand.

The present invention aims to protect the core sequences of the sequences in Table 1. A core sequence is any segment of at least 15 consecutive nucleotides of the above sequences, wherein "at least 15" means 15, 16, 17, 18, 19, 20, 21, 22, 23 nucleotides, etc. In some embodiments, the sense strand is represented by Formula (1): 5'-X core sequence Y-3', at least 85% of the bases between the antisense strand and the sense strand are complementary, X and Y comprise 0, 1, 2, 3, 4, 5, or 6 nucleotides, and 0, 1, 2, or 3 unpaired bases may be tolerated at the terminal positions in the double strand.

The antisense strand comprises consecutive nucleotides differing by 0, 1, 2 or 3 nucleotides from Formula (2): 5'-X' core sequence Y'-3'. At least 85% of the bases between the antisense strand and the sense strand are complementary; X' and Y' comprise 0, 1, 2, 3, 4, 5, or 6 nucleotides; and 0, 1, 2, or 3 unpaired bases may be tolerated at the terminal positions in the double strand.

In one embodiment, the core sequence may differ by 0, 1, 2 or 3 nucleotides. Such differences may be base pairs formed according to Watson-Crick principles or mismatches.

In some embodiments, the RNA inhibitor may be delivered into cell lines for sequence screening by cell transfection methods well known to those skilled in the art or by liposome-nucleic acid nanoparticles. The methods for preparing lipid compounds and liposome-nucleic acid nanoparticles described in US9233971B2, US9080186B2, CN102985548B and CN103189057B are incorporated herein by reference in their entirety.

In some embodiments, the amphipathic lipids among the lipid compounds are preferably macrocyclic lipid compounds D1C1, T1C1, T1C6, T4C4, B2C1, B2C6, B2C7 and M10C1.

It is well known to those skilled in the art that dsRNAs with a duplex structure of about 20 to 23 base pairs, e.g., 21 base pairs, have been found to be particularly effective in inducing RNA inhibition (Elbashir et al., EMBO 2001, 20:6877-6888). However, others have found that shorter or longer RNA duplex structures are also effective (Chu and Rana (2007) RNA 14:1714-1719; Kim et al. (2005) Nat Biotech 23:222-226). It can be reasonably expected that duplexes obtained by removing or adding a few nucleotides at one or both ends of a sequence in Tables 1 and 2 may be similarly effective compared to the dsRNAs. Therefore, dsRNAs having a sequence of at least 15, 16, 17, 18, 19, 20, 21 or more consecutive nucleotides derived from a sequence in Tables 1 and 2, and whose ability to inhibit complement system gene expression differs by no more than about 5, 10, 15, 20, 25 or 30% inhibition from that of a dsRNA comprising the full sequence, are all within the scope of the present application.

The dsRNAs described herein may further comprise one or more single-stranded nucleotide overhangs, e.g., 1, 2, 3 or 4 nucleotides. Nucleotide overhangs may comprise nucleotide/nucleoside analogs or combinations thereof, including deoxynucleotides. Such overhangs may be on the sense strand, the antisense strand, or a combination thereof. Furthermore, the nucleotides of the overhang may be present at the 5'-end, 3'-end or both ends of the antisense strand or sense strand of the dsRNA. Such overhangs may be formed by one strand being longer than the other, or by two strands of the same length being staggered. When the overhang is on the antisense strand, it may form a mismatch or complement with the complement system mRNA, or may be another sequence. For example, the overhang is at the 3'-end of the sense strand, or alternatively at the 3'-end of the antisense strand.

The dsRNA may also have blunt ends, meaning that no unpaired nucleotides are present at that end of the dsRNA, i.e., no nucleotide overhangs. Blunt ends may be located at the 5'-end of the antisense strand and the 3'-end of the sense strand, or vice versa, or a double-blunt ended molecule, a dsRNA that is fully double-stranded along its entire length, i.e., no nucleotide overhangs at either end of the molecule.

In some embodiments, the sense strand or antisense strand of the dsRNA has a nucleotide overhang of 1, 2, 3 or 4 nucleotides at the 3'-end, and the 5'-end is blunt.

In some embodiments, overhangs are present at the 3'-ends of both the sense strand and the antisense strand, each containing 1, 2, 3 or 4 nucleotides, including but not limited to: TT, UU, AU or UA.

In some embodiments, the dsRNA is a double-blunt ended molecule of 19, 21 or 23 nucleotides in length, a dsRNA that is fully double-stranded along its entire length, i.e., no nucleotide overhangs at either end of the molecule.

In some embodiments, the dsRNA is 21 nucleotides in length, and both the sense strand and the antisense strand have a 2-nucleotide overhang at the 3'-end.

To enhance the in vivo stability of the RNA inhibitors described herein without impairing or even enhancing their activity, the sense strand and antisense strand of the RNA inhibitors may be modified, and the nucleotides may have modifying groups, either on the entire strand or partially. In some embodiments, one or more nucleotides on the sense strand and/or antisense strand are modified to form modified nucleotides.

In some embodiments, the sense strand and antisense strand of the RNA inhibitors (e.g., dsRNAs) described herein are unmodified. In other embodiments, the sense strand and antisense strand of the RNA inhibitors described herein are chemically modified or conjugated as known in the art and described in the present invention to enhance stability or other beneficial properties. In other embodiments of the present application, all or substantially all nucleotides of the RNA inhibitors described herein may be modified, i.e., the strand of the RNA inhibitor has no more than 5, 4, 3, 2 or 1 unmodified nucleotides.

The sense strand and antisense strand of the RNA inhibitors described herein may be synthesized and/or modified by methods well known in the art, such as those described in "Current protocols in nucleic acid chemistry", Beaucage, S.L. et al. (Eds.), John Wiley & Sons, Inc., New York, NY, USA, which is incorporated herein by reference. In the RNA inhibitors provided herein, the sense strand and antisense strand of the RNA inhibitors need not be uniformly modified, and one or more modifications may be incorporated into individual nucleotides thereof.

In some embodiments, nucleotide modifications include: 5'-terminal modified nucleotides, 3'-terminal modified nucleotides, base modifications, sugar modifications or sugar replacements, backbone modifications. 5'-terminal modifications refer to phosphorylation, conjugation, inverted linkage. 3'-terminal modifications refer to conjugation, DNA nucleotides, inverted linkage, etc.; base modifications refer to: replacement with stabilizing bases, destabilizing bases, or bases pairing with an expanded partner library, abasic nucleotides (abasic) or conjugated bases. Sugar modifications are generally at the 2'-position or 4'-position. Backbone modifications refer to: modification or replacement of phosphodiester linkages.

Specific nucleotide modifications may include, but are not limited to: 5'-terminal phosphorus-containing nucleotide modifications, vinylphosphonate deoxyribonucleotides, vinylphosphonate-containing nucleotides and cyclopropylphosphonate-containing nucleotides, 3'-terminal deoxythymidine (dT) nucleotides, 2'-O-methyl modified nucleotides, 2'-fluoro modified nucleotides, 2'-deoxy modified nucleotides, 2'-amino modified nucleotides, 2'-O-allyl modified nucleotides, 2'-C-alkyl modified nucleotides, 2'-hydroxy modified nucleotides, 2'-methoxyethyl modified nucleotides, 2'-O-alkyl modified nucleotides, 2-O-(N-methylacetamide) modified nucleotides, 3'-O-methoxy (2'-internucleotide linkage) nucleotides, 2'-F-arabinose nucleotides, 5'-Me/2'-fluoro nucleotides, locked nucleotides, unlocked nucleotides, unlocked nucleobase analogs, conformationally restricted nucleotides, constrained ethyl nucleotides, abasic nucleotides, morpholino nucleotides, phosphoramidates, nucleotides containing non-natural bases, tetrahydropyran modified nucleotides, 1,5-anhydrohexitol modified nucleotides, cyclohexenyl modified nucleotides, nucleotides containing methylphosphonate groups, thermolabile nucleotides, GNA, deoxyribonucleotides, nucleotide analogs, morpholino nucleotides, abasic nucleotides, 3'-to-3' linked (inverted) nucleotides, bridged nucleotides, peptide nucleic acids (PNA).

5'-terminal phosphorus-containing nucleotide modification may be 5'-phosphate nucleotide or deoxynucleotide containing 5'-phosphate analog; including but not limited to: 5'-phosphate (5'-P), 5'-thiophosphate (5'-PS), 5'-dithiophosphate (5'-PS2), 5'-vinylphosphonate (5'-VP), 5'-methylphosphonate (MePhos) or 5'-deoxy-5'-C-malonyl. When the 5'-terminal phosphorus group is 5'-vinylphosphonate (5'-VP), the 5'-VP may be the 5'-E-VP isomer (i.e., trans-vinylphosphonate), 5'-Z-VP isomer (i.e., cis-vinylphosphonate) or a mixture thereof.

Internucleotide modifications may include, but are not limited to: phosphorothioate, chiral phosphorothioate, phosphorodithioate, phosphotriester, aminoalkyl phosphotriester, alkylphosphonate, phosphinate, phosphoramidate, thiocarbonylphosphoramidate, thiocarbonylalkylphosphonate, thiocarbonylalkyl phosphotriester, boranophosphate, including various salts and free acids.

Terminal modifications of the sense strand or antisense strand can prevent exonuclease degradation and enhance nuclease stability, such as a cap structure: inverted deoxy abasiccap (invAb). invAb is well known in the art, and specific performance verification can be found in F. Czauderna, Nucleic Acids Res., 2003, 31(11), 2705-16.

In some embodiments, at least two or more even-numbered nucleotides starting from the 5'-end of the antisense strand have fluoro at the 2'-position of the sugar moiety.

In some embodiments, all even-numbered nucleotides starting from the 5'-end of the antisense strand have fluoro at the 2'-position of the sugar moiety.

In some embodiments, at least one of the 2nd, 4th, 6th, 8th, 12th, 14th nucleotides starting from the 5'-end of the antisense strand has fluoro at the 2'-position of the sugar moiety. For example, the 2nd, 4th, 6th, 8th, 12th, 14th nucleotides starting from the 5'-end of the antisense strand all have fluoro at the 2'-position of the sugar moiety.

In some embodiments, the antisense strand, except for the 2nd, 6th, 8th, 10th, 14th, 16th nucleotides starting from the 5'-end, has at least one of the remaining nucleotides with methoxy at the 2'-position of the sugar moiety.

In some embodiments, the antisense strand, except for the 2nd, 4th, 6th, 8th, 14th, 16th nucleotides starting from the 5'-end, has at least one of the remaining nucleotides with methoxy at the 2'-position of the sugar moiety.

In some embodiments, the antisense strand, except for the 2nd, 4th, 6th, 8th, 14th, 16th, 18th, 20th nucleotides starting from the 5'-end, has at least one of the remaining nucleotides with methoxy at the 2'-position of the sugar moiety.

In some embodiments, at least two or more odd-numbered nucleotides starting from the 5'-end of the sense strand have fluoro at the 2'-position of the sugar moiety.

In some embodiments, all odd-numbered nucleotides starting from the 5'-end of the sense strand have fluoro at the 2'-position of the sugar moiety.

In some embodiments, at least one of the 5th, 7th, 8th, 9th nucleotides starting from the 5'-end of the sense strand has fluoro at the 2'-position of the sugar moiety. For example, the 5th, 7th, 8th, 9th nucleotides starting from the 5'-end of the sense strand all have fluoro at the 2'-position of the sugar moiety.

In some embodiments, the sense strand, except for the 5th, 7th, 8th, 9th nucleotides starting from the 5'-end, has at least one of the remaining nucleotides with methoxy at the 2'-position of the sugar moiety.

In some embodiments, at least one of the 7th, 9th, 10th, 11th nucleotides starting from the 5'-end of the sense strand has fluoro at the 2'-position of the sugar moiety. For example, the 7th, 9th, 10th, 11th nucleotides starting from the 5'-end of the sense strand all have fluoro at the 2'-position of the sugar moiety.

In some embodiments, the sense strand, except for the 7th, 9th, 10th, 11th nucleotides starting from the 5'-end, has at least one of the remaining nucleotides with methoxy at the 2'-position of the sugar moiety.

In some embodiments, at least one of the 3rd, 5th, 7th, 9th, 10th, 11th, 13th, 15th nucleotides starting from the 5'-end of the sense strand has fluoro at the 2'-position of the sugar moiety. For example, the 3rd, 5th, 7th, 9th, 10th, 11th, 13th, 15th nucleotides starting from the 5'-end of the sense strand all have fluoro at the 2'-position of the sugar moiety.

In some embodiments, the sense strand, except for the 3rd, 5th, 7th, 9th, 10th, 11th, 13th, 15th nucleotides starting from the 5'-end, has at least one of the remaining nucleotides with methoxy at the 2'-position of the sugar moiety.

In some embodiments, the sense strand, except for the 7th, 8th, 9th, 10th nucleotides starting from the 5'-end, has at least one of the remaining nucleotides with methoxy at the 2'-position of the sugar moiety.

For example, the -OH at the 2'-position of the sugar moiety of some or all nucleotides of the sense strand and/or antisense strand may be substituted, wherein the substituent group is fluoro or methoxy. Preferably, the 9th, 10th, 11th nucleotides starting from the 5'-end of the sense strand have fluoro at the 2'-position of the sugar moiety, and the 2nd, 4th, 6th, 12th, 14th, 16th, 18th, 20th nucleotides starting from the 5'-end of the antisense strand have fluoro at the 2'-position, and all remaining nucleotides have methoxy at the 2'-position of the sugar moiety. Alternatively, preferably, the 5th, 7th, 8th, 9th nucleotides starting from the 5'-end of the sense strand have fluoro at the 2'-position, and the 2nd, 4th, 8th, 14th, 16th nucleotides starting from the 5'-end of the antisense strand have fluoro at the 2'-position of the sugar moiety, and all remaining nucleotides have methoxy at the 2'-position of the sugar moiety.

In some embodiments, there are at least two consecutive phosphorothioate linkages between the nucleotides of the sense strand and/or antisense strand.

In some embodiments, there are at least two consecutive phosphorothioate linkages between three consecutive nucleotides at one or both ends of the sense strand and/or antisense strand.

For example, there are at least two consecutive phosphorothioate linkages between three consecutive nucleotides at the 5'-end and 3'-end of both the sense strand and the antisense strand.

For another example, the 9th, 10th, 11th nucleotides starting from the 5'-end of the sense strand have fluoro at the 2'-position of the sugar moiety, and the 2nd, 4th, 6th, 12th, 14th, 16th, 18th, 20th nucleotides starting from the 5'-end of the antisense strand have fluoro at the 2'-position of the sugar moiety, all remaining nucleotides have methoxy at the 2'-position of the sugar moiety, and there are at least two consecutive phosphorothioate linkages between three consecutive nucleotides at the 5'-end and 3'-end of both the sense strand and the antisense strand.

In some embodiments, some nucleotides of the sense strand have fluoro or methoxy at the 2'-position, and the phosphate linkages between at least three adjacent nucleotides at the end of the antisense strand may be phosphorothioated. The 5th, 7th, 8th, 9th or 3rd, 5th, 7th, 8th, 9th, 11th, 13th, 15th nucleotides starting from the 5'-end of the sense strand have fluoro at the 2'-position, remaining nucleotides have methoxy at the 2'-position, and the phosphate linkages between at least three adjacent nucleotides at the end of the antisense strand may be phosphorothioated.

In some embodiments, some nucleotides of the sense strand have fluoro or methoxy at the 2'-position, and the phosphate linkages between at least three adjacent nucleotides at the end of the antisense strand may be phosphorothioated. The 9th, 10th, 11th or 3rd, 5th, 7th, 8th, 9th, 11th, 13th, 15th and/or 17th nucleotides starting from the 5'-end of the sense strand have fluoro at the 2'-position, remaining nucleotides have methoxy at the 2'-position, and the phosphate linkages between at least three adjacent nucleotides at the end of the antisense strand may be phosphorothioated.

In some embodiments, the CFB modified sequence RNA inhibitors of the present invention are selected from Table 2-1 below, C5 modified sequence RNA inhibitors from Table 2-2 below, and C3 modified sequence RNA inhibitors from Table 2-3 below:

**Table 2-1 CFB Modified Sequence RNA Inhibitors**

| siRNA Inhibitor | SEQ ID No: | Sense Strand (5'-3') | SEQ ID No: | Antisense Strand (5'-3') |
|---|---|---|---|---|
| Kylo-17-DS2 69 | 537 | GCAAfCAfUfGfUGUUCAAAGUCAA | 566 | UGACUUfUGAACfAC AfUGUUGCUA |
| Kylo-17-DS2 70 | 538 | GCfAAfCAfUfGfUGfUUfCAfAAGUCAA | 567 | UfGAfCUfUUfGAACA CfAUfGUUGCUA |
| Kylo-17-DS2 71 | 539 | CUUAfAUfUfGfAGAAGGUGGCAAA | 568 | UGCCACfCUUCUfCAf AUUAAGAA |
| Kylo-17-DS2 72 | 540 | CUfUAfAUfUfGfAGfAAfGGfUGGCAAA | 569 | UfGCfCAfCCfUUCUC AfAUfUAAGAA |
| Kylo-17-DS2 73 | 541 | GsCsfAAfCAfUfGfUGfUUfCAfAAGsUs C | 570 | UsfGsAfCUfUUfGAA CACfAUfGUUGCsUs A |
| Kylo-17-DS2 74 | 542 | GsCsAACAUGfUfGfUUCAAAGUCsAsA | 571 | UsfGsAfCUfUUGAAC CfAUfGUUGCsUsA |
| Kylo-17-DS2 75 | 543 | GsCsAACAUGfUfGfUUCAAAGUCsAsA | 572 | UsfUsGfACfUUUGAA fCAfCAfUGUUsGsC |
| Kylo-17-DS2 76 | 544 | CsAsACAUGUfGfUfUCAAAGUCAsAsU | 573 | CsfUsUfGAfCUUUGA fACfACfAUGUsUsG |
| Kylo-17-DS8 30 | 168 1 | GsCsfAAfCAfUfGfUGfUUfUAfAAGUCs AsA | 168 4 | UsfGsAfCUfUUfGAA CACfAUfGUUGCsUs A |
| Kylo-17-DS8 31 | 168 2 | GsCsfAAfCAfUfGfUGfUUfCAfAAGUCs AsA | 168 5 | UsfGsAfCUfUUfGAA CACfAUfGUUGCsUs A |
| Kylo-17-DS8 32 | 168 3 | GsCsfAAfCAfUfGfUGfUUfCAfAAGUCs AsA | 168 6 | UsfGsAfCUfUUfGAA CACfAUfGUUGCsUs A |
| Kylo-17-DS2 77 | 545 | GsCsfAAfCAfUfGfUGfUUfCAfAAGUCs AsA | 574 | UsfGsAfCUfUUfGAA CACfAUfGUUGCsUs A |
| Kylo-17-DS2 78 | 546 | GsCsfAAfCAfUfGfUGfUUfCAfAAGUCs AsA | 575 | UsfGsAfCUfUUfGAA CACfAUfGUUGCsUs A |
| Kylo-17-DS2 79 | 547 | GsCsfAAfCAfUfGfUGfUUfCAfAAGUCs AsA | 576 | UsfGsAfCUfUUfGAA CACfAUfGUUGCsUs A |
| Kylo-17-DS2 80 | 548 | CsCsUGAUfCAfAfGfCUCAAGAAUsAsA | 577 | UsfUsAfUUfCUfUGA GCUfUGfAUCAGGsG sC |
| Kylo-17-DS2 81 | 549 | CsCsUGAUfCAfAfGfCUCAAGAAUsAsA | 578 | UsfUsAfUUfCUfUGA GCUfUGfAUCAsGsG |
| Kylo-17-DS2 82 | 550 | GsAsAGCAfGGfAfAfUUCCUGAAUsUs U | 579 | AsfAsAfUUfCAfGGA AUUfCCfUGCUUCsU sU |
| Kylo-17-DS2 83 | 551 | GsCsUGUGfGUfGfUfCUGAGUACUsUs U | 580 | AsfAsAfGUfACfUCAG ACfACfCACAGCsCsC |
| Kylo-17-DS2 84 | 552 | AsGsCUGUfGAfGfAfGAGAUGCUCsAs A | 581 | UsfUsGfAGfCAfUCU CUCfUCfACAGCUsGs C |
| Kylo-17-DS2 85 | 553 | AsGsCCAAfAAfAfGfUGUCUAGUCsAsA | 582 | UsfUsGfACfUAfGAC ACUfUUfUUGGCUsC sC |
| Kylo-17-DS2 86 | 554 | GsUsCUAGfUCfAfAfCUUAAUUGAsGs A | 583 | UsfCsUfCAfAUfUAA GUUfGAfCUAGACsA sC |
| Kylo-17-DS2 87 | 555 | GsUsCUAGfUCfAfAfCUUAAUUGAsGs A | 584 | UsfCsUfCAfAUfUAA GUUfGAfCUAGsAsC |
| Kylo-17-DS2 88 | 556 | CsAsACAUGUfGfUfUCAAAGUCAsAsU | 585 | AsTsUGdACTUUGAd ACfACAUGUsUsG |
| Kylo-17-DS2 89 | 557 | CsAsACAUfGUfGfUfUCAAAGUCAsAs U | 586 | AsTsUGdACTUUGAd ACfACAUGUsUsG |
| Kylo-17-DS2 90 | 558 | GsAsAGCAfGGfAfAfUUCCUGAAUsUs U | 587 | AsdAsAUTCdAGGAA TUfCCUGCUUCsUsU |
| Kylo-17-DS2 91 | 559 | GsUsCUAGfUCfAfAfCUUAAUUGAsGs A | 588 | UsdCsUCdAATUAAG TUfGACUAGACsAsC |
| Kylo-17-DS2 92 | 560 | CsAsACAUGUfGfUfUCAAAGUCAsAsU | 589 | AsfUsUGAfCUfUfUG AACfACfAUGUsUsG |
| Kylo-17-DS2 93 | 561 | GsAsAGCAfGGfAfAfUUCCUGAAUsUs U | 590 | AsfAsAUUfCAfGfGA AUUfCCfUGCUUCsU sU |
| Kylo-17-DS2 94 | 562 | GsUsCUAGfUCfAfAfCUUAAUUGAsGs A | 591 | UsfCsUCAfAUfUfAA GUUfGAfCUAGACsA sC |
| Kylo-17-DS2 95 | 563 | GsUsCUAG UCfAfAfCU UAAU UGAsGsA | 592 | UsfCsUfCAfAUfUAA GUUfGAfCUfAGsfAs C |
| Kylo-17-DS2 96 | 564 | GsUsCUAGfUCfAfAfCUUAAUUGAsGs A | 593 | UsfCsUfCAfAUfUAA GUUfGAfCUAGACsA sCTT |
| Kylo-17-DS2 97 | 565 | GsUsCUAGfUCfAfAfCUUAAUUGAsGs A | 594 | UsfCsUCAfAUfUfAA GUUfGAfCUAGACsA sC |

**Table 2-2 C5 Modified Sequence RNA Inhibitors**

| siRNA Inhibitor | SEQ ID No: | Sense Strand (5'-3') | SEQ ID No: | Antisense Strand (5'-3') |
|---|---|---|---|---|
| Kylo-19-D S750 | 1499 | GsCsAAACfAUfAfUfGUCAUUUC AsGsU | 1570 | AsfCsUfGAfAA fUGACAUfAUf GUUUGCsUsC |
| Kylo-19-D S751 | 1500 | UsCsAUUUfCAfGfCfACCAAAAA UsAsU | 1571 | AsfUsAfUUfU UfUGGUGCfU GfAAAUGAsC sA |
| Kylo-19-D S752 | 1501 | GsUsUUAGfAAfGfAfUUUAAACA AsCsA | 1572 | UsfGsUfUGfU UfUAAAUCfU UfCUAAACsUs G |
| Kylo-19-D S753 | 1502 | GsGsAAGUfUUfGfCfAGCUUUUA UsUsU | 1573 | AsfAsAfUAfA AfAGCUGCfA AfACUUCCsUs C |
| Kylo-19-D S754 | 1503 | AsGsCAUUfUUfUfAfGCUAAUUU AsGsA | 1574 | UsfCsUfAAfAU fUAGCUAfAAf AAUGCUsUsG |
| Kylo-19-D S755 | 1504 | GsAsAGfUUfAfAfAGAAUAUGUC UsUsG | 1575 | AsGsACAUfA UUCUfUUfAA CUUCsAsA |
| Kylo-19-D S756 | 1505 | GsAsAUfAUfGfUfCUUGCCACAU UsUsU | 1576 | AsUsGUGGfC AAGAfCAfUA UUCUsUsU |
| Kylo-19-D S757 | 1506 | CsAsAGfAAfCfUfUUAAGAAUUU UsGsA | 1577 | AsAsAAUUfC UUAAfAGfUU CUUGsUsA |
| Kylo-19-D S758 | 1507 | GsAsACfUUfUfAfAGAAUUUUGA AsAsU | 1578 | UsUsCAAAfA UUCUfUAfAA GUUCsUsU |
| Kylo-19-D S759 | 1508 | CsUsUUfAAfGfAfAUUUUGAAAU UsCsU | 1579 | AsAsUUUCfA AAAUfUCfUU AAAGsUsU |
| Kylo-19-D S760 | 1509 | GsAsAUfUUfUfGfAAAUUCUAUA AsAsA | 1580 | AsAsUUUCfA AAAUfUCfUU AAAGsUsU |
| Kylo-19-D S761 | 1510 | GsAsAAfUUfCfUfAUAAAAAGCA AsGsA | 1581 | UsUsGCUUfU UUAUfAGfAA UUUCsAsA |
| Kylo-19-D S762 | 1511 | CsUsAUfAAfAfAfAGCAAGAUAU UsUsU | 1582 | AsAsUAUCfU UGCUfUUfUU AUAGsAsA |
| Kylo-19-D S763 | 1512 | GsCsAAfGAfUfAfUUUUUAUAAU AsAsA | 1583 | UsAsUUAUfA AAAAfUAfUC UUGCsUsU |
| Kylo-19-D S764 | 1513 | CsAsAGfAUfAfUfUUUUAUAAUA AsAsG | 1584 | UsUsAUUAfU AAAAfAUfAU CUUGsCsU |
| Kylo-19-D S765 | 1514 | GsAsUAfUUfUfUfUAUAAUAAAG UsAsG | 1585 | AsCsUUUAfU UAUAfAAfAA UAUCsUsU |
| Kylo-19-D S766 | 1515 | AsAsAGfCAfAfGfAUAUUUUAU AsAsU | 1586 | UsAsUAAAfA AUAUfCUfUG CUUUsUsU |
| Kylo-19-D S767 | 1516 | CsAsAAfAUfAfAfCUCACUAUAA UsUsA | 1587 | AsUsUAUAfG UGAGfUUfAU UUGsUsC |
| Kylo-19-D S768 | 1517 | AsCsAAfAAfUfAfACUCACUAUA AsUsU | 1588 | UsUsAUAGfU GAGUfUAfUU UUGUsCsA |
| Kylo-19-D S769 | 1518 | AsGsCAfAGfAfUfAUUUUUAUAA UsAsA | 1589 | AsUsUAUAfA AAAUfAUfCU UGCUsUsU |
| Kylo-19-D S770 | 1519 | GsAsfAGfUUfAfAfAGfAAfUAfUG UCUsUsG | 1590 | AsfGsAfCAfUA fUUCUUUfAAf CUUCsAsA |
| Kylo-19-D S771 | 1520 | GsAsfAUfAUfGfUfCUfUGfCCfAC AUUsUsU | 1591 | AsfUsGfUGfGC fAAGACAfUAf UUCUsUsU |
| Kylo-19-D S772 | 1521 | CsAsfAGfAAfCfUfUfAAfGAfAU UUUsGsA | 1592 | AsfAsAfAUfUC fUUAAAGfUUf CUUGsUsA |
| Kylo-19-D S773 | 1522 | GsAsfACfUUfUfAfAGfAAfUUfUU GAAsAsU | 1593 | UsfUsCfAAfAA fUUCUUAfAAf GUUCsUsU |
| Kylo-19-D S774 | 1523 | CsUsfUUfAAfGfAfAUfUUfUGfAA AUUsCsU | 1594 | AsfAsUfUUfCA fAAAUUCfUUf AAAGsUsU |
| Kylo-19-D S775 | 1524 | GsAsfAUfUUfUfGfAAfAUfUCfUA UAAsAsA | 1595 | AsfAsUfUUfCA fAAAUUCfUUf AAAGsUsU |
| Kylo-19-D S776 | 1525 | GsAsfAAfUUfCfUfAUfAAfAAfAG CAAsGsA | 1596 | UsfUsGfCUfUU fUUAUAGfAAf UUUCsAsA |
| Kylo-19-D S777 | 1526 | CsUsfAUfAAfAfAfAGfCAfAGfAU AUUsUsU | 1597 | AsfAsUfAUfCU fUGCUUUfUUf AUAGsAsA |
| Kylo-19-D S778 | 1527 | GsCsfAAfGAfUfAfUUfUUfUAfUA AUAsAsA | 1598 | UsfAsUfUAfU AfAAAUAfU CfUUGCsUsU |
| Kylo-19-D S779 | 1528 | CsAsfAGfAUfAfUfUUfUUfAUfAA UAAsAsG | 1599 | UsfUsAfUUfA UfAAAAUfA UfCUUGsCsU |
| Kylo-19-D S780 | 1529 | GsAsfUAfUUfUfUfUAfUAfAUfAA AGUsAsG | 1600 | AsfCsUfUUfAU fUAUAAAfAAf UAUCsUsU |
| Kylo-19-D S781 | 1530 | AsAsfAGfCAfAfGfAUfAUfUUfUU AUAsAsU | 1601 | UsfAsUfAAfA AfAUAUCUfU GfCUUUsUsU |
| Kylo-19-D S782 | 1531 | CsAsfAAfAUfAfAfCUfCAfCUfAU AAUsUsA | 1602 | AsfUsUfAUfA GfUGAGUUfA UfUUUGsUsC |
| Kylo-19-D S783 | 1532 | AsCsfAAfAAfUfAfACfUCfACfUA UAAsUsU | 1603 | UsfUsAfUAfG UfGAGUUAfU UfUUGUsCsA |
| Kylo-19-D S784 | 1533 | AsGsfCAfAGfAfUfAUfUUfUUfAU AAUsAsA | 1604 | AsfUsUfAUfA AfAAAUAUfC UfUGCUsUsU |
| Kylo-19-D S785 | 1534 | AsUsGGAUfUUfCfUfCUUCAUUC AsUsA | 1605 | UsfAsUGAfAU fGfAAGAGfAA fAUCCAUsUsG |
| Kylo-19-D S786 | 1535 | UsUsGUCCfCAfGfUfAUUCUAUG UsUsU | 1606 | AsfAsACAfUAf GfAAUACfUGf GGACAAsCsG |
| Kylo-19-D S787 | 1536 | AsUsGGAUfUUfCfUfCUUCAUUC AsUsA | 1607 | UsdAsUGdAAT GAAGdAGfAA AUCCAUsUsG |
| Kylo-19-D S788 | 1537 | UsUsGUCCfCAfGfUfAUUCUAUG UsUsU | 1608 | AsdAsACdAUd AGAAUdACfU GGGACAAsCs G |
| Kylo-19-D S789 | 1538 | GsAsCAAUfGGfAfUfUUCUCUUC AsUsU | 1609 | AsfAsUfGAfA GfAGAAAUfC CfAUUGUCsAs U |
| Kylo-19-D S790 | 1539 | AsUsGGAUfUUfCfUfCUUCAUUC AsUsA | 1610 | UsfAsUfGAfA UfGAAGAGfA AfAUCCAUsUs G |
| Kylo-19-D S791 | 1540 | CsAsAUGGfAUfUfUfCUCUUCAU UsCsA | 1611 | UsfGsAfAUfG AfAGAGAAfA UfCCAUUGsUs C |
| Kylo-19-D S792 | 1541 | AsUsGCAAfCAfAfUfCUCUAUUA AsAsA | 1612 | UsfUsUfUAfA UfAGAGAUfU GfUUGCAUsCs A |
| Kylo-19-D S793 | 1542 | CsCsCAGUfAUfUfCfUAUGUUUU UsCsA | 1613 | UsfGsAfAAfA AfCAUAGAfA UfACUGGGsA sC |
| Kylo-19-D S794 | 1543 | CsUsUUGCfUUfGfAfAAGAAAAU AsCsC | 1614 | GsfGsUfAUfU UfUCUUUCfA AfGCAAAGsG sC |
| Kylo-19-D S795 | 1544 | AsAsUGGAfUUfUfCfUCUUCAUU CsAsU | 1615 | AsfUsGfAAfU GfAAGAGAfA AfUCCAUUsGs U |
| Kylo-19-D S796 | 1545 | AsGsAGAAfCAfGfCfUUAUAUCU UsAsC | 1616 | GsfUsAfAGfA UfAUAAGCfU GfUUCUCUsCs G |
| Kylo-19-D S797 | 1546 | UsGsGACAfCCfGfGfAAACAAUA AsAsU | 1617 | AsfUsUfUAfU UfGUUUCCfG GfUGUCCAsAs U |
| Kylo-19-D S798 | 1547 | GsGsACACfCGfGfAfAACAAUAA AsUsU | 1618 | AsfAsUfUUfA UfUGUUUCfC GfGUGUCCsAs A |
| Kylo-19-D S799 | 1548 | UsGsGUCUfUAfUfUfUGUAAAGC UsCsA | 1619 | UsfGsAfGCfUU fUACAAAfUAf AGACCAsGsC |
| Kylo-19-D S800 | 1549 | UsUsGUCCfCAfGfUfAUUCUAUG UsUsU | 1620 | AsfAsAfCAfUA fGAAUACfUGf GGACAAsCsG |
| Kylo-19-D S801 | 1550 | UsAsUCCUfCAfGfAfGAAUAAAU UsCsC | 1621 | GsfGsAfAUfU UfAUUCUCfU GfAGGAUAsA sA |
| Kylo-19-D S802 | 1551 | GsAsUACAfCUfGfAfAGCAUUUG AsUsG | 1622 | CsfAsUfCAfAA fUGCUUCfAGf UGUAUCsCsA |
| Kylo-19-D S803 | 1552 | GsGsAAAUfCAfUfUfGGAACAUU UsUsU | 1623 | AsfAsAfAAfU GfUUCCAAfU GfAUUUCCsUs G |
| Kylo-19-D S804 | 1553 | CsCsCUUGfGAfAfAfGAGUAUUU CsAsA | 1624 | UsfUsGfAAfA UfACUCUUfU CfCAAGGGsCs U |
| Kylo-19-D S805 | 1554 | CsAsGGAAfAUfCfAfUUGGAACA UsUsU | 1625 | AsfAsAfUGfU UfCCAAUGfA UfUUCCUGsUs U |
| Kylo-19-D S806 | 1555 | GsCsCCUUfGGfAfAfAGAGUAUU UsCsA | 1626 | UsfGsAfAAfU AfCUCUUUfC CfAAGGGCsUs U |
| Kylo-19-D S807 | 1556 | GsCsAGUUfCUfGfUfGUUAAAAU GsUsC | 1627 | GsfAsCfAUfUU fUAACACfAGf AACUGCsAsU |
| Kylo-19-D S808 | 1557 | GsGsAGCAfAAfCfAfUAUGUCAU UsUsC | 1628 | GsfAsAfAUfG AfCAUAUGfU UfUGCUCCsUs G |
| Kylo-19-D S809 | 1558 | CsAsGGAGfCAfAfAfCAUAUGUC AsUsU | 1629 | AsfAsUfGAfCA fUAUGUUfUGf CUCCUGsUsC |
| Kylo-19-D S810 | 1559 | AsAsUGAUfGAfAfCfCUUGUAAA GsAsA | 1630 | UsfUsCfUUfUA fCAAGGUfUCf AUCAUUsUsU |
| Kylo-19-D S811 | 1560 | CsAsfAGfAUfAUfUfUfUUAUAAU AAsAsG | 1631 | UsfUsAfUUfA UfAAAAUfA UfCUUGsCsU |
| Kylo-19-D S812 | 1561 | CsAsAGAUAUfUfUfUUAUAAUsA sA | 1632 | UsfUsAfUUfA UfAAAAUfA UfCUfUGsCsU |
| Kylo-19-D S813 | 1562 | GsCsAAGAUAfUfUfUUUAUAAU AsAsA | 1633 | UsfUsUfAUfU AfUAAAAAfU AfUCfUUGCsU sU |
| Kylo-19-D S814 | 1563 | CsUsUUfAAfGfAfAUUUUGAAAU UsCsU | 1634 | AsAsUUUCfA AAAUfUCfUU AAAGsUsU |
| Kylo-19-D S815 | 1564 | CsUsUUAAfGfAfAUUUUGAAAsU sU | 1635 | AsfAsUfUUfCA fAAAUUCfUUf AAfAGsUsU |
| Kylo-19-D S816 | 1565 | CsUsfUUfAAfGfAfAUfUUfUGfAA AUUsCsU | 1636 | AsfAsUfUUfCA fAAAUUCfUUf AAfAGsUsU |
| Kylo-19-D S817 | 1566 | CsUsUUAAGAfAfUfUUUGAAAU UsCsU | 1637 | AsfAsUfUUfCA fAAAUUCfUUf AAfAGsUsU |
| Kylo-19-D S818 | 1567 | CsUsfUUfAAfGfAfAUfUUUGAAA UUsCsC | 1638 | AsfAsUfUUfCA fAAAUUCfUUf AAAGsUsU |
| Kylo-19-D S819 | 1568 | AsAsCUUUAAfGfAfAUUUUGAA AsUsU | 1639 | AsfAsUfUUfCA fAAAUfUCfUU fAAfAGsUsU |
| Kylo-19-D S820 | 1569 | CsUsUUAAGAfAfUfUUUGAAAU UsAsC | 1640 | GsfUsAfAUfU UfCAAAfAUfU CfUUfAAsAsG |

**Table 2-3 C3 Modified Sequence RNA Inhibitors**

| RNA Inhibitor | SEQ ID No: | Sense Strand Sequence 5' to 3' | SEQ ID No: | Antisense Strand Sequence 5' to 3' |
|---|---|---|---|---|
| Kylo-27-DS810 | 1641 | GsGsUGUUfGAfCfAfGAUACAUsCsU | 1661 | AsdGsAUdGUdAUC UGTCfAACACCsAsU |
| Kylo-27-DS811 | 1642 | GGUGUUfGAfCfAfGAUACAUsCsU | 1662 | AsfGsAUGfUAfUCUG UCfAAfCACCsAsU |
| Kylo-27-DS81 2 | 164 3 | GsGsUGUUfGAfCfAfGAUACAUsCsU | 1663 | AsfGsAUGfUAfUCUG UCfAAfCACCAU |
| Kylo-27-DS81 3 | 164 4 | GsCsUGAGfGAfGfAfAUUGCUUCAsU sA | 1664 | UsdAsUGdAAdGCAA UTCfUCCUCAGCsAs C |
| Kylo-27-DS81 4 | 164 5 | AsGsAAAUfUCfUfAfCUACAUCUAsU sA | 1665 | UsdAsUAdGATGUA GTAfGAAUUUCUsCs U |
| Kylo-27-DS82 0 | 165 1 | GsCsUGAGfGAfGfAfAUUGCUUCAsU sA | 1671 | UsfAsUGAfAGfCAAU UCfUCfCUCAGCsAsC |
| Kylo-27-DS81 6 | 164 7 | AsGsAAAUfUCfUfAfCUACAUCUAsU sA | 1667 | UsfAsUAGfAUfGUA GUAfGAfAUUUCUsC sU |
| Kylo-27-DS81 7 | 164 8 | GsGsUGUUfGAfCfAfGAUACAUsCsU | 1668 | AsfGsAUGfUAfUfCU GUCfAAfCACCsAsU |
| Kylo-27-DS81 8 | 164 9 | GsCsUGAGfGAfGfAfAUUGCUUCAsU sA | 1669 | UsfAsUGAfAGfCfAA UUCfUCfCUCAGCsAs C |
| Kylo-27-DS81 9 | 165 0 | AsGsAAAUfUCfUfAfCUACAUCUAsU sA | 1670 | UsfAsUAGfAUfGfUA GUAfGAfAUUUCUsC sU |
| Kylo-27-DS82 1 | 165 2 | GsGsUGUUfGAfCfAfGAUACAUsCsU | 1672 | AsdGsAUdGUdAUC UGTCfAACACCsAsU |
| Kylo-27-DS82 2 | 165 3 | GsGsUGUUfGAfCfAfGAUACAUsCsU | 1673 | AsfGsAUGfUAfUCUG UCfAAfCACCsAsU |
| Kylo-27-DS82 3 | 165 4 | GsCsUGAGfGAfGfAfAUUGCUUCAsU sA | 1674 | UsfAsUGAfAGfCAAU UCfUCfCUCAGCsAsC TT |
| Kylo-27-DS82 4 | 165 5 | AsGsAAAUfUCfUfAfCUACAUCUAsU sA | 1675 | UsfAsUAGfAUfGUA GUAfGAfAUUUCUsC sU |
| Kylo-27-DS82 5 | 165 6 | AsGsAAAUfUCfUfAfCUACAUCUAsU sA | 1676 | UsfAsUAGfAUfGUA GUAfGAfAUUUCUsC sUTT |
| Kylo-27-DS82 6 | 165 7 | GsCsUGAGGAfGfAfAUUGCUUCAsUs A | 1677 | UsfAsUfGAfAGfCAA UUCfUCfCUfCAsfGsC |
| Kylo-27-DS82 7 | 1658 | AsGsAAAUUCfUfAfCUACAUCUAsUs A | 1678 | UsfAsUfAGfAUfGUA GUAfGAfAUfUUsfCs U |
| Kylo-27-DS82 8 | 1659 | AsUsGGUGUUfGfAfCAGAUACAUsCs U | 1679 | AsfGsAfUGfUAfUCU GUCfAAfCAfCCsfAsU |
| Kylo-27-DS82 9 | 1660 | AsUsGGUGfUUfGfAfCAGAUACAUsC sU | 1680 | AsfGsAUGfUAfUCUG UCfAAfCACCAUsUsG TT |
| Kylo-27-DS82 9 | 286 3 | GsGsUGUUGAfCfAfGAUACAUsCsU | 3109 | AsfGsAfUGfUAUCUG fUCfAAfCAfCCsfAsU |
| Kylo-27-DS83 0 | 286 4 | GsGsUGfUUfGfAfCAGAUACAUsCsU | 3110 | AsfGsAUGfUAfUfCU GUCfAAfCACCsAsU |
| Kylo-27-DS83 1 | 286 5 | GsGsfUGfUUfGAfCfAfGAfUAfCAUsC sU | 3111 | AsfGsAfUGUAfUCUG UCfAAfCACCsAsU |
| Kylo-27-DS83 2 | 286 6 | GsGsAGCCfUAfCfAfGAGAAAUUCsU sA | 3112 | AsdGsAUdGUdAUC UGdTCfAACACCsAs U |
| Kylo-27-DS83 3 | 286 7 | GsGsfAGfCCfUfAfCAfGAfGAfAAUUC sUsA | 3113 | UsfAsGfAAfUUUCUf CUfGUfAGfGCfUCCs AsC |
| Kylo-27-DS83 4 | 286 8 | GsGsfAGfCCfUAfCfAfGAfGAfAAUUC sUsA | 3114 | UsfAsGfAAUUfUfCU CUfGUfAGGCUCCsA sC |
| Kylo-27-DS83 5 | 286 9 | AsGsAAAUfUCfUfAfCUACAUCUAsU sA | 3115 | UsfAsGfAAUUfUCUC UfGUfAGGCUCCsAs C |
| Kylo-27-DS83 6 | 287 0 | AsGsAAAUUCfUfAfCUACAUCUAsUs A | 3116 | UsdAsGAdAUdTUCU CdTGfUAGGCUCCsA sC |
| Kylo-27-DS83 7 | 287 1 | AsGsfAAfAUfUCfUfAfCUfACfAUCUA sUsA | 3117 | UsfAsUfAGfAUGUA GfUAfGAfAUfUUfCU sCsU |
| Kylo-27-DS83 8 | 287 2 | GsCsUGAGfGAfGfAfAUUGCUUCAsU sA | 3118 | UsfAUfAGfAUfGfUA GUAfGAfAUUUCUsC sU |
| Kylo-27-DS83 9 | 287 3 | GsCsUGAGGAfGfAfAUUGCUUCAsUs A | 3119 | UsfAsUfAGAUGfUA GUAfGAfAUUUCUsC sU |
| Kylo-27-DS84 0 | 287 4 | GsCsUGfAGfGfAfGAAUUGCUUCAsU sA | 3120 | UsdAsUAdGAdTGUA GdTAfGAAUUUCUsC sU |
| Kylo-27-DS84 1 | 287 5 | GsGsAGAAfUUfGfCfUUCAUACAAsA sA | 3121 | UsfAsUfGAfAGfCAA UfUCfUCfCUfCAfGCs AsC |
| Kylo-27-DS84 2 | 287 6 | GsGsAGAAUUfGfCfUUCAUACAAsAs A | 3122 | UsfAsUfGAfAGfCfAA UUCfUCfCUCAGCsAs C |
| Kylo-27-DS84 3 | 2877 | GsGsAGfAAfUfUfGCUUCAUACAAsA sA | 3123 | UsfAsUGAAGfCAAU UCfUCfCUCAGCsAsC |
| Kylo-27-DS84 4 | 287 8 | GsGsfAGfAAfUUfGfCfUUfCAfUACAA sAsA | 3124 | dTsdAsUGAAGCAAU UCfUCfCUCAGCsAsC |
| Kylo-27-DS84 5 | 287 9 | GsAsGCCGUUfCfUfCUACAAUUAsCs U | 3125 | AsfGsUfUUfGUUCU UfCUfUAfUUfCAfGC sAsC |
| Kylo-27-DS84 6 | 288 0 | GsAsGCfCGfUfUfCUCUACAAUUAsCs U | 3126 | AsfGsUUUGUfUfCU UCUfUAfUUCAGCsA sC |
| Kylo-27-DS84 7 | 288 1 | GsAsfGCfCGfUUfCfUfCUfACfAAUUA sCsU | 3127 | AsfGsUUUGUfUCUU CUfUAfUUCAGCsAs C |
| Kylo-27-DS84 8 | 288 2 | GsGsUGUUGAfCfAfGAUACAUsCsU | 3128 | AsfGsAfUGfUAUCUG fUCfAAfCAfCCsfAsU |
| Kylo-27-DS84 9 | 288 3 | GsGsUGUUfGAfCfAfGAUACAUsCsU | 3129 | AsfGsAUGfUAfUfCU GUCfAAfCACCsAsU |
| Kylo-27-DS85 0 | 288 4 | GsGsUGfUUfGfAfCAGAUACAUsCsU | 3130 | AsfGsAfUGUAfUCUG UCfAAfCACCsAsU |
| Kylo-27-DS85 1 | 288 5 | GsGsfUGfUUfGAfCfAfGAfUAfCAUsC sU | 3131 | AsdGsAUdGUdAUC UGdTCfAACACCsAs U |
| Kylo-27-DS85 2 | 288 6 | GsCsAGCUfAAfAfAfGACUUUGACsU sU | 3132 | AsfAsGUCfAAfAGUC UUfUUfAGCUGCsAs G |
| Kylo-27-DS85 3 | 288 7 | GsCsAGCUAAfAfAfGACUUUGACsUs U | 3133 | AsfAsGfUCfAAAGUC fUUfUUfAGfCUfGCs AsG |
| Kylo-27-DS85 4 | 288 8 | GsCsAGCUfAAfAfAfGACUUUGACsU sU | 3134 | AsfAsGUCAAfAfGUC UUfUUfAGCUGCsAs G |
| Kylo-27-DS85 5 | 288 9 | GsCsAGfCUfAfAfAAGACUUUGACsU sU | 3135 | AsfAsGfUCAAfAGUC UUfUUfAGCUGCsAs G |
| Kylo-27-DS85 6 | 289 0 | GsCsfAGfCUfAAfAfAfGAfCUfUUGAC sUsU | 3136 | AsdAsGUdCAdAAGU CdTUfUUAGCUGCsA sG |
| Kylo-27-DS85 7 | 289 1 | AsGsAAAUUCfUfAfCUACAUCUAsUs A | 3137 | UsfAsUfAGfAUGUA GfUAfGAfAUfUUfCU sCsU |
| Kylo-27-DS85 8 | 289 2 | AsGsAAAUfUCfUfAfCUACAUCUAsU sA | 3138 | UsfAUAGfAUfGfUAG UAfGAfAUUUCUsCs U |
| Kylo-27-DS85 9 | 289 3 | AsGsAAfAUfUfCfUACUACAUCUAsU sA | 3139 | UsfAsUfAGAUfGUA GUAfGAfAUUUCUsC sU |
| Kylo-27-DS86 0 | 289 4 | AsGsfAAfAUfUCfUfAfCUfACfAUCUA sUsA | 3140 | UsdAsUAdGAdTGUA GdTAfGAAUUUCUsC sU |
| Kylo-27-DS86 1 | 289 5 | GsGsAGCCfUAfCfAfGAGAAAUUCsU sA | 3141 | UsfAsGAAfUUfUCUC UGfUAfGGCUCCsAs C |
| Kylo-27-DS86 2 | 289 6 | GsGsAGCCUAfCfAfGAGAAAUUCsUs A | 3142 | UsfAsGfAAfUUUCUC fUGfUAfGGfCUfCCs AsC |
| Kylo-27-DS86 3 | 289 7 | GsGsAGCCfUAfCfAfGAGAAAUUCsU sA | 3143 | UsfAsGAAfUUfUfCU CUGfUAfGGCUCCsA sC |
| Kylo-27-DS86 4 | 289 8 | GsGsAGfCCfUfAfCAGAGAAAUUCsU sA | 3144 | UsfAsGAAUUfUCUC UGfUAfGGCUCCsAs C |
| Kylo-27-DS86 5 | 289 9 | GsGsfAGfCCfUAfCfAfGAfGAfAAUUC sUsA | 3145 | UsdAsGAdAUdTUCU CdTGfUAGGCUCCsA sC |
| Kylo-27-DS86 6 | 290 0 | CsCsAGACfAGfAfCfAAGACCAUCsUs A | 3146 | UsfAsGAUfGGfUCU UGUfCUfGUCUGGsA sU |
| Kylo-27-DS86 7 | 290 1 | CsCsAGACAGfAfCfAAGACCAUCsUsA | 3147 | UsfAsGfAUfGGUCU UfGUfCUfGUfCUfGG sAsU |
| Kylo-27-DS86 8 | 290 2 | CsCsAGACfAGfAfCfAAGACCAUCsUs A | 3148 | UsfAsGAUfGGfUfCU UGUfCUfGUCUGGsA sU |
| Kylo-27-DS86 9 | 290 3 | CsCsAGfACfAfGfACAAGACCAUCsUs A | 3149 | UsfAsGfAUGGfUCU UGUfCUfGUCUGGsA sU |
| Kylo-27-DS87 0 | 290 4 | CsCsfAGfACfAGfAfCfAAfGAfCCAUCs UsA | 3150 | UsdAsGAdTGdGUCU UdGUfCUGUCUGGs AsU |
| Kylo-27-DS87 1 | 290 5 | CsCsAGACfAGfAfCfAAGACCAUCsUs A | 3151 | AsfGsUUUfGUfUCU UCUfAfUUCAGCsA sC |
| Kylo-27-DS87 2 | 290 6 | CsCsAGACAGfAfCfAAGACCAUCsUsA | 3152 | AsfGsUfUUfGUUCU UfCUfUAfUUfCAfGC sAsC |
| Kylo-27-DS87 3 | 290 7 | CsCsAGACfAGfAfCfAAGACCAUCsUs A | 3153 | AsfGsUUUfGUfUCU UCUfAfUUCAGCsA sC |
| Kylo-27-DS87 4 | 290 8 | CsCsAGfACfAfGfACAAGACCAUCsUs A | 3154 | AsfGsUUUGUfUCUU CUfUAfUUCAGCsAs C |
| Kylo-27-DS87 5 | 290 9 | CsCsfAGfACfAGfAfCfAAfGAfCCAUCs UsA | 3155 | AsdGsUUdTGdTUCU UdCUfUAUUCAGCsA sC |
| Kylo-27-DS87 6 | 291 0 | GsAsGCCGfUUfCfUfCUACAAUUAsCs U | 3156 | AsfGsUAAfUUfGfUA GAGfAAfCGGCUCsG sG |
| Kylo-27-DS87 7 | 291 1 | GsGsAGAAfUUfGfCfUUCAUACAAsA sA | 3157 | UsfUsUUGfUAfUGA AGCfAAfUUCUCCsU sC |
| Kylo-27-DS87 8 | 291 2 | GsGsAGAAUUfGfCfUUCAUACAAsAs A | 3158 | AsfGsUfUUfGUUCU UfCUfUAfUUfCAfGC sAsC |
| Kylo-27-DS87 9 | 291 3 | GsGsAGAAfUUfGfCfUUCAUACAAsA sA | 3159 | AsfGsUUUfGUfUfCU UCUfAfUUCAGCsA sC |
| Kylo-27-DS88 0 | 291 4 | GsGsAGfAAfUfUfGCUUCAUACAAsA sA | 3160 | AsfGUfUUGUfUCUU CUfUAfUUCAGCsAs C |
| Kylo-27-DS88 1 | 291 5 | GsGsfAGfAAfUUfGfCfUUfCAfUACAA sAsA | 3161 | AsdGsUUdTGdTUCU UdCUfUAUUCAGCsA sC |
| Kylo-27-DS88 2 | 291 6 | GsCsUGAGfGAfGfAfAUUGCUUCAsU sA | 3162 | UsfAsUGAfAGfCAAU UCfUCfCUCAGCsAsC |
| Kylo-27-DS88 3 | 291 7 | GsCsUGAGGAfGfAfAUUGCUUCAsUs A | 3163 | UsfAsUfGAfAGCAAU fUCfUCfCUfCAfGCsA sC |
| Kylo-27-DS88 4 | 291 8 | GsCsUGAGfGAfGfAfAUUGCUUCAsU sA | 3164 | UsfAsUGAfAGfCfAA UUCfUCfCUCAGCsAs C |
| Kylo-27-DS88 5 | 291 9 | GsCsUGfAGfGfAfGAAUUGCUUCAsU sA | 3165 | UsfAsUfGAAGfCAAU UCfUCfCUCAGCsAsC |
| Kylo-27-DS88 6 | 292 0 | GsCsfUGfAGfGAfGfAfAUfUGfCUUC AsUsA | 3166 | UsdAsUGdAAdGCAA UdTCfUCCUCAGCsA sC |
| Kylo-27-DS88 7 | 292 1 | GsGsUGUUfGAfCfAfGAUACAUsCsU | 3167 | AsfGsAUGfUAfUCUG UCfAAfCACCsAsU |
| Kylo-27-DS88 8 | 292 2 | GsGsUGUUfGAfCfAfGAUACAUsCsU | 3168 | AsfGsAUGfUAfUCUG UCfAAfCACCsAsU |
| Kylo-27-DS88 9 | 292 3 | GsGsUGUUfGAfCfAfGAUACAUsCsU | 3169 | AsfGsAUGfUAfUCUG UCfAAfCACCsAsU |
| Kylo-27-DS89 0 | 292 4 | GsGsUGUUfGAfCfAfGAUACAUsCsU | 3170 | AsfGsAUGfUAfUCUG UCfAAfCACCsAsU |
| Kylo-27-DS89 1 | 292 5 | GsGsUGUUGAfCfAfGAUACAUsCsU | 3171 | AsfGsAfUGfUAUCUG fUCfAAfCAfCCsfAsU |
| Kylo-27-DS89 2 | 292 6 | GsGsUGUUGAfCfAfGAUACAUsCsU | 3172 | AsfGsAfUGfUAUCUG fUCfAAfCAfCCsfAsU |
| Kylo-27-DS89 3 | 292 7 | GsGsUGUUGAfCfAfGAUACAUsCsU | 3173 | AsfGsAfUGfUAUCUG fUCfAAfCAfCCsfAsU |
| Kylo-27-DS89 4 | 292 8 | GsGsUGUUGAfCfAfGAUACAUsCsU | 3174 | AsfGsAfUGfUAUCUG fUCfAAfCAfCCsfAsU |
| Kylo-27-DS89 5 | 2929 | GsGsUGUUGAfCfAfGAUACAUsCsU | 3175 | AsfGsAfUGfUAUCUG fUCfAAfCAfCCsfAsU |
| Kylo-27-DS89 6 | 2930 | GsGsUGUUGAfCfAfGAUACAUsCsU | 3176 | AsfGsAfUGfUAUCUG fUCfAAfCAfCCsfAsU |
| Kylo-27-DS89 7 | 2931 | GsGsUGUUfGAfCfAfGAUACAUsCsU | 3177 | AsfGsAUGfUAfUfCU GUCfAAfCACCsAsU |
| Kylo-27-DS89 8 | 293 2 | GsGsUGUUfGAfCfAfGAUACAUsCsU | 3178 | AsfGsAUGfUAfUfCU GUCfAAfCACCsAsU |
| Kylo-27-DS89 9 | 2933 | GsGsUGUUfGAfCfAfGAUACAUsCsU | 3179 | AsfGsAUGfUAfUfCU GUCfAAfCACCsAsU |
| Kylo-27-DS90 0 | 2934 | GsGsUGUUfGAfCfAfGAUACAUsCsU | 3180 | AsfGsAUGfUAfUfCU GUCfAAfCACCsAsU |
| Kylo-27-DS90 1 | 293 5 | GsGsUGUUfGAfCfAfGAUACAUsCsU | 3181 | AsfGsAUGfUAfUfCU GUCfAAfCACCsAsU |
| Kylo-27-DS90 2 | 293 6 | GsGsUGUUfGAfCfAfGAUACAUsCsU | 3182 | AsfGsAUGfUAfUfCU GUCfAAfCACCsAsU |
| Kylo-27-DS90 3 | 293 7 | GsGsUGfUUfGfAfCAGAUACAUsCsU | 3183 | AsfGsAUGfUAfUfCU GUCfAAfCACCsAsU |
| Kylo-27-DS90 4 | 293 8 | GsGsUGfUUfGfAfCAGAUACAUsCsU | 3184 | AsfGsAUGfUAfUfCU GUCfAAfCACCsAsU |
| Kylo-27-DS90 5 | 293 9 | GsGsUGfUUfGfAfCAGAUACAUsCsU | 3185 | AsfGsAUGfUAfUfCU GUCfAAfCACCsAsU |
| Kylo-27-DS90 6 | 294 0 | GsGsUGfUUfGfAfCAGAUACAUsCsU | 3186 | AsfGsAUGfUAfUfCU GUCfAAfCACCsAsU |
| Kylo-27-DS90 7 | 294 1 | GsGsUGfUUfGfAfCAGAUACAUsCsU | 3187 | AsfGsAUGfUAfUfCU GUCfAAfCACCsAsU |
| Kylo-27-DS90 8 | 294 2 | GsGsUGfUUfGfAfCAGAUACAUsCsU | 3188 | AsfGsAUGfUAfUfCU GUCfAAfCACCsAsU |
| Kylo-27-DS90 9 | 294 3 | GsGsfUGfUUfGAfCfAfGAfUAfCAUsC sU | 3189 | AsdGsAUdGUdAUC UGdTCfAACACCsAs U |
| Kylo-27-DS91 0 | 294 4 | GsGsfUGfUUfGfAfCAfGAfUAfCAUsC sU | 3190 | AsdGsAUdGUdAUC UGdTCfAACACCsAs U |
| Kylo-27-DS91 1 | 294 5 | GsGsfUGfUUfGfAfCAfGAfUAfCAUsC sU | 3191 | AsdGsAUdGUdAUC UGdTCfAACACCsAs U |
| Kylo-27-DS91 2 | 294 6 | GsGsfUGfUUfGfAfCAfGAfUAfCAUsC sU | 3192 | AsdGsAUdGUdAUC UGdTCfAACACCsAs U |
| Kylo-27-DS91 3 | 294 7 | GsGsfUGfUUfGfAfCAfGAfUAfCAUsC sU | 3193 | AsdGsAUdGUdAUC UGdTCfAACACCsAs U |
| Kylo-27-DS91 4 | 294 8 | GsGsfUGfUUfGfAfCAfGAfUAfCAUsC sU | 3194 | AsdGsAUdGUdAUC UGdTCfAACACCsAs U |
| Kylo-27-DS91 5 | 294 9 | AsGsAAAUfUCfUfAfCUACAUCUAsU sA | 3195 | UsfAsUAGfAUfGUA GUAfGAfAUUUCUsC sU |
| Kylo-27-DS91 6 | 295 0 | AsGsAAAUfUCfUfAfCUACAUCUAsU sA | 3196 | UsfAsUAGfAUfGUA GUAfGAfAUUUCUsC sU |
| Kylo-27-DS91 7 | 295 1 | AsGsAAAUfUCfUfAfCUACAUCUAsU sA | 3197 | UsfAsUAGfAUfGUA GUAfGAfAUUUCUsC sU |
| Kylo-27-DS91 8 | 295 2 | AsGsAAAUfUCfUfAfCUACAUCUAsU sA | 3198 | UsfAsUAGfAUfGUA GUAfGAfAUUUCUsC sU |
| Kylo-27-DS91 9 | 295 3 | AsGsAAAUfUCfUfAfCUACAUCUAsU sA | 3199 | UsfAsUAGfAUfGUA GUAfGAfAUUUCUsC sU |
| Kylo-27-DS92 0 | 295 4 | AsGsAAAUUCfUfAfCUACAUCUAsUs A | 3200 | UsfAsUfAGfAUGUA GfUAfGAfAUfUUfCU sCsU |
| Kylo-27-DS92 1 | 295 5 | AsGsAAAUUCfUfAfCUACAUCUAsUs A | 3201 | UsfAsUfAGfAUGUA GfUAfGAfAUfUUfCU sCsU |
| Kylo-27-DS92 2 | 295 6 | AsGsAAAUUCfUfAfCUACAUCUAsUs A | 3202 | UsfAsUfAGfAUGUA GfUAfGAfAUfUUfCU sCsU |
| Kylo-27-DS92 3 | 295 7 | AsGsAAAUUCfUfAfCUACAUCUAsUs A | 3203 | UsfAsUfAGfAUGUA GfUAfGAfAUfUUfCU sCsU |
| Kylo-27-DS92 4 | 295 8 | AsGsAAAUUCfUfAfCUACAUCUAsUs A | 3204 | UsfAsUfAGfAUGUA GfUAfGAfAUfUUfCU sCsU |
| Kylo-27-DS92 5 | 295 9 | AsGsAAAUUCfUfAfCUACAUCUAsUs A | 3205 | UsfAsUfAGfAUGUA GfUAfGAfAUfUUfCU sCsU |
| Kylo-27-DS92 6 | 296 0 | AsGsAAAUfUCfUfAfCUACAUCUAsU sA | 3206 | UsfAUAGfAUfGfUAG UAfGAfAUUUCUsCs U |
| Kylo-27-DS92 7 | 296 1 | AsGsAAAUfUCfUfAfCUACAUCUAsU sA | 3207 | UsfAUAGfAUfGfUAG UAfGAfAUUUCUsCs U |
| Kylo-27-DS92 8 | 296 2 | AsGsAAAUfUCfUfAfCUACAUCUAsU sA | 3208 | UsfAUAGfAUfGfUAG UAfGAfAUUUCUsCs U |
| Kylo-27-DS92 9 | 296 3 | AsGsAAAUfUCfUfAfCUACAUCUAsU sA | 3209 | UsfAUAGfAUfGfUAG UAfGAfAUUUCUsCs U |
| Kylo-27-DS93 0 | 296 4 | AsGsAAAUfUCfUfAfCUACAUCUAsU sA | 3210 | UsfAUAGfAUfGfUAG UAfGAfAUUUCUsCs U |
| Kylo-27-DS93 1 | 296 5 | AsGsAAAUfUCfUfAfCUACAUCUAsU sA | 3211 | UsfAUAGfAUfGfUAG UAfGAfAUUUCUsCs U |
| Kylo-27-DS93 2 | 296 6 | AsGsAAfAUfUfCfUACUACAUCUAsU sA | 3212 | UsfAsUfAGAUfGUA GUAfGAfAUUUCUsC sU |
| Kylo-27-DS93 3 | 296 7 | AsGsAAfAUfUfCfUACUACAUCUAsU sA | 3213 | UsfAsUfAGAUfGUA GUAfGAfAUUUCUsC sU |
| Kylo-27-DS93 4 | 296 8 | AsGsAAfAUfUfCfUACUACAUCUAsU sA | 3214 | UsfAsUfAGAUfGUA GUAfGAfAUUUCUsC sU |
| Kylo-27-DS93 5 | 296 9 | AsGsAAfAUfUfCfUACUACAUCUAsU sA | 3215 | UsfAsUfAGAUfGUA GUAfGAfAUUUCUsC sU |
| Kylo-27-DS93 6 | 297 0 | AsGsAAfAUfUfCfUACUACAUCUAsU sA | 3216 | UsfAsUfAGAUfGUA GUAfGAfAUUUCUsC sU' |
| Kylo-27-DS93 7 | 297 1 | AsGsAAfAUfUfCfUACUACAUCUAsU sA | 3217 | UsfAsUfAGAUfGUA GUAfGAfAUUUCUsC sU |
| Kylo-27-DS93 8 | 297 2 | AsGsfAAfAUfUCfUfAfCUfACfAUCUA sUsA | 3218 | UsdAsUAdGAdTGUA GdTAfGAAUUUCUsC sU |
| Kylo-27-DS93 9 | 297 3 | AsGsfAAfAUfUfCfUAfCUfACfAUCUA sUsA | 3219 | UsdAsUAdGAdTGUA GdTAfGAAUUUCUsC sU |
| Kylo-27-DS94 0 | 297 4 | AsGsfAAfAUfUfCfUAfCUfACfAUCUA sUsA | 3220 | UsdAsUAdGAdTGUA GdTAfGAAUUUCUsC sU |
| Kylo-27-DS94 1 | 297 5 | AsGsfAAfAUfUfCfUAfCUfACfAUCUA sUsA | 3221 | UsdAsUAdGAdTGUA GdTAfGAAUUUCUsC sU |
| Kylo-27-DS94 2 | 297 6 | AsGsfAAfAUfUfCfUAfCUfACfAUCUA sUsA | 3222 | UsdAsUAdGAdTGUA GdTAfGAAUUUCUsC sU |
| Kylo-27-DS94 3 | 297 7 | AsGsfAAfAUfUfCfUAfCUfACfAUCUA sUsA | 3223 | UsdAsUAdGAdTGUA GdTAfGAAUUUCUsC sU |
| Kylo-27-DS94 4 | 297 8 | GsGsAGCCfUAfCfAfGAGAAAUUCsU sA | 3224 | UsfAsGAAfUUfUCUC UGfUAfGGCUCCsAs C |
| Kylo-27-DS94 5 | 297 9 | GsGsAGCCfUAfCfAfGAGAAAUUCsU sA | 3225 | UsfAsGAAfUUfUCUC UGfUAfGGCUCCsAs C |
| Kylo-27-DS94 6 | 298 0 | GsGsAGCCfUAfCfAfGAGAAAUUCsU sA | 3226 | UsfAsGAAfUUfUCUC UGfUAfGGCUCCsAs C |
| Kylo-27-DS94 7 | 298 1 | GsGsAGCCfUAfCfAfGAGAAAUUCsU sA | 3227 | UsfAsGAAfUUfUCUC UGfUAfGGCUCCsAs C |
| Kylo-27-DS94 8 | 298 2 | GsGsAGCCfUAfCfAfGAGAAAUUCsU sA | 3228 | UsfAsGAAfUUfUCUC UGfUAfGGCUCCsAs C |
| Kylo-27-DS94 9 | 298 3 | GsGsAGCCfUAfCfAfGAGAAAUUCsU sA | 3229 | UsfAsGAAfUUfUCUC UGfUAfGGCUCCsAs C |
| Kylo-27-DS95 0 | 298 4 | GsGsAGCCfUAfCfAfGAGAAAUUCsU sA | 3230 | UsfAsGAAfUUfUCUC UGfUAfGGCUCCsAs C |
| Kylo-27-DS95 1 | 298 5 | GsGsAGCCfUAfCfAfGAGAAAUUCsU sA | 3231 | UsfAsGAAfUUfUCUC UGfUAfGGCUCCsAs C |
| Kylo-27-DS95 2 | 298 6 | GsGsAGCCfUAfCfAfGAGAAAUUCsU sA | 3232 | UsfAsGAAfUUfUCUC UGfUAfGGCUCCsAs C |
| Kylo-27-DS95 3 | 298 7 | GsGsAGCCfUAfCfAfGAGAAAUUCsU sA | 3233 | UsfAsGAAfUUfUCUC UGfUAfGGCUCCsAs C |
| Kylo-27-DS95 4 | 298 8 | GsGsAGCCUAfCfAfGAGAAAUUCsUs A | 3234 | UsfAsGfAAfUUUCUC fUGfUAfGGfCUfCCs AsC |
| Kylo-27-DS95 5 | 298 9 | GsGsAGCCUAfCfAfGAGAAAUUCsUs A | 3235 | UsfAsGfAAfUUUCUC fUGfUAfGGfCUfCCs AsC |
| Kylo-27-DS95 6 | 299 0 | GsGsAGCCUAfCfAfGAGAAAUUCsUs A | 3236 | UsfAsGfAAfUUUCUC fUGfUAfGGfCUfCCs AsC |
| Kylo-27-DS95 7 | 299 1 | GsGsAGCCUAfCfAfGAGAAAUUCsUs A | 3237 | UsfAsGfAAfUUUCUC fUGfUAfGGfCUfCCs AsC |
| Kylo-27-DS95 8 | 299 2 | GsGsAGCCUAfCfAfGAGAAAUUCsUs A | 3238 | UsfAsGfAAfUUUCUC fUGfUAfGGfCUfCCs AsC |
| Kylo-27-DS95 9 | 299 3 | GsGsAGCCUAfCfAfGAGAAAUUCsUs A | 3239 | UsfAsGfAAfUUUCUC fUGfUAfGGfCUfCCs AsC |
| Kylo-27-DS96 0 | 299 4 | GsGsAGCCfUAfCfAfGAGAAAUUCsU sA | 3240 | UsfAsGAAfUUfUfCU CUGfUAfGGCUCCsA sC |
| Kylo-27-DS96 1 | 299 5 | GsGsAGCCfUAfCfAfGAGAAAUUCsU sA | 3241 | UsfAsGAAfUUfUfCU CUGfUAfGGCUCCsA sC |
| Kylo-27-DS96 2 | 299 6 | GsGsAGCCfUAfCfAfGAGAAAUUCsU sA | 3242 | UsfAsGAAfUUfUfCU CUGfUAfGGCUCCsA sC |
| Kylo-27-DS96 3 | 299 7 | GsGsAGCCfUAfCfAfGAGAAAUUCsU sA | 3243 | UsfAsGAAfUUfUfCU CUGfUAfGGCUCCsA sC |
| Kylo-27-DS96 4 | 299 8 | GsGsAGCCfUAfCfAfGAGAAAUUCsU sA | 3244 | UsfAsGAAfUUfUfCU CUGfUAfGGCUCCsA sC |
| Kylo-27-DS96 5 | 299 9 | GsGsAGCCfUAfCfAfGAGAAAUUCsU sA | 3245 | UsfAsGAAfUUfUfCU CUGfUAfGGCUCCsA sC |
| Kylo-27-DS96 6 | 300 0 | GsGsAGCCfUAfCfAfGAGAAAUUCsU sA | 3246 | UsfAsGAAUUfUCUC UGfUAfGGCUCCsAs C |
| Kylo-27-DS96 7 | 300 1 | GsGsAGCCfUAfCfAfGAGAAAUUCsU sA | 3247 | UsfAsGAAUUfUCUC UGfUAfGGCUCCsAs C |
| Kylo-27-DS96 8 | 300 2 | GsGsAGCCfUAfCfAfGAGAAAUUCsU sA | 3248 | UsfAsGAAUUfUCUC UGfUAfGGCUCCsAs C |
| Kylo-27-DS96 9 | 300 3 | GsGsAGCCfUAfCfAfGAGAAAUUCsU sA | 3249 | UsfAsGAAUUfUCUC UGfUAfGGCUCCsAs C |
| Kylo-27-DS97 0 | 300 4 | GsGsAGCCUAfCfAfGAGAAAUUCsUs A | 3250 | UsfAsGAAUUfUCUC UGfUAfGGCUCCsAs C |
| Kylo-27-DS97 1 | 300 5 | GsGsAGCCUAfCfAfGAGAAAUUCsUs A | 3251 | UsfAsGAAUUfUCUC UGfUAfGGCUCCsAs C |
| Kylo-27-DS97 2 | 300 6 | GsGsAGCCUAfCfAfGAGAAAUUCsUs A | 3252 | UsdAsGAdAUdTUCU CdTGfUAGGCUCCsA sC |
| Kylo-27-DS97 3 | 300 7 | GsGsAGCCUAfCfAfGAGAAAUUCsUs A | 3253 | UsdAsGAdAUdTUCU CdTGfUAGGCUCCsA sC |
| Kylo-27-DS97 4 | 300 8 | GsGsAGCCUAfCfAfGAGAAAUUCsUs A | 3254 | UsdAsGAdAUdTUCU CdTGfUAGGCUCCsA sC |
| Kylo-27-DS97 5 | 300 9 | GsGsAGCCUAfCfAfGAGAAAUUCsUs A | 3255 | UsdAsGAdAUdTUCU CdTGfUAGGCUCCsA sC |
| Kylo-27-DS97 6 | 301 0 | GsGsAGfCCfUfAfCAGAGAAAUUCsU sA | 3256 | UsdAsGAdAUdTUCU CdTGfUAGGCUCCsA sC |
| Kylo-27-DS97 7 | 301 1 | GsGsAGfCCfUfAfCAGAGAAAUUCsU sA | 3257 | UsdAsGAdAUdTUCU CdTGfUAGGCUCCsA sC |
| Kylo-27-DS97 8 | 301 2 | GsGsAGfCCfUfAfCAGAGAAAUUCsU sA | 3258 | AsfGsUAAfUUfGfUA GAGfAAfCGGCUCsG sG |
| Kylo-27-DS97 9 | 301 3 | GsGsAGfCCfUfAfCAGAGAAAUUCsU sA | 3259 | AsfGsUAAfUUfGfUA GAGfAAfCGGCUCsG sG |
| Kylo-27-DS98 0 | 301 4 | GsGsAGfCCfUfAfCAGAGAAAUUCsU sA | 3260 | AsfGsUAAfUUfGfUA GAGfAAfCGGCUCsG sG |
| Kylo-27-DS98 1 | 301 5 | GsGsAGfCCfUfAfCAGAGAAAUUCsU sA | 3261 | AsfGsUAAfUUfGfUA GAGfAAfCGGCUCsG sG |
| Kylo-27-DS98 2 | 301 6 | GsGsfAGfCCfUAfCfAfGAfGAfAAUUC sUsA | 3262 | AsfGsUAAfUUfGfUA GAGfAAfCGGCUCsG sG |
| Kylo-27-DS98 3 | 301 7 | GsGsfAGfCCfUfAfCAfGAfGAfAAUUC sUsA | 3263 | AsfGsUAAfUUfGfUA GAGfAAfCGGCUCsG sG |
| Kylo-27-DS98 4 | 301 8 | GsGsfAGfCCfUfAfCAfGAfGAfAAUUC sUsA | 3264 | AsfGsUAAfUUfGfUA GAGfAAfCGGCUCsG sG |
| Kylo-27-DS98 5 | 301 9 | GsGsfAGfCCfUfAfCAfGAfGAfAAUUC sUsA | 3265 | AsfGsUAAfUUfGfUA GAGfAAfCGGCUCsG sG |
| Kylo-27-DS98 6 | 302 0 | GsGsfAGfCCfUfAfCAfGAfGAfAAUUC sUsA | 3266 | AsfGsUAAfUUfGfUA GAGfAAfCGGCUCsG sG |
| Kylo-27-DS98 7 | 302 1 | GsGsfAGfCCfUfAfCAfGAfGAfAAUUC sUsA | 3267 | AsfGsUAAfUUfGfUA GAGfAAfCGGCUCsG sG |
| Kylo-27-DS98 8 | 302 2 | GsAsGCCGfUUfCfUfCUACAAUUAsCs U | 3268 | AsfGsUfAAfUUGUA GfAGfAAfCGfGCfUCs GsG |
| Kylo-27-DS98 9 | 302 3 | CsCsAGACfAGfAfCfAAGACCAUCsUs A | 3269 | AsfGsUfAAfUUGUA GfAGfAAfCGfGCfUCs GsG |
| Kylo-27-DS99 0 | 302 4 | CsCsAGACfAGfAfCfAAGACCAUCsUs A | 3270 | AsfGsUfAAfUUGUA GfAGfAAfCGfGCfUCs GsG |
| Kylo-27-DS99 1 | 302 5 | CsCsAGACfAGfAfCfAAGACCAUCsUs A | 3271 | AsfGsUfAAfUUGUA GfAGfAAfCGfGCfUCs GsG |
| Kylo-27-DS99 2 | 302 6 | CsCsAGACfAGfAfCfAAGACCAUCsUs A | 3272 | AsfGsUfAAfUUGUA GfAGfAAfCGfGCfUCs GsG |
| Kylo-27-DS99 3 | 302 7 | CsCsAGACfAGfAfCfAAGACCAUCsUs A | 3273 | UsfAsGAUfGGfUCU UGUfCUfGUCUGGsA sU |
| Kylo-27-DS99 4 | 302 8 | GsAsGCCGfUUfCfUfCUACAAUUAsCs U | 3274 | AsfGsUAAfUUfGfUA GAGfAAfCGGCUCsG sG |
| Kylo-27-DS99 5 | 302 9 | CsCsAGACfAGfAfCfAAGACCAUCsUs A | 3275 | AsfGsUAAfUUfGfUA GAGfAAfCGGCUCsG sG |
| Kylo-27-DS99 6 | 303 0 | CsCsAGACfAGfAfCfAAGACCAUCsUs A | 3276 | AsfGsUAAfUUfGfUA GAGfAAfCGGCUCsG sG |
| Kylo-27-DS99 7 | 303 1 | CsCsAGACfAGfAfCfAAGACCAUCsUs A | 3277 | AsfGsUAAfUUfGfUA GAGfAAfCGGCUCsG sG |
| Kylo-27-DS99 8 | 303 2 | CsCsAGACfAGfAfCfAAGACCAUCsUs A | 3278 | AsfGsUAAfUUfGfUA GAGfAAfCGGCUCsG sG |
| Kylo-27-DS99 9 | 303 3 | CsCsAGACfAGfAfCfAAGACCAUCsUs A | 3279 | AsfGsUAAfUUfGfUA GAGfAAfCGGCUCsG sG |
| Kylo-27-DS10 00 | 303 4 | GsAsGCfCGfUfUfCUCUACAAUUAsCs U | 3280 | AsfGsUfAAUUfGUA GAGfAAfCGGCUCsG sG |
| Kylo-27-DS10 01 | 303 5 | GsAsGCfCGfUfUfCUCUACAAUUAsCs U | 3281 | AsfGsUfAAUUfGUA GAGfAAfCGGCUCsG sG |
| Kylo-27-DS10 02 | 303 6 | GsAsGCfCGfUfUfCUCUACAAUUAsCs U | 3282 | AsfGsUfAAUUfGUA GAGfAAfCGGCUCsG sG |
| Kylo-27-DS10 03 | 303 7 | GsAsGCfCGfUfUfCUCUACAAUUAsCs U | 3283 | AsfGsUfAAUUfGUA GAGfAAfCGGCUCsG sG |
| Kylo-27-DS10 04 | 303 8 | GsAsGCfCGfUfUfCUCUACAAUUAsCs U | 3284 | AsfGsUfAAUUfGUA GAGfAAfCGGCUCsG sG |
| Kylo-27-DS10 05 | 303 9 | GsAsGCfCGfUfUfCUCUACAAUUAsCs U | 3285 | AsfGsUfAAUUfGUA GAGfAAfCGGCUCsG sG |
| Kylo-27-DS10 06 | 304 0 | GsAsfGCfCGfUUfCfUfCUfACfAAUUA sCsU | 3286 | AsdGsUAdAUdTGUA GdAGfAACGGCUCsG sG |
| Kylo-27-DS10 07 | 304 1 | GsAsGCfCGfUfUfCUCUACAAUUAsCs U | 3287 | AsdGsUAdAUdTGUA GdAGfAACGGCUCsG sG |
| Kylo-27-DS10 08 | 304 2 | GsAsGCfCGfUfUfCUCUACAAUUAsCs U | 3288 | AsdGsUAdAUdTGUA GdAGfAACGGCUCsG sG |
| Kylo-27-DS10 09 | 304 3 | GsAsGCfCGfUfUfCUCUACAAUUAsCs U | 3289 | AsdGsUAdAUdTGUA GdAGfAACGGCUCsG sG |
| Kylo-27-DS10 10 | 304 4 | GsAsGCfCGfUfUfCUCUACAAUUAsCs U | 3290 | AsdGsUAdAUdTGUA GdAGfAACGGCUCsG sG |
| Kylo-27-DS10 11 | 304 5 | GsAsGCfCGfUfUfCUCUACAAUUAsCs U | 3291 | AsdGsUAdAUdTGUA GdAGfAACGGCUCsG sG |
| Kylo-27-DS10 12 | 304 6 | GsGsAGAAfUUfGfCfUUCAUACAAsA sA | 3292 | UsfUsUUGfUAfUGA AGCfAAfUUCUCCsU sC |
| Kylo-27-DS10 13 | 304 7 | GsGsAGAAfUUfGfCfUUCAUACAAsA sA | 3293 | UsfUsUUGfUAfUGA AGCfAAfUUCUCCsU sC |
| Kylo-27-DS10 14 | 304 8 | GsGsAGAAfUUfGfCfUUCAUACAAsA sA | 3294 | UsfUsUUGfUAfUGA AGCfAAfUUCUCCsU sC |
| Kylo-27-DS10 15 | 304 9 | GsGsAGAAfUUfGfCfUUCAUACAAsA sA | 3295 | UsfUsUUGfUAfUGA AGCfAAfUUCUCCsU sC |
| Kylo-27-DS10 16 | 305 0 | GsGsAGAAfUUfGfCfUUCAUACAAsA sA | 3296 | UsfUsUUGfUAfUGA AGCfAAfUUCUCCsU sC |
| Kylo-27-DS10 17 | 305 1 | GsGsAGAAfUUfGfCfUUCAUACAAsA sA | 3297 | UsfUsUUGfUAfUGA AGCfAAfUUCUCCsU sC |
| Kylo-27-DS10 18 | 305 2 | GsGsAGAAfUUfGfCfUUCAUACAAsA sA | 3298 | UsfUsUUGfUAfUGA AGCfAAfUUCUCCsU sC |
| Kylo-27-DS10 19 | 305 3 | GsGsAGAAfUUfGfCfUUCAUACAAsA sA | 3299 | UsfUsUUGfUAfUGA AGCfAAfUUCUCCsU sC |
| Kylo-27-DS10 20 | 305 4 | GsGsAGAAfUUfGfCfUUCAUACAAsA sA | 3300 | UsfUsUUGfUAfUGA AGCfAAfUUCUCCsU sC |
| Kylo-27-DS10 21 | 305 5 | GsGsAGAAfUUfGfCfUUCAUACAAsA sA | 3301 | UsfUsUUGfUAfUGA AGCfAAfUUCUCCsU sC |
| Kylo-27-DS10 22 | 305 6 | GsGsAGAAUUfGfCfUUCAUACAAsAs A | 3302 | AsfGsUfUUfGUUCU UfCUfUAfUUfCAfGC sAsC |
| Kylo-27-DS10 23 | 305 7 | GsGsAGAAUUfGfCfUUCAUACAAsAs A | 3303 | AsfGsUfUUfGUUCU UfCUfUAfUUfCAfGC sAsC |
| Kylo-27-DS10 24 | 305 8 | GsGsAGAAUUfGfCfUUCAUACAAsAs A | 3304 | AsfGsUfUUfGUUCU UfCUfUAfUUfCAfGC sAsC |
| Kylo-27-DS10 25 | 305 9 | GsGsAGAAUUfGfCfUUCAUACAAsAs A | 3305 | AsfGsUfUUfGUUCU UfCUfUAfUUfCAfGC sAsC |
| Kylo-27-DS10 26 | 306 0 | GsGsAGAAUUfGfCfUUCAUACAAsAs A | 3306 | AsfGsUfUUfGUUCU UfCUfUAfUUfCAfGC sAsC |
| Kylo-27-DS10 27 | 306 1 | GsGsAGAAUUfGfCfUUCAUACAAsAs A | 3307 | AsfGsUfUUfGUUCU UfCUfUAfUUfCAfGC sAsC |
| Kylo-27-DS10 28 | 306 2 | GsGsAGAAfUUfGfCfUUCAUACAAsA sA | 3308 | AsfGsUUUfGUfUfCU UCUfUAfUUCAGCsA sC |
| Kylo-27-DS10 29 | 306 3 | GsGsAGAAfUUfGfCfUUCAUACAAsA sA | 3309 | AsfGsUUUfGUfUfCU UCUfUAfUUCAGCsA sC |
| Kylo-27-DS10 30 | 306 4 | GsGsAGAAfUUfGfCfUUCAUACAAsA sA | 3310 | AsfGsUUUfGUfUfCU UCUfUAfUUCAGCsA sC |
| Kylo-27-DS10 31 | 306 5 | GsGsAGAAfUUfGfCfUUCAUACAAsA sA | 3311 | AsfGsUUUfGUfUfCU UCUfUAfUUCAGCsA sC |
| Kylo-27-DS10 32 | 306 6 | GsGsAGAAfUUfGfCfUUCAUACAAsA sA | 3312 | AsfGsUUUfGUfUfCU UCUfUAfUUCAGCsA sC |
| Kylo-27-DS10 33 | 306 7 | GsGsAGAAfUUfGfCfUUCAUACAAsA sA | 3313 | AsfGsUUUfGUfUfCU UCUfUAfUUCAGCsA sC |
| Kylo-27-DS10 34 | 306 8 | GsGsAGAAfUUfGfCfUUCAUACAAsA sA | 3314 | AsfGUfUUGUfUCUU CUfUAfUUCAGCsAs C |
| Kylo-27-DS10 35 | 306 9 | GsGsAGAAfUUfGfCfUUCAUACAAsA sA | 3315 | AsfGUfUUGUfUCUU CUfUAfUUCAGCsAs C |
| Kylo-27-DS10 36 | 307 0 | GsGsAGAAfUUfGfCfUUCAUACAAsA sA | 3316 | AsfGUfUUGUfUCUU CUfUAfUUCAGCsAs C |
| Kylo-27-DS10 37 | 307 1 | GsGsAGAAfUUfGfCfUUCAUACAAsA sA | 3317 | AsfGUfUUGUfUCUU CUfUAfUUCAGCsAs C |
| Kylo-27-DS10 38 | 307 2 | GsGsAGAAUUfGfCfUUCAUACAAsAs A | 3318 | AsfGUfUUGUfUCUU CUfUAfUUCAGCsAs C |
| Kylo-27-DS10 39 | 307 3 | GsGsAGAAUUfGfCfUUCAUACAAsAs A | 3319 | AsfGUfUUGUfUCUU CUfUAfUUCAGCsAs C |
| Kylo-27-DS10 40 | 307 4 | GsGsAGAAUUfGfCfUUCAUACAAsAs A | 3320 | AsdGsUUdTGdTUCU UdCUfUAUUCAGCsA sC |
| Kylo-27-DS10 41 | 307 5 | GsGsAGAAUUfGfCfUUCAUACAAsAs A | 3321 | AsdGsUUdTGdTUCU UdCUfUAUUCAGCsA sC |
| Kylo-27-DS10 42 | 307 6 | GsGsAGAAUUfGfCfUUCAUACAAsAs A | 3322 | AsdGsUUdTGdTUCU UdCUfUAUUCAGCsA sC |
| Kylo-27-DS10 43 | 307 7 | GsGsAGAAUUfGfCfUUCAUACAAsAs A | 3323 | AsdGsUUdTGdTUCU UdCUfUAUUCAGCsA sC |
| Kylo-27-DS10 44 | 307 8 | GsGsAGfAAfUfUfGCUUCAUACAAsA sA | 3324 | AsdGsUUdTGdTUCU UdCUfUAUUCAGCsA sC |
| Kylo-27-DS10 45 | 307 9 | GsGsAGfAAfUfUfGCUUCAUACAAsA sA | 3325 | AsdGsUUdTGdTUCU UdCUfUAUUCAGCsA sC |
| Kylo-27-DS10 46 | 308 0 | GsGsAGfAAfUfUfGCUUCAUACAAsA sA | 3326 | UsfAsUGAfAGfCAAU UCfUCfCUCAGCsAsC |
| Kylo-27-DS10 47 | 308 1 | GsGsfAGfAAfUfUfGCfUUfCAfUACAA sAsA | 3327 | UsfAsUGAfAGfCAAU UCfUCfCUCAGCsAsC |
| Kylo-27-DS10 48 | 308 2 | GsGsfAGfAAfUfUfGCfUUfCAfUACAA sAsA | 3328 | UsfAsUGAfAGfCAAU UCfUCfCUCAGCsAsC |
| Kylo-27-DS10 49 | 308 3 | GsGsfAGfAAfUfUfGCfUUfCAfUACAA sAsA | 3329 | UsfAsUGAfAGfCAAU UCfUCfCUCAGCsAsC |
| Kylo-27-DS10 50 | 308 4 | GsGsfAGfAAfUfUfGCfUUfCAfUACAA sAsA | 3330 | UsfAsUGAfAGfCAAU UCfUCfCUCAGCsAsC |
| Kylo-27-DS10 51 | 308 5 | GsCsUGAGfGAfGfAfAUUGCUUCAsU sA | 3331 | UsfAsUfGAfAGCAAU fUCfUCfCUfCAfGCsA sC |
| Kylo-27-DS10 52 | 308 6 | GsCsUGAGfGAfGfAfAUUGCUUCAsU sA | 3332 | UsfAsUfGAfAGCAAU fUCfUCfCUfCAfGCsA sC |
| Kylo-27-DS10 53 | 308 7 | GsCsUGAGfGAfGfAfAUUGCUUCAsU sA | 3333 | UsfAsUfGAfAGCAAU fUCfUCfCUfCAfGCsA sC |
| Kylo-27-DS10 54 | 308 8 | GsCsUGAGfGAfGfAfAUUGCUUCAsU sA | 3334 | UsfAsUfGAfAGCAAU fUCfUCfCUfCAfGCsA sC |
| Kylo-27-DS10 55 | 308 9 | GsCsUGAGfGAfGfAfAUUGCUUCAsU sA | 3335 | UsfAsUfGAfAGCAAU fUCfUCfCUfCAfGCsA sC |
| Kylo-27-DS10 56 | 309 0 | GsCsUGAGfGAfGfAfAUUGCUUCAsU sA | 3336 | UsfAsUfGAfAGCAAU fUCfUCfCUfCAfGCsA sC |
| Kylo-27-DS10 57 | 309 1 | GsCsUGAGfGAfGfAfAUUGCUUCAsU sA | 3337 | UsfAsUGAfAGfCfAA UUCfUCfCUCAGCsAs C |
| Kylo-27-DS10 58 | 309 2 | GsCsUGAGfGAfGfAfAUUGCUUCAsU sA | 3338 | UsfAsUGAfAGfCfAA UUCfUCfCUCAGCsAs C |
| Kylo-27-DS10 59 | 309 3 | GsCsUGAGfGAfGfAfAUUGCUUCAsU sA | 3339 | UsfAsUGAfAGfCfAA UUCfUCfCUCAGCsAs C |
| Kylo-27-DS10 60 | 309 4 | GsCsUGAGfGAfGfAfAUUGCUUCAsU sA | 3340 | UsfAsUGAfAGfCfAA UUCfUCfCUCAGCsAs C |
| Kylo-27-DS10 61 | 309 5 | GsCsUGAGfGAfGfAfAUUGCUUCAsU sA | 3341 | UsfAsUGAfAGfCfAA UUCfUCfCUCAGCsAs C |
| Kylo-27-DS10 62 | 309 6 | GsCsUGAGfGAfGfAfAUUGCUUCAsU sA | 3342 | UsfAsUGAfAGfCfAA UUCfUCfCUCAGCsAs C |
| Kylo-27-DS10 63 | 309 7 | GsCsUGAGfGAfGfAfAUUGCUUCAsU sA | 3343 | UsfAsUfGAAGfCAAU UCfUCfCUCAGCsAsC |
| Kylo-27-DS10 64 | 309 8 | GsCsUGAGfGAfGfAfAUUGCUUCAsU sA | 3344 | UsfAsUfGAAGfCAAU UCfUCfCUCAGCsAsC |
| Kylo-27-DS10 65 | 309 9 | GsCsUGAGfGAfGfAfAUUGCUUCAsU sA | 3345 | UsfAsUfGAAGfCAAU UCfUCfCUCAGCsAsC |
| Kylo-27-DS10 66 | 310 0 | GsCsUGAGfGAfGfAfAUUGCUUCAsU sA | 3346 | UsfAsUfGAAGfCAAU UCfUCfCUCAGCsAsC |
| Kylo-27-DS10 67 | 310 1 | GsCsUGAGGAfGfAfAUUGCUUCAsUs A | 3347 | UsfAsUfGAAGfCAAU UCfUCfCUCAGCsAsC |
| Kylo-27-DS10 68 | 310 2 | GsCsUGAGGAfGfAfAUUGCUUCAsUs A | 3348 | UsfAsUfGAAGfCAAU UCfUCfCUCAGCsAsC |
| Kylo-27-DS10 69 | 310 3 | GsCsUGAGGAfGfAfAUUGCUUCAsUs A | 3349 | UsdAsUGdAAdGCAA UdTCfUCCUCAGCsA sC |
| Kylo-27-DS10 70 | 310 4 | GsCsUGAGGAfGfAfAUUGCUUCAsUs A | 3350 | UsdAsUGdAAdGCAA UdTCfUCCUCAGCsA sC |
| Kylo-27-DS10 71 | 310 5 | GsCsUGAGGAfGfAfAUUGCUUCAsUs A | 3351 | UsdAsUGdAAdGCAA UdTCfUCCUCAGCsA sC |
| Kylo-27-DS10 72 | 310 6 | GsCsUGAGGAfGfAfAUUGCUUCAsUs A | 3352 | UsdAsUGdAAdGCAA UdTCfUCCUCAGCsA sC |
| Kylo-27-DS10 73 | 310 7 | GsCsUGfAGfGfAfGAAUUGCUUCAsU sA | 3353 | UsdAsUGdAAdGCAA UdTCfUCCUCAGCsA sC |
| Kylo-27-DS10 74 | 310 8 | GsCsUGfAGfGfAfGAAUUGCUUCAsU sA | 3354 | UsdAsUGdAAdGCAA UdTCfUCCUCAGCsA sC |

wherein G = 2'-O-methylguanosine monophosphate, A = 2'-O-methyladenosine monophosphate, U = 2'-O-methyluridine monophosphate, C = 2'-O-methylcytidine monophosphate; Gs = 2'-O-methyl-3'-phosphorothioguanosine monophosphate, As = 2'-O-methyl-3'-phosphorothioadenosine monophosphate, Us = 2'-O-methyl-3'-phosphorothiouridine monophosphate, Ts = 2'-O-methyl-3'-phosphorothiothymidine monophosphate, Cs = 2'-O-methyl-3'-phosphorothiocytidine monophosphate; fG = 2'-fluoroguanosine monophosphate, fA = 2'-fluoroadenosine monophosphate, fU = 2'-fluorouridine monophosphate, fC = 2'-fluorocytidine monophosphate, fT = 2'-fluorothymidine monophosphate; fGs = 2'-fluoro-3'-phosphorothioguanosine monophosphate, fAs = 2'-fluoro-3'-phosphorothioadenosine monophosphate, fUs = 2'-fluoro-3'-phosphorothiouridine monophosphate, fCs = 2'-fluoro-3'-phosphorothiocytidine monophosphate, T = deoxythymidine monophosphate, dA = 2'-deoxyadenosine monophosphate, invAb = inverted deoxy abasic cap, Tgn = triethylene glycol deoxythymidine monophosphate.

In some embodiments, the sense strand or antisense strand of the RNA inhibitor of the present invention is a sequence having at least 15 identical consecutive nucleotides as the sense strand or antisense strand in Tables 1 to 2, or a sequence differing by one, two or three nucleotides therefrom.

In some embodiments, the distribution, targeting or stability of the RNA inhibitor is modified by incorporating a ligand for a target tissue receptor into the carrier. For example, a specific ligand may provide enhanced affinity for a selected target (e.g., a molecule, cell or cell type, compartment (e.g., cellular or organelle), body tissue, organ or region) as compared to a species without the ligand.

Ligands may include naturally occurring substances such as proteins (e.g., human serum albumin (HSA), low-density lipoprotein (LDL) or globulin); carbohydrates (e.g., dextran, pullulan, chitin, chitosan, inulin, cyclodextrin, N-acetylglucosamine, N-acetylgalactosamine or hyaluronic acid); or lipids. Ligands may also be recombinant or synthetic molecules, such as synthetic polymers, e.g., synthetic polyamino acids.

Ligands may also include targeting groups, e.g., cell or tissue targeting agents that bind to specified cell types such as kidney cells, e.g., lectins, glycoproteins, lipids or proteins, e.g., antibodies. Targeting groups may be thyroid-stimulating hormone, melanocortin, lectins, glycoproteins, surfactant protein A, mucin carbohydrates, multivalent lactose, multivalent galactose, N-acetylgalactosamine, N-acetylglucosamine, multivalent mannose, multivalent fucose, glycosylated polyamino acids, multivalent galactose, transferrin, bisphosphonates, polyglutamic acid, polyaspartic acid, lipids, cholesterol, steroids, cholic acid, folic acid, vitamin B12, vitamin A, biotin, or RGD peptides or RGD peptide mimetics. In some embodiments, the ligand is multivalent galactose, e.g., N-acetylgalactosamine.

The sense strand and antisense strand comprised in the RNA inhibitor of the present invention can be conveniently and routinely prepared by well-known techniques of solid-phase synthesis. Any other methods known in the art for such synthesis, such as liquid-phase synthesis or fermentation, may additionally or alternatively be used.

In some embodiments, in addition to commercially available and routinely used standard nucleoside phosphoramidite monomers and non-standard nucleoside phosphoramidite monomers, the sense strand and antisense strand comprised in the RNA inhibitor of the present application can be synthesized using the phosphoramidite method derived from carrier-nucleoside phosphoramidite monomers with an automated synthesizer.

In some embodiments, the ligand of the present invention is coupled to the 5'-end and/or 3'-end of the antisense strand, and/or the 5'-end and/or 3'-end of the sense strand via a carrier structure.

For example, the carrier structure may be coupled to the 5'-end and/or 3'-end of the sense strand; or the carrier structure may be coupled to the 5'-end of the antisense strand and the carrier structure is coupled to the 3'-end of the sense strand; or the carrier structure may be coupled to the 3'-end of the antisense strand, and the ligand is coupled to the 5'-end of the sense strand.

In some embodiments, the carrier structure comprises 5'MVIP and 3'MVIP, wherein the 5'MVIP is coupled to the 5'-end of the sense strand and/or the antisense strand, the 3'MVIP is coupled to the 3'-end of the antisense strand and/or the sense strand, the structure of the 5'MVIP is shown in Formula I, and the structure of the 3'MVIP is shown in Formula II.

**(X-L)ₙ-B-D-R₁-,** I

**(X-L)ₘ-B-D-R₂-,** II

Wherein
X is a liver-targeting specific ligand;
L is a branching chain;B is a linker;
D is a linking chain;
R₁ and R₂ are junction points;

The 5'MVIP is connected to the 5'-end of the sense strand or the 5'-end of the antisense strand via the junction point R₁, the 3'MVIP is connected to the 3'-end of the sense strand or the 3'-end of the antisense strand via the junction point R₂, n and m are each independently any integer from 0 to 4, and n+m is an integer from 2 to 6, preferably n+m = 2, 3 or 4, more preferably 4.

In some embodiments, the connection between R₁ or R₂ and the sense strand or antisense strand is via a phosphate ester or a modified phosphate ester, and R₁ or R₂ is preferably connected to the sense strand or antisense strand via a phosphate ester or a phosphorothioate ester.

In some embodiments, m or n may be 0, i.e., 3'MVIP or 5'MVIP is absent.

In some embodiments, when n=0 (i.e., 5'MVIP is absent), the structure of the 3'MVIP may be:

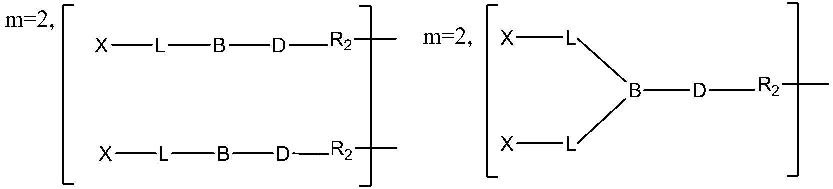

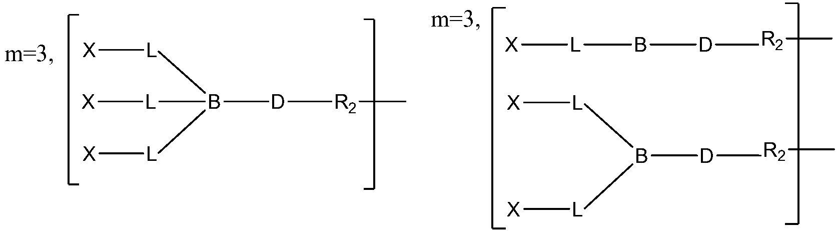

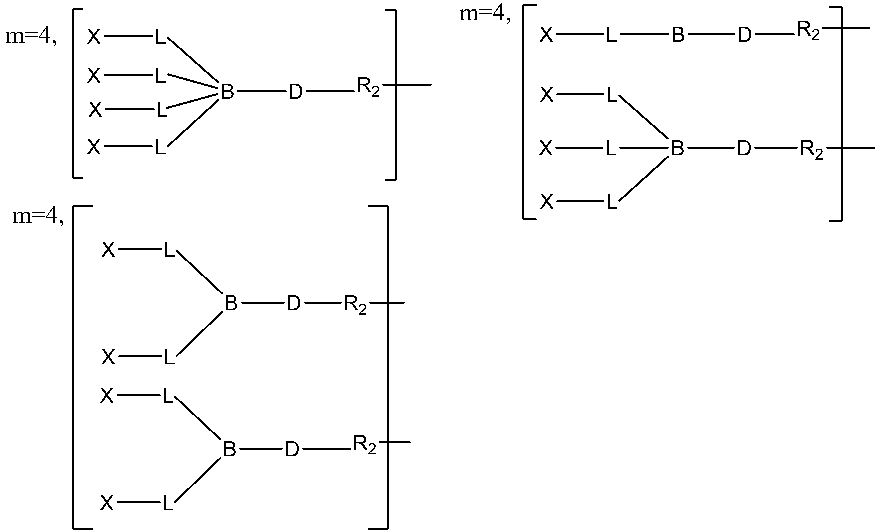

In some embodiments, when n=1, the structure of the 3'MVIP may be:

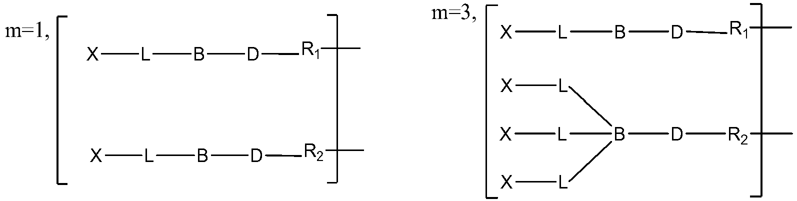

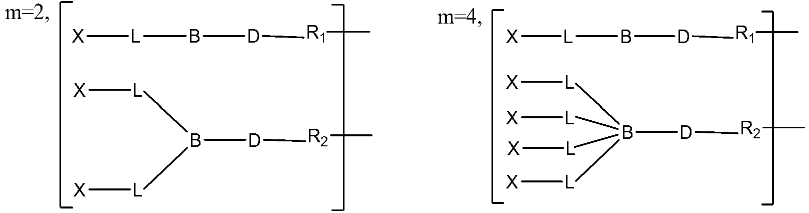

In some embodiments, when n=2, the structure of the 3'MVIP may be:

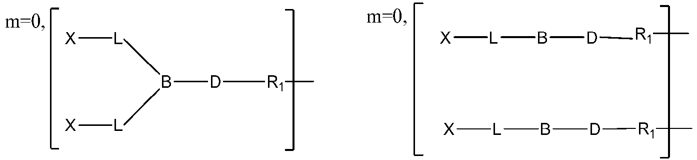

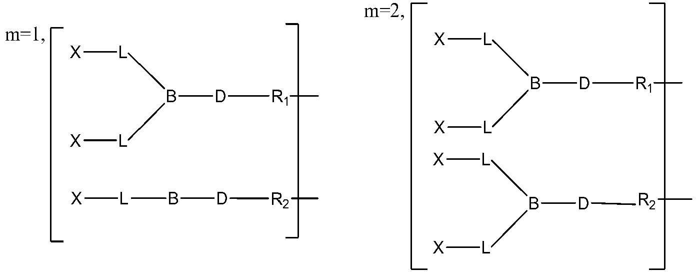

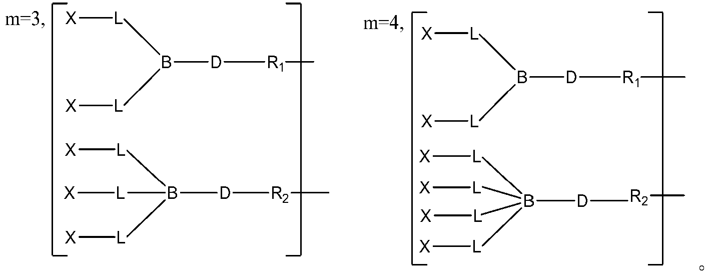

In some embodiments, when n=3, the structure of the 3'MVIP may be:

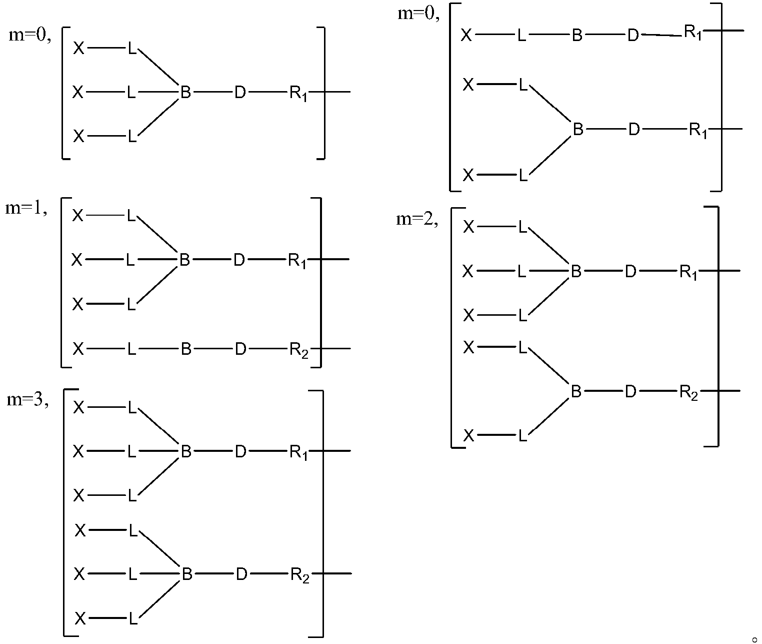

In some embodiments, when n=4, the structure of the 3'MVIP may be:

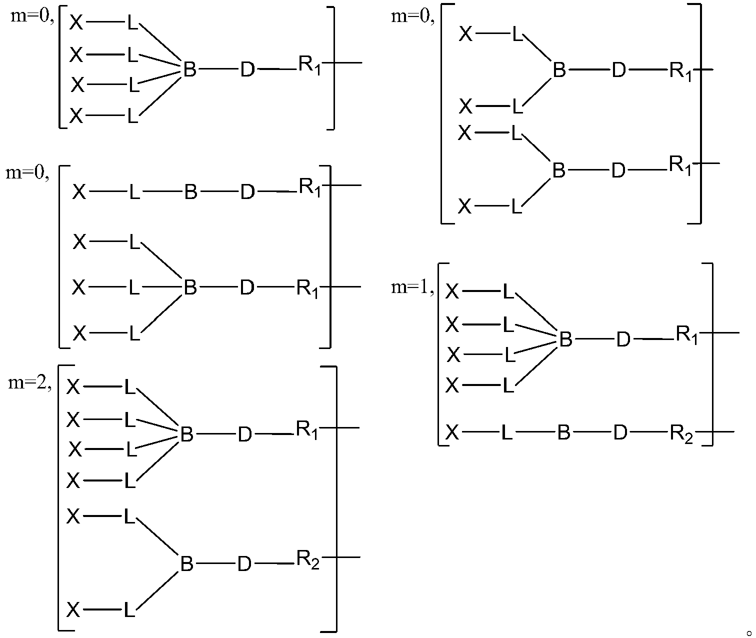

In some embodiments, n refers to the sum of n in the 5'MVIP placed at the 5'-ends of both the sense strand and the antisense strand of the RNA inhibitor, and m refers to the sum of m in the 3'MVIP placed at the 3'-ends of both the sense strand and the antisense strand of the RNA inhibitor.

In some embodiments, R₁ and R₂ have -NH-, -S- and/or -O- in their structures. R₁ and R₂ are connected to the linking chain D and the 5'- and 3'-ends of the sense strand and/or the antisense strand via -NH-, -S- or -O- in the structures, respectively, and R₁ and R₂ are the same or different.

In some embodiments, R₁ and R₂ are optionally straight carbon chains, or straight carbon chains or cyclic structures with amide, carboxyl or alkyl branches, wherein the cyclic structures include saturated or unsaturated aliphatic carbocyclic groups, or five- or six-membered heterocyclic groups or aromatic hydrocarbon groups containing sulfur, oxygen or nitrogen atoms.

In some embodiments, R₁ and/or R₂ is -E₁(CH₂)ₓCH₂E₂-, wherein x is any integer from 3 to 12, and the groups E₁ and E₂ may each be -NH-, -S- or -O-.

In some embodiments, R₁ and/or R₂ is -E₁(CH₂)ₓ₁CH(OH)(CH₂)ₓ₂E₂-, wherein x1 or x2 are each independently any integer from 3 to 10, and E₁ and E₂ may each be -NH-, -S- or -O-.

In some embodiments, R₁ is a heterocyclic or carbocyclic structure containing N, S or O as shown below:

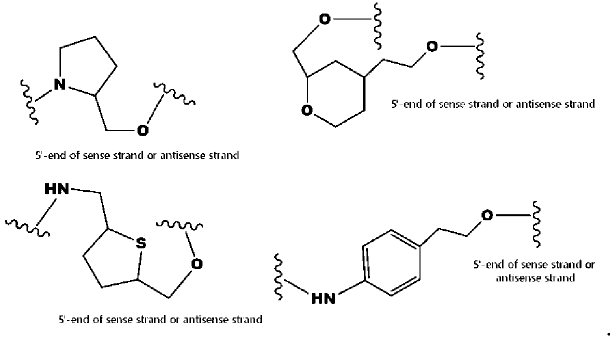

In some embodiments, the junction point R₁ is -NH(CH₂)ₓCH₂O-, wherein x is any integer from 3 to 12, preferably any integer from 4 to 6, which may be introduced via the following two phosphoramidite monomers.
i. One -O- or -S- in the R₁ structure is used for the synthesis of R₁ phosphoramidite monomers, which are attached to the 5'-end of the sense strand or antisense strand of the RNA inhibitor by solid-phase synthesis. -NH-, -S- or -O- in the structure is used for connection to the linking chain D in the 5'MVIP, thereby introducing the liver-targeting specific ligand X at the 5'-end of the sense strand or antisense strand in the RNA inhibitor. Exemplary structures of monomers introduced to the 5'-end of the sense strand or antisense strand of the RNA inhibitor are as follows:

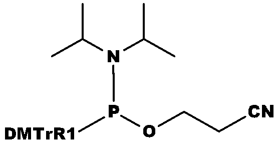

In some embodiments, the following structures are preferred:

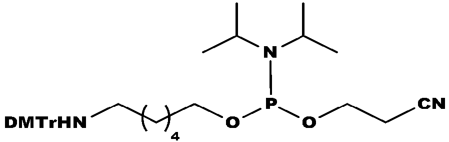

ii. One -NH-, -S- or -O- in the R₁ structure is first connected to the linking chain D, and another -NH-, -S- or -O- is used for esterification with phosphoramidite in the synthesis of 5'MVIP phosphoramidite monomers. Exemplary structures of 5'MVIP phosphoramidite monomers for the sense strand or antisense strand are as follows:

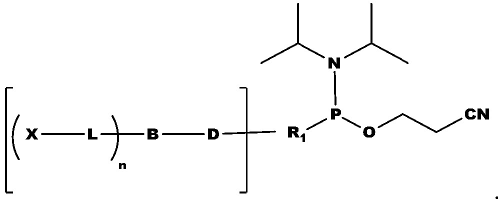

In some embodiments, the following structures are preferred for the 5'MVIP phosphoramidite monomers of the sense strand or antisense strand:

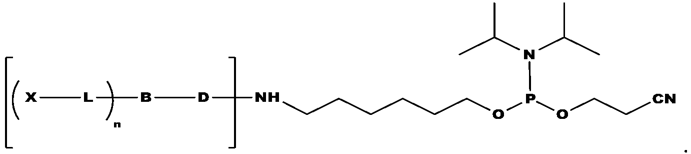

When n in the general formula is 1 to 4, the linker B moiety in the monomer is branched 1 to 4 times, respectively, to obtain the corresponding monomer compound, by means of which the liver-targeting specific ligand X is introduced to the 5'-end of the sense strand or antisense strand by solid-phase synthesis.

In some embodiments, the junction point R₁ is -NH(CH₂)ₓCH₂O-, wherein x may be any integer from 3 to 12, preferably any integer from 4 to 6.

In some embodiments, the structure of the 5'MVIP phosphoramidite monomer is selected from the following structures:

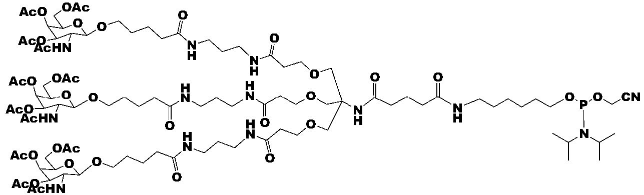

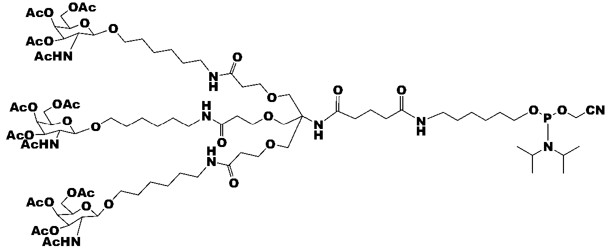

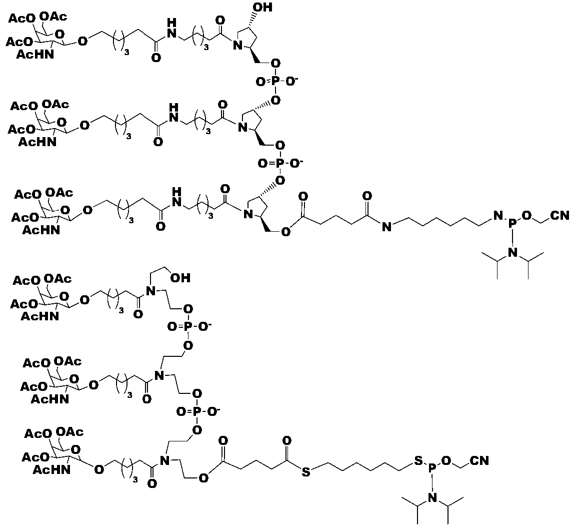

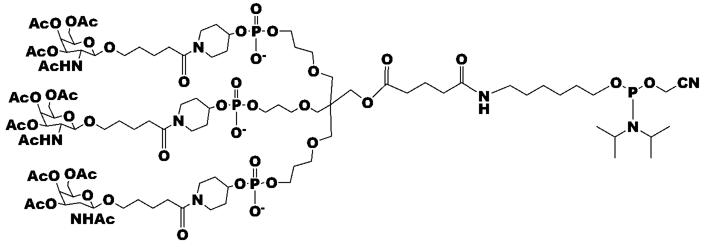

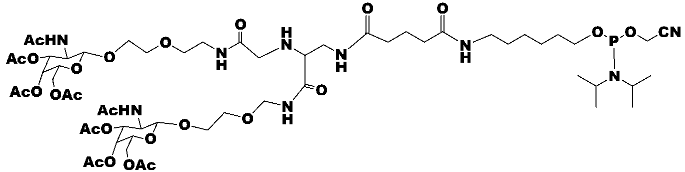

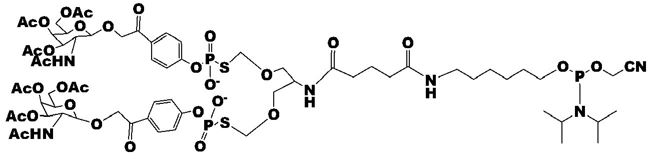

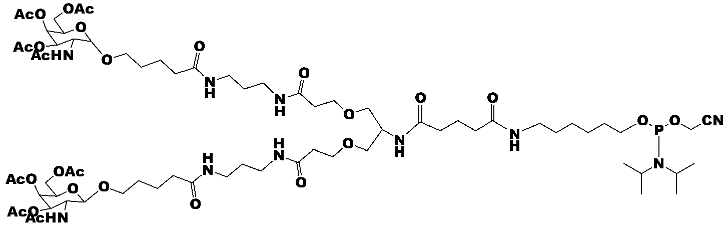

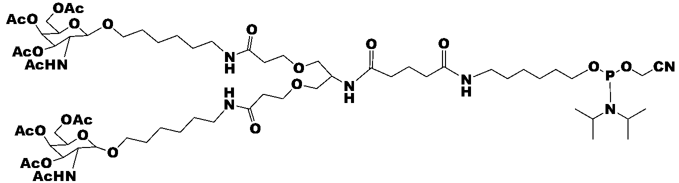

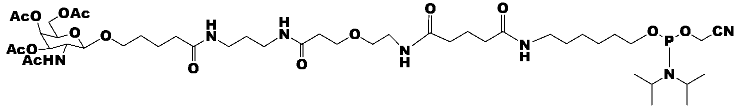

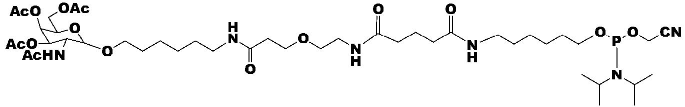

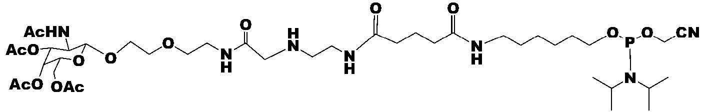

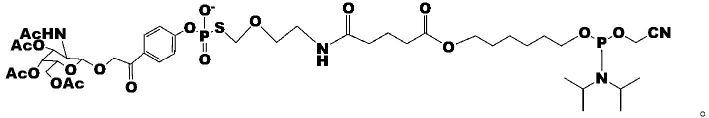

In some embodiments, the junction point R₂ is a heterocyclic or carbocyclic structure containing N, S or O as shown below:

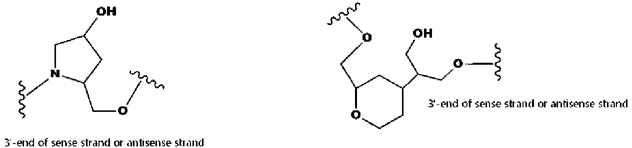

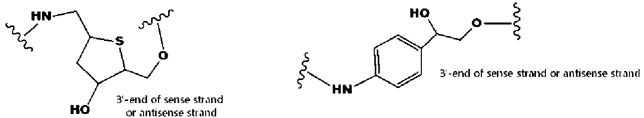

In some embodiments, the junction point R₂ is -NH(CH₂)ₓ₁CH(OH)(CH₂)ₓ₂CH₂O-, wherein x1 is any integer from 1 to 4, and x2 is any integer from 0 to 4.

The junction point R₂ of the present application is coupled with -NH-, -S- or -O- in the R₂ structure via succinic anhydride to form an ester or amide, and simultaneously coupled with -NH- in the blank Solid Support to form a 3'MVIP solid support, and then the 3'MVIP is introduced to the 3'-end of the sense strand or antisense strand by the phosphoramidite solid-phase synthesis method.

In some embodiments, the heterocycle in the junction point R₂ structure is a pyrrole ring or a piperidine ring, which is connected to the linking chain D of the 3'MVIP via a nitrogen heteroatom in the ring. Exemplary structures of the introduced 3'MVIP solid support are as follows:

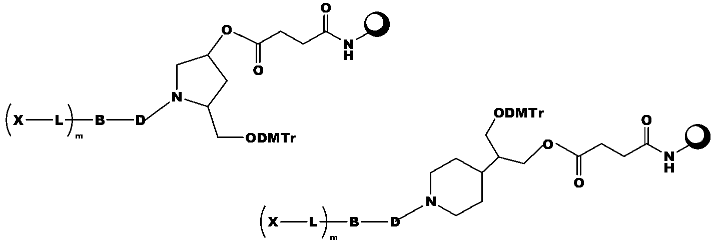

When m in the general formula is 1 to 4, the linker B moiety in the monomer is branched 1 to 4 times, respectively, to obtain the corresponding Solid Support.

In some embodiments, the junction point R₂ is -B₄(CH₂)ₓ₁CH(OH)(CH₂)ₓ₂CH₂B₅-, wherein x1 is any integer from 1 to 4, x2 is any integer from 0 to 4, and B₄ and B₅ are each -NH-, -S- or -O-. Exemplary structures of the introduced 3'MVIP solid support are as follows:

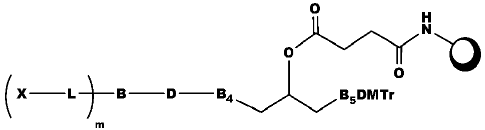

When m in the general formula is 1 to 4, the linker B moiety in the monomer is branched 1 to 4 times, respectively, to obtain the corresponding Solid Support.

In some embodiments, R₂ is -NHCH₂CH(OH)CH₂O-. Exemplary structures of the introduced 3'MVIP solid support are as follows:

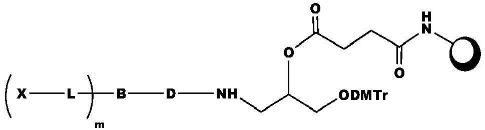

When m in the general formula is 1 to 4, the linker B moiety in the monomer is branched 1 to 4 times, respectively, to obtain the corresponding Solid Support.

In some embodiments, the structure of the 3'MVIP solid support is as follows:

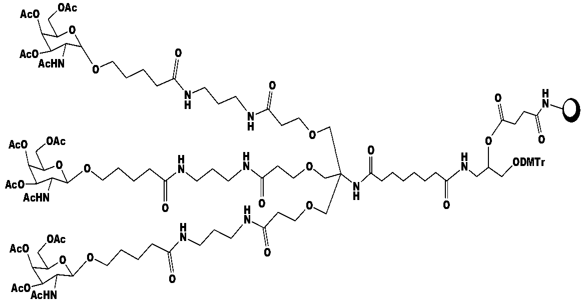

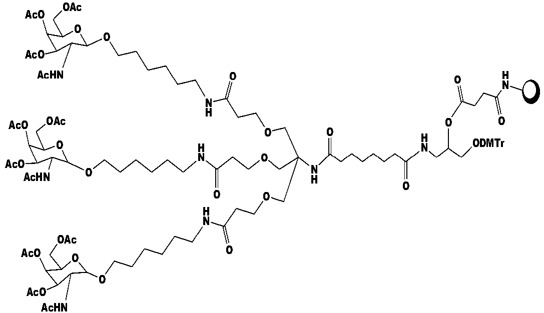

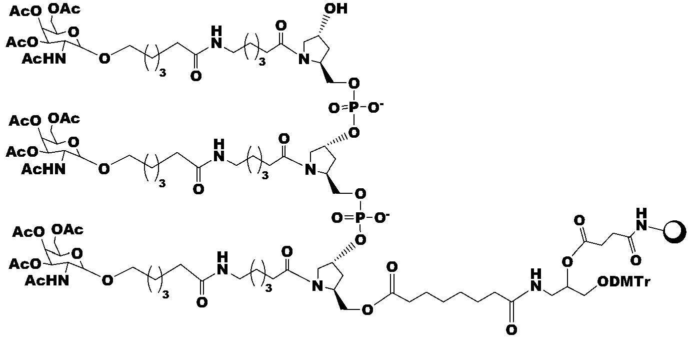

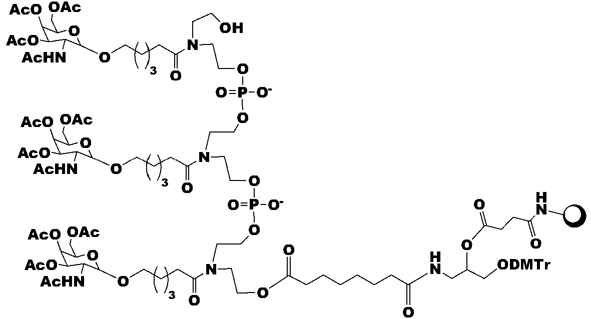

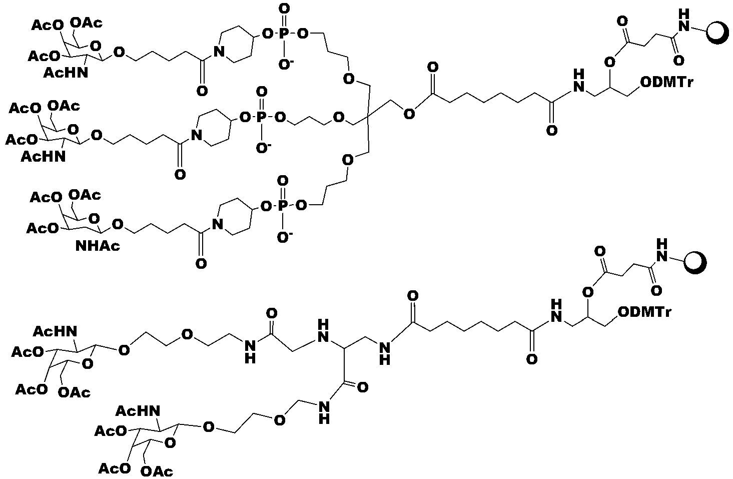

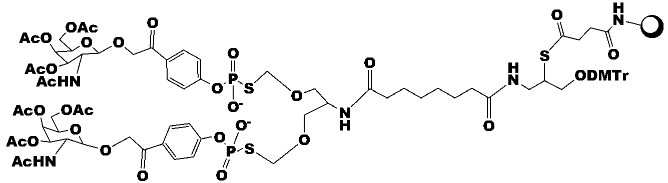

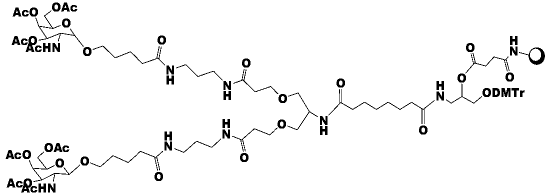

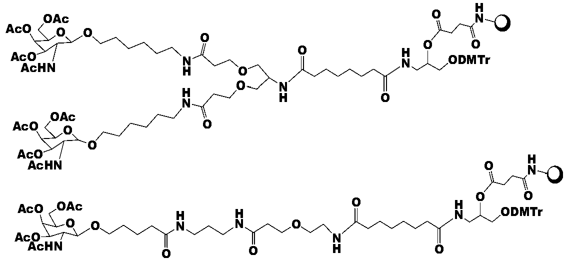

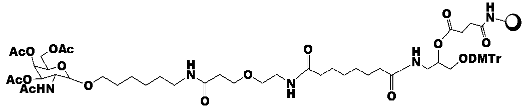

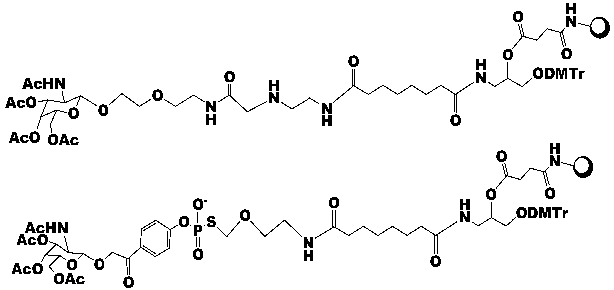

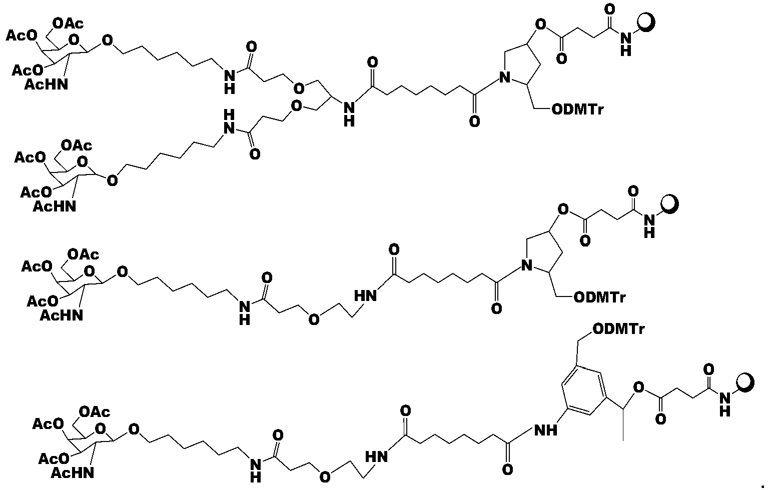

In some embodiments, the liver-targeting specific ligand X is selected from structures for enhancing the uptake of RNA inhibitors by hepatocytes, and may be lipids, steroids, vitamins, sugars, proteins, peptides, polyamines and peptidomimetic structures. In the RNA inhibitors provided in the present application, the liver-targeting specific ligands X introduced to the ends of the sense strand or antisense strand of the RNA inhibitor may be the same or different. For example, in terms of properties, some may enhance liver targeting, some may be structures for regulating the in vivo pharmacokinetics of the RNA inhibitor, and some may be structures with in vivo lytic activity. In some embodiments, the liver-targeting specific ligand X is selected from one or more monosaccharides and derivatives thereof of the following structures.

In some embodiments, the monosaccharide is selected from one or more of the following structures: mannose, galactose, D-arabinose, glucose, fructose, xylose, glucosamine, ribose. The monosaccharide derivative is selected from mannose derivatives, galactose derivatives, glucose derivatives, ribose derivatives and other derivatives.

In some embodiments, the liver-targeting specific ligand X is selected from galactose, galactosamine, N-acetylgalactosamine and derivatives thereof, and its general structure is as follows: 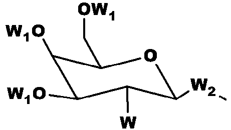 wherein W₁ is hydrogen or a hydroxyl protecting group, which may be the same or different; W is -OH, -NHCOOH or -NHCO(CH₂)_{q}CH₃, wherein q is an integer from 0 to 4; W₂ is -NH-, O, S or C.

In some embodiments, the liver-targeting specific ligand X is N-acetylgalactosamine and derivatives thereof.

In some embodiments, the liver-targeting specific ligand X is selected from the following structures: 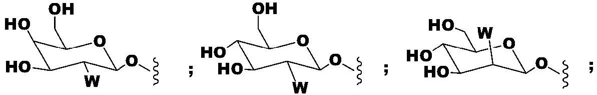 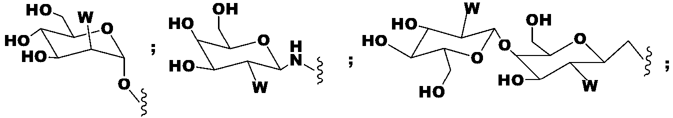 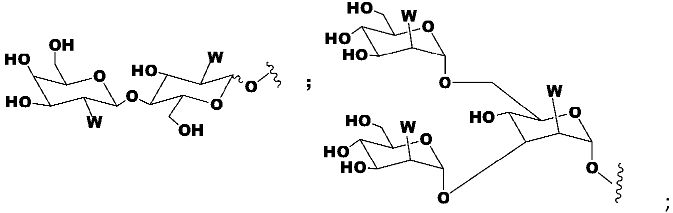 wherein W is selected from one or two of -OH, -NHCOOH and -NHCO(CH₂)_{q}CH₃, wherein q is an integer from 0 to 4.

In some embodiments, the liver-targeting specific ligands X in the same 5'MVIP or 3'MVIP structure may be the same or different.

In some embodiments, the X of 5'MVIP and 3'MVIP may be the same or different from each other.

In some embodiments, the branching chain L is a C₄-C₁₈ carbon chain containing -NH-, -C(=O)-, -O-, -S-, amide, phosphoryl, thiophosphoryl, C₄-C₁₀ aliphatic carbocyclic, phenyl or a combination of these groups.

In some embodiments, the branching chain L further has a hydroxyethyl or carboxylic acid side chain.

In some embodiments, the branching chain L is a C₇-C₁₈ carbon chain containing an amide group or a six-membered aliphatic carbocyclic group.

In some embodiments, the branching chain L is selected from one or more of the following structures:

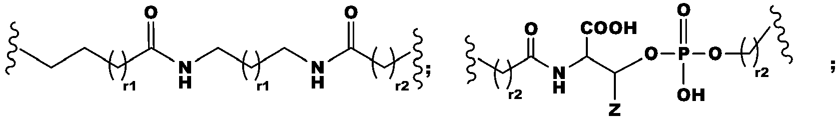

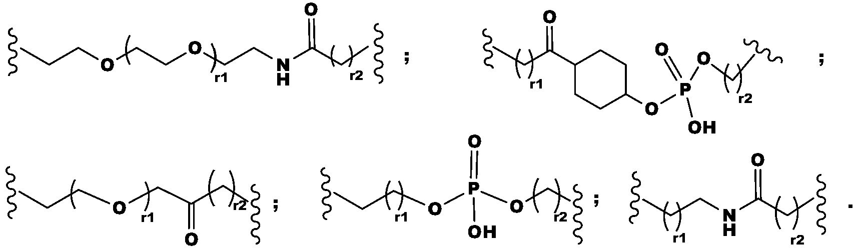

Wherein, r1 is any integer from 1 to 12, r2 is any integer from 0 to 20, and Z is H, an alkyl group or an amido group, and the alkyl group is, for example, C₁-C₅ alkyl group.

In some embodiments, the structure of the linker B is related to the number of X that can be introduced, and the linker B contains -NH-, C, O, S, amido group, phosphoryl group and thiophosphoryl group; when n or m is 1, it is a straight carbon chain, and when n or m is 2, 3 or 4, the number of branches is 2, 3 or 4, respectively.

In some embodiments, the linker B is selected from the following structures:

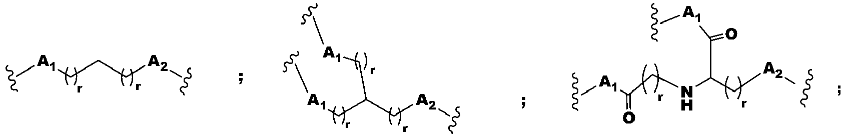

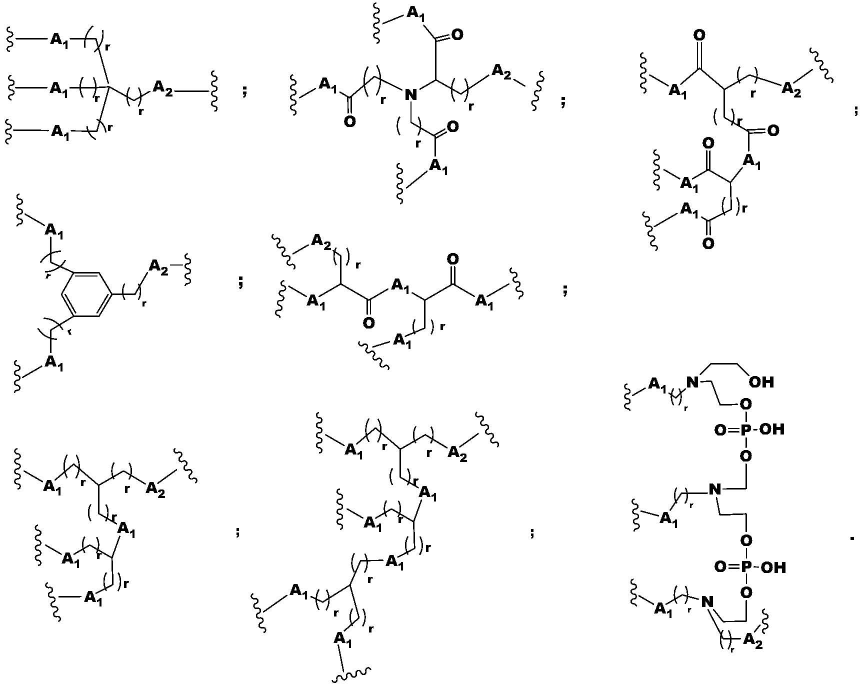

Wherein, A₁ and A₂ are each independently C, O, S, -NH-, carbonyl group, amido group, phosphoryl group or thiophosphoryl group, and r is an integer from 0 to 4.

In some embodiments, the linker B is selected from the following structures:

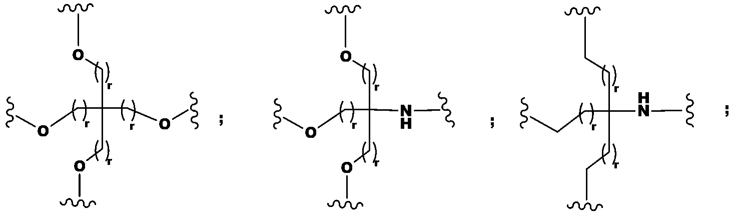

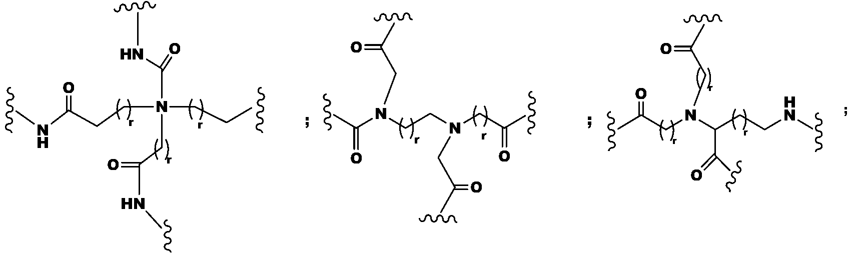

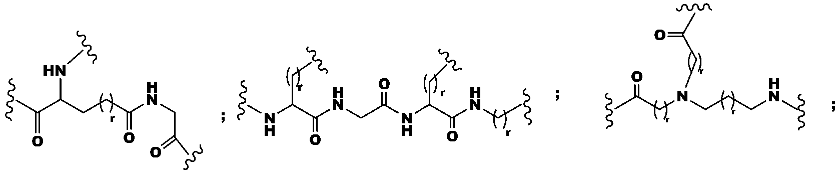

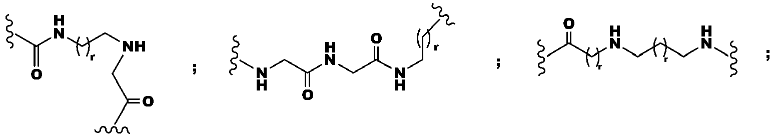

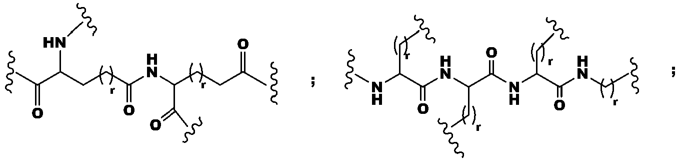

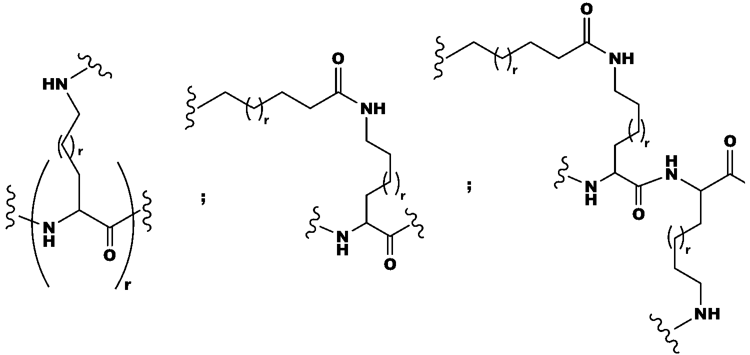

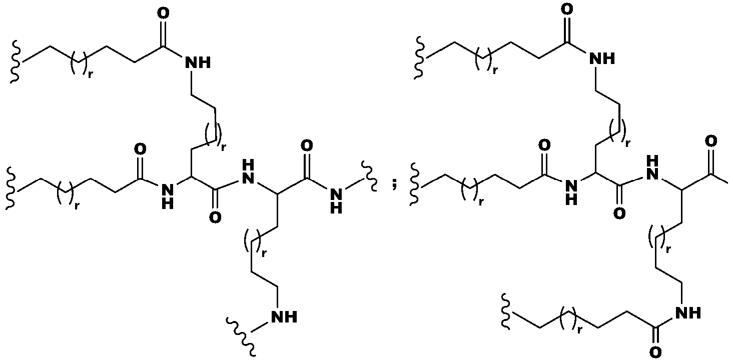

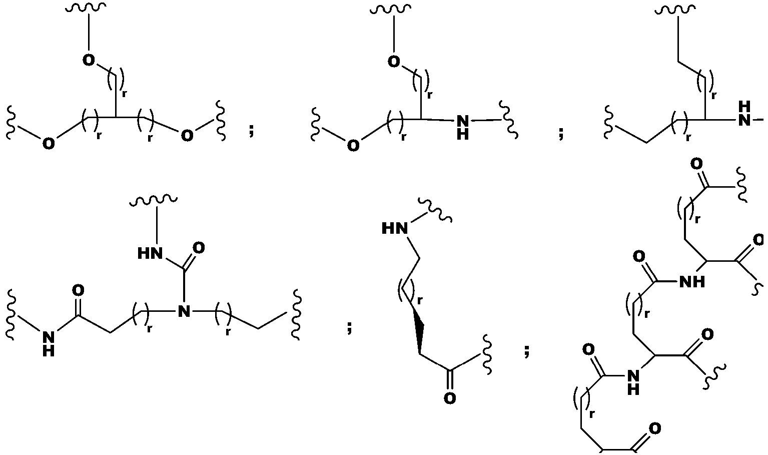

Wherein, r is any integer from 0 to 4.

In some embodiments, the linker B is selected from the following structures: 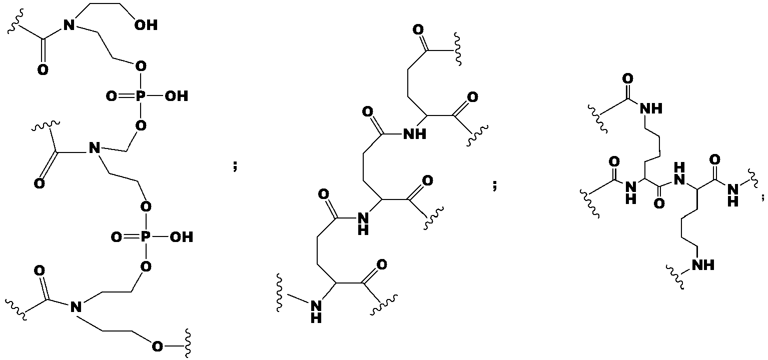 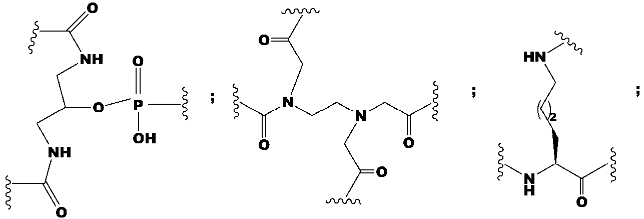 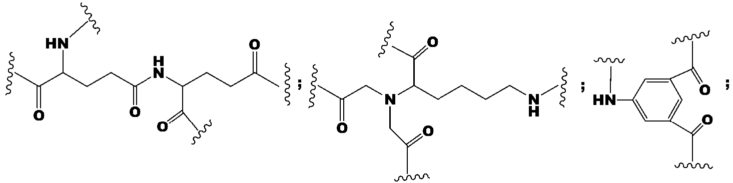 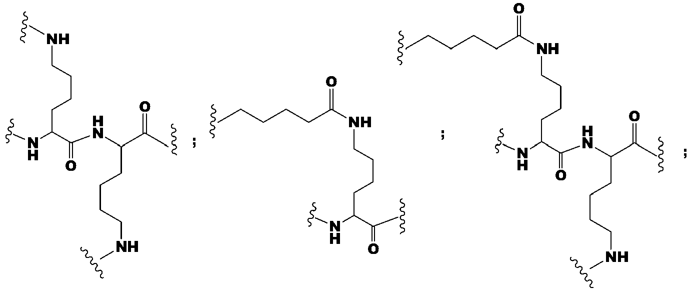 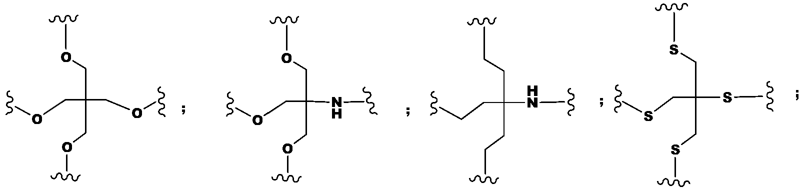 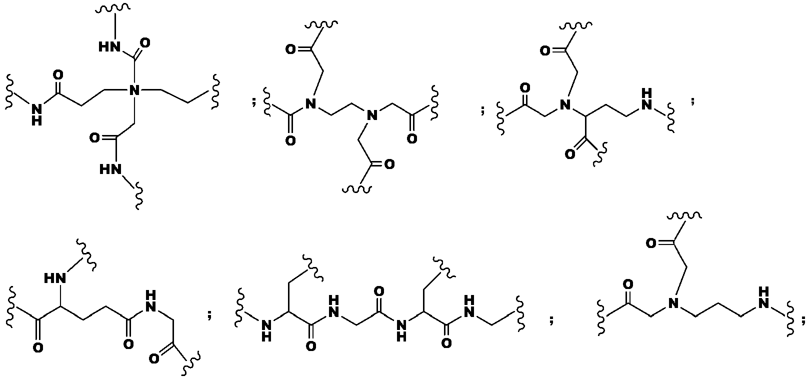 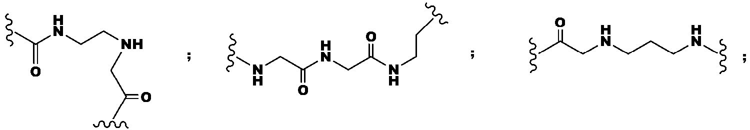 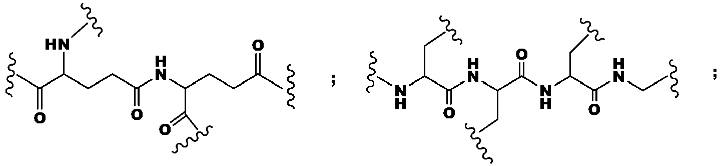 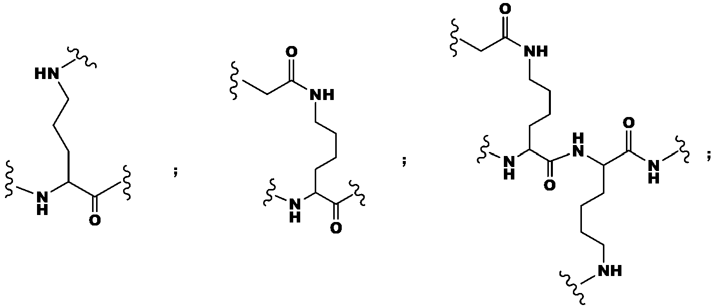 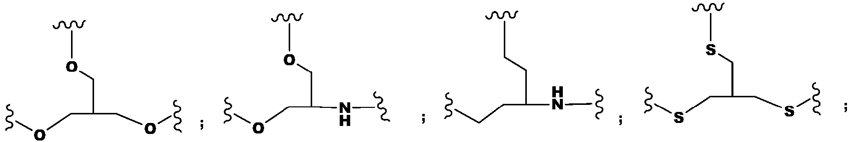 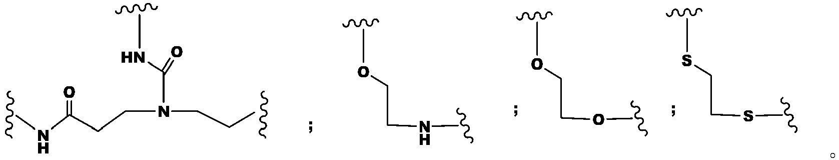 In some embodiments, the linker B is selected from the following structures:

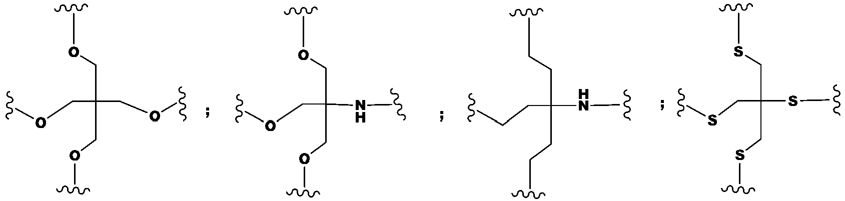

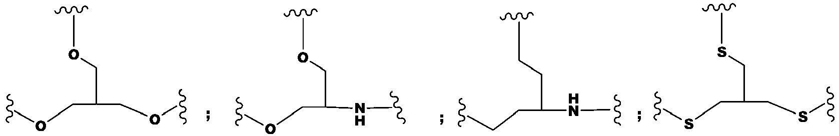

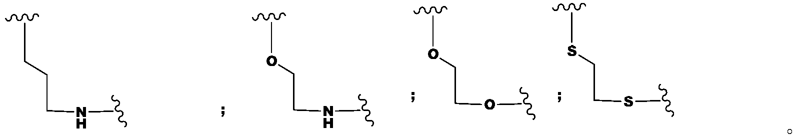

In some embodiments, the linking chain D is a C₃-C₁₈ carbon chain containing -NH-, C=O, O, S, amido group, phosphoryl group, thiophosphoryl group, aromatic hydrocarbon group, C₄-C₁₀ aliphatic carbocyclic group, five-membered or six-membered heterocyclic group containing 1 to 3 nitrogen atoms, or combination of these groups.

In some embodiments, the linking chain D further has side chain of hydroxymethyl group, methyl tert-butyl group, methylphenol group, or C₅-C₆ aliphatic cyclic group.

In some embodiments, the linking chain D is a C₃-C₁₀ carbon chain containing two C=O groups, six-membered aliphatic carbocyclic group or phenyl group.

In some embodiments, the linking chain D is a C₃-C₁₀ carbon chain containing two C=O groups.

In some embodiments, the linking chain D is selected from the following structures:

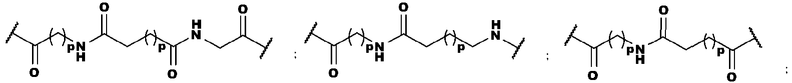

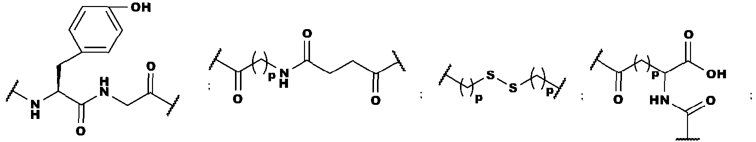

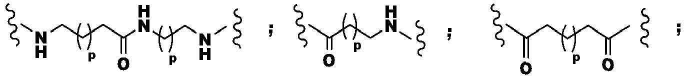

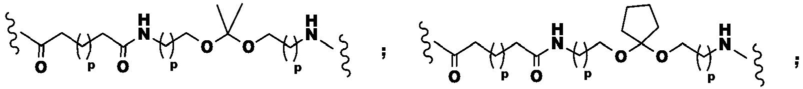

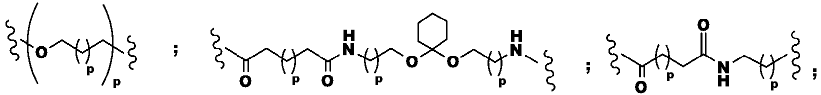

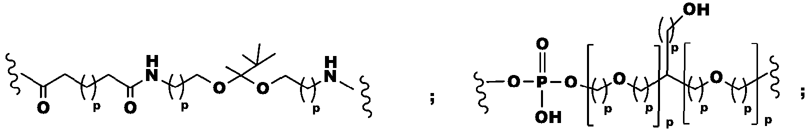

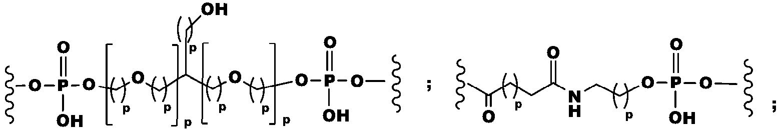

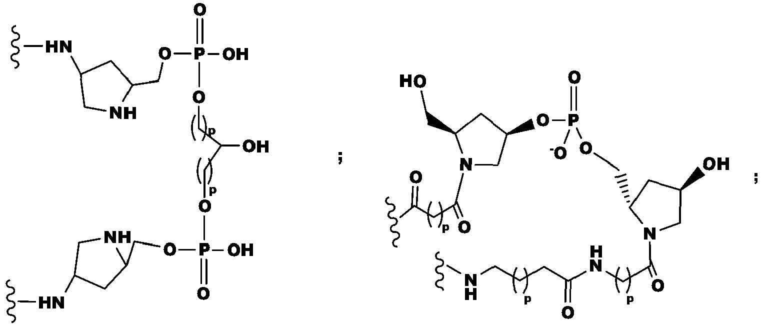

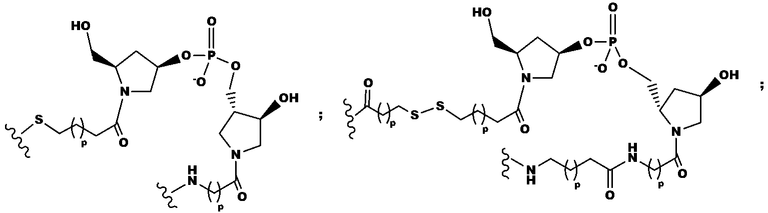

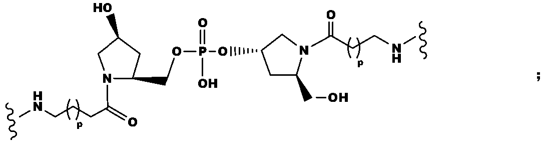

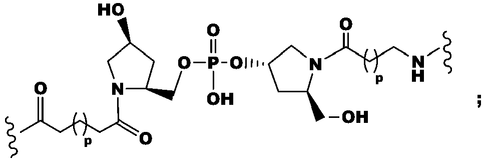

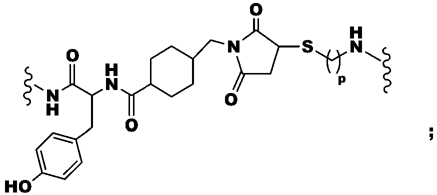

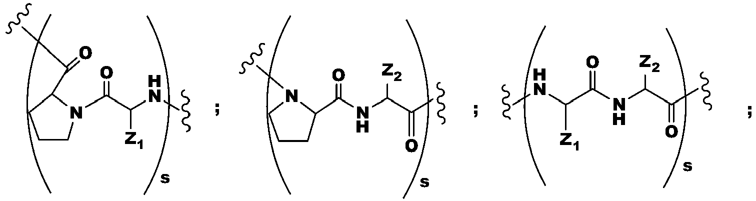

Wherein, each p is each independently any integer from 1 to 20; s is an integer from 2 to 13; Z₁ and Z₂ are identical or different substituent groups, such as C₃-C₁₀ alkyl group.

In some embodiments, the linking chain D is selected from the following structures: 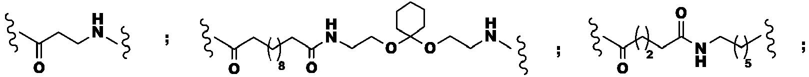 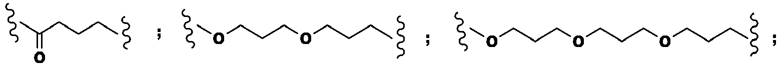 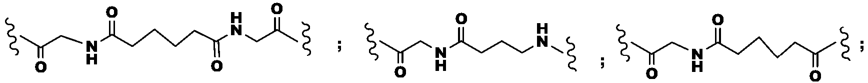 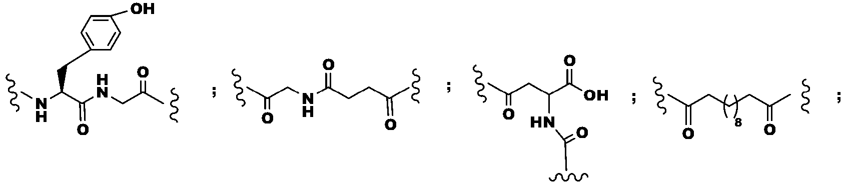 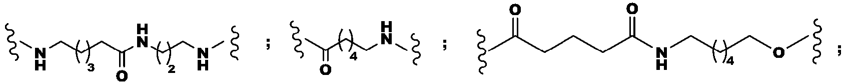 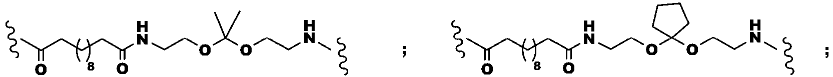 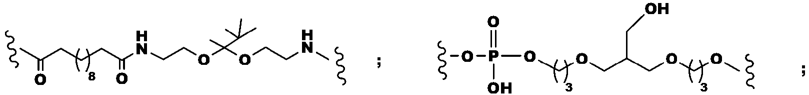 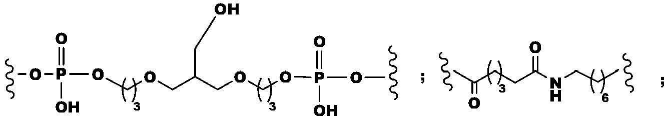 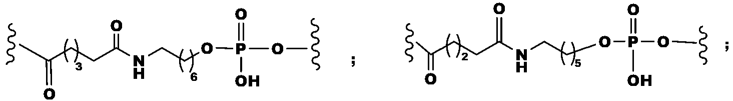 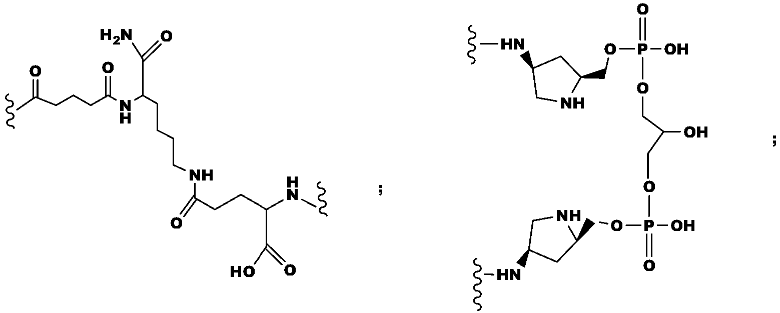 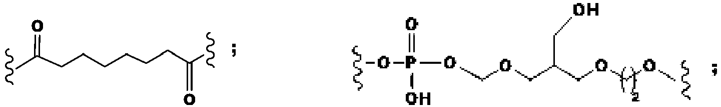 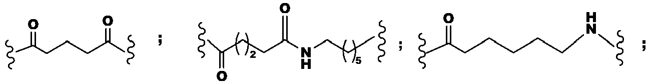 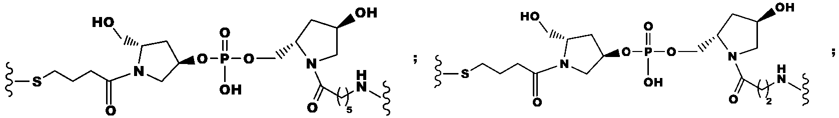 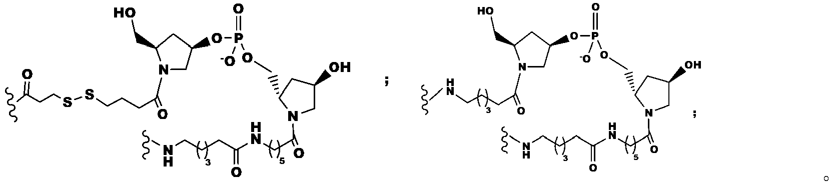 In some embodiments, the linking chain D is selected from the following structures:

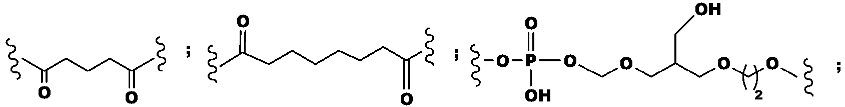

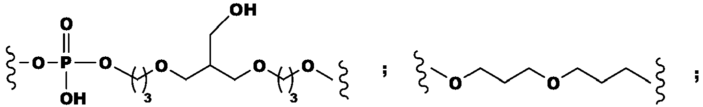

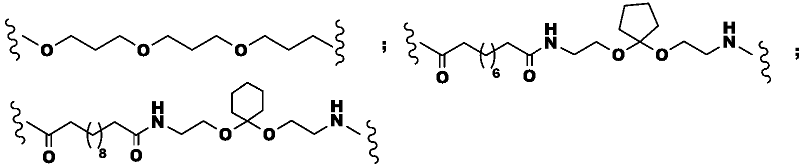

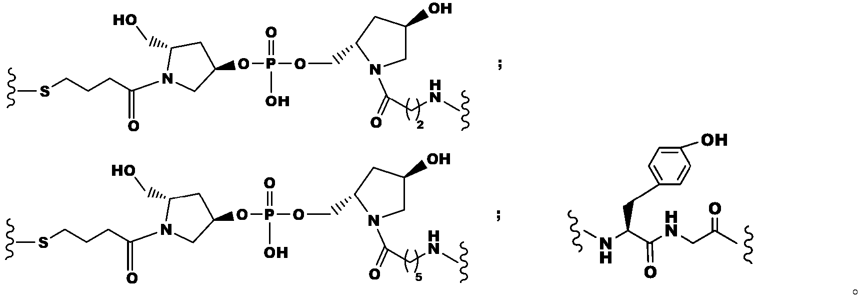

In some embodiments, **(X-L)ₙ-B-D-** in the 5'-MVIP structure and **(X-L)ₘ-B-D-** in the 3'-MVIP structure are selected from one or more of the following structures:

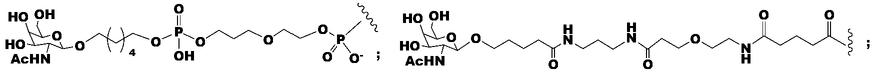

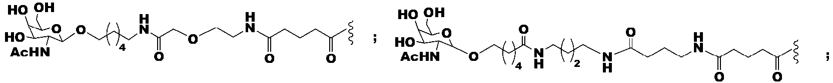

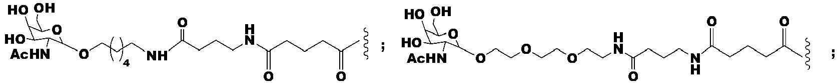

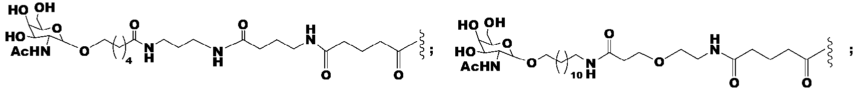

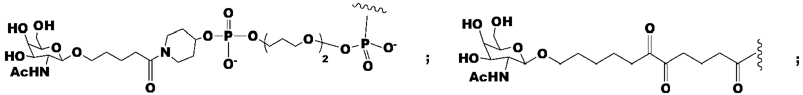

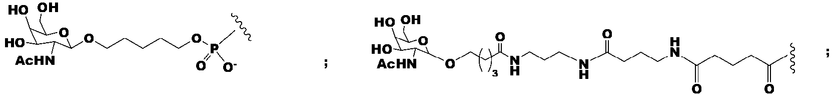

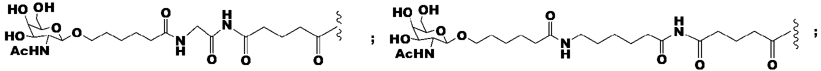

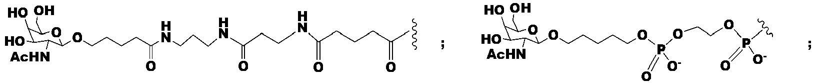

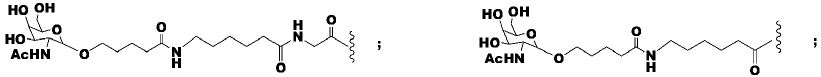

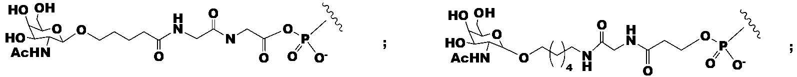

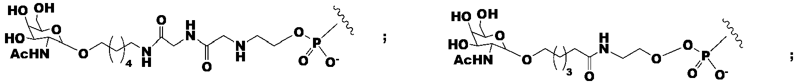

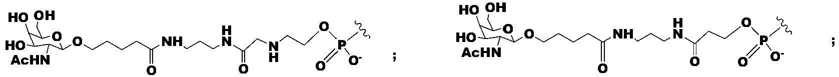

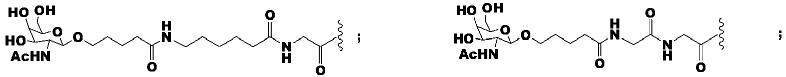

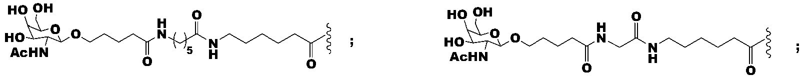

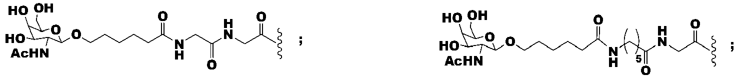

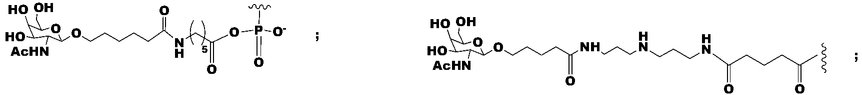

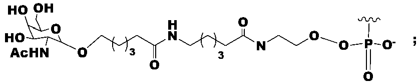

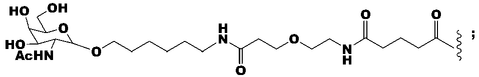

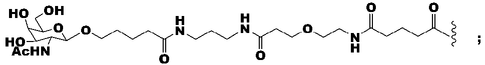

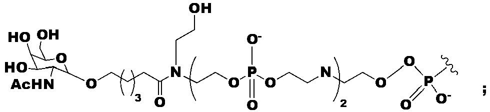

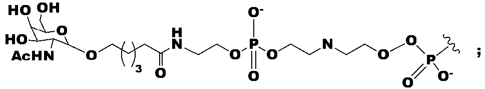

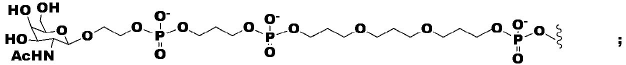

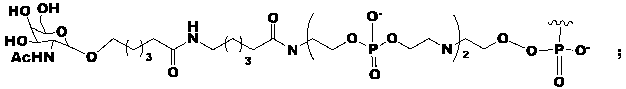

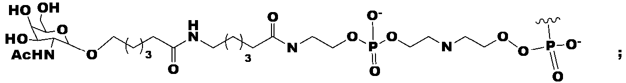

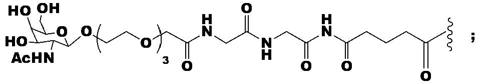

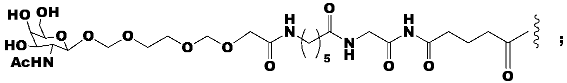

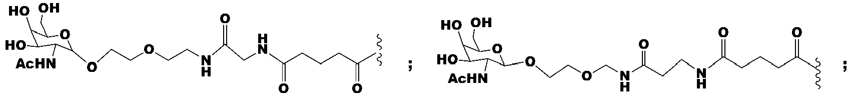

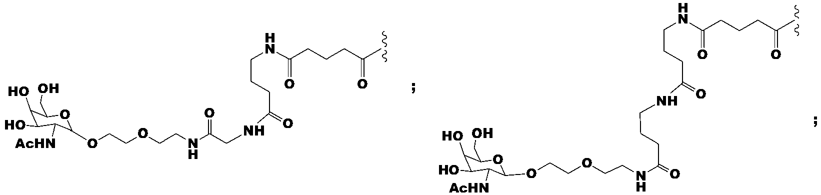

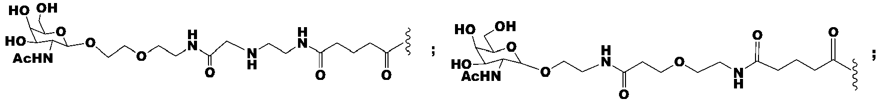

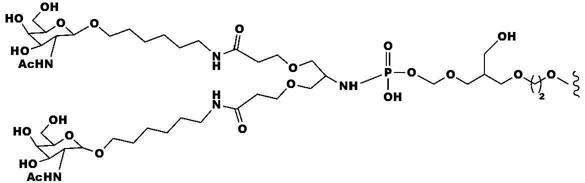

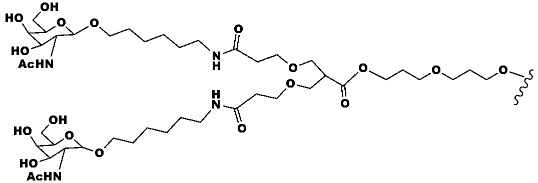

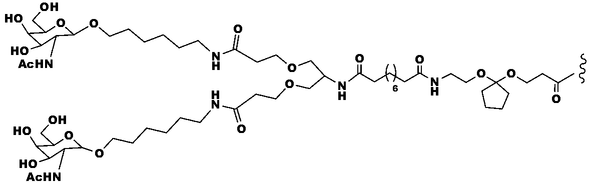

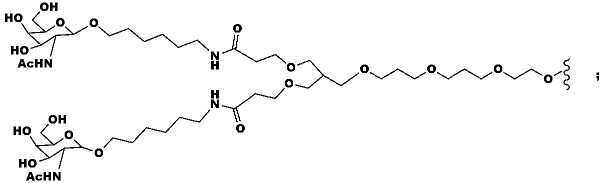

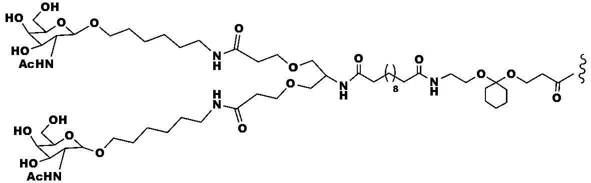

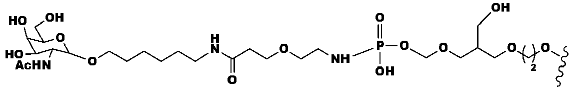

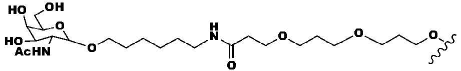

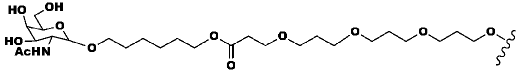

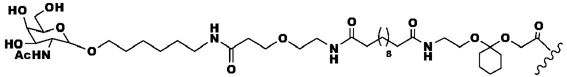

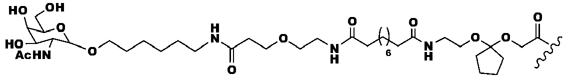

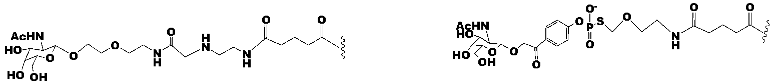

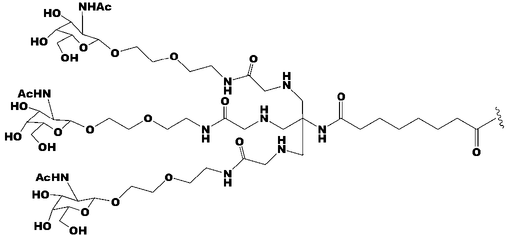

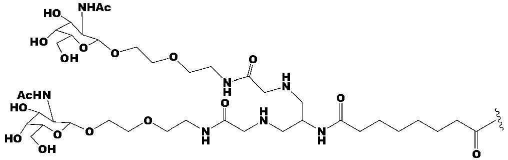

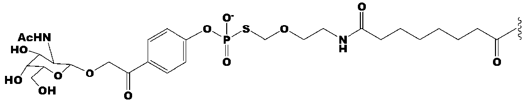

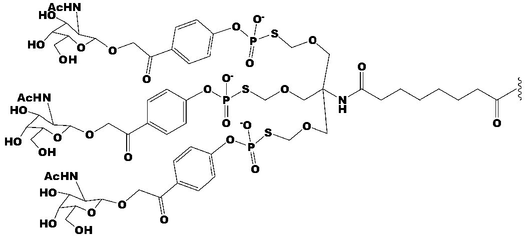

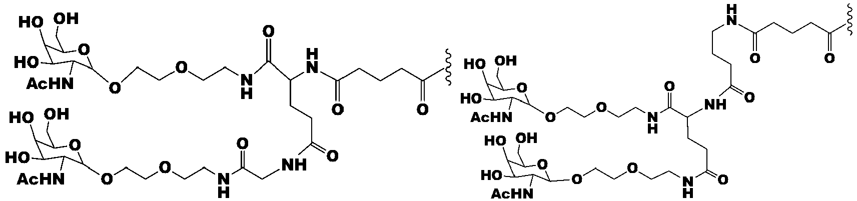

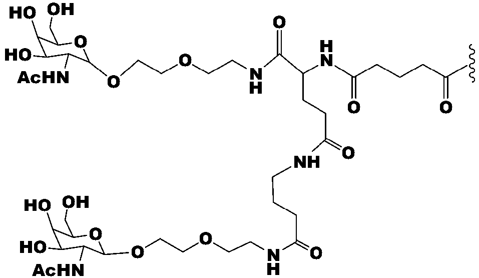

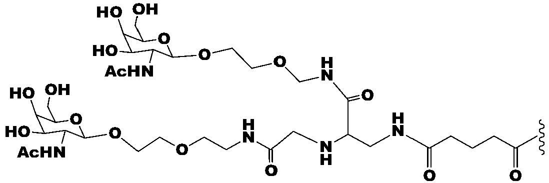

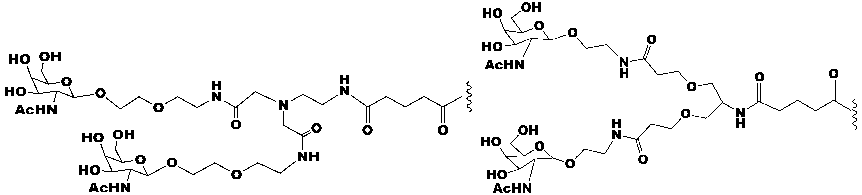

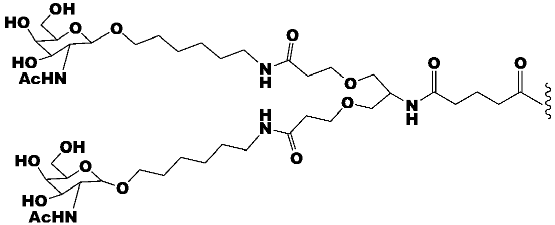

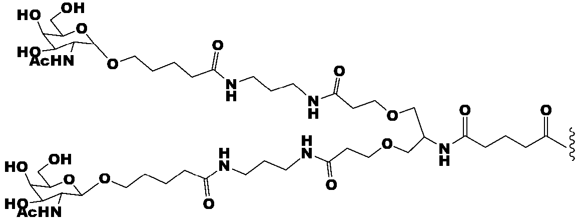

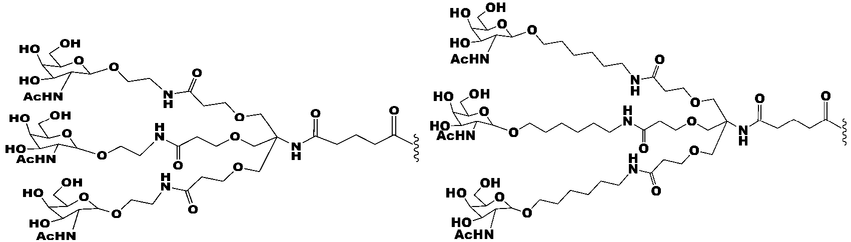

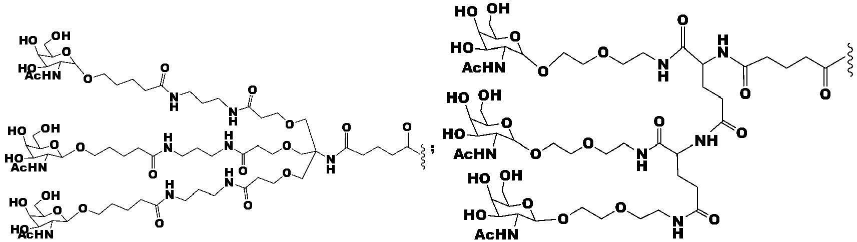

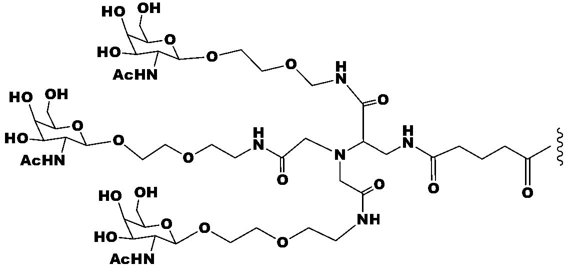

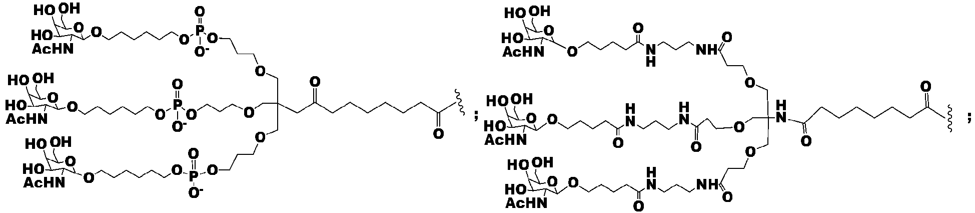

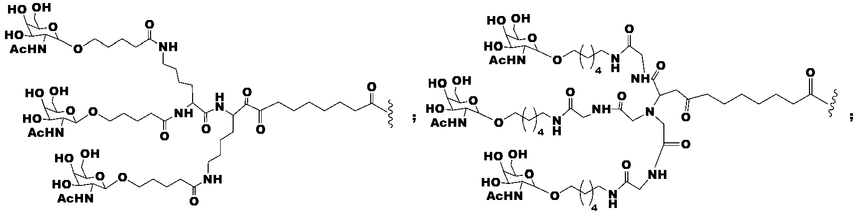

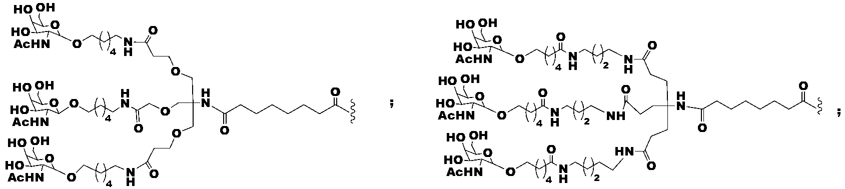

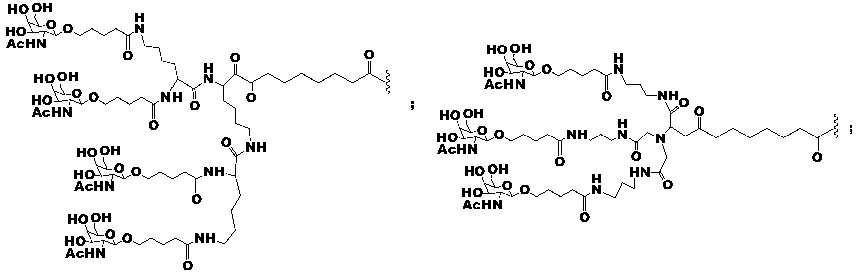

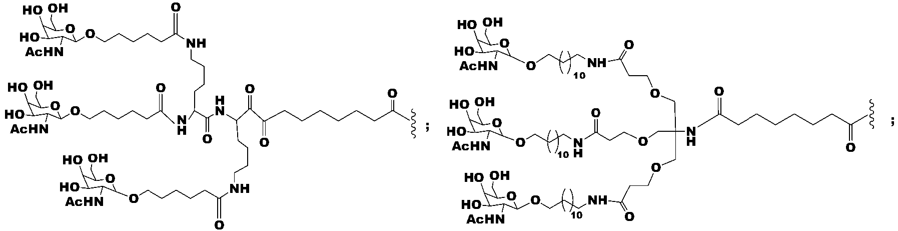

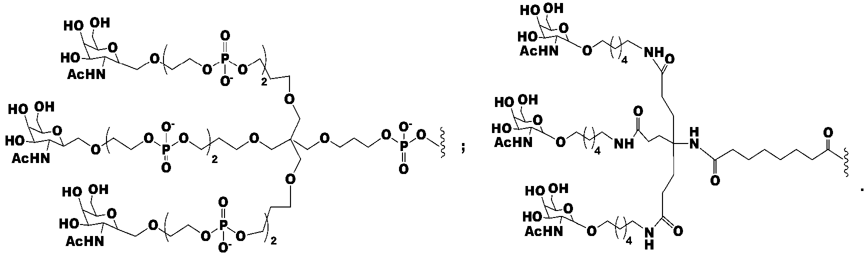

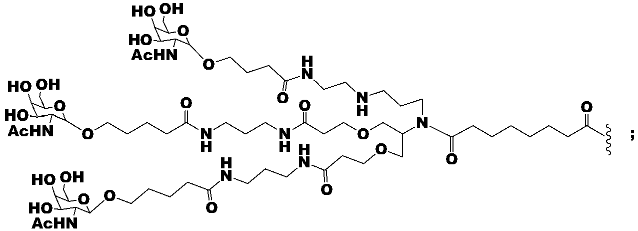

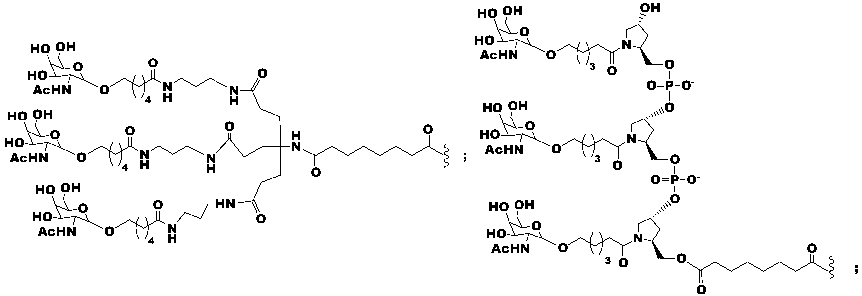

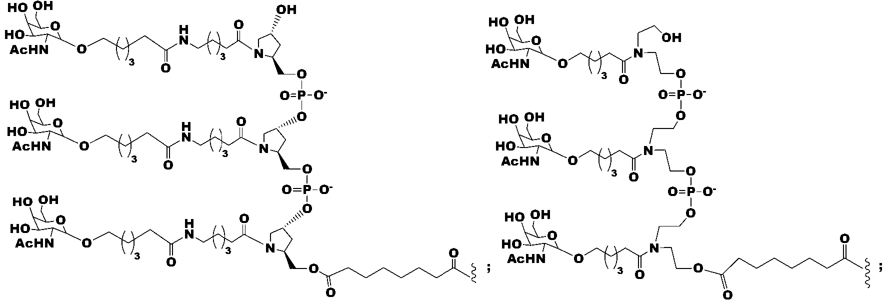

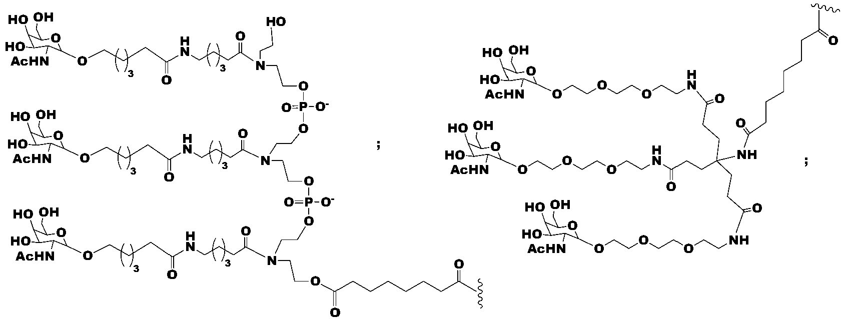

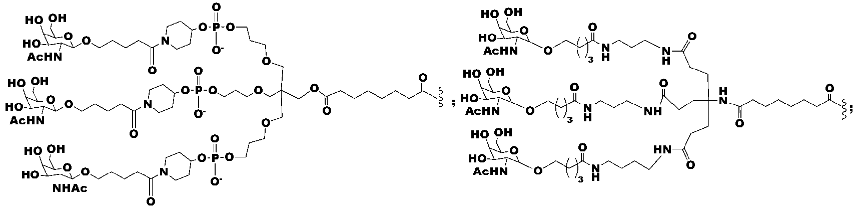

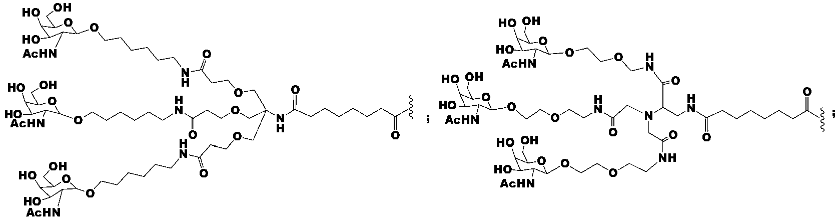

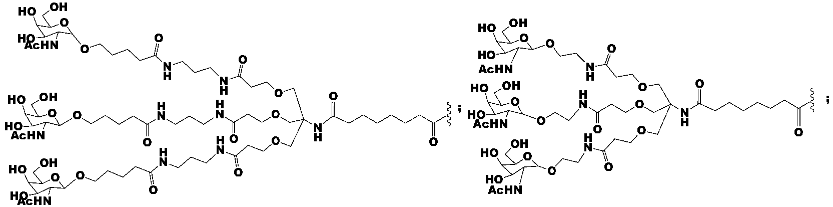

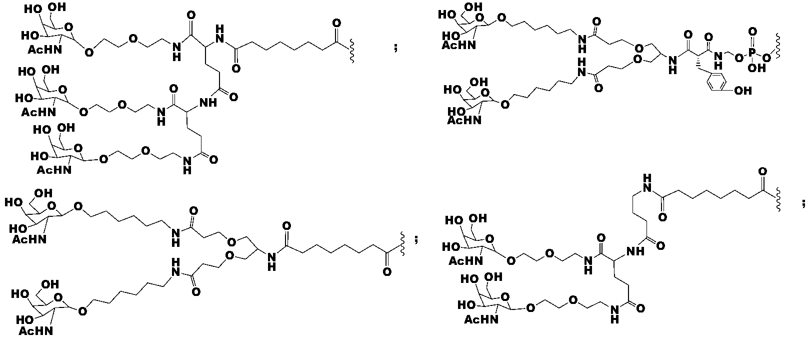

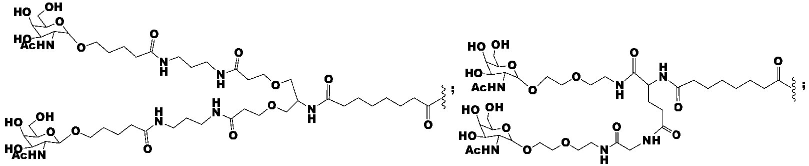

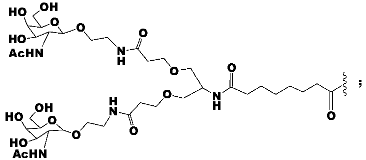

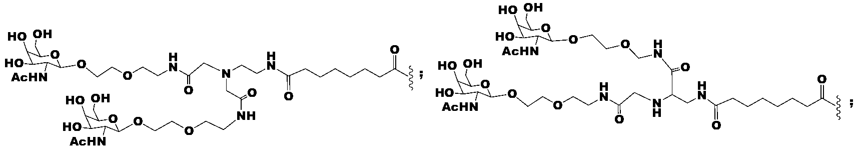

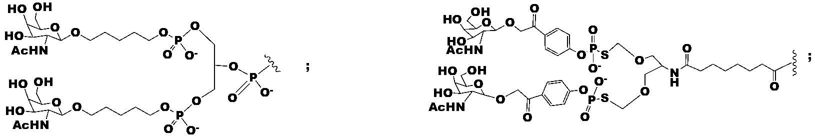

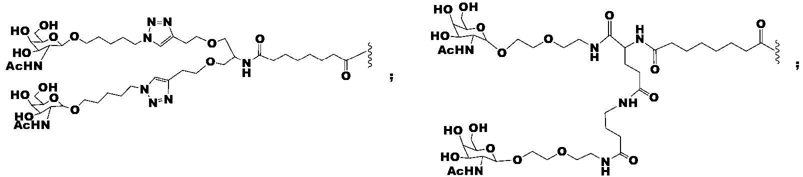

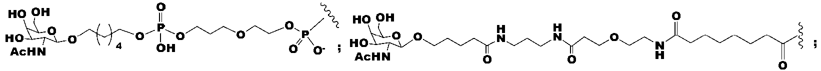

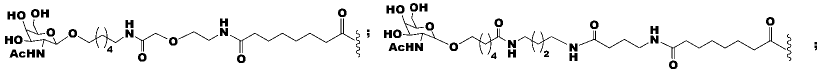

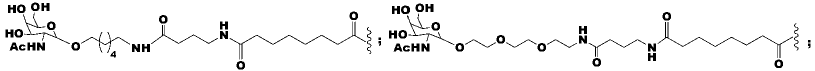

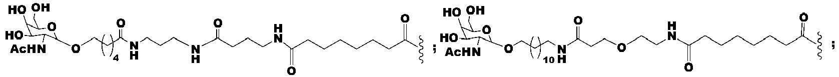

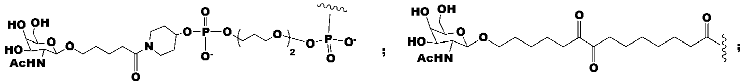

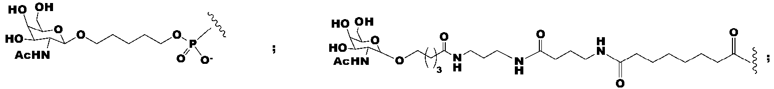

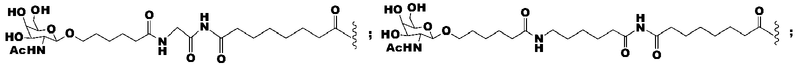

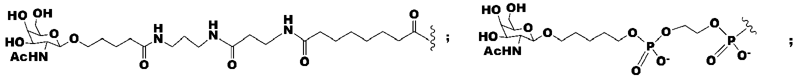

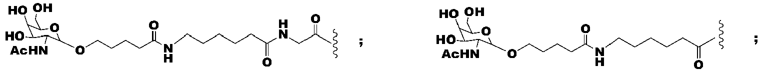

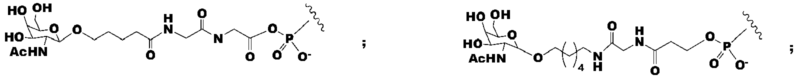

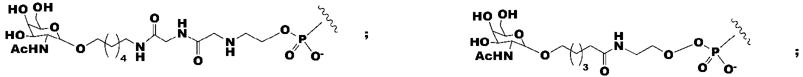

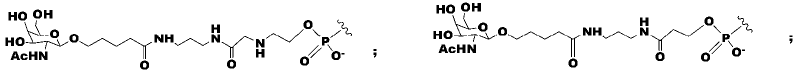

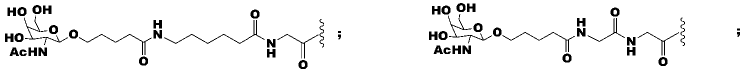

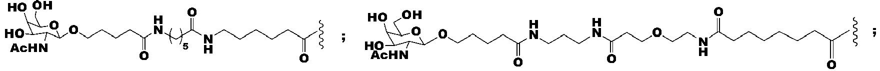

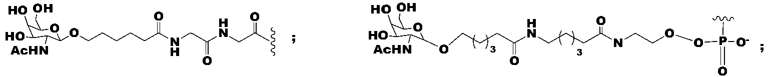

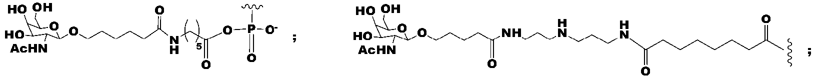

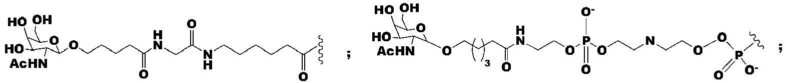

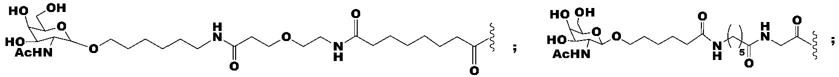

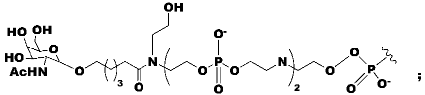

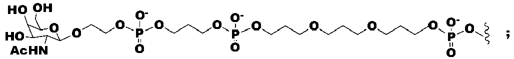

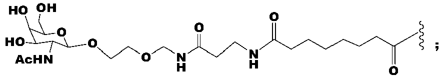

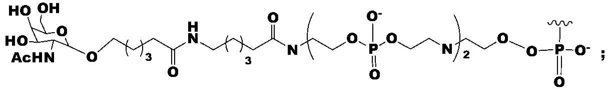

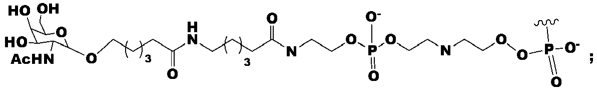

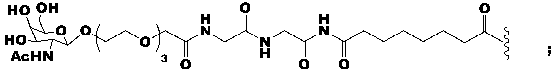

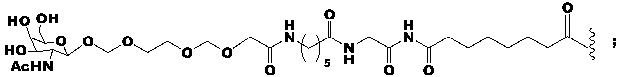

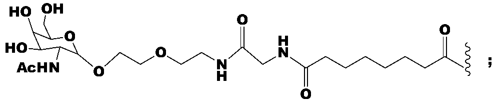

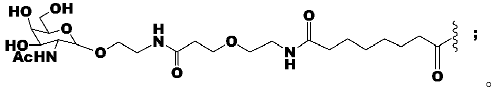

In some embodiments, said X, L, B and D are the same or different within each of 5'MVIP and 3'MVIP or between 5'MVIP and 3'MVIP.

In some embodiments, **(X-L)ₙ-B-D-** in said 5'MVIP structure is selected from structures shown in Table 3.

**Table 3 Structures of (X-L)ₙ-B-D- of 5'MVIP**

| No. | Code | Structural Formula |
|---|---|---|
| 1 | 5'YICdd-01 | 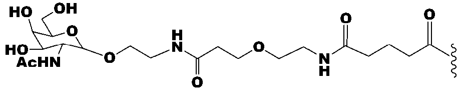 |
| 2 | 5'YICd-01 | 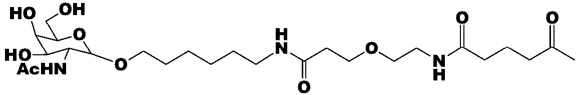 |
| 3 | 5'YICc-01 | 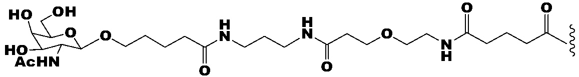 |
| 4 | 5'YICa-01 | 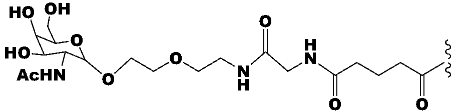 |
| 5 | 5'YICa-02 | 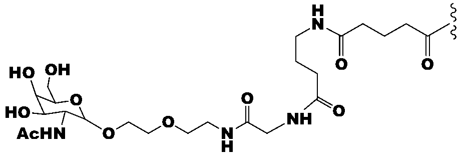 |
| 6 | 5'YICa-03 | 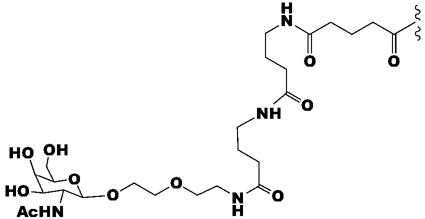 |
| 7 | 5'YICa-04 | 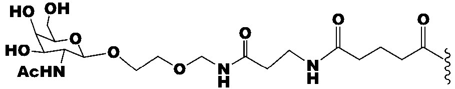 |
| 8 | 5'YICa-05 | 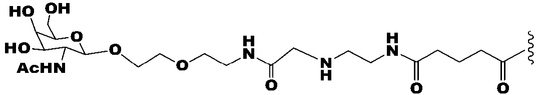 |
| 9 | 5'ERCa-01 | 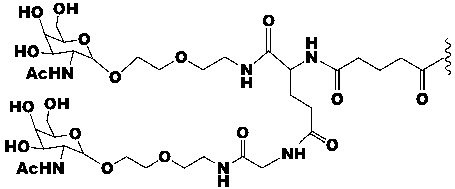 |
| 10 | 5'ERCa-02 | 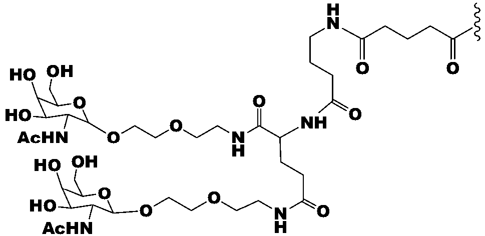 |
| 11 | 5'ERCa-03 | 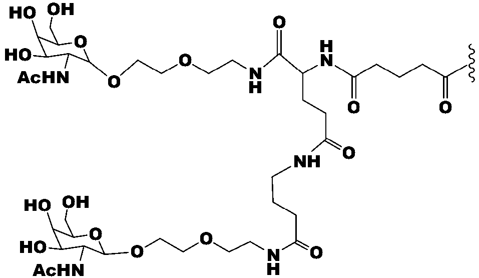 |
| 12 | 5'ERCa-04 | 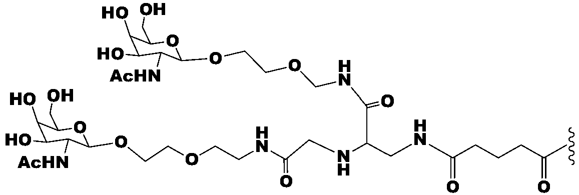 |
| 13 | 5'ERCa-05 | 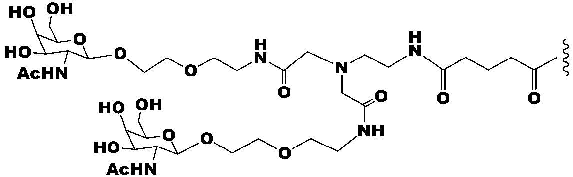 |
| 14 | 5'ERCdd-01 | 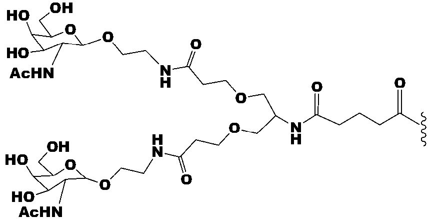 |
| 15 | 5'ERCd-01 | 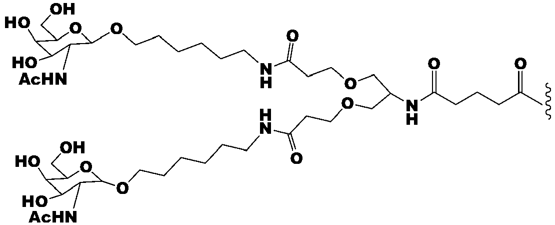 |
| 16 | 5'ERCc-01 | 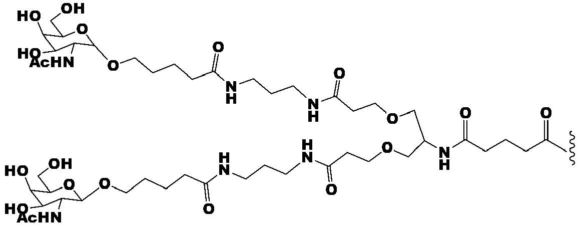 |
| 17 | 5'SANCdd-01 | 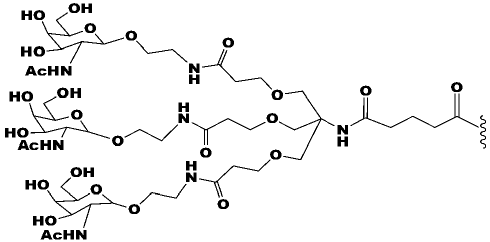 |
| 18 | 5'SANCd-01 | 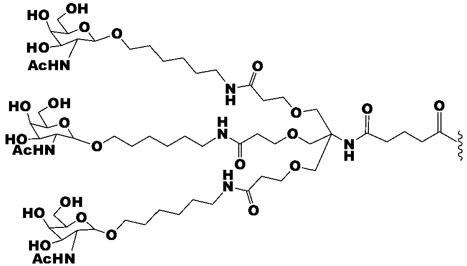 |
| 19 | 5'SANCc-01 | 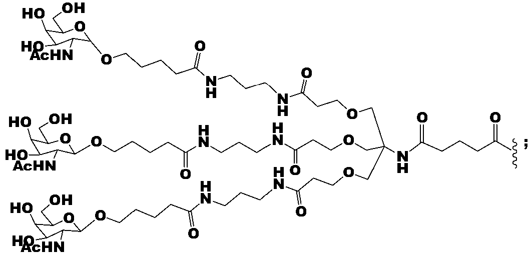 |
| 20 | 5'SANCa-01 | 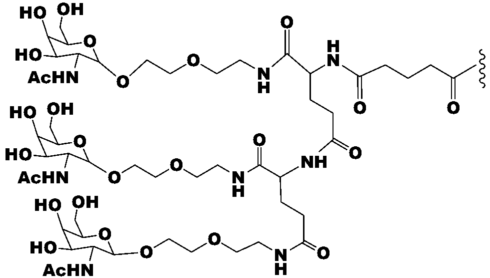 |
| 21 | 5'SANCa-02 | 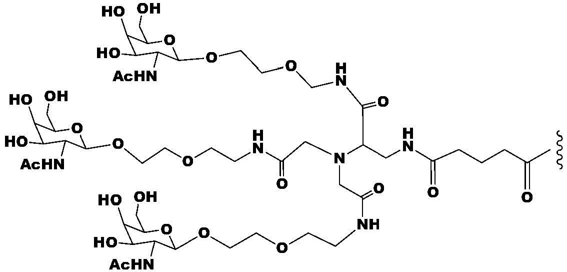 |
| 22 | 5'SANCa-03 | 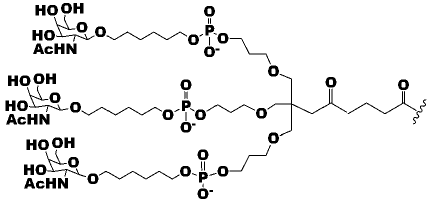 |

In some embodiments, 5'MVIP may also be absent, and m may be any integer from 2 to 4 in this case.

In some embodiments, **(X-L)ₘ-B-D-** in said 3'MVIP structure is selected from structures shown in Table 4:

**Table 4 Structures of (X-L)ₘ-B-D- of 3'MVIP**

| No. | Code | Structure |
|---|---|---|
| 1 | 3'SANCdd-01 | 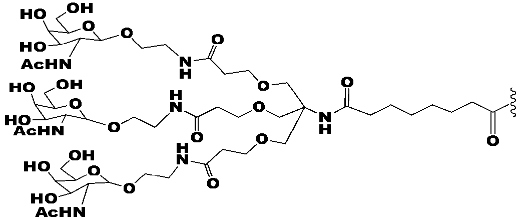 |
| 2 | 3'SANCd-01 | 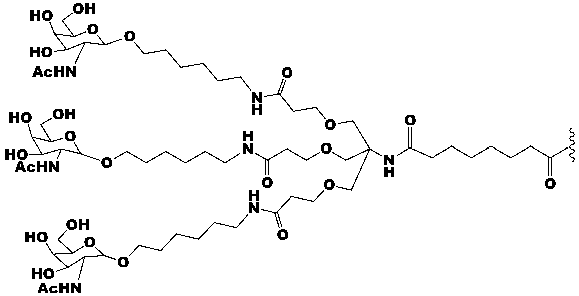 |
| 3 | 3'SANCc-01 | 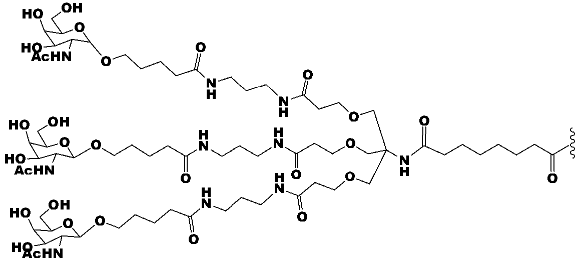 |
| 4 | 3'SANCa-01 | 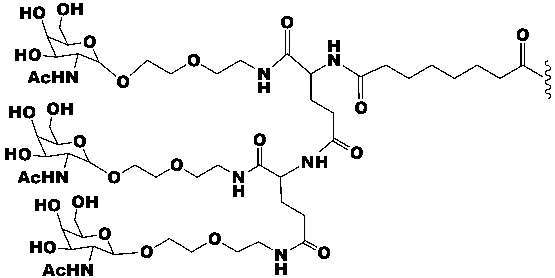 |
| 5 | 3'SANCa-02 | 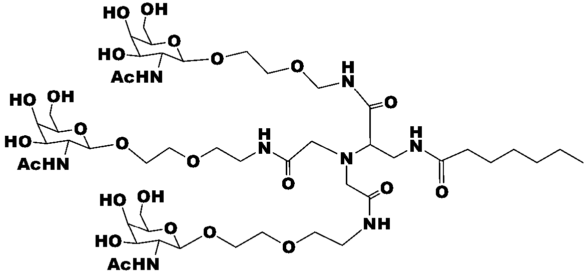 |
| 6 | 3'SANCa-03 | 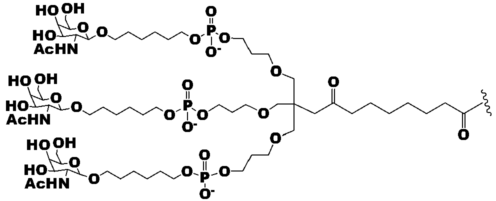 |
| 7 | 3'ERCdd-01 | 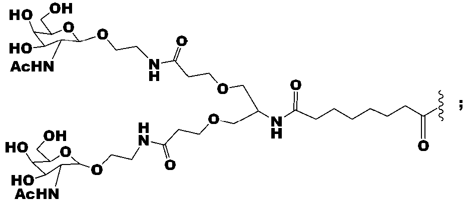 |
| 8 | 3'ERCd-01 | 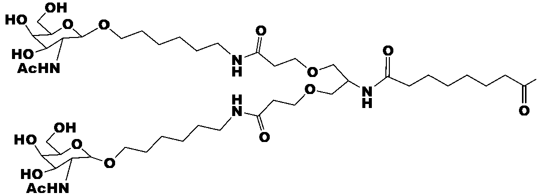 |
| 9 | 3'ERCc-01 | 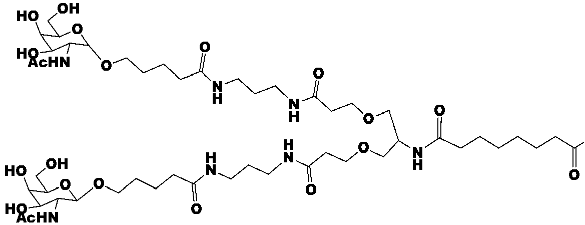 |
| 10 | 3'ERCa-01 | 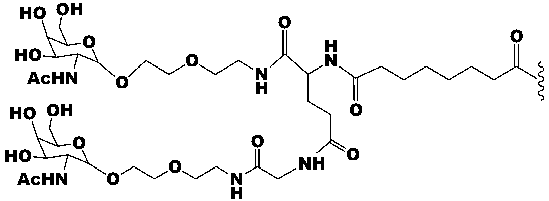 |
| 11 | 3'ERCa-02 | 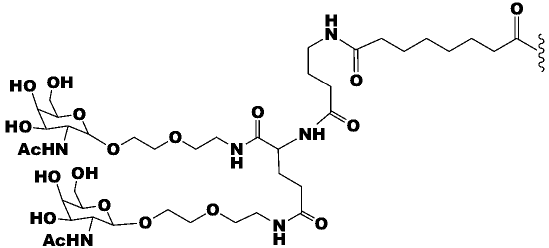 |
| 12 | 3'ERCa-03 | 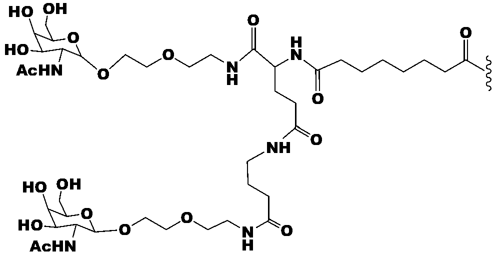 |
| 13 | 3'ERCa-04 | 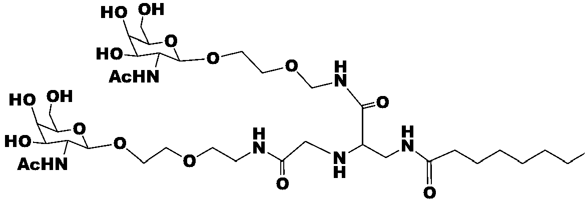 |
| 14 | 3'ERCa-05 | 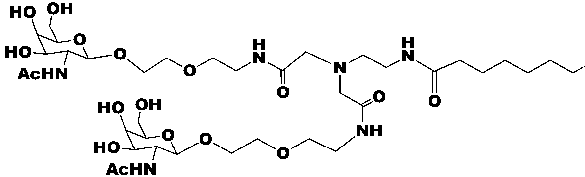 |
| 15 | 3'YICa-01 | 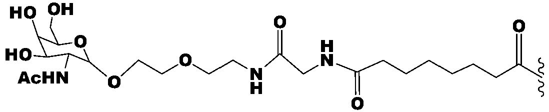 |
| 16 | 3'YICa-02 | 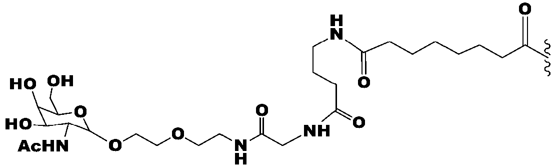 |
| 17 | 3'YICa-03 | 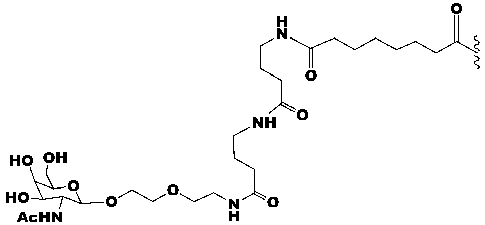 |
| 18 | 3'YICa-04 | 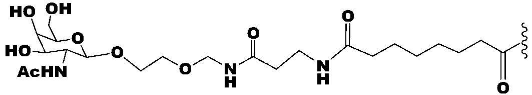 |
| 19 | 3'YICa-05 | 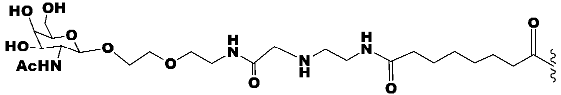 |
| 20 | 3'YICdd-01 | 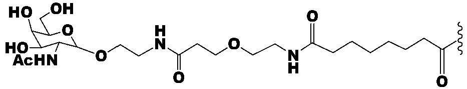 |
| 21 | 3'YICd-01 | 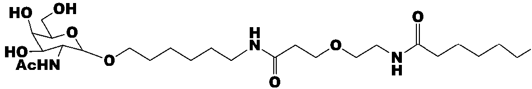 |
| 22 | 3'YICc-01 | 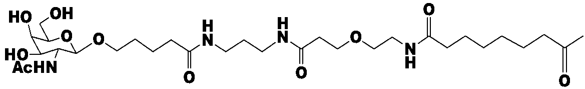 |

In some embodiments, combinations of **(X-L)ₙ-B-D-** and R₁ in said carrier structure 5'MVIP are shown in Table 5.

**Table 5 Combinations of (X-L)ₙ-B-D- and R₁ in 5'MVIP**

| No. | **(X-L)ₙ-B-D-**Code | R₁ | 5'MVIP Code |
|---|---|---|---|
| 1 | 5'YICd-01 | -NH(CH₂)₆O- | 5'MVIP01 |
| 2 | 5'YICc-01 | -NH(CH₂)₆O- | 5'MVIP02 |
| 3 | 5'YICa-01 | -O(CH₂)₆O- | 5'MVIP03 |
| 4 | 5'YICa-02 | -O(CH₂)₆O- | 5'MVIP04 |
| 5 | 5'YICa-03 | -S(CH₂)₆O- | 5'MVIP05 |
| 6 | 5'YICa-04 | -NH(CH₂)₆S- | 5'MVIP06 |
| 7 | 5'YICa-05 | -NH(CH₂)₈ O- | 5'MVIP07 |
| 8 | 5'YICr-06 | -NH(CH₂)₈O- | 5'MVIP08 |
| 9 | 5'ERCd-01 | -NH(CH₂)₆O- | 5'MVIP09 |
| 10 | 5'ERCc-01 | -NH(CH₂)₆O- | 5'MVIP10 |
| 11 | 5'ERCa-01 | -NH(CH₂)₃CH(CH₂CH₃)O- | 5'MVIP11 |
| 12 | 5'ERCa-02 | -O(CH₂)₆O- | 5'MVIP12 |
| 13 | 5'ERCa-03 | -S(CH₂)₆O- | 5'MVIP13 |
| 14 | 5'ERCa-04 | -O(CH₂)₆O- | 5'MVIP14 |
| 15 | 5'ERCa-05 | -O(CH₂)₆O- | 5'MVIP15 |
| 16 | 5'ERCr-06 | -S(CH₂)₄CH(CH₃)O- | 5'MVIP16 |
| 17 | 5'SANCd-01 | -NH(CH₂)₆O- | 5'MVIP17 |
| 18 | 5'SANCc-01 | -NH(CH₂)₆O- | 5'MVIP18 |
| 19 | 5'ERCd-01 | 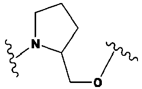 | 5'MVIP19 |
| 20 | 5'ERCd-01 | 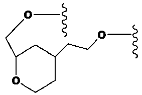 | 5'MVIP20 |
| 21 | 5'YICd-01 | 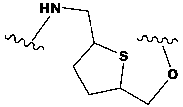 | 5'MVIP21 |
| 22 | 5'SANCd-01 | 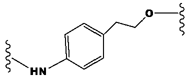 | 5'MVIP22 |

In some embodiments, 3'MVIP may be absent, and n may be any integer from 2 to 4 in this case.

In some embodiments, combinations of **(X-L)ₘ-B-D-** and R₂ in said carrier structure 3'MVIP are shown in Table 6.

**Table 6 Combination of (X-L)ₘ-B-D- and R₂ in 3'MVIP**

| No. | **(X-L)ₘ-B-D-** Code | R₂ | 3'MVIP Code |
|---|---|---|---|
| 1 | 3'YICd-01 | 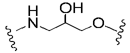 | 3'MVIP01 |
| 2 | 3'YICc-01 | 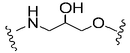 | 3'MVIP02 |
| 3 | 3'YICa-01 | 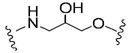 | 3'MVIP03 |
| 4 | 3'YICa-02 | 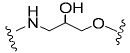 | 3'MVIP04 |
| 5 | 3'YICa-03 | 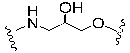 | 3'MVIP05 |
| 6 | 3'YICa-04 | 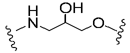 | 3'MVIP06 |
| 7 | 3'YICa-05 | 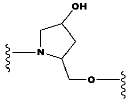 | 3'MVIP07 |
| 8 | 3'YICr-06 | 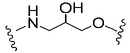 | 3'MVIP08 |
| 9 | 3'ERCd-01 | 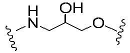 | 3'MVIP09 |
| 10 | 3'ERCc-01 | 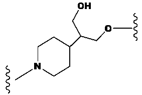 | 3'MVIP10 |
| 11 | 3'ERCa-01 | 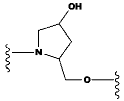 | 3'MVIP11 |
| 12 | 3'ERCa-02 | 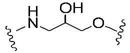 | 3'MVIP12 |
| 13 | 3'ERCa-03 | 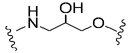 | 3'MVIP13 |
| 14 | 3'ERCa-04 | 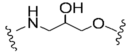 | 3'MVIP14 |
| 15 | 3'ERCa-05 | 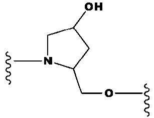 | 3'MVIP15 |
| 16 | 3'ERCr-06 | 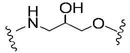 | 3'MVIP16 |
| 17 | 3'SANCd-01 | 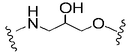 | 3'MVIP17 |
| 18 | 3'SANCc-01 | 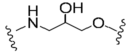 | 3'MVIP18 |
| 19 | 3'SANCa-01 | 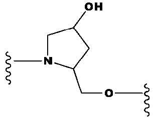 | 3'MVIP19 |
| 20 | 3'ERCd-01 | 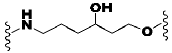 | 3'MVIP20 |
| 21 | 3'ERCd-01 | 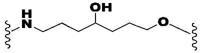 | 3'MVIP21 |
| 22 | 3'ERCd-01 | 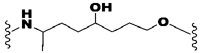 | 3'MVIP22 |
| 23 | 3'ERCd-01 | 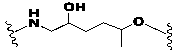 | 3'MVIP23 |
| 24 | 3'ERCd-01 | 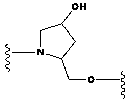 | 3'MVIP24 |
| 25 | 3'ERCd-01 | 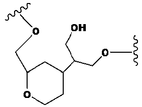 | 3'MVIP25 |
| 26 | 3'ERCd-01 | 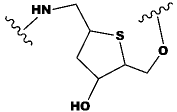 | 3'MVIP26 |
| 27 | 3'ERCd-01 | 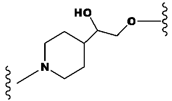 | 3'MVIP27 |

In some embodiments, said 5'MVIP is selected from any one or more of 5'MVIP01 to 5'MVIP22 in Table 5.

In some embodiments, said 3'MVIP is selected from any one or more of 3'MVIP01 to 3'MVIP27 in Table 6.

In some embodiments, any combination of 5'MVIP in Table 5 and 3'MVIP in Table 6 is possible, wherein n+m=2, 3, 4, 5 or 6.

In some embodiments, the sense strand and antisense strand conjugated to the carrier in said CFB RNA inhibitor may be selected from the following Table 7-1, the sense strand and antisense strand conjugated to the carrier in said C5 RNA inhibitor may be selected from the following Table 7-2, and the sense strand and antisense strand conjugated to the carrier in said C3 RNA inhibitor may be selected from the following Table 7-3:

**Table 7-1 Sense strand or antisense strand conjugated to carrier in CFB RNA inhibitor**

| siRNA inhibitor | SEQ ID NO : | Sense strand (5'-3') | SEQ ID NO : | Antisense strand (5'-3') |
|---|---|---|---|---|
| Kylo-17-D S2731 | 541 | 5'MVIP09-GsCsfAAfCAfUfGfU GfUUfCAfAAGsUsC | 570 | UsfGsAfCUfUUfGAAC ACfAUfGUUGCsUsA-3' MVIP09 |
| Kylo-17-D S2741 | 542 | 5'MVIP09-GsCsAACAUGfUfGf UUCAAAGUCsAsA | 571 | UsfGsAfCUfUUGAACC fAUfGUUGCsUsA-3'M VIP09 |
| Kylo-17-D S2751 | 543 | 5'MVIP09-GsCsAACAUGfUfGf UUCAAAGUCsAsA | 572 | UsfUsGfACfUUUGAAf CAfCAfUGUUsGsC-3' MVIP09 |
| Kylo-17-D S2761 | 544 | 5'MVIP09-CsAsACAUGUfGfUf UCAAAGUCAsAsU | 573 | CsfUsUfGAfCUUUGAf ACfACfAUGUsUsG-3' MVIP09 |
| Kylo-17-D S8301 | 1681 | 5'MVIP09-GsCsfAAfCAfUfGfU GfUUfUAfAAGUCsAsA | 1684 | UsfGsAfCUfUUfGAAC ACfAUfGUUGCsUsA-3' MVIP09 |
| Kylo-17-D S8311 | 1682 | 5'MVIP09-GsCsfAAfCAfUfGfU GfUUfCAfAAGUCsAsA-3'MVIP 09 | 1685 | UsfGsAfCUfUUfGAAC ACfAUfGUUGCsUsA |
| Kylo-17-D S8321 | 1683 | 5'MVIP01-GsCsfAAfCAfUfGfU GfUUfCAfAAGUCsAsA-3'MVIP 09 | 1686 | UsfGsAfCUfUUfGAAC ACfAUfGUUGCsUsA |
| Kylo-17-D S2771 | 545 | 5'MVIP09-GsCsfAAfCAfUfGfU GfUUfCAfAAGUCsAsA-3'MVIP 01 | 574 | UsfGsAfCUfUUfGAAC ACfAUfGUUGCsUsA |
| Kylo-17-D S2781 | 546 | 5'MVIP01-GsCsfAAfCAfUfGfU GfUUfCAfAAGUCsAsA | 575 | UsfGsAfCUfUUfGAAC ACfAUfGUUGCsUsA-3' MVIP17 |
| Kylo-17-D S2791 | 547 | 5'MVIP17-GsCsfAAfCAfUfGfU GfUUfCAfAAGUCsAsA | 576 | UsfGsAfCUfUUfGAAC ACfAUfGUUGCsUsA-3' MVIP01 |
| Kylo-17-D S2801 | 548 | 5'MVIP09-CsCsUGAUfCAfAfGf CUCAAGAAUsAsA | 577 | UsfUsAfUUfCUfUGAG CUfUGfAUCAGGsGsC- 3'MVIP09 |
| Kylo-17-D S2811 | 549 | 5'MVIP09-CsCsUGAUfCAfAfGf CUCAAGAAUsAsA | 578 | UsfUsAfUUfCUfUGAG CUfUGfAUCAsGsG-3' MVIP09 |
| Kylo-17-D S2821 | 550 | 5'MVIP09-GsAsAGCAfGGfAfAf UUCCUGAAUsUsU | 579 | AsfAsAfUUfCAfGGAA UUfCCfUGCUUCsUsU- 3'MVIP09 |
| Kylo-17-D S2831 | 551 | 5'MVIP09-GsCsUGUGfGUfGfU fCUGAGUACUsUsU | 580 | AsfAsAfGUfACfUCAGA CfACfCACAGCsCsC-3' MVIP09 |
| Kylo-17-D S2841 | 552 | 5'MVIP09-AsGsCUGUfGAfGfA fGAGAUGCUCsAsA | 581 | UsfUsGfAGfCAfUCUC UCfUCfACAGCUsGsC- 3'MVIP09 |
| Kylo-17-D S2851 | 553 | 5'MVIP09-AsGsCCAAfAAfAfGf UGUCUAGUCsAsA | 582 | UsfUsGfACfUAfGACAC UfUUfUUGGCUsCsC-3' MVIP09 |
| Kylo-17-D S2861 | 554 | 5'MVIP09-GsUsCUAGfUCfAfAf CUUAAUUGAsGsA | 583 | UsfCsUfCAfAUfUAAG UUfGAfCUAGACsAsC- 3'MVIP09 |
| Kylo-17-D S2871 | 555 | 5'MVIP09-GsUsCUAGfUCfAfAf CUUAAUUGAsGsA | 584 | UsfCsUfCAfAUfUAAG UUfGAfCUAGsAsC-3' MVIP09 |
| Kylo-17-D S2881 | 556 | 5'MVIP09-CsAsACAUGUfGfUf UCAAAGUCAsAsU | 585 | AsTsUGdACTUUGAdA CfACAUGUsUsG-3'MV IP09 |
| Kylo-17-D S2891 | 557 | 5'MVIP09-CsAsACAUfGUfGfUf UCAAAGUCAsAsU | 586 | AsTsUGdACTUUGAdA CfACAUGUsUsG-3'MV IP09 |
| Kylo-17-D S2901 | 558 | 5'MVIP09-GsAsAGCAfGGfAfAf UUCCUGAAUsUsU | 587 | AsdAsAUTCdAGGAAT UfCCUGCUUCsUsU-3' MVIP09 |
| Kylo-17-D S2911 | 559 | 5'MVIP09-GsUsCUAGfUCfAfAf CUUAAUUGAsGsA | 588 | UsdCsUCdAATUAAGT UfGACUAGACsAsC-3' MVIP09 |
| Kylo-17-D S2921 | 560 | 5'MVIP09-CsAsACAUGUfGfUf UCAAAGUCAsAsU | 589 | AsfUsUGAfCUfUfUGA ACfACfAUGUsUsG-3' MVIP09 |
| Kylo-17-D S2931 | 561 | 5'MVIP09-GsAsAGCAfGGfAfAf UUCCUGAAUsUsU | 590 | AsfAsAUUfCAfGfGAA UUfCCfUGCUUCsUsU- 3'MVIP09 |
| Kylo-17-D S2941 | 562 | 5'MVIP09-GsUsCUAGfUCfAfAf CUUAAUUGAsGsA | 591 | UsfCsUCAfAUfUfAAG UUfGAfCUAGACsAsC- 3'MVIP09 |
| Kylo-17-D S2951 | 563 | 5'MVIP09-GsUsCUAGUCfAfAf CUUAAUUGAsGsA | 592 | UsfCsUfCAfAUfUAAG UUfGAfCUfAGsfAsC-3' MVIP09 |
| Kylo-17-D S2961 | 564 | 5'MVIP09-GsUsCUAGfUCfAfAf CUUAAUUGAsGsA | 593 | UsfCsUfCAfAUfUAAG UUfGAfCUAGACsAsCT T-3'MVIP09 |
| Kylo-17-D S2971 | 565 | 5'MVIP09-GsUsCUAGfUCfAfAf CUUAAUUGAsGsA | 594 | UsfCsUCAfAUfUfAAG UUfGAfCUAGACsAsC- 3'MVIP09 |

**Table 7-2 Sense strand or antisense strand conjugated to carrier in C5 RNA inhibitor**

| siRNA inhibitor | SEQ ID No: | Sense strand (5'-3') | SEQ ID No: | Antisense strand (5'-3') |
|---|---|---|---|---|
| Kylo-19-DS 7501 | 1499 | 5'MVIP09-GsCsAAACfA UfAfUfGUCAUUUCAsGs U | 1560 | AsfCsUfGAfAAfUGACAUfA UfGUUUGCsUsC-3'MVIP0 9 |
| Kylo-19-DS 7511 | 1500 | 5'MVIP09-UsCsAUUUfC AfGfCfACCAAAAUsAs U | 1561 | AsfUsAfUUfUUfUGGUGCf UGfAAAUGAsCsA-3'MVIP 09 |
| Kylo-19-DS 7521 | 1501 | 5'MVIP09-GsUsUUAGf AAfGfAfUUUAAACAAsC sA | 1562 | UsfGsUfUGfUUfUAAAUCf UUfCUAAACsUsG-3'MVIP 09 |
| Kylo-19-DS 7531 | 1502 | 5'MVIP09-GsGsAAGUf UUfGfCfAGCUUUUAUs UsU | 1563 | AsfAsAfUAfAAfAGCUGCfA AfACUUCCsUsC-3'MVIP09 |
| Kylo-19-DS 7541 | 1503 | 5'MVIP09-AsGsCAUUfU UfUfAfGCUAAUUUAsG sA | 1564 | UsfCsUfAAfAUfUAGCUAfA AfAAUGCUsUsG-3'MVIP09 |
| Kylo-19-DS 7851 | 1534 | 5'MVIP09-AsUsGGAUf UUfCfUfCUUCAUUCAs UsA | 1595 | UsfAsUGAfAUfGfAAGAGf AAfAUCCAUsUsG-3'MVIP0 9 |
| Kylo-19-DS 7861 | 1535 | 5'MVIP09-UsUsGUCCfC AfGfUfAUUCUAUGUsU sU | 1596 | AsfAsACAfUAfGfAAUACfU GfGGACAAsCsG-3'MVIP09 |
| Kylo-19-DS 7871 | 1536 | 5'MVIP09-AsUsGGAUf UUfCfUfCUUCAUUCAs UsA | 1597 | UsdAsUGdAATGAAGdAGf AAAUCCAUsUsG-3'MVIP0 9 |
| Kylo-19-DS 7881 | 1537 | 5'MVIP09-UsUsGUCCfC AfGfUfAUUCUAUGUsU sU | 1598 | AsdAsACdAUdAGAAUdAC fUGGGACAAsCsG-3'MVIP 09 |
| Kylo-19-DS 7891 | 1538 | 5'MVIP09-GsAsCAAUfG GfAfUfUUCUCUUCAsUs U | 1599 | AsfAsUfGAfAGfAGAAAUfC CfAUUGUCsAsU-3'MVIP09 |
| Kylo-19-DS 7901 | 1539 | 5'MVIP09-AsUsGGAUf UUfCfUfCUUCAUUCAs UsA | 1600 | UsfAsUfGAfAUfGAAGAGf AAfAUCCAUsUsG-3'MVIP0 9 |
| Kylo-19-DS 7911 | 1540 | 5'MVIP09-CsAsAUGGfA UfUfUfCUCUUCAUUsCs A | 1601 | UsfGsAfAUfGAfAGAGAAfA UfCCAUUGsUsC-3'MVIP09 |
| Kylo-19-DS 7921 | 1541 | 5'MVIP09-AsUsGCAAfC AfAfUfCUCUAUUAAsAs A | 1602 | UsfUsUfUAfAUfAGAGAUf UGfUUGCAUsCsA-3'MVIP 09 |
| Kylo-19-DS 7931 | 1542 | 5'MVIP09-CsCsCAGUfA UfUfCfUAUGUUUUUsC sA | 1603 | UsfGsAfAAfAAfCAUAGAfA UfACUGGGsAsC-3'MVIP09 |
| Kylo-19-DS 7941 | 1543 | 5'MVIP09-CsUsUUGCfU UfGfAfAAGAAAAUAsCs C | 1604 | GsfGsUfAUfUUfUCUUUCf AAfGCAAAGsGsC-3'MVIP0 9 |
| Kylo-19-DS 7951 | 1544 | 5'MVIP09-AsAsUGGAfU UfUfCfUCUUCAUUCsAs U | 1605 | AsfUsGfAAfUGfAAGAGAfA AfUCCAUUsGsU-3'MVIP09 |
| Kylo-19-DS 7961 | 1545 | 5'MVIP09-AsGsAGAAfC AfGfCfUUAUAUCUUsAs C | 1606 | GsfUsAfAGfAUfAUAAGCfU GfUUCUCUsCsG-3'MVIP09 |
| Kylo-19-DS 7971 | 1546 | 5'MVIP09-UsGsGACAfC CfGfGfAAACAAUAAsAs U | 1607 | AsfUsUfUAfUUfGUUUCCf GGfUGUCCAsAsU-3'MVIP 09 |
| Kylo-19-DS 7981 | 1547 | 5'MVIP09-GsGsACACfC GfGfAfAACAAUAAAsUs U | 1608 | AsfAsUfUUfAUfGUUUCf CGfGUGUCCsAsA-3'MVIP0 9 |
| Kylo-19-DS 7991 | 1548 | 5'MVIP09-UsGsGUCUf UAfUfUfUGUAAAGCUs CsA | 1609 | UsfGsAfGCfUUfUACAAAfU AfAGACCAsGsC-3'MVIP09 |
| Kylo-19-DS 8001 | 1549 | 5'MVIP09-UsUsGUCCfC AfGfUfAUUCUAUGUsU sU | 1610 | AsfAsAfCAfUAfGAAUACfU GfGGACAAsCsG-3'MVIP09 |
| Kylo-19-DS 8011 | 1550 | 5'MVIP09-UsAsUCCUfC AfGfAfGAAUAAAUUsCs C | 1611 | GsfGsAfAUfUUfAUUCUCf UGfAGGAUAsAsA-3'MVIP 09 |
| Kylo-19-DS 8021 | 1551 | 5'MVIP09-GsAsUACAfC UfGfAfAGCAUUUGAsU sG | 1612 | CsfAsUfCAfAAfUGCUUCfA GfUGUAUCsCsA-3'MVIP09 |
| Kylo-19-DS 8031 | 1552 | 5'MVIP09-GsGsAAAUfC AfUfUfGGAACAUUUsU sU | 1613 | AsfAsAfAAfUGfUUCCAAfU GfAUUUCCsUsG -3'MVIP09 |
| Kylo-19-DS 8041 | 1553 | 5'MVIP09-CsCsCUUGfG AfAfAfGAGUAUUUCsAs A | 1614 | UsfUsGfAAfAUfACUCUUf UCfCAAGGGsCsU-3'MVIP0 9 |
| Kylo-19-DS 8051 | 1554 | 5'MVIP09-CsAsGGAAfA UfCfAfUUGGAACAUsUs U | 1615 | AsfAsAfUGfUUfCCAAUGfA UfUUCCUGsUsU-3'MVIP0 9 |
| Kylo-19-DS 8061 | 1555 | 5'MVIP09-GsCsCCUUfG GfAfAfAGAGUAUUUsCs A | 1616 | UsfGsAfAAfUAfCUCUUUfC CfAAGGGCsUsU-3'MVIP09 |
| Kylo-19-DS 8071 | 1556 | 5'MVIP09-GsCsAGUUfC UfGfUfGUUAAAAUGsU sC | 1617 | GsfAsCfAUfUUfUAACACfA GfAACUGCsAsU-3'MVIP09 |
| Kylo-19-DS 8081 | 1557 | 5'MVIP09-GsGsAGCAfA AfCfAfUAUGUCAUUsUs C | 1618 | GsfAsAfAUfGAfCAUAUGfU UfUGCUCCsUsG-3'MVIP09 |
| Kylo-19-DS 8091 | 1558 | 5'MVIP09-CsAsGGAGfC AfAfAfCAUAUGUCAsUs U | 1619 | AsfAsUfGAfCAfUAUGUUf UGfCUCCUGsUsC-3'MVIP0 9 |
| Kylo-19-DS 8101 | 1559 | 5'MVIP09-AsAsUGAUfG AfAfCfCUUGUAAAGsAs A | 1620 | UsfUsCfUUfUAfCAAGGUf UCfAUCAUUsUsU-3'MVIP 09 |
| Kylo-19-DS 8111 | 1560 | 5'MVIP09-CsAsfAGfAUf AUfUfUfUUAUAAUAAs AsG | 1631 | UsfUsAfUUfAUfAAAAAUf AUfCUUGsCsU-3'MVIP09 |
| Kylo-19-DS 8121 | 1561 | 5'MVIP09-CsAsAGAUA UfUfUfUUAUAAUsAsA | 1632 | UsfUsAfUUfAUfAAAAAUf AUfCUfUGsCsU-3'MVIP09 |
| Kylo-19-DS 8131 | 1562 | 5'MVIP09-GsCsAAGAU AfUfUfUUUAUAAUAsA sA | 1633 | UsfUsUfAUfUAfUAAAAAf UAfUCfUUGCsUsU-3'MVIP 09 |
| Kylo-19-DS 8141 | 1563 | 5'MVIP09-CsUsUUfAAf GfAfAUUUUGAAAUUsC sU | 1634 | AsAsUUUCfAAAAUfUCfUU AAAGsUsU -3'MVIP09 |
| Kylo-19-DS 8151 | 1564 | 5'MVIP09-CsUsUUAAfG fAfAUUUUGAAAsUsU | 1635 | AsfAsUfUUfCAfAAAUUCfU UfAAfAGsUsU -3'MVIP09 |
| Kylo-19-DS 8161 | 1565 | 5'MVIP09-CsUsfUUfAAf GfAfAUfUUfUGfAAAUU sCsU | 1636 | AsfAsUfUUfCAfAAAUUCfU UfAAfAGsUsU -3'MVIP09 |
| Kylo-19-DS 8171 | 1566 | 5'MVIP09-CsUsUUAAG AfAfUfUUUGAAAUUsCs U, | 1637 | AsfAsUfUUfCAfAAAUUCfU UfAAfAGsUsU -3'MVIP09 |
| Kylo-19-DS 8181 | 1567 | 5'MVIP09-CsUsfUUfAAf GfAfAUfUUUGAAAUUs CsC | 1638 | AsfAsUfUUfCAfAAAUUCfU UfAAAGsUsU-3'MVIP09 |
| Kylo-19-DS 8191 | 1568 | 5'MVIP09-AsAsCUUUA AfGfAfAUUUUGAAAsUs U | 1639 | AsfAsUfUUfCAfAAAUfUCf UUfAAfAGsUsU-3'MVIP09 |
| Kylo-19-DS 8201 | 1569 | 5'MVIP09-CsUsUUAAG AfAfUfUUUGAAAUUsA sC | 1640 | GsfUsAfAUfU UfCAAAfAUf UCfUUfAAsAsG -3'MVIP09 |

**Table 7-3 Sense strand or antisense strand conjugated to carrier in C3 RNA inhibitor**

| siRNA inhibitor | SEQ ID No: | Sense strand 5' to 3' | SEQ ID No: | Antisense strand 5' to 3' |
|---|---|---|---|---|
| Kylo-27-DS 8101 | 1641 | 5'MVIP09-GsGsU GUUfGAfCfAfGA UACAUsCsU | 1661 | AsdGsAUdGUdAUCUGTCfAACACCs AsU-3'MVIP09 |
| Kylo-27-DS 8111 | 1642 | 5'MVIP09-GGUG UUfGAfCfAfGAUA CAUsCsU | 1662 | AsfGsAUGfUAfUCUGUCfAAfCACCs AsU-3'MVIP09 |
| Kylo-27-DS 8121 | 1643 | 5'MVIP09-GsGsU GUUfGAfCfAfGA UACAUsCsU | 1663 | AsfGsAUGfUAfUCUGUCfAAfCACCA U-3'MVIP09 |
| Kylo-27-DS 8131 | 1644 | 5'MVIP09-GsCsU GAGfGAfGfAfAU UGCUUCAsUsA | 1664 | UsdAsUGdAAdGCAAUTCfUCCUCA GCsAsC-3'MVIP09 |
| Kylo-27-DS 8141 | 1645 | 5'MVIP09-AsGsA AAUfUCfUfAfCUA CAUCUAsUsA | 1665 | UsdAsUAdGATGUAGTAfGAAUUUC UsCsU-3'MVIP09 |
| Kylo-27-DS 8201 | 1651 | 5'MVIP09-GsCsU GAGfGAfGfAfAU UGCUUCAsUsA | 1671 | UsfAsUGAfAGfCAAUUCfUCfCUCAG CsAsC-3'MVIP09 |
| Kylo-27-DS 8171 | 1648 | 5'MVIP09-GsGsU GUUfGAfCfAfGA UACAUsCsU | 1668 | AsfGsAUGfUAfUfCUGUCfAAfCACCs AsU-3'MVIP09 |
| Kylo-27-DS 8181 | 1649 | 5'MVIP09-GsCsU GAGfGAfGfAfAU UGCUUCAsUsA | 1669 | UsfAsUGAfAGfCfAAUUCfUCfCUCA GCsAsC-3'MVIP09 |
| Kylo-27-DS 8191 | 1650 | 5'MVIP09-AsGsA AAUfUCfUfAfCUA CAUCUAsUsA | 1670 | UsfAsUAGfAUfGfUAGUAfGAfAUUU CUsCsU-3'MVIP09 |
| Kylo-27-DS 8211 | 1652 | 5'MVIP09-GsGsU GUUfGAfCfAfGA UACAUsCsU | 1672 | AsdGsAUdGUdAUCUGTCfAACACCs AsU-3'MVIP09 |
| Kylo-27-DS 8221 | 1653 | 5'MVIP09-GsGsU GUUfGAfCfAfGA UACAUsCsU | 1673 | AsfGsAUGfUAfUCUGUCfAAfCACCs AsU-3'MVIP09 |
| Kylo-27-DS 8231 | 1654 | 5'MVIP09-GsCsU GAGfGAfGfAfAU UGCUUCAsUsA | 1674 | UsfAsUGAfAGfCAAUUCfUCfCUCAG CsAsCTT-3'MVIP09 |
| Kylo-27-DS 8241 | 1655 | 5'MVIP09-AsGsA AAUfUCfUfAfCUA CAUCUAsUsA | 1675 | UsfAsUAGfAUfGUAGUAfGAfAUUU CUsCsU-3'MVIP09 |
| Kylo-27-DS 8251 | 1656 | 5'MVIP09-AsGsA AAUfUCfUfAfCUA CAUCUAsUsA | 1676 | UsfAsUAGfAUfGUAGUAfGAfAUUU CUsCsUTT-3'MVIP09 |
| Kylo-27-DS 8261 | 1657 | 5'MVIP09-GsCsU GAGGAfGfAfAUU GCUUCAsUsA | 1677 | UsfAsUfGAfAGfCAAUUCfUCfCUfCA sfGsC-3'MVIP09 |
| Kylo-27-DS 8271 | 1658 | 5'MVIP09-AsGsA AAUUCfUfAfCUA CAUCUAsUsA | 1678 | UsfAsUfAGfAUfGUAGUAfGAfAUfU UsfCsU-3'MVIP09 |
| Kylo-27-DS 8281 | 1659 | 5'MVIP09-AsUsG GUGUUfGfAfCAG AUACAUsCsU | 1679 | AsfGsAfUGfUAfUCUGUCfAAfCAfCC sfAsU-3'MVIP09 |
| Kylo-27-DS 8291 | 1660 | 5'MVIP09-AsUsG GUGfUUfGfAfCA GAUACAUsCsU | 1680 | AsfGsAUGfUAfUCUGUCfAAfCACCA UsUsGTT-3'MVIP09 |
| Kylo-27-DS 8501 | 2884 | 5'MVIP09-GsGsU GfUUfGfAfCAGA UACAUsCsU | 3130 | AsfGsAfUGUAfUCUGUCfAAfCACCs AsU-3'MVIP09 |
| Kylo-27-DS 8511 | 2885 | 5'MVIP09-GsGsfU GfUUfGAfCfAfGAf UAfCAUsCsU | 3131 | AsdGsAUdGUdAUCUGdTCfAACACC sAsU-3'MVIP09 |
| Kylo-27-DS 8521 | 2886 | 5'MVIP09-GsCsA GCUfAAfAfAfGAC UUUGACsUsU | 3132 | AsfAsGUCfAAfAGUCUUfUUfAGCU GCsAsG-3'MVIP09 |
| Kylo-27-DS 8531 | 2887 | 5'MVIP09-GsCsA GCUAAfAfAfGAC UUUGACsUsU | 3133 | AsfAsGfUCfAAAGUCfU UfUUfAGfC UfGCsAsG-3'MVIP09 |
| Kylo-27-DS 8541 | 2888 | 5'MVIP09-GsCsA GCUfAAfAfAfGAC UUUGACsUsU | 3134 | AsfAsGUCAAfAfGUCUUfUUfAGCU GCsAsG-3'MVIP09 |
| Kylo-27-DS 8551 | 2889 | 5'MVIP09-GsCsA GfCUfAfAfAAGAC UUUGACsUsU | 3135 | AsfAsGfUCAAfAGUCUUfUUfAGCU GCsAsG-3'MVIP09 |
| Kylo-27-DS 8561 | 2890 | 5'MVIP09-GsCsfA GfCUfAAfAfAfGAf CUfUUGACsUsU | 3136 | AsdAsGUdCAdAAGUCdTUfUUAGC UGCsAsG-3'MVIP09 |
| Kylo-27-DS 8571 | 2891 | 5'MVIP09-AsGsA AAUUCfUfAfCUA CAUCUAsUsA | 3137 | UsfAsUfAGfAUGUAGfUAfGAfAUfU UfCUsCsU-3'MVIP09 |
| Kylo-27-DS 8581 | 2892 | 5'MVIP09-AsGsA AAUfUCfUfAfCUA CAUCUAsUsA | 3138 | UsfAUAGfAUfGfUAGUAfGAfAUUU CUsCsU-3'MVIP09 |
| Kylo-27-DS 8591 | 2893 | 5'MVIP09-AsGsA AfAUfUfCfUACUA CAUCUAsUsA | 3139 | UsfAsUfAGAUfGUAGUAfGAfAUUU CUsCsU-3'MVIP09 |
| Kylo-27-DS 8601 | 2894 | 5'MVIP09-AsGsfA AfAUfUCfUfAfCUf ACfAUCUAsUsA | 3140 | UsdAsUAdGAdTGUAGdTAfGAAUU UCUsCsU-3'MVIP09 |
| Kylo-27-DS 8611 | 2895 | 5'MVIP09-GsGsA GCCfUAfCfAfGAG AAAUUCsUsA | 3141 | UsfAsGAAfUUfUCUCUGfUAfGGCU CCsAsC-3'MVIP09 |
| Kylo-27-DS 8621 | 2896 | 5'MVIP09-GsGsA GCCUAfCfAfGAG AAAUUCsUsA | 3142 | UsfAsGfAAfUUUCUCfUGfUAfGGfC UfCCsAsC-3'MVIP09 |
| Kylo-27-DS 8631 | 2897 | 5'MVIP09-GsGsA GCCfUAfCfAfGAG AAAUUCsUsA | 3143 | UsfAsGAAfUUfUfCUCUGfUAfGGCU CCsAsC-3'MVIP09 |
| Kylo-27-DS 8641 | 2898 | 5'MVIP09-GsGsA GfCCfUfAfCAGAG AAAUUCsUsA | 3144 | UsfAsGAAUUfUCUCUGfUAfGGCUC CsAsC-3'MVIP09 |
| Kylo-27-DS 8651 | 2899 | 5'MVIP09-GsGsfA GfCCfUAfCfAfGAf GAfAAUUCsUsA | 3145 | UsdAsGAdAUdTUCUCdTGfUAGGC UCCsAsC-3'MVIP09 |
| Kylo-27-DS 8661 | 2900 | 5'MVIP09-CsCsA GACfAGfAfCfAAG ACCAUCsUsA | 3146 | UsfAsGAUfGGfUCUUGUfCUfGUCU GGsAsU-3'MVIP09 |
| Kylo-27-DS 8671 | 2901 | 5'MVIP09-CsCsA GACAGfAfCfAAG ACCAUCsUsA | 3147 | UsfAsGfAUfGGUCUUfGUfCUfGUfC UfGGsAsU-3'MVIP09 |
| Kylo-27-DS 8681 | 2902 | 5'MVIP09-CsCsA GACfAGfAfCfAAG ACCAUCsUsA | 3148 | UsfAsGAUfGGfUfCUUGUfCUfGUCU GGsAsU-3'MVIP09 |
| Kylo-27-DS 8691 | 2903 | 5'MVIP09-CsCsA GfACfAfGfACAAG ACCAUCsUsA | 3149 | UsfAsGfAUGGfUCUUGUfCUfGUCU GGsAsU-3'MVIP09 |
| Kylo-27-DS 8701 | 2904 | 5'MVIP09-CsCsfA GfACfAGfAfCfAAf GAfCCAUCsUsA | 3150 | UsdAsGAdTGdGUCUUdGUfCUGUC UGGsAsU-3'MVIP09 |
| Kylo-27-DS 8711 | 2905 | 5'MVIP09-CsCsA GACfAGfAfCfAAG ACCAUCsUsA | 3151 | AsfGsUUUfGUfUCUUCUfUAfUUCA GCsAsC-3'MVIP09 |
| Kylo-27-DS 8721 | 2906 | 5'MVIP09-CsCsA GACAGfAfCfAAG ACCAUCsUsA | 3152 | AsfGsUfUUfGUUCUUfCUfUAfUUfC AfGCsAsC-3'MVIP09 |
| Kylo-27-DS 8731 | 2907 | 5'MVIP09-CsCsA GACfAGfAfCfAAG ACCAUCsUsA | 3153 | AsfGsUUUfGUfUCUUCUfUAfUUCA GCsAsC-3'MVIP09 |
| Kylo-27-DS 8741 | 2908 | 5'MVIP09-CsCsA GfACfAfGfACAAG ACCAUCsUsA | 3154 | AsfGsUUUGUfCUUCUfAfUUCAG CsAsC-3'MVIP09 |
| Kylo-27-DS 8751 | 2909 | 5'MVIP09-CsCsfA GfACfAGfAfCfAAf GAfCCAUCsUsA | 3155 | AsdGsUUdTGdTUCUUdCUfUAUUC AGCsAsC-3'MVIP09 |
| Kylo-27-DS 8761 | 2910 | 5'MVIP09-GsAsG CCGfUUfCfUfCUA CAAUUAsCsU | 3156 | AsfGsUAAfUUfGfUAGAGfAAfCGGC UCsGsG-3'MVIP09 |
| Kylo-27-DS 8771 | 2911 | 5'MVIP09-GsGsA GAAfUUfGfCfUUC AUACAAsAsA | 3157 | UsfUsUUGfUAfUGAAGCfAAfUUCU CCsUsC-3'MVIP09 |
| Kylo-27-DS 8781 | 2912 | 5'MVIP09-GsGsA GAAUUfGfCfUUC AUACAAsAsA | 3158 | AsfGsUfUUfGUUCUUfCUfUAfUUfC AfGCsAsC-3'MVIP09 |
| Kylo-27-DS 8791 | 2913 | 5'MVIP09-GsGsA GAAfUUfGfCfUUC AUACAAsAsA | 3159 | AsfGsUUUfGUfUfCUUCUfUAfUUCA GCsAsC-3'MVIP09 |
| Kylo-27-DS 8801 | 2914 | 5'MVIP09-GsGsA GfAAfUfUfGCUUC AUACAAsAsA | 3160 | AsfGUfUUGUfCUUCUfAfUUCAG CsAsC-3'MVIP09 |
| Kylo-27-DS 8811 | 2915 | 5'MVIP09-GsGsfA GfAAfUUfGfCfUU fCAfUACAAsAsA | 3161 | AsdGsUUdTGdTUCUUdCUfUAUUC AGCsAsC-3'MVIP09 |
| Kylo-27-DS 8821 | 2916 | 5'MVIP09-GsCsU GAGfGAfGfAfAU UGCUUCAsUsA | 3162 | UsfAsUGAfAGfCAAUUCfUCfCUCAG CsAsC-3'MVIP09 |
| Kylo-27-DS 8831 | 2917 | 5'MVIP09-GsCsU GAGGAfGfAfAUU GCUUCAsUsA | 3163 | UsfAsUfGAfAGCAAUfUCfUCfCUfCA fGCsAsC-3'MVIP09 |
| Kylo-27-DS 8841 | 2918 | 5'MVIP09-GsCsU GAGfGAfGfAfAU UGCUUCAsUsA | 3164 | UsfAsUGAfAGfCfAAUUCfUCfCUCA GCsAsC-3'MVIP09 |
| Kylo-27-DS 8851 | 2919 | 5'MVIP09-GsCsU GfAGfGfAfGAAU UGCUUCAsUsA | 3165 | UsfAsUfGAAGfCAAUUCfUCfCUCAG CsAsC-3'MVIP09 |
| Kylo-27-DS 8861 | 2920 | 5'MVIP09-GsCsfU GfAGfGAfGfAfAU fUGfCUUCAsUsA | 3166 | UsdAsUGdAAdGCAAUdTCfUCCUCA GCsAsC-3'MVIP09 |
| Kylo-27-DS 8871 | 2921 | 5'MVIP09-GsGsU GUUfGAfCfAfGA UACAUsCsU | 3167 | AsfGsAUGfUAfUCUGUCfAAfCACCs AsU-3'MVIP09 |
| Kylo-27-DS 8881 | 2922 | 5'MVIP10-GsGsU GUUfGAfCfAfGA UACAUsCsU | 3168 | AsfGsAUGfUAfUCUGUCfAAfCACCs AsU-3'MVIP02 |
| Kylo-27-DS 8891 | 2923 | 5'MVIP18-GsGsU GUUfGAfCfAfGA UACAUsCsU | 3169 | AsfGsAUGfUAfUCUGUCfAAfCACCs AsU-3'MVIP11 |
| Kylo-27-DS 8901 | 2924 | 5'MVIP19-GsGsU GUUfGAfCfAfGA UACAUsCsU | 3170 | AsfGsAUGfUAfUCUGUCfAAfCACCs AsU-3'MVIP18 |
| Kylo-27-DS 8911 | 2925 | 5'MVIP09-GsGsU GUUGAfCfAfGAU ACAUsCsU | 3171 | AsfGsAfUGfUAUCUGfUCfAAfCAfCC sfAsU-3'MVIP09 |
| Kylo-27-DS 8921 | 2926 | 5'MVIP17-GsGsU GUUGAfCfAfGAU ACAUsCsU | 3172 | AsfGsAfUGfUAUCUGfUCfAAfCAfCC sfAsU-3'MVIP01 |
| Kylo-27-DS 8931 | 2927 | 5'MVIP01-GsGsU GUUGAfCfAfGAU ACAUsCsU | 3173 | AsfGsAfUGfUAUCUGfUCfAAfCAfCC sfAsU-3'MVIP17 |
| Kylo-27-DS 8941 | 2928 | 5'MVIP17-GsGsU GUUGAfCfAfGAU ACAUsCsU | 3174 | AsfGsAfUGfUAUCUGfUCfAAfCAfCC sfAsU-3'MVIP09 |
| Kylo-27-DS 8951 | 2929 | 5'MVIP09-GsGsU GUUGAfCfAfGAU ACAUsCsU | 3175 | AsfGsAfUGfUAUCUGfUCfAAfCAfCC sfAsU-3'MVIP17 |
| Kylo-27-DS 8961 | 2930 | 5'MVIP17-GsGsU GUUGAfCfAfGAU ACAUsCsU | 3176 | AsfGsAfUGfUAUCUGfUCfAAfCAfCC sfAsU-3'MVIP17 |
| Kylo-27-DS 8971 | 2931 | 5'MVIP09-GsGsU GUUfGAfCfAfGA UACAUsCsU | 3177 | AsfGsAUGfUAfUfCUGUCfAAfCACCs AsU-3'MVIP09 |
| Kylo-27-DS 8981 | 2932 | 5'MVIP01-GsGsU GUUfGAfCfAfGA UACAUsCsU | 3178 | AsfGsAUGfUAfUfCUGUCfAAfCACCs AsU-3'MVIP01 |
| Kylo-27-DS 8991 | 2933 | 5'MVIP17-GsGsU GUUfGAfCfAfGA UACAUsCsU | 3179 | AsfGsAUGfUAfUfCUGUCfAAfCACCs AsU-3'MVIP17 |
| Kylo-27-DS 9001 | 2934 | 5'MVIP09-GsGsU GUUfGAfCfAfGA UACAUsCsU | 3180 | AsfGsAUGfUAfUfCUGUCfAAfCACCs AsU-3'MVIP17 |
| Kylo-27-DS 9011 | 2935 | 5'MVIP17-GsGsU GUUfGAfCfAfGA UACAUsCsU | 3181 | AsfGsAUGfUAfUfCUGUCfAAfCACCs AsU-3'MVIP09 |
| Kylo-27-DS 9021 | 2936 | 5'MVIP17-GsGsU GUUfGAfCfAfGA UACAUsCsU | 3182 | AsfGsAUGfUAfUfCUGUCfAAfCACCs AsU-3'MVIP17 |
| Kylo-27-DS 9031 | 2937 | 5'MVIP09-GsGsU GfUUfGfAfCAGA UACAUsCsU | 3183 | AsfGsAUGfUAfUfCUGUCfAAfCACCs AsU-3'MVIP09 |
| Kylo-27-DS 9041 | 2938 | 5'MVIP17-GsGsU GfUUfGfAfCAGA UACAUsCsU | 3184 | AsfGsAUGfUAfUfCUGUCfAAfCACCs AsU-3'MVIP17 |
| Kylo-27-DS 9051 | 2939 | 5'MVIP01-GsGsU GfUUfGfAfCAGA UACAUsCsU | 3185 | AsfGsAUGfUAfUfCUGUCfAAfCACCs AsU-3'MVIP01 |
| Kylo-27-DS 9061 | 2940 | 5'MVIP09-GsGsU GfUUfGfAfCAGA UACAUsCsU | 3186 | AsfGsAUGfUAfUfCUGUCfAAfCACCs AsU-3'MVIP17 |
| Kylo-27-DS 9071 | 2941 | 5'MVIP17-GsGsU GfUUfGfAfCAGA UACAUsCsU | 3187 | AsfGsAUGfUAfUfCUGUCfAAfCACCs AsU-3'MVIP09 |
| Kylo-27-DS 9081 | 2942 | 5'MVIP17-GsGsU GfUUfGfAfCAGA UACAUsCsU | 3188 | AsfGsAUGfUAfUfCUGUCfAAfCACCs AsU-3'MVIP17 |
| Kylo-27-DS 9091 | 2943 | 5'MVIP09-GsGsfU GfUUfGAfCfAfGAf UAfCAUsCsU | 3189 | AsdGsAUdGUdAUCUGdTCfAACACC sAsU-3'MVIP09 |
| Kylo-27-DS 9101 | 2944 | 5'MVIP01-GsGsfU GfUUfGfAfCAfGAf UAfCAUsCsU | 3190 | AsdGsAUdGUdAUCUGdTCfAACACC sAsU-3'MVIP17 |
| Kylo-27-DS 9111 | 2945 | 5'MVIP17-GsGsfU GfUUfGfAfCAfGAf UAfCAUsCsU | 3191 | AsdGsAUdGUdAUCUGdTCfAACACC sAsU-3'MVIP01 |
| Kylo-27-DS 9121 | 2946 | 5'MVIP09-GsGsfU GfUUfGfAfCAfGAf UAfCAUsCsU | 3192 | AsdGsAUdGUdAUCUGdTCfAACACC sAsU-3'MVIP09 |
| Kylo-27-DS 9131 | 2947 | 5'MVIP17-GsGsfU GfUUfGfAfCAfGAf UAfCAUsCsU | 3193 | AsdGsAUdGUdAUCUGdTCfAACACC sAsU-3'MVIP17 |
| Kylo-27-DS 9141 | 2948 | 5'MVIP17-GsGsfU GfUUfGfAfCAfGAf UAfCAUsCsU | 3194 | AsdGsAUdGUdAUCUGdTCfAACACC sAsU-3'MVIP17 |
| Kylo-27-DS 9151 | 2949 | 5'MVIP09-AsGsA AAUfUCfUfAfCUA CAUCUAsUsA | 3195 | UsfAsUAGfAUfGUAGUAfGAfAUUU CUsCsU-3'MVIP09 |
| Kylo-27-DS 9161 | 2950 | 5'MVIP02-AsGsA AAUfUCfUfAfCUA CAUCUAsUsA | 3196 | UsfAsUAGfAUfGUAGUAfGAfAUUU CUsCsU-3'MVIP10 |
| Kylo-27-DS 9171 | 2951 | 5'MVIP10-AsGsA AAUfUCfUfAfCUA CAUCUAsUsA | 3197 | UsfAsUAGfAUfGUAGUAfGAfAUUU CUsCsU-3'MVIP02 |
| Kylo-27-DS 9181 | 2952 | 5'MVIP18-AsGsA AAUfUCfUfAfCUA CAUCUAsUsA | 3198 | UsfAsUAGfAUfGUAGUAfGAfAUUU CUsCsU-3'MVIP11 |
| Kylo-27-DS 9191 | 2953 | 5'MVIP19-AsGsA AAUfUCfUfAfCUA CAUCUAsUsA | 3199 | UsfAsUAGfAUfGUAGUAfGAfAUUU CUsCsU-3'MVIP18 |
| Kylo-27-DS 9201 | 2954 | 5'MVIP09-AsGsA AAUUCfUfAfCUA CAUCUAsUsA | 3200 | UsfAsUfAGfAUGUAGfUAfGAfAUfU UfCUsCsU-3'MVIP09 |
| Kylo-27-DS 9211 | 2955 | 5'MVIP01-AsGsA AAUUCfUfAfCUA CAUCUAsUsA | 3201 | UsfAsUfAGfAUGUAGfUAfGAfAUfU UfCUsCsU-3'MVIP17 |
| Kylo-27-DS 9221 | 2956 | 5'MVIP17-AsGsA AAUUCfUfAfCUA CAUCUAsUsA | 3202 | UsfAsUfAGfAUGUAGfUAfGAfAUfU UfCUsCsU-3'MVIP01 |
| Kylo-27-DS 9231 | 2957 | 5'MVIP09-AsGsA AAUUCfUfAfCUA CAUCUAsUsA | 3203 | UsfAsUfAGfAUGUAGfUAfGAfAUfU UfCUsCsU-3'MVIP17 |
| Kylo-27-DS 9241 | 2958 | 5'MVIP17-AsGsA AAUUCfUfAfCUA CAUCUAsUsA | 3204 | UsfAsUfAGfAUGUAGfUAfGAfAUfU UfCUsCsU-3'MVIP09 |
| Kylo-27-DS 9251 | 2959 | 5'MVIP17-AsGsA AAUUCfUfAfCUA CAUCUAsUsA | 3205 | UsfAsUfAGfAUGUAGfUAfGAfAUfU UfCUsCsU-3'MVIP17 |
| Kylo-27-DS 9261 | 2960 | 5'MVIP09-AsGsA AAUfUCfUfAfCUA CAUCUAsUsA | 3206 | UsfAUAGfAUfGfUAGUAfGAfAUUU CUsCsU-3'MVIP09 |
| Kylo-27-DS 9271 | 2961 | 5'MVIP01-AsGsA AAUfUCfUfAfCUA CAUCUAsUsA | 3207 | UsfAUAGfAUfGfUAGUAfGAfAUUU CUsCsU-3'MVIP17 |
| Kylo-27-DS 9281 | 2962 | 5'MVIP17-AsGsA AAUfUCfUfAfCUA CAUCUAsUsA | 3208 | UsfAUAGfAUfGfUAGUAfGAfAUUU CUsCsU-3'MVIP01 |
| Kylo-27-DS 9291 | 2963 | 5'MVIP09-AsGsA AAUfUCfUfAfCUA CAUCUAsUsA | 3209 | UsfAUAGfAUfGfUAGUAfGAfAUUU CUsCsU-3'MVIP17 |
| Kylo-27-DS 9301 | 2964 | 5'MVIP17-AsGsA AAUfUCfUfAfCUA CAUCUAsUsA | 3210 | UsfAUAGfAUfGfUAGUAfGAfAUUU CUsCsU-3'MVIP09 |
| Kylo-27-DS 9311 | 2965 | 5'MVIP17-AsGsA AAUfUCfUfAfCUA CAUCUAsUsA | 3211 | UsfAUAGfAUfGfUAGUAfGAfAUUU CUsCsU-3'MVIP09 |
| Kylo-27-DS 9321 | 2966 | 5'MVIP09-AsGsA AfAUfUfCfUACUA CAUCUAsUsA | 3212 | UsfAsUfAGAUfGUAGUAfGAfAUUU CUsCsU-3'MVIP09 |
| Kylo-27-DS 9331 | 29676 | 5'MVIP01-AsGsA AfAUfUfCfUACUA CAUCUAsUsA | 3213 | UsfAsUfAGAUfGUAGUAfGAfAUUU CUsCsU-3'MVIP17 |
| Kylo-27-DS 9341 | 2968 | 5'MVIP17-AsGsA AfAUfUfCfUACUA CAUCUAsUsA | 3214 | UsfAsUfAGAUfGUAGUAfGAfAUUU CUsCsU-3'MVIP01 |
| Kylo-27-DS 9351 | 2969 | 5'MVIP019-AsGs AAfAUfUfCfUACU ACAUCUAsUsA | 3215 | UsfAsUfAGAUfGUAGUAfGAfAUUU CUsCsU-3'MVIP17 |
| Kylo-27-DS 9361 | 2970 | 5'MVIP17-AsGsA AfAUfUfCfUACUA CAUCUAsUsA | 3216 | UsfAsUfAGAUfGUAGUAfGAfAUUU CUsCsU-3'MVIP09 |
| Kylo-27-DS 9371 | 2971 | 5'MVIP09-AsGsA AfAUfUfCfUACUA CAUCUAsUsA | 3217 | UsfAsUfAGAUfGUAGUAfGAfAUUU CUsCsU-3'MVIP17 |
| Kylo-27-DS 9381 | 2972 | 5'MVIP09-AsGsfA AfAUfUCfUfAfCUf ACfAUCUAsUsA | 3218 | UsdAsUAdGAdTGUAGdTAfGAAUU UCUsCsU-3'MVIP09 |
| Kylo-27-DS 9391 | 2973 | 5'MVIP01-AsGsfA AfAUfUfCfUAfCUf ACfAUCUAsUsA | 3219 | UsdAsUAdGAdTGUAGdTAfGAAUU UCUsCsU-3'MVIP17 |
| Kylo-27-DS 9401 | 2974 | 5'MVIP17-AsGsfA AfAUfUfCfUAfCUf ACfAUCUAsUsA | 3220 | UsdAsUAdGAdTGUAGdTAfGAAUU UCUsCsU-3'MVIP01 |
| Kylo-27-DS 9411 | 2975 | 5'MVIP01-AsGsfA AfAUfUfCfUAfCUf ACfAUCUAsUsA | 3221 | UsdAsUAdGAdTGUAGdTAfGAAUU UCUsCsU-3'MVIP17 |
| Kylo-27-DS 9421 | 2976 | 5'MVIP17-AsGsfA AfAUfUfCfUAfCUf ACfAUCUAsUsA | 3222 | UsdAsUAdGAdTGUAGdTAfGAAUU UCUsCsU-3'MVIP09 |
| Kylo-27-DS 9431 | 2977 | 5'MVIP17-AsGsfA AfAUfUfCfUAfCUf ACfAUCUAsUsA | 3223 | UsdAsUAdGAdTGUAGdTAfGAAUU UCUsCsU-3'MVIP17 |
| Kylo-27-DS 9441 | 2978 | 5'MVIP09-GsGsA GCCfUAfCfAfGAG AAAUUCsUsA | 3224 | UsfAsGAAfUUfUCUCUGfUAfGGCU CCsAsC-3'MVIP09 |
| Kylo-27-DS 9451 | 2979 | 5'MVIP01-GsGsA GCCfUAfCfAfGAG AAAUUCsUsA | 3225 | UsfAsGAAfUUfUCUCUGfUAfGGCU CCsAsC-3'MVIP17 |
| Kylo-27-DS 9461 | 2980 | 5'MVIP17-GsGsA GCCfUAfCfAfGAG AAAUUCsUsA | 3226 | UsfAsGAAfUUfUCUCUGfUAfGGCU CCsAsC-3'MVIP01 |
| Kylo-27-DS 9471 | 2981 | 5'MVIP09-GsGsA GCCfUAfCfAfGAG AAAUUCsUsA | 3227 | UsfAsGAAfUUfUCUCUGfUAfGGCU CCsAsC-3'MVIP17 |
| Kylo-27-DS 9481 | 2982 | 5'MVIP17-GsGsA GCCfUAfCfAfGAG AAAUUCsUsA | 3228 | UsfAsGAAfUUfUCUCUGfUAfGGCU CCsAsC-3'MVIP09 |
| Kylo-27-DS 9491 | 2983 | 5'MVIP17-GsGsA GCCfUAfCfAfGAG AAAUUCsUsA | 3229 | UsfAsGAAfUUfUCUCUGfUAfGGCU CCsAsC-3'MVIP17 |
| Kylo-27-DS 9501 | 2984 | 5'MVIP02-GsGsA GCCfUAfCfAfGAG AAAUUCsUsA | 3230 | UsfAsGAAfUUfUCUCUGfUAfGGCU CCsAsC-3'MVIP10 |
| Kylo-27-DS 9511 | 2985 | 5'MVIP10-GsGsA GCCfUAfCfAfGAG AAAUUCsUsA | 3231 | UsfAsGAAfUUfUCUCUGfUAfGGCU CCsAsC-3'MVIP02 |
| Kylo-27-DS 9521 | 2986 | 5'MVIP18-GsGsA GCCfUAfCfAfGAG AAAUUCsUsA | 3232 | UsfAsGAAfUUfUCUCUGfUAfGGCU CCsAsC-3'MVIP11 |
| Kylo-27-DS 9531 | 2987 | 5'MVIP19-GsGsA GCCfUAfCfAfGAG AAAUUCsUsA | 3233 | UsfAsGAAfUUfUCUCUGfUAfGGCU CCsAsC-3'MVIP18 |
| Kylo-27-DS 9541 | 2988 | 5'MVIP09-GsGsA GCCUAfCfAfGAG AAAUUCsUsA | 3234 | UsfAsGfAAfUUUCUCfUGfUAfGGfC UfCCsAsC-3'MVIP09 |
| Kylo-27-DS 9551 | 2989 | 5'MVIP01-GsGsA GCCUAfCfAfGAG AAAUUCsUsA | 3235 | UsfAsGfAAfUUUCUCfUGfUAfGGfC UfCCsAsC-3'MVIP17 |
| Kylo-27-DS 9561 | 2990 | 5'MVIP17-GsGsA GCCUAfCfAfGAG AAAUUCsUsA | 3236 | UsfAsGfAAfUUUCUCfUGfUAfGGfC UfCCsAsC-3'MVIP01 |
| Kylo-27-DS 9571 | 2991 | 5'MVIP09-GsGsA GCCUAfCfAfGAG AAAUUCsUsA | 3237 | UsfAsGfAAfUUUCUCfUGfUAfGGfC UfCCsAsC-3'MVIP17 |
| Kylo-27-DS 9581 | 2992 | 5'MVIP17-GsGsA GCCUAfCfAfGAG AAAUUCsUsA | 3238 | UsfAsGfAAfUUUCUCfUGfUAfGGfC UfCCsAsC-3'MVIP09 |
| Kylo-27-DS 9591 | 2993 | 5'MVIP17-GsGsA GCCUAfCfAfGAG AAAUUCsUsA | 3239 | UsfAsGfAAfUUUCUCfUGfUAfGGfC UfCCsAsC-3'MVIP17 |
| Kylo-27-DS 9601 | 2994 | 5'MVIP09-GsGsA GCCfUAfCfAfGAG AAAUUCsUsA | 3240 | UsfAsGAAfUUfUfCUCUGfUAfGGCU CCsAsC-3'MVIP09 |
| Kylo-27-DS 9611 | 2995 | 5'MVIP01-GsGsA GCCfUAfCfAfGAG AAAUUCsUsA | 3241 | UsfAsGAAfUUfUfCUCUGfUAfGGCU CCsAsC-3'MVIP01 |
| Kylo-27-DS 9621 | 2996 | 5'MVIP17-GsGsA GCCfUAfCfAfGAG AAAUUCsUsA | 3242 | UsfAsGAAfUUfUfCUCUGfUAfGGCU CCsAsC-3'MVIP17 |
| Kylo-27-DS 9631 | 2997 | 5'MVIP09-GsGsA GCCfUAfCfAfGAG AAAUUCsUsA | 3243 | UsfAsGAAfUUfUfCUCUGfUAfGGCU CCsAsC-3'MVIP17 |
| Kylo-27-DS 9641 | 2998 | 5'MVIP17-GsGsA GCCfUAfCfAfGAG AAAUUCsUsA | 3244 | UsfAsGAAfUUfUfCUCUGfUAfGGCU CCsAsC-3'MVIP09 |
| Kylo-27-DS 9651 | 2999 | 5'MVIP17-GsGsA GCCfUAfCfAfGAG AAAUUCsUsA | 3245 | UsfAsGAAfUUfUfCUCUGfUAfGGCU CCsAsC-3'MVIP17 |
| Kylo-27-DS 9661 | 3000 | 5'MVIP02-GsGsA GCCfUAfCfAfGAG AAAUUCsUsA | 3246 | UsfAsGAAUUfUCUCUGfUAfGGCUC CsAsC-3'MVIP09 |
| Kylo-27-DS 9671 | 3001 | 5'MVIP10-GsGsA GCCfUAfCfAfGAG AAAUUCsUsA | 3247 | UsfAsGAAUUfUCUCUGfUAfGGCUC CsAsC-3'MVIP17 |
| Kylo-27-DS 9681 | 3002 | 5'MVIP18-GsGsA GCCfUAfCfAfGAG AAAUUCsUsA | 3248 | UsfAsGAAUUfUCUCUGfUAfGGCUC CsAsC-3'MVIP01 |
| Kylo-27-DS 9691 | 3003 | 5'MVIP19-GsGsA GCCfUAfCfAfGAG AAAUUCsUsA | 3249 | UsfAsGAAUUfUCUCUGfUAfGGCUC CsAsC-3'MVIP17 |
| Kylo-27-DS 9701 | 3004 | 5'MVIP09-GsGsA GCCUAfCfAfGAG AAAUUCsUsA | 3250 | UsfAsGAAUUfUCUCUGfUAfGGCUC CsAsC-3'MVIP09 |
| Kylo-27-DS 9711 | 3005 | 5'MVIP01-GsGsA GCCUAfCfAfGAG AAAUUCsUsA | 3251 | UsfAsGAAUUfUCUCUGfUAfGGCUC CsAsC-3'MVIP17 |
| Kylo-27-DS 9721 | 3006 | 5'MVIP17-GsGsA GCCUAfCfAfGAG AAAUUCsUsA | 3252 | UsdAsGAdAUdTUCUCdTGfUAGGC UCCsAsC-3'MVIP09 |
| Kylo-27-DS 9731 | 3007 | 5'MVIP09-GsGsA GCCUAfCfAfGAG AAAUUCsUsA | 3253 | UsdAsGAdAUdTUCUCdTGfUAGGC UCCsAsC-3'MVIP17 |
| Kylo-27-DS 9741 | 3008 | 5'MVIP17-GsGsA GCCUAfCfAfGAG AAAUUCsUsA | 3254 | UsdAsGAdAUdTUCUCdTGfUAGGC UCCsAsC-3'MVIP01 |
| Kylo-27-DS 9751 | 3009 | 5'MVIP17-GsGsA GCCUAfCfAfGAG AAAUUCsUsA | 3255 | UsdAsGAdAUdTUCUCdTGfUAGGC UCCsAsC-3'MVIP17 |
| Kylo-27-DS 9761 | 3010 | 5'MVIP09-GsGsA GfCCfUfAfCAGAG AAAUUCsUsA | 3256 | UsdAsGAdAUdTUCUCdTGfUAGGC UCCsAsC-3'MVIP09 |
| Kylo-27-DS 9771 | 3011 | 5'MVIP01-GsGsA GfCCfUfAfCAGAG AAAUUCsUsA | 3257 | UsdAsGAdAUdTUCUCdTGfUAGGC UCCsAsC-3'MVIP17 |
| Kylo-27-DS 9781 | 3012 | 5'MVIP17-GsGsA GfCCfUfAfCAGAG AAAUUCsUsA | 3258 | AsfGsUAAfUUfGfUAGAGfAAfCGGC UCsGsG-3'MVIP09 |
| Kylo-27-DS 9791 | 3013 | 5'MVIP09-GsGsA GfCCfUfAfCAGAG AAAUUCsUsA | 3259 | AsfGsUAAfUUfGfUAGAGfAAfCGGC UCsGsG-3'MVIP17 |
| Kylo-27-DS 9801 | 3014 | 5'MVIP17-GsGsA GfCCfUfAfCAGAG AAAUUCsUsA | 3260 | AsfGsUAAfUUfGfUAGAGfAAfCGGC UCsGsG-3'MVIP01 |
| Kylo-27-DS 9811 | 3015 | 5'MVIP17-GsGsA GfCCfUfAfCAGAG AAAUUCsUsA | 3261 | AsfGsUAAfUUfGfUAGAGfAAfCGGC UCsGsG-3'MVIP17 |
| Kylo-27-DS 9821 | 3016 | 5'MVIP09-GsGsfA GfCCfUAfCfAfGAf GAfAAUUCsUsA | 3262 | AsfGsUAAfUUfGfUAGAGfAAfCGGC UCsGsG-3'MVIP09 |
| Kylo-27-DS 9831 | 3017 | 5'MVIP01-GsGsfA GfCCfUfAfCAfGAf GAfAAUUCsUsA | 3263 | AsfGsUAAfUUfGfUAGAGfAAfCGGC UCsGsG-3'MVIP17 |
| Kylo-27-DS 9841 | 3018 | 5'MVIP17-GsGsfA GfCCfUfAfCAfGAf GAfAAUUCsUsA | 3264 | AsfGsUAAfUUfGfUAGAGfAAfCGGC UCsGsG-3'MVIP10 |
| Kylo-27-DS 9851 | 3019 | 5'MVIP09-GsGsfA GfCCfUfAfCAfGAf GAfAAUUCsUsA | 3265 | AsfGsUAAfUUfGfUAGAGfAAfCGGC UCsGsG-3'MVIP02 |
| Kylo-27-DS 9861 | 3020 | 5'MVIP17-GsGsfA GfCCfUfAfCAfGAf GAfAAUUCsUsA | 3266 | AsfGsUAAfUUfGfUAGAGfAAfCGGC UCsGsG-3'MVIP11 |
| Kylo-27-DS 9871 | 3021 | 5'MVIP17-GsGsfA GfCCfUfAfCAfGAf GAfAAUUCsUsA | 3267 | AsfGsUAAfUUfGfUAGAGfAAfCGGC UCsGsG-3'MVIP18 |
| Kylo-27-DS 9881 | 3022 | 5'MVIP09-GsAsG CCGfUUfCfUfCUA CAAUUAsCsU | 3268 | AsfGsUfAAfUUGUAGfAGfAAfCGfG CfUCsGsG-3'MVIP09 |
| Kylo-27-DS 9891 | 3023 | 5'MVIP01-CsCsA GACfAGfAfCfAAG ACCAUCsUsA | 3269 | AsfGsUfAAfUUGUAGfAGfAAfCGfG CfUCsGsG-3'MVIP17 |
| Kylo-27-DS 9901 | 3024 | 5'MVIP17-CsCsA GACfAGfAfCfAAG ACCAUCsUsA | 3270 | AsfGsUfAAfUUGUAGfAGfAAfCGfG CfUCsGsG-3'MVIP01 |
| Kylo-27-DS 9911 | 3025 | 5'MVIP09-CsCsA GACfAGfAfCfAAG ACCAUCsUsA | 3271 | AsfGsUfAAfUUGUAGfAGfAAfCGfG CfUCsGsG-3'MVIP17 |
| Kylo-27-DS 9921 | 3026 | 5'MVIP17-CsCsA GACfAGfAfCfAAG ACCAUCsUsA | 3272 | AsfGsUfAAfUUGUAGfAGfAAfCGfG CfUCsGsG-3'MVIP09 |
| Kylo-27-DS 9931 | 3027 | 5'MVIP17-CsCsA GACfAGfAfCfAAG ACCAUCsUsA | 3273 | UsfAsGAUfGGfUCUUGUfCUfGUCU GGsAsU-3'MVIP17 |
| Kylo-27-DS 00 | 3028 | 5'MVIP09-GsAsG CCGfUUfCfUfCUA CAAUUAsCsU | 3274 | AsfGsUAAfUUfGfUAGAGfAAfCGGC UCsGsG-3'MVIP09 |
| Kylo-27-DS 9951 | 3029 | 5'MVIP01-CsCsA GACfAGfAfCfAAG ACCAUCsUsA | 3275 | AsfGsUAAfUUfGfUAGAGfAAfCGGC UCsGsG-3'MVIP17 |
| Kylo-27-DS 9961 | 3030 | 5'MVIP17-CsCsA GACfAGfAfCfAAG ACCAUCsUsA | 3276 | AsfGsUAAfUUfGfUAGAGfAAfCGGC UCsGsG-3'MVIP01 |
| Kylo-27-DS 9971 | 3031 | 5'MVIP09-CsCsA GACfAGfAfCfAAG ACCAUCsUsA | 3277 | AsfGsUAAfUUfGfUAGAGfAAfCGGC UCsGsG-3'MVIP17 |
| Kylo-27-DS 9981 | 3032 | 5'MVIP17-CsCsA GACfAGfAfCfAAG ACCAUCsUsA | 3278 | AsfGsUAAfUUfGfUAGAGfAAfCGGC UCsGsG-3'MVIP09 |
| Kylo-27-DS 9991 | 3033 | 5'MVIP17-CsCsA GACfAGfAfCfAAG ACCAUCsUsA | 3279 | AsfGsUAAfUUfGfUAGAGfAAfCGGC UCsGsG-3'MVIP17 |
| Kylo-27-DS 10001 | 3034 | 5'MVIP09-GsAsG CfCGfUfUfCUCUA CAAUUAsCsU | 3280 | AsfGsUfAAUUfGUAGAGfAAfCGGC UCsGsG-3'MVIP09 |
| Kylo-27-DS 10011 | 3035 | 5'MVIP01-GsAsG CfCGfUfUfCUCUA CAAUUAsCsU | 3281 | AsfGsUfAAUUfGUAGAGfAAfCGGC UCsGsG-3'MVIP17 |
| Kylo-27-DS 10021 | 3036 | 5'MVIP17-GsAsG CfCGfUfUfCUCUA CAAUUAsCsU | 3282 | AsfGsUfAAUUfGUAGAGfAAfCGGC UCsGsG-3'MVIP01 |
| Kylo-27-DS 10031 | 3037 | 5'MVIP09-GsAsG CfCGfUfUfCUCUA CAAUUAsCsU | 3283 | AsfGsUfAAUUfGUAGAGfAAfCGGC UCsGsG-3'MVIP17 |
| Kylo-27-DS 10041 | 3038 | 5'MVIP17-GsAsG CfCGfUfUfCUCUA CAAUUAsCsU | 3284 | AsfGsUfAAUUfGUAGAGfAAfCGGC UCsGsG-3'MVIP09 |
| Kylo-27-DS 10051 | 3039 | 5'MVIP17-GsAsG CfCGfUfUfCUCUA CAAUUAsCsU | 3285 | AsfGsUfAAUUfGUAGAGfAAfCGGC UCsGsG-3'MVIP17 |
| Kylo-27-DS 10061 | 3040 | 5'MVIP09-GsAsfG CfCGfUUfCfUfCUf ACfAAUUAsCsU | 3286 | AsdGsUAdAUdTGUAGdAGfAACGG CUCsGsG-3'MVIP09 |
| Kylo-27-DS 10071 | 3041 | 5'MVIP01-GsAsG CfCGfUfUfCUCUA CAAUUAsCsU | 3287 | AsdGsUAdAUdTGUAGdAGfAACGG CUCsGsG-3'MVIP17 |
| Kylo-27-DS 10081 | 3042 | 5'MVIP17-GsAsG CfCGfUfUfCUCUA CAAUUAsCsU | 3288 | AsdGsUAdAUdTGUAGdAGfAACGG CUCsGsG-3'MVIP01 |
| Kylo-27-DS 10091 | 3043 | 5'MVIP09-GsAsG CfCGfUfUfCUCUA CAAUUAsCsU | 3289 | AsdGsUAdAUdTGUAGdAGfAACGG CUCsGsG-3'MVIP17 |
| Kylo-27-DS 10101 | 3044 | 5'MVIP17-GsAsG CfCGfUfUfCUCUA CAAUUAsCsU | 3290 | AsdGsUAdAUdTGUAGdAGfAACGG CUCsGsG-3'MVIP09 |
| Kylo-27-DS 10111 | 3045 | 5'MVIP17-GsAsG CfCGfUfUfCUCUA CAAUUAsCsU | 3291 | AsdGsUAdAUdTGUAGdAGfAACGG CUCsGsG-3'MVIP17 |
| Kylo-27-DS 10121 | 3046 | 5'MVIP09-GsGsA GAAfUUfGfCfUUC AUACAAsAsA | 3292 | UsfUsUUGfUAfUGAAGCfAAfUUCU CCsUsC-3'MVIP09 |
| Kylo-27-DS 10131 | 3047 | 5'MVIP01-GsGsA GAAfUUfGfCfUUC AUACAAsAsA | 3293 | UsfUsUUGfUAfUGAAGCfAAfUUCU CCsUsC-3'MVIP17 |
| Kylo-27-DS 10141 | 3048 | 5'MVIP17-GsGsA GAAfUUfGfCfUUC AUACAAsAsA | 3294 | UsfUsUUGfUAfUGAAGCfAAfUUCU CCsUsC-3'MVIP01 |
| Kylo-27-DS 10151 | 3049 | 5'MVIP09-GsGsA GAAfUUfGfCfUUC AUACAAsAsA | 3295 | UsfUsUUGfUAfUGAAGCfAAfUUCU CCsUsC-3'MVIP17 |
| Kylo-27-DS 10161 | 3050 | 5'MVIP17-GsGsA GAAfUUfGfCfUUC AUACAAsAsA | 3296 | UsfUsUUGfUAfUGAAGCfAAfUUCU CCsUsC-3'MVIP09 |
| Kylo-27-DS 10171 | 3051 | 5'MVIP17-GsGsA GAAfUUfGfCfUUC AUACAAsAsA | 3297 | UsfUsUUGfUAfUGAAGCfAAfUUCU CCsUsC-3'MVIP17 |
| Kylo-27-DS 10181 | 3052 | 5'MVIP02-GsGsA GAAfUUfGfCfUUC AUACAAsAsA | 3298 | UsfUsUUGfUAfUGAAGCfAAfUUCU CCsUsC-3'MVIP10 |
| Kylo-27-DS 10191 | 3053 | 5'MVIP10-GsGsA GAAfUUfGfCfUUC AUACAAsAsA | 3299 | UsfUsUUGfUAfUGAAGCfAAfUUCU CCsUsC-3'MVIP02 |
| Kylo-27-DS 10201 | 3054 | 5'MVIP18-GsGsA GAAfUUfGfCfUUC AUACAAsAsA | 3300 | UsfUsUUGfUAfUGAAGCfAAfUUCU CCsUsC-3'MVIP11 |
| Kylo-27-DS 10211 | 3055 | 5'MVIP19-GsGsA GAAfUUfGfCfUUC AUACAAsAsA | 3301 | UsfUsUUGfUAfUGAAGCfAAfUUCU CCsUsC-3'MVIP18 |
| Kylo-27-DS 10221 | 3056 | 5'MVIP09-GsGsA GAAUUfGfCfUUC AUACAAsAsA | 3302 | AsfGsUfUUfGUUCUUfCUfUAfUUfC AfGCsAsC-3'MVIP09 |
| Kylo-27-DS 10231 | 3057 | 5'MVIP01-GsGsA GAAUUfGfCfUUC AUACAAsAsA | 3303 | AsfGsUfUUfGUUCUUfCUfUAfUUfC AfGCsAsC-3'MVIP17 |
| Kylo-27-DS 10241 | 3058 | 5'MVIP17-GsGsA GAAUUfGfCfUUC AUACAAsAsA | 3304 | AsfGsUfUUfGUUCUUfCUfUAfUUfC AfGCsAsC-3'MVIP01 |
| Kylo-27-DS 10251 | 3059 | 5'MVIP09-GsGsA GAAUUfGfCfUUC AUACAAsAsA | 3305 | AsfGsUfUUfGUUCUUfCUfUAfUUfC AfGCsAsC-3'MVIP17 |
| Kylo-27-DS 10261 | 3060 | 5'MVIP17-GsGsA GAAUUfGfCfUUC AUACAAsAsA | 3306 | AsfGsUfUUfGUUCUUfCUfUAfUUfC AfGCsAsC-3'MVIP09 |
| Kylo-27-DS 10271 | 3061 | 5'MVIP17-GsGsA GAAUUfGfCfUUC AUACAAsAsA | 3307 | AsfGsUfUUfGUUCUUfCUfUAfUUfC AfGCsAsC-3'MVIP17 |
| Kylo-27-DS 10281 | 3062 | 5'MVIP09-GsGsA GAAfUUfGfCfUUC AUACAAsAsA | 3308 | AsfGsUUUfGUfUfCUUCUfUAfUUCA GCsAsC-3'MVIP09 |
| Kylo-27-DS 10291 | 3063 | 5'MVIP01-GsGsA GAAfUUfGfCfUUC AUACAAsAsA | 3309 | AsfGsUUUfGUfUfCUUCUfUAfUUCA GCsAsC-3'MVIP17 |
| Kylo-27-DS 10301 | 3064 | 5'MVIP17-GsGsA GAAfUUfGfCfUUC AUACAAsAsA | 3310 | AsfGsUUUfGUfUfCUUCUfUAfUUCA GCsAsC-3'MVIP01 |
| Kylo-27-DS 10311 | 3065 | 5'MVIP09-GsGsA GAAfUUfGfCfUUC AUACAAsAsA | 3311 | AsfGsUUUfGUfUfCUUCUfUAfUUCA GCsAsC-3'MVIP17 |
| Kylo-27-DS 10321 | 3066 | 5'MVIP17-GsGsA GAAfUUfGfCfUUC AUACAAsAsA | 3312 | AsfGsUUUfGUfUfCUUCUfUAfUUCA GCsAsC-3'MVIP09 |
| Kylo-27-DS 10331 | 3067 | 5'MVIP17-GsGsA GAAfUUfGfCfUUC AUACAAsAsA | 3313 | AsfGsUUUfGUfUfCUUCUfUAfUUCA GCsAsC-3'MVIP17 |
| Kylo-27-DS 10341 | 3068 | 5'MVIP02-GsGsA GAAfUUfGfCfUUC AUACAAsAsA | 3314 | AsfGUfUUGUfCUUCUfAfUUCAG CsAsC-3'MVIP09 |
| Kylo-27-DS 10351 | 3069 | 5'MVIP10-GsGsA GAAfUUfGfCfUUC AUACAAsAsA | 3315 | AsfGUfUUGUfCUUCUfUAfUUCAG CsAsC-3'MVIP17 |
| Kylo-27-DS 10361 | 3070 | 5'MVIP18-GsGsA GAAfUUfGfCfUUC AUACAAsAsA | 3316 | AsfGUfUUGUfCUUCUfAfUUCAG CsAsC-3'MVIP01 |
| Kylo-27-DS 10371 | 3071 | 5'MVIP19-GsGsA GAAfUUfGfCfUUC AUACAAsAsA | 3317 | AsfGUfUUGUfCUUCUfUAfUUCAG CsAsC-3'MVIP17 |
| Kylo-27-DS 10381 | 3072 | 5'MVIP09-GsGsA GAAUUfGfCfUUC AUACAAsAsA | 3318 | AsfGUfUUGUfCUUCUfAfUUCAG CsAsC-3'MVIP09 |
| Kylo-27-DS 10391 | 3073 | 5'MVIP01-GsGsA GAAUUfGfCfUUC AUACAAsAsA | 3319 | AsfGUfUUGUfCUUCUfUAfUUCAG CsAsC-3'MVIP17 |
| Kylo-27-DS 10401 | 3074 | 5'MVIP17-GsGsA GAAUUfGfCfUUC AUACAAsAsA | 3320 | AsdGsUUdTGdTUCUUdCUfUAUUC AGCsAsC-3'MVIP09 |
| Kylo-27-DS 10411 | 3075 | 5'MVIP09-GsGsA GAAUUfGfCfUUC AUACAAsAsA | 3321 | AsdGsUUdTGdTUCUUdCUfUAUUC AGCsAsC-3'MVIP17 |
| Kylo-27-DS 10421 | 3076 | 5'MVIP17-GsGsA GAAUUfGfCfUUC AUACAAsAsA | 3322 | AsdGsUUdTGdTUCUUdCUfUAUUC AGCsAsC-3'MVIP01 |
| Kylo-27-DS 10431 | 3077 | 5'MVIP17-GsGsA GAAUUfGfCfUUC AUACAAsAsA | 3323 | AsdGsUUdTGdTUCUUdCUfUAUUC AGCsAsC-3'MVIP17 |
| Kylo-27-DS 10441 | 3078 | 5'MVIP09-GsGsA GfAAfUfUfGCUUC AUACAAsAsA | 3324 | AsdGsUUdTGdTUCUUdCUfUAUUC AGCsAsC-3'MVIP09 |
| Kylo-27-DS 10451 | 3079 | 5'MVIP01-GsGsA GfAAfUfUfGCUUC AUACAAsAsA | 3325 | AsdGsUUdTGdTUCUUdCUfUAUUC AGCsAsC-3'MVIP17 |
| Kylo-27-DS 10461 | 3080 | 5'MVIP17-GsGsA GfAAfUfUfGCUUC AUACAAsAsA | 3326 | UsfAsUGAfAGfCAAUUCfUCfCUCAG CsAsC-3'MVIP09 |
| Kylo-27-DS 10471 | 3081 | 5'MVIP17-GsGsfA GfAAfUfUfGCfUU fCAfUACAAsAsA | 3327 | UsfAsUGAfAGfCAAUUCfUCfCUCAG CsAsC-3'MVIP10 |
| Kylo-27-DS 10481 | 3082 | 5'MVIP09-GsGsfA GfAAfUfUfGCfUU fCAfUACAAsAsA | 3328 | UsfAsUGAfAGfCAAUUCfUCfCUCAG CsAsC-3'MVIP02 |
| Kylo-27-DS 10491 | 3083 | 5'MVIP17-GsGsfA GfAAfUfUfGCfUU fCAfUACAAsAsA | 3329 | UsfAsUGAfAGfCAAUUCfUCfCUCAG CsAsC-3'MVIP11 |
| Kylo-27-DS 10501 | 3084 | 5'MVIP17-GsGsfA GfAAfUfUfGCfUU fCAfUACAAsAsA | 3330 | UsfAsUGAfAGfCAAUUCfUCfCUCAG CsAsC-3'MVIP18 |
| Kylo-27-DS 10511 | 3085 | 5'MVIP09-GsCsU GAGfGAfGfAfAU UGCUUCAsUsA | 3331 | UsfAsUfGAfAGCAAUfUCfUCfCUfCA fGCsAsC-3'MVIP09 |
| Kylo-27-DS 10521 | 3086 | 5'MVIP01-GsCsU GAGfGAfGfAfAU UGCUUCAsUsA | 3332 | UsfAsUfGAfAGCAAUfUCfUCfCUfCA fGCsAsC-3'MVIP17 |
| Kylo-27-DS 10531 | 3087 | 5'MVIP17-GsCsU GAGfGAfGfAfAU UGCUUCAsUsA | 3333 | UsfAsUfGAfAGCAAUfUCfUCfCUfCA fGCsAsC-3'MVIP01 |
| Kylo-27-DS 10541 | 3088 | 5'MVIP09-GsCsU GAGfGAfGfAfAU UGCUUCAsUsA | 3334 | UsfAsUfGAfAGCAAUfUCfUCfCUfCA fGCsAsC-3'MVIP17 |
| Kylo-27-DS 10551 | 3089 | 5'MVIP17-GsCsU GAGfGAfGfAfAU UGCUUCAsUsA | 3335 | UsfAsUfGAfAGCAAUfUCfUCfCUfCA fGCsAsC-3'MVIP09 |
| Kylo-27-DS 10561 | 3090 | 5'MVIP17-GsCsU GAGfGAfGfAfAU UGCUUCAsUsA | 3336 | UsfAsUfGAfAGCAAUfUCfUCfCUfCA fGCsAsC-3'MVIP17 |
| Kylo-27-DS 10571 | 3091 | 5'MVIP09-GsCsU GAGfGAfGfAfAU UGCUUCAsUsA | 3337 | UsfAsUGAfAGfCfAAUUCfUCfCUCA GCsAsC-3'MVIP09 |
| Kylo-27-DS 10581 | 3092 | 5'MVIP01-GsCsU GAGfGAfGfAfAU UGCUUCAsUsA | 3338 | UsfAsUGAfAGfCfAAUUCfUCfCUCA GCsAsC-3'MVIP17 |
| Kylo-27-DS 10591 | 3093 | 5'MVIP17-GsCsU GAGfGAfGfAfAU UGCUUCAsUsA | 3339 | UsfAsUGAfAGfCfAAUUCfUCfCUCA GCsAsC-3'MVIP01 |
| Kylo-27-DS 10601 | 3094 | 5'MVIP09-GsCsU GAGfGAfGfAfAU UGCUUCAsUsA | 3340 | UsfAsUGAfAGfCfAAUUCfUCfCUCA GCsAsC-3'MVIP17 |
| Kylo-27-DS 10611 | 3095 | 5'MVIP17-GsCsU GAGfGAfGfAfAU UGCUUCAsUsA | 3341 | UsfAsUGAfAGfCfAAUUCfUCfCUCA GCsAsC-3'MVIP09 |
| Kylo-27-DS 10621 | 3096 | 5'MVIP17-GsCsU GAGfGAfGfAfAU UGCUUCAsUsA | 3342 | UsfAsUGAfAGfCfAAUUCfUCfCUCA GCsAsC-3'MVIP17 |
| Kylo-27-DS 10631 | 3097 | 5'MVIP02-GsCsU GAGfGAfGfAfAU UGCUUCAsUsA | 3343 | UsfAsUfGAAGfCAAUUCfUCfCUCAG CsAsC-3'MVIP09 |
| Kylo-27-DS 10641 | 3098 | 5'MVIP10-GsCsU GAGfGAfGfAfAU UGCUUCAsUsA | 3344 | UsfAsUfGAAGfCAAUUCfUCfCUCAG CsAsC-3'MVIP17 |
| Kylo-27-DS 10651 | 3099 | 5'MVIP18-GsCsU GAGfGAfGfAfAU UGCUUCAsUsA | 3345 | UsfAsUfGAAGfCAAUUCfUCfCUCAG CsAsC-3'MVIP01 |
| Kylo-27-DS 10661 | 3100 | 5'MVIP19-GsCsU GAGfGAfGfAfAU UGCUUCAsUsA | 3346 | UsfAsUfGAAGfCAAUUCfUCfCUCAG CsAsC-3'MVIP17 |
| Kylo-27-DS 10671 | 3101 | 5'MVIP09-GsCsU GAGGAfGfAfAUU GCUUCAsUsA | 3347 | UsfAsUfGAAGfCAAUUCfUCfCUCAG CsAsC-3'MVIP09 |
| Kylo-27-DS 10681 | 3102 | 5'MVIP01-GsCsU GAGGAfGfAfAUU GCUUCAsUsA | 3348 | UsfAsUfGAAGfCAAUUCfUCfCUCAG CsAsC-3'MVIP17 |
| Kylo-27-DS 10691 | 3103 | 5'MVIP17-GsCsU GAGGAfGfAfAUU GCUUCAsUsA | 3349 | UsdAsUGdAAdGCAAUdTCfUCCUCA GCsAsC-3'MVIP09 |
| Kylo-27-DS 10701 | 3104 | 5'MVIP09-GsCsU GAGGAfGfAfAUU GCUUCAsUsA | 3350 | UsdAsUGdAAdGCAAUdTCfUCCUCA GCsAsC-3'MVIP17 |
| Kylo-27-DS 10711 | 3105 | 5'MVIP17-GsCsU GAGGAfGfAfAUU GCUUCAsUsA | 3351 | UsdAsUGdAAdGCAAUdTCfUCCUCA GCsAsC-3'MVIP01 |
| Kylo-27-DS 10721 | 3106 | 5'MVIP17-GsCsU GAGGAfGfAfAUU GCUUCAsUsA | 3352 | UsdAsUGdAAdGCAAUdTCfUCCUCA GCsAsC-3'MVIP17 |
| Kylo-27-DS 10731 | 3107 | 5'MVIP09-GsCsU GfAGfGfAfGAAU UGCUUCAsUsA | 3353 | UsdAsUGdAAdGCAAUdTCfUCCUCA GCsAsC-3'MVIP09 |
| Kylo-27-DS 10741 | 3108 | 5'MVIP01-GsCsU GfAGfGfAfGAAU UGCUUCAsUsA | 3354 | UsdAsUGdAAdGCAAUdTCfUCCUCA GCsAsC-3'MVIP17 |
| Kylo-27-DS 8481 | 2882 | 5'MVIP09-GsGsU GUUGAfCfAfGAU ACAUsCsU | 3128 | AsfGsAfUGfUAUCUGfUCfAAfCAfCC sfAsU-3'MVIP09 |
| Kylo-27-DS 8491 | 2883 | 5'MVIP09-GsGsU GUUfGAfCfAfGA UACAUsCsU | 3129 | AsfGsAUGfUAfUfCUGUCfAAfCACCs AsU-3'MVIP09 |

In some embodiments, the sense strand and antisense strand of the RNA inhibitor described in the present application are sequences having at least 15 consecutive nucleotides identical to those of the sense strand and antisense strand in Table 7, or sequences differing from the sense strand and antisense strand in Table 7 by one, two or three nucleotides.

It should be emphasized that the combination manner of the sense strand and the antisense strand is not limited to the double-strand combination manner in Table 7, and any one sense strand in Table 7 can be complementarily paired with any one antisense strand. For example, GsUsCUAGfUCfAfAfCUUAAUUGAsGsA (SEQ ID NO: 554) in Table 7-1 can be complementarily paired with UsfCsUfCAfAUfUAAGUUfGAfCUAGACsAsC (SEQ ID NO : 583) , UsfCsUfCAfAUfUAAGUUfGAfCUAGsAsC (SEQ ID NO : 584) , UsdCsUCdAATUAAGTUfGACUAGACsAsC (SEQ ID NO : 588) , UsfCsUCAfAUfUfAAGUUfGAfCUAGACsAsC (SEQ ID NO : 591) , UsfCsUfCAfAUfUAAGUUfGAfCUAGACsAsCTT (SEQ ID NO : 593) , UsfCsUCAfAUfUfAAGUUfGAfCUAGACsAsC (SEQ ID NO: 594) Any one sense strand or antisense strand in the present invention is an independent entity, the combination manner is not limited, and double-stranded inhibitors can be formed as long as at least 85% of the nucleotides can be complementarily paired.

In some embodiments, any one sense strand or antisense strand of the present invention can be linked to carriers of different structures. As an example, the 5'-end and/or 3'-end of any one sense strand or antisense strand of the present invention is linked to 5'MVIP and/or 3'MVIP of different structures, and the conjugation combination manner is not limited.

In some embodiments, the double-stranded RNA inhibitor of the present invention can be optionally conjugated to one or more ligands. Any ligand that can enhance the activity, cellular distribution or cellular uptake (e.g., entry into cells) of the double-stranded RNA inhibitor can be applied to the double-stranded RNA inhibitor of the present invention. The ligand can be attached to the sense strand, the antisense strand, or both strands at the 3'-end, 5'-end, or both ends. The carrier is not limited to the MVIP exemplified in the present invention, and may also include, but is not limited to: GalNac carriers of any structure, cationic lipid vehicles, viral vectors, lipophilic moieties, amphiphilic moieties, targeting groups, small molecule drugs, proteins, peptides, and antibodies.

In some embodiments, the antisense strand of the RNA inhibitor described in the present invention can be obtained by conjugating the antisense strand in Tables 1 to 2 with 5'MVIP and/or 3'MVIP.

In some embodiments, the antisense strand of the RNA inhibitor described in the present invention is obtained by conjugating a sequence having at least 15 consecutive nucleotides identical to those of the antisense strand in Tables 1 to 2, or a sequence differing from the antisense strand in Tables 1 to 2 by one, two or three nucleotides, with 5'MVIP and/or 3'MVIP.

In some embodiments, the double-stranded RNA inhibitor of the present invention can be optionally conjugated to one or more ligands. Any ligand that can enhance the activity, cellular distribution or cellular uptake (e.g., entry into cells) of the double-stranded RNA inhibitor can be applied to the double-stranded RNA inhibitor of the present invention. The ligand can be attached to the sense strand, the antisense strand, or both strands at the 3'-end, 5'-end, or both ends. The carrier is not limited to the MVIP exemplified in the present invention, and may also include, but is not limited to: GalNac carriers of any structure, cationic lipid vehicles, viral vectors, lipophilic moieties, amphiphilic moieties, targeting groups, small molecule drugs, proteins, peptides, and antibodies.

Patent CN113171371B has investigated in detail the effects of different X, L, B, D, R₁ and R₂ in the 5'MVIP and/or 3'MVIP structures on the activity of RNA inhibitors, and the entire content of this patent is incorporated into the present invention.

When X is galactose, galactosamine, N-acetylgalactosamine and derivatives thereof respectively, N-acetylgalactosamine and derivatives thereof are preferred as liver-targeting specific ligands in the RNA inhibitors provided by the present invention, as shown in Table 8:

**Table 8**

| X Code | X Structure |
|---|---|
| X1 | 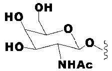 |
| X2 | 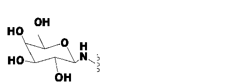 |
| X3 | 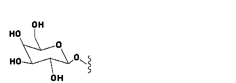 |
| X4 | 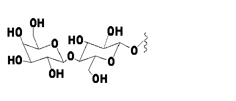 |
| X5 | 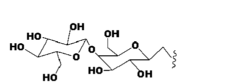 |
| X6 | 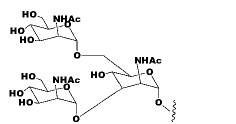 |

The length of L has a great influence on the effect of the RNA inhibitor, and the L chain cannot be too short or too long. When the L chain contains -NH-, C=O, O, S, amido group, phosphoryl group, thiophosphoryl group, aliphatic carbocyclic group such as cyclohexane, or a combination of these groups, or when the L structures are different in the same 5'MVIP/3'MVIP structure or between 5'MVIP and 3'MVIP, the activities of the obtained RNA inhibitors are not significantly different within the carbon chain length range of C7-C18, as shown in Table 9.

**Table 9**

| L Code | L Structure |
|---|---|
| L1 | 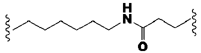 |
| L2 | 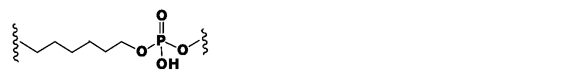 |
| L3 | 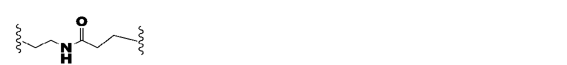 |
| L4 | 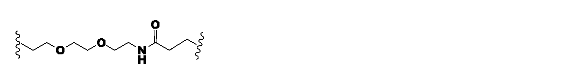 |
| L5 | 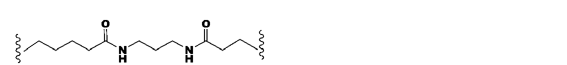 |
| L6 | 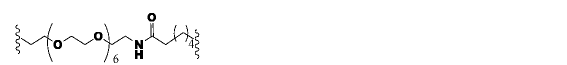 |
| L7 | 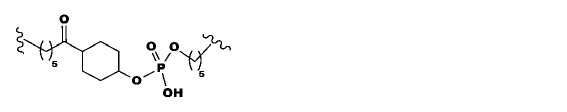 |
| L8 | 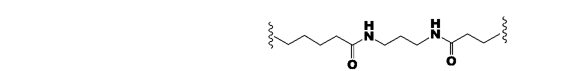 |
| L9 | 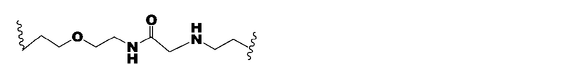 |
| L10 | 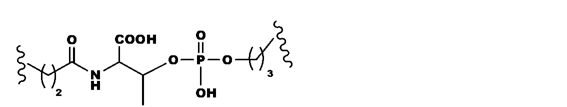 |
| L11 | L in 5' MVIP of S: 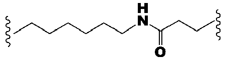 |
| | L in 3' MVIP of AS : 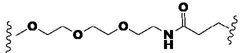 |
| L12 | L in 5' MVIP of S: 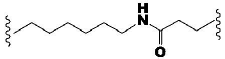 |
| | L in 3' MVIP of AS : 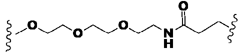 |
| L13 | L in 5' MVIP of S : 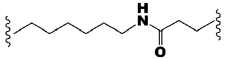 |
| | L in 3' MVIP of AS : 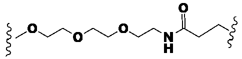 |
| L14 | 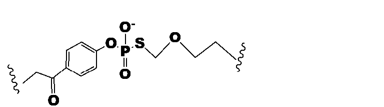 |

When X, L, D and R₁/R₂ are the same as those in the combination 5'MVIP09/3'MVIP09 except for the change of the linker B structure, A, and A₂ in the general formula of linker B are each independently C, O, S, -NH-, carbonyl group, amido group, phosphoryl group or thiophosphoryl group, r is any integer from 0 to 4, and the activities of the obtained RNA inhibitors are not significantly different when linker B is the same or different between 5'MVIP and 3'MVIP.

**Table 10**

| B Code | B Structure |
|---|---|
| B1 | 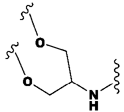 |
| B2 | 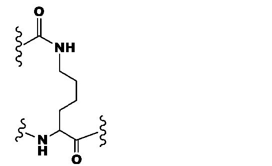 |
| B3 | 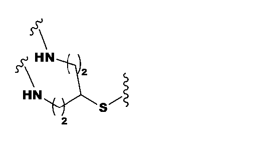 |
| B4 | 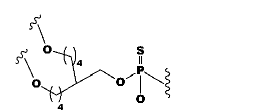 |
| B5 | 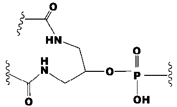 |
| B6 | 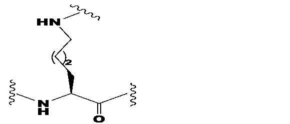 |
| B7 | 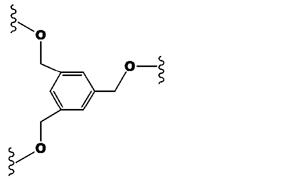 |
| B8 | B in 5'MVIP of S: 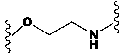 |
| | B in 3'MVIP of AS: 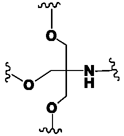 |
| B9 | B in 5'MVIP of S: 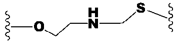 |
| | B in 3'MVIP of AS: 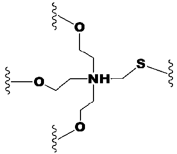 |
| B10 | B in 5'MVIP of S: 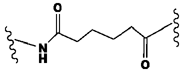 |
| | B in 3'MVIP of AS: 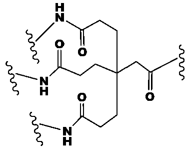 |
| B11 | B in 5'MVIP of S: 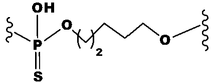 |
| | B in 3'MVIP of AS: 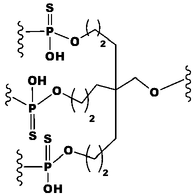 |
| B12 | B in 5'MVIP of S: 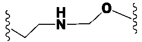 |
| | B in 3'MVIP of AS: 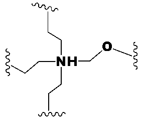 |
| B13 | Bin 5'MVIP of S: 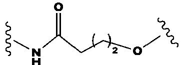 |
| | B in 3'MVIP of AS: 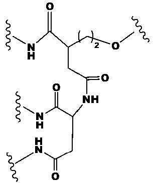 |
| B14 | Bin 5'MVIP of S: 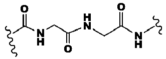 |
| | B in 3'MVIP of AS: 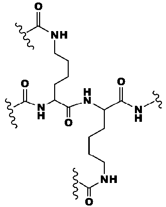 |
| B15 | B in 5'MVIP of S: 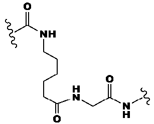 |
| | B in 3'MVIP of AS: 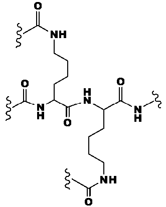 |
| B16 | B in 5'MVIP of S: 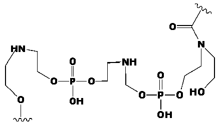 |
| | B in 3'MVIP of AS: 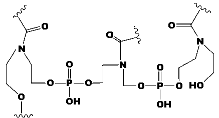 |
| B17 | B in 5'MVIP of S: 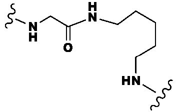 |
| | B in 3'MVIP of AS: 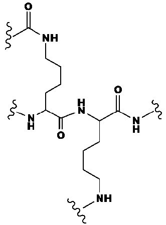 |
| B18 | B in 5'MVIP of S: 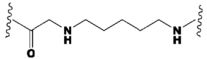 |
| | B in 3'MVIP of AS: 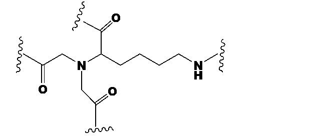 |
| B19 | B in 5'MVIP of S: 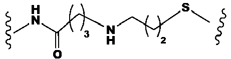 |
| | B in 3'MVIP of AS: 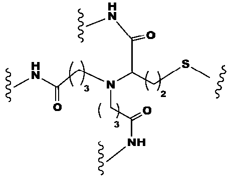 |
| B20 | 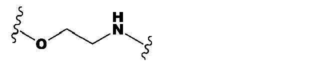 |
| B21 | 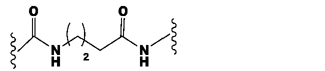 |
| B22 | 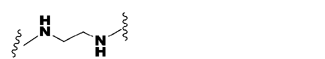 |
| B23 | 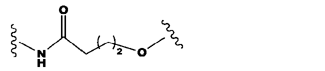 |
| B24 | 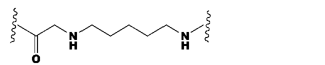 |
| B25 | 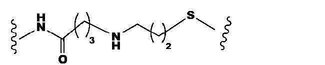 |
| B26 | 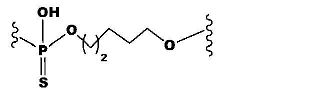 |
| B27 | B in 5'MVIP of S: 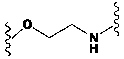 |
| | B in 3'MVIP of AS: 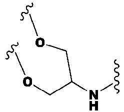 |
| B28 | B in 5'MVIP of S: 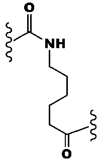 |
| | B in 3'MVIP of AS: 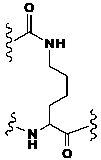 |
| B29 | B in 5'MVIP of S: 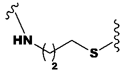 |
| | B in 3'MVIP of AS: 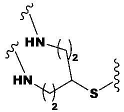 |
| B30 | B in 5'MVIP of S: 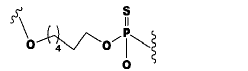 |
| | B in 3'MVIP of AS: 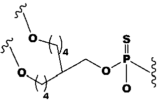 |
| B31 | B in 5'MVIP of S: 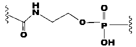 |
| | B in 3'MVIP of AS: 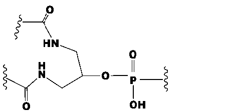 |
| B32 | B in 5'MVIP of S: 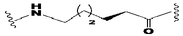 |
| | B in 3'MVIP of AS: 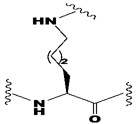 |
| B33 | B in 5'MVIP of S: 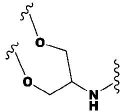 |
| | B in 3'MVIP of AS: 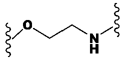 |
| B34 | B in 5'MVIP of S: 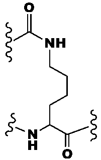 |
| | B in 3'MVIP of AS: 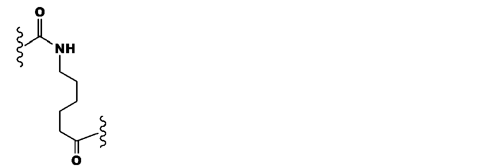 |
| B35 | B in 5'MVIP of S: 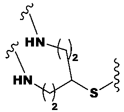 |
| | B in 3'MVIP of AS: 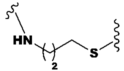 |
| B36 | B in 5'MVIP of S: 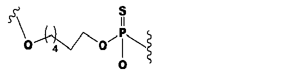 |
| | B in 3'MVIP of AS: 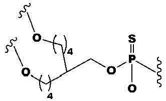 |
| B37 | B in 5'MVIP of S: 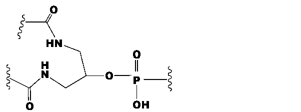 |
| | B in 3'MVIP of AS: 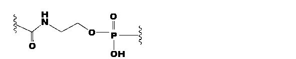 |
| B38 | B in 5'MVIP of S: 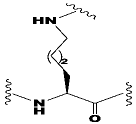 |
| | B in 3'MVIP of AS: 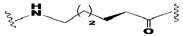 |

Under the condition that the MVIP structure and the RNA inhibitor are the same, different linking chains D have an influence on the activity of the RNA inhibitor, wherein the effects of D1, D2 and D4 are similar and superior to that of D3, and the D structures are shown in Table 11.

**Table 11**

| D Code | D Structure |
|---|---|
| D1 | D in 5'MVIP of S: 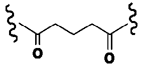 |
| | D in 3'MVIP of AS: 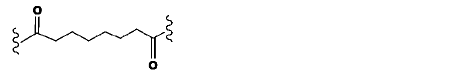 |
| D2 | D in 5'MVIP of S: 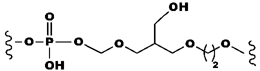 |
| | D in 3'MVIP of AS: 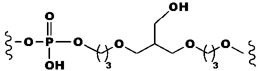 |
| D3 | D in 5'MVIP of S: 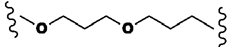 |
| | D in 3'MVIP of AS: 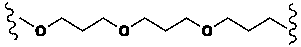 |
| D4 | D in 5'MVIP of S: 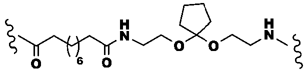 |
| | D in 3'MVIP of AS: 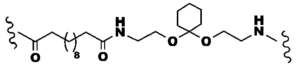 |
| D5 | D in 5'MVIP of S: 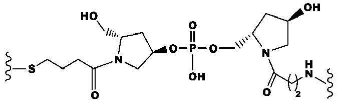 |
| | D in 3'MVIP of AS: 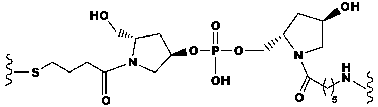 |

Different linking sites R₁ have an influence on the activity of the RNA inhibitor, wherein the RNA inhibitor obtained with R1-1 as the linking site has the best activity, and the R, structures are shown in Table 12.

**Table 12**

| R₁ Code | R₁ Structure |
|---|---|
| R1-1 | -NH(CH₂)₆O- |
| R1-2 | -O(CH₂)₆O- |
| R1-3 | -S(CH₂)₆O- |
| R1-4 | -NH(CH₂)₈ O- |
| R1-5 | -NH(CH₂)₃CH(CH₂CH₃)O- |
| R1-6 | -S(CH₂)₄CH(CH₃)O- |

Different linking sites R₂ have an influence on the activity of the RNA inhibitor, wherein the RNA inhibitor has the best effect with R2-1 as the linking site, and the R₂ structures are shown in Table 13.

**Table 13**

| R₂ Code | R₂ Structure |
|---|---|
| R2-1 | 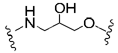 |
| R2-2 | 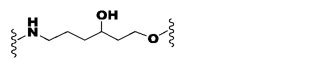 |
| R2-3 | 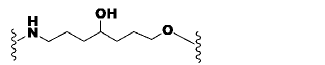 |
| R2-4 | 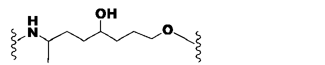 |
| R2-5 | 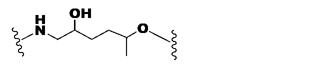 |
| R2-6 | 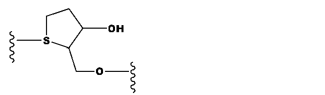 |
| R2-7 | 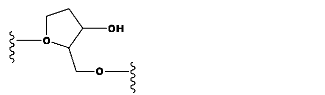 |
| R2-8 | 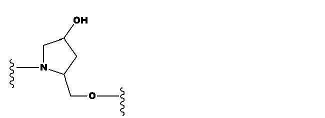 |
| R2-9 | 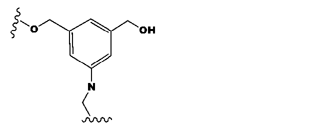 |
| R2-10 | 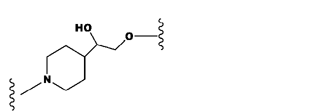 |
| R2-11 | 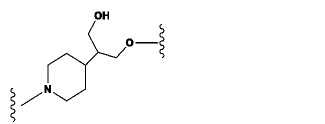 |
| R2-12 | 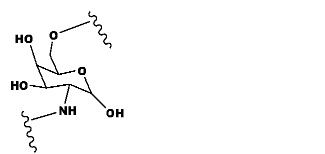 |

In some embodiments, n+m in the RNA inhibitor described in the present invention is 2, 3, 4, 5 and 6, respectively. The conjugation sites of 5'MVIP and/or 3'MVIP include the 5'-end and/or 3'-end of the antisense strand, the 5'-end and/or 3'-end of the sense strand, the 5'-end of the antisense strand and the 3'-end of the sense strand, and the 5'-end of the sense strand and the 3'-end of the antisense strand.

In some embodiments, n+m in the RNA inhibitor described in the present invention is 2, 3, 4, 5 and 6, respectively. The conjugation sites of 5'MVIP and/or 3'MVIP include the 5'-end and/or 3'-end of the antisense strand in Tables 1-2, the 5'-end and/or 3'-end of the sense strand in Tables 1-2, the 5'-end of the antisense strand and the 3'-end of the sense strand in Tables 1-2, and the 5'-end of the sense strand and the 3'-end of the antisense strand in Tables 1-2. The obtained combinations of 5'MVIP and 3'MVIP are shown in Table 14.

**Table 14 List of 5'MVIP and 3'MVIP Combinations**

| Carrier Structure | | Carrier Structure | | n+ m | Conjugation Site of Carrier Structure with Sense Strand and/or Antisense Strand (S:AS) |
|---|---|---|---|---|---|
| 5'MVIP09 | n=2 | 3'MVIP09 | m=2 | 4 | n:m |
| 5'MVIP01 | n=1 | 3' MVIP01 | m=1 | 2 | n:m |
| 5'MVIP21 | n=1 | 3' MVIP01 | m=1 | 2 | n:m |
| 5'MVIP01 | n=1 | 3' MVIP07 | m=1 | 2 | n:m |
| 5'MVIP01/3'MVIP01 | n=1/m=1 | / | / | 2 | nm:/ |
| 3'MVIP01 | m=1 | 5'MVIP01 | n=1 | 2 | m:n |
| 3' MVIP07 | m=1 | 5'MVIP21 | n=1 | 2 | m:n |
| 5'MVIP01 | n=1 | 3'MVIP09 | m=2 | 3 | n:m |
| 5' MVIP08 | n=1 | 3'MVIP15 | m=2 | 3 | n:m |
| 5'MVIP09 | n=2 | 3'MVIP01 | m=1 | 3 | n:m |
| 5' MVIP19 | n=2 | 3' MVIP07 | m=1 | 3 | n:m |
| 5'MVIP01/3'MVIP09 | n=1/m=2 | / | / | 3 | nm:/ |
| 5'MVIP09/3'MVIP01 | n=2/m=1 | / | / | 3 | nm:/ |
| 3'MVIP09 | m=2 | 5'MVIP01 | n=1 | 3 | m:n |
| 3'MVIP11 | m=2 | 5'MVIP06 | n=1 | 3 | m:n |
| 3'MVIP01 | m=1 | 5' MVIP20 | n=2 | 3 | m:n |
| 3' MVIP07 | m=1 | 5' MVIP19 | n=2 | 3 | m:n |
| 5'MVIP01 | n=1 | 3' MVIP17 | m=3 | 4 | n:m |
| 5' MVIP21 | n=1 | 3' MVIP17 | m=3 | 4 | n:m |
| 5'MVIP17 | n=3 | 3' MVIP01 | m=1 | 4 | n:m |
| 5' MVIP22 | n=3 | 3' MVIP07 | m=1 | 4 | n:m |
| 5' MVIP20 | n=2 | 3' MVIP10 | m=2 | 4 | n:m |
| 5' MVIP19 | n=2 | 3'MVIP15 | m=2 | 4 | n:m |
| 5' MVIP10 | n=2 | 3'MVIP12 | m=2 | 4 | n:m |
| 5'MVIP09/3'MVIP09 | n=2/m=2 | / | / | 4 | nm:/ |
| 3'MVIP09 | m=2 | 5' MVIP09 | n=2 | 4 | m:n |
| 3'MVIP10 | m=2 | 5' MVIP20 | n=2 | 4 | m:n |
| 3'MVIP01 | m=1 | 5' MVIP17 | n=3 | 4 | m:n |
| 3'MVIP02 | m=1 | 5' MVIP18 | n=3 | 4 | m:n |
| 3'MVIP17 | m=3 | 5' MVIP01 | n=1 | 4 | m:n |
| 3'MVIP18 | m=3 | 5' MVIP21 | n=1 | 4 | m:n |
| 5'MVIP09 | n=2 | 3' MVIP17 | m=3 | 5 | n:m |
| 5'MVIP20 | n=2 | 3' MVIP19 | m=3 | 5 | n:m |
| 5'MVIP17 | n=3 | 3'MVIP09 | m=2 | 5 | n:m |
| 5'MVIP22 | n=3 | 3'MVIP11 | m=2 | 5 | n:m |
| 5'MVIP09/3'MVIP17 | n=2/m=3 | / | / | 5 | nm:/ |
| 5'MVIP17/3'MVIP09 | n=3/m=2 | / | / | 5 | nm:/ |
| 3'MVIP17 | m=3 | 5' MVIP20 | n=2 | 5 | m:n |
| 3'MVIP19 | m=3 | 5' MVIP16 | n=2 | 5 | m:n |
| 3'MVIP09 | m=2 | 5' MVIP17 | n=3 | 5 | m:n |
| 3' MVIP15 | m=2 | 5' MVIP17 | n=3 | 5 | m:n |
| 5'MVIP17 | n=3 | 3' MVIP17 | m=3 | 6 | n:m |
| 5' MVIP22 | n=3 | 3' MVIP19 | m=3 | 6 | n:m |
| 5'MVIP17/3'MVIP17 | n=3/m=3 | / | | 6 | nm:/ |
| 3'MVIP17 | m=3 | 5' MVIP17 | n=3 | 6 | m:n |
| 3'MVIP18 | m=3 | 5' MVIP22 | n=3 | 6 | m:n |

In some embodiments, n and m are each independently any integer from 0 to 4, each independently preferably an integer from 1 to 3, and n+m is an integer from 2 to 6, preferably n+m=2, 3 or 4, more preferably 4.

In some embodiments, the RNA inhibitor described in the present application or a pharmaceutically acceptable salt thereof is preferably prepared or synthesized in the form of a sodium salt, a triethylamine salt or other pharmaceutically acceptable salts.

In some embodiments, the RNA inhibitor described in the present application or pharmaceutically acceptable salt thereof is more preferably sodium salt or triethylamine salt thereof.

In another aspect, the present application further provides a pharmaceutical composition comprising said RNA inhibitor or pharmaceutically acceptable salt thereof.

In some embodiments, the present invention provides a pharmaceutical composition comprising said RNA inhibitor or pharmaceutically acceptable salt thereof and optionally a pharmaceutically acceptable adjuvant. Use of the RNA inhibitor provided by the present invention or a pharmaceutically acceptable salt thereof in the manufacture of a medicament for the treatment and/or prevention of diseases associated with elevated levels of the complement system, including but not limited to lipid metabolism disorders.

In some embodiments, the complement system regulates plasma triglyceride levels by inhibiting lipoprotein lipase (LPL) activity in liver and adipose tissue and limiting fatty acid release. It affects insulin resistance, fat metabolism and overall energy balance. Factors that may inhibit complement system mRNA expression in vivo and/or in vitro include PPAR δ , statins, insulin, leptin, thyroid hormones, lipopolysaccharides and the like 5-10. The inhibitors of the present invention can be used in combination with these drugs, and have the prospect of further improving the therapeutic effects of lipid metabolism, glucose metabolism, cardiovascular diseases and the like.

One exemplary embodiment is to formulate compositions for systemic administration by parenteral delivery, e.g., subcutaneous (SC), intramuscular (IM) or intravenous (IV) delivery. The pharmaceutical composition provided by the present application can be administered at a dose sufficient to inhibit complement system gene expression.

A pharmaceutically acceptable "adjuvant" or "excipient" is a pharmaceutically acceptable solvent, suspending agent or any other pharmaceutically inert vehicle for delivering one or more nucleic acids to an animal. Excipients can be liquid or solid and are selected with regard to the intended mode of administration to provide the desired volume, consistency, etc., when combined with the nucleic acid and other components in a given pharmaceutical composition. The RNA inhibitor described in the present invention can be delivered in a manner targeted to specific tissues (e.g., hepatocytes).

In some embodiments, the pharmaceutical composition described in the present invention further comprises a delivery vehicle (such as nanoparticles, dendrimers, polymers, liposomes or cationic delivery systems).

In some embodiments, the delivery vehicle described in the present invention comprises liposomes.

In some embodiments, the delivery vehicle described in the present invention comprises nanolipids capable of forming liposome-nucleic acid nanoparticles with nucleic acid molecules.

In some embodiments, the delivery vehicle described in the present invention comprises the amphiphilic lipid compound M10C1.

Pharmaceutical compositions provided by the present invention include, but are not limited to, solutions, emulsions and liposome-containing formulations. These compositions can be produced from a variety of components including, but not limited to, preformed liquids, self-emulsifying solids and self-emulsifying semi-solids. Said formulations include those targeted to the liver. Pharmaceutical formulations of the present application, which may conveniently exist in unit dosage form, can be prepared according to conventional techniques well known in the pharmaceutical industry. Such techniques include the step of bringing into association the active ingredient with pharmaceutically acceptable adjuvants or excipients.

### Use

In another aspect, the present application provides a method of reducing complement system mRNA or protein expression in a cell or tissue, comprising contacting the cell or tissue with an effective amount of the aforementioned RNA inhibitor inhibiting complement system gene expression or a pharmaceutically acceptable salt thereof, and/or the aforementioned pharmaceutical composition.

Cells suitable for treatment using the method of the present application can be any cells expressing complement system genes, such as liver cells, brain cells, gallbladder cells, heart cells or kidney cells, but are preferably liver cells. Cells suitable for use in the method of the present application can be mammalian cells. When contacted with a cell expressing a complement system gene, the RNA inhibitor inhibits expression of a complement system gene (e.g., human, primate, non-primate or rat complement system gene) by at least about 50%, as can be determined by PCR or branched DNA (bDNA)-based methods, or by protein-based methods such as immunofluorescence assay, Western blotting or flow cytometry.

In some embodiments, the tissue is liver tissue.

In some embodiments, the cells and tissues are ex vivo.

In some embodiments, the cells and tissues are in a subject.

The term "inhibition" as used herein is interchangeable with "reduction", "lowering", "silencing", "down-regulation", "suppression" and other similar terms, and includes any level of inhibition. Expression of a complement system gene can be assessed by the level or change in level of any variable associated with complement system gene expression, e.g., complement system mRNA level. This level can be analyzed in a single cell or a population of cells (including, e.g., a sample derived from a subject). Control levels can be any type of control level employed in the art, e.g., baseline levels before administration or levels measured in similar subjects, cells or samples that are untreated or treated with a control (e.g., buffer-only control or no active agent control).

Inhibition of complement system gene expression can be demonstrated by a reduction in the amount of mRNA expressed by a first cell or population of cells (such cells can be present, e.g., in a sample derived from a subject) in which the complement system gene is transcribed and which has been treated (e.g., by contacting one or more cells with the RNA inhibitor of the present application, or by administering the RNA inhibitor of the present application to a subject in which such cells are present) compared to a second cell or population of cells (control cells not treated with the RNA inhibitor or not treated with an RNA inhibitor targeting the gene of interest) that is substantially identical to said first cell or population of cells but not so treated.

In a preferred embodiment, evaluation is performed in a cell line highly expressing the complement system by using an appropriate concentration of siRNA, and the mRNA level in the intervened cells is expressed as a percentage of the mRNA level in the non-intervened control cells.

In other embodiments, inhibition of complement system gene expression can be assessed by a reduction in a parameter functionally related to complement system gene expression, e.g., complement system levels in the blood or serum of a subject. Inhibition of complement system gene expression can be determined in any cell expressing the complement system (endogenous or exogenous from an expression construct) and by any assay known in the art.

Inhibition of complement system expression can be demonstrated by a reduction in the level of complement system expressed by a cell or population of cells or a subject sample (e.g., protein level in a blood sample derived from a subject).

Control cells, cell populations or subject samples that can be used to assess complement system gene inhibition include cells, cell populations or subject samples not contacted with the RNA inhibitor of the present application. For example, control cells, cell populations or subject samples can be derived from a single subject (e.g., human or animal subject) prior to treatment with the RNA inhibitor or an appropriately matched population control.

Complement system mRNA levels expressed by a cell or population of cells can be determined using any method known in the art for assessing mRNA expression, e.g., qRT-PCR to assess the reduction in gene expression. The reduction in protein production can be assessed by any method known in the art, e.g., ELISA. In some embodiments, a puncture liver biopsy sample is used as tissue material to monitor the reduction in complement system gene expression. In other embodiments, a blood sample is used as a subject sample to monitor the reduction in complement system expression.

In another aspect, the present application provides use of the aforementioned RNA inhibitor inhibiting complement system gene expression or a pharmaceutically acceptable salt thereof, or the aforementioned pharmaceutical composition, in the manufacture of a medicament for the prevention and/or treatment of a disease or disorder or reducing the risk of a disease or disorder.

In another aspect, the present application provides a method of preventing and/or treating a disease or disorder, said method comprising administering to a subject in need thereof an effective amount of the aforementioned RNA inhibitor inhibiting complement system gene expression or a pharmaceutically acceptable salt thereof, and/or the aforementioned pharmaceutical composition.

The in vivo methods of the present application may comprise administering to a subject a pharmaceutical composition comprising an RNA inhibitor, wherein the RNA inhibitor comprises a nucleotide sequence complementary to at least a portion of the complement system mRNA of a mammal to which the RNA inhibitor is administered. The pharmaceutical composition described in the present invention can be administered in any manner known in the art, including but not limited to: oral, intraperitoneal or parenteral routes, including intracranial (e.g., intraventricular, intraparenchymal and intrathecal), intravenous, intramuscular, subcutaneous, transdermal, airway (aerosol), nasal, rectal and topical (including buccal and sublingual) administration. In some embodiments, said pharmaceutical composition is administered by intravenous infusion or injection. In some embodiments, said pharmaceutical composition is administered by subcutaneous injection. In some embodiments, the composition is administered by intramuscular injection.

The RNA inhibitor provided by the present application can also be administered as "free RNA inhibitor". Free RNA inhibitor is administered in the absence of a pharmaceutical composition. Naked RNA inhibitor can be in a suitable buffer. Said buffer may comprise acetate, citrate, prolamine, carbonate or phosphate, or any combination thereof. In one embodiment, said buffer is phosphate buffered saline (PBS). The pH and osmotic pressure of the buffer containing said RNA inhibitor can be adjusted to be suitable for administration to a subject.

Alternatively, the RNA inhibitor provided by the present application can be administered as a pharmaceutical composition, such as a liposomal formulation.

The pharmaceutical composition provided by the present application can be administered at a dose sufficient to inhibit complement system gene expression. Typically, a suitable dose of the RNA inhibitor described in the present application is in the range of about 0.001 to about 200.0 mg per kilogram of subject body weight per day, usually in the range of about 1 to 50 mg per kilogram of body weight per day. Typically, a suitable dose of the RNA inhibitor described in the present application is in the range of about 0.1 mg/kg to about 5.0 mg/kg, e.g., about 0.3 mg/kg to about 3.0 mg/kg.

In some embodiments, the method further comprises determining the complement system level in a sample from said subject.

For example, the method further comprises determining the complement system level in a blood sample, serum sample or urine sample from the subject.

In yet another aspect, the present application provides a cell comprising the aforementioned RNA inhibitor inhibiting complement system gene expression or pharmaceutically acceptable salt thereof.

In another aspect, the present application provides a kit comprising the aforementioned RNA inhibitor inhibiting complement system gene expression or a pharmaceutically acceptable salt thereof, or the aforementioned pharmaceutical composition.

Without being bound by any theory, the following examples are merely for illustrating the RNA inhibitor provided by the present application, the preparation method and use thereof, and are not intended to limit the scope of the invention of the present application.

### Examples

### Notes:

DMSO: dimethyl sulfoxide;
DMF: N,N-dimethylformamide;
HOBt: 1-hydroxybenzotriazole;
HBTU: O-benzotriazole-tetramethyluronium hexafluorophosphate;
DIPEA (DIEA): N,N-diisopropylethylamine;
DCM: dichloromethane;
DMAP: 4-dimethylaminopyridine;
DMT-CI: 4,4'-dimethoxytriphenylmethyl chloride;
MeOH: methanol;
TBTU: O-benzotriazole-N,N,N',N'-tetramethyluronium tetrafluoroborate;
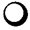 : solid support, such as macroporous aminomethyl resin.

### Example 1 Synthesis of RNA Inhibitor

The sense strand and antisense strand without conjugated carrier structure are synthesized using a standard solid-phase phosphoramidite method with a multi-channel solid-phase synthesizer, and then the sense strand is complementary annealed with the corresponding antisense strand to prepare the corresponding RNA inhibitor.

### Basic steps of the solid-phase phosphoramidite method:

1) Deprotection: removing the hydroxyl protecting group (DMTr) of the Solid Support in the starting monomer;
2) Coupling: adding the first phosphoramidite monomer, and performing a coupling reaction in the 3' to 5' direction;
3) Oxidation: oxidizing the resulting nucleoside phosphite to a more stable nucleoside phosphate (i.e., oxidizing trivalent phosphorus to pentavalent phosphorus);
4) Capping: capping the 5'-OH of the failed nucleotide sequence in the previous step to prevent further participation in the reaction; Repeat the above steps until the last phosphoramidite monomer is attached; then cleave the ester bond between the Solid Support and the starting monomer with methylamine aqueous solution and ammonia water, and remove the protecting groups on each base and phosphate in the resulting nucleotide sequence; After HPLC separation and purification, filter for sterilization, and lyophilize to obtain the corresponding sense strand or antisense strand.

### Description of the synthesis process of RNA inhibitor:

The lyophilized powders of the sense strand and antisense strand are reconstituted respectively, mixed in equimolar amounts, added with an appropriate amount of water for injection, and added with an appropriate amount of TRIS buffer solution. Gently shake for about 1 to 2 minutes to mix the solution uniformly. Heat the water bath to 92°C to 95°C. Place the reaction solution in the water bath and heat for 3 to 5 minutes, gently shake to make the solution heated uniformly. Cool naturally to room temperature to obtain a colorless or slightly yellow transparent liquid, take a sample for inspection, and measure the concentration.

### Example 2-1 In Vitro Assay of CFB, C5 RNA Inhibitors for Inhibiting Complement System Gene Expression

The RNA inhibitors of this example are selected from Table 1-1 and Table 1-2, and prepared by the method described in Example 1.

Hep3B and huh7 cells are digested with trypsin and adjusted to an appropriate density, then seeded into 96-well plates at a viable cell concentration of 0.2×10⁶ cells/mL. At the time of seeding, cells are transfected with test siRNA or control siRNA using Lipofectamine RNAiMax (Invitrogen-13778150). siRNA is tested at the following concentrations: 0.1 nM, 0.02 nM, 0.015 nM, 0.001 nM, 10 nM, 1 nM, 0.01 nM in triplicate, while a no-siRNA control group containing only cells and RNAiMax and a positive control group are set.

24 hours after transfection, the medium is removed and cells are harvested for RNA extraction. Total RNA is extracted using a 96-channel fully automatic nucleic acid extraction and purification instrument or Promega Total RNA Extraction Kit.
cDNA synthesis is performed using the TransScript^{®} II All-in-One First-Strand cDNA Synthesis SuperMix for qPCR (One-Step gDNA Removal) kit according to the manual. The target cDNA is detected by qPCR, and GAPDH cDNA is detected in parallel as an internal control. The fluorescence quantitative PCR instrument (Thermo QuantStudio 1) is operated as follows: 30 seconds at 95°C, followed by 40 cycles of 10 seconds at 95°C and 30 seconds at 60°C.

### Data analysis:

The mRNA expression level of the target gene in the sample is calculated according to the Ct value of each sample, using the ΔΔCt relative quantification method, and the relative expression level of the target gene is expressed as 2^{(-ΔΔCt)}.

The equations are listed as follows: $ΔCT = average Ct of target gene - average Ct of GAPDH$ $ΔΔCT = ΔCT \left(sample\right) - ΔCT \left(random control or Lipofectamine RNAiMax control\right) ;$

Relative quantification of target gene mRNA = 2^{(-ΔΔCT)} 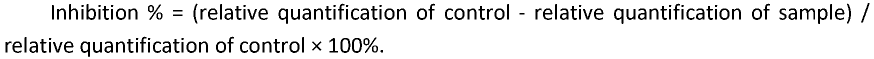 Inhibition % = (relative quantification of control - relative quantification of sample) / relative quantification of control × 100%.

The transfection assay results of CFB RNA inhibitors in Hep3B cells are shown in Tables 15-1 to 15-4 below.

The transfection assay results of C5 RNA inhibitors in Hep3B cells are shown in Tables 15-5 to 15-11 below.

**Table 15-1 Inhibitory effect of 0.1 nM CFB RNA inhibitor in Hep3B cells**

| siRNA inhibitor (0.1nM) | Inhibition rate % | SD |
|---|---|---|
| Kylo-17-DS01 | 88.28 | 2.11 |
| Kylo-17-DS02 | 59.30 | 11.67 |
| Kylo-17-DS03 | 85.59 | 3.18 |
| Kylo-17-DS04 | 85.38 | 3.34 |
| Kylo-17-DS05 | 89.19 | 2.99 |
| Kylo-17-DS06 | 86.15 | 2.18 |
| Kylo-17-DS07 | 87.07 | 2.77 |
| Kylo-17-DS08 | 85.04 | 1.80 |
| Kylo-17-DS09 | 81.54 | 1.58 |
| Kylo-17-DS10 | 73.16 | 7.45 |
| Kylo-17-DS11 | 76.29 | 0.64 |
| Kylo-17-DS12 | 75.90 | 3.54 |
| Kylo-17-DS13 | 85.92 | 3.25 |
| Kylo-17-DS14 | 85.05 | 1.23 |
| Kylo-17-DS15 | 85.63 | 0.41 |
| Kylo-17-DS16 | 80.28 | 5.36 |
| Kylo-17-DS17 | 86.87 | 2.81 |
| Kylo-17-DS18 | 74.22 | 6.58 |
| Kylo-17-DS19 | 86.52 | 1.01 |
| Kylo-17-DS20 | 82.83 | 0.40 |
| Kylo-17-DS21 | 81.46 | 4.56 |
| Kylo-17-DS22 | 64.79 | 9.75 |
| Kylo-17-DS23 | 86.69 | 2.71 |
| Kylo-17-DS24 | 72.32 | 6.76 |
| Kylo-17-DS25 | 85.28 | 1.20 |
| Kylo-17-DS26 | 81.23 | 3.61 |
| Kylo-17-DS27 | 60.98 | 5.72 |
| Kylo-17-DS28 | 66.49 | 4.65 |

**Table 15-2 Inhibitory effect of 0.02 nM CFB RNA inhibitor in Hep3B cells**

| siRNA inhibitor (0.02 nM) | Inhibition rate % | SD | siRNA inhibitor (0.02 nM) | Inhibition rate % | SD |
|---|---|---|---|---|---|
| Kylo-17-DS100 | 36.36 | 10.5 | Kylo-17-DS34 | 70.4 | 1.17 |
| Kylo-17-DS101 | 48.87 | 12.44 | Kylo-17-DS265 | 70.33 | 2.33 |
| Kylo-17-DS102 | 53.92 | 5.23 | Kylo-17-DS157 | 70.26 | 5.13 |
| Kylo-17-DS103 | 53.96 | 5.55 | Kylo-17-DS183 | 70.24 | 6.7 |
| Kylo-17-DS106 | 39.93 | 4.39 | Kylo-17-DS75 | 70.02 | 2.54 |
| Kylo-17-DS107 | 50.64 | 5.19 | Kylo-17-DS257 | 69.94 | 3.4 |
| Kylo-17-DS113 | 79.73 | 2.79 | Kylo-17-DS260 | 69.91 | 3.1 |
| Kylo-17-DS114 | 78.87 | 1.76 | Kylo-17-DS155 | 69.82 | 2.1 |
| Kylo-17-DS115 | 62.17 | 2.81 | Kylo-17-DS150 | 69.76 | 4.9 |
| Kylo-17-DS118 | 73.14 | 1.98 | Kylo-17-DS226 | 69.58 | 3.31 |
| Kylo-17-DS119 | 54.24 | 11.01 | Kylo-17-DS206 | 68.64 | 3.99 |
| Kylo-17-DS120 | 57.53 | 7.42 | Kylo-17-DS153 | 68.44 | 1.18 |
| Kylo-17-DS121 | 31.82 | 7.59 | Kylo-17-DS170 | 67.88 | 4.15 |
| Kylo-17-DS122 | 48.7 | 8.29 | Kylo-17-DS256 | 67.74 | 1.98 |
| Kylo-17-DS123 | 48.04 | 5.89 | Kylo-17-DS109 | 67.49 | 4.04 |
| Kylo-17-DS124 | 49.46 | 5.18 | Kylo-17-DS261 | 67.32 | 7.23 |
| Kylo-17-DS125 | 39.86 | 6.38 | Kylo-17-DS252 | 67 | 1.48 |
| Kylo-17-DS126 | 30.92 | 4 | Kylo-17-DS210 | 65.9 | 4.7 |
| Kylo-17-DS127 | 52.41 | 4.01 | Kylo-17-DS207 | 65.87 | 2.37 |
| Kylo-17-DS129 | 57.09 | 6.57 | Kylo-17-DS42 | 65.79 | 3.39 |
| Kylo-17-DS130 | 61.43 | 5.29 | Kylo-17-DS59 | 65.24 | 2.59 |
| Kylo-17-DS132 | 45.54 | 7.27 | Kylo-17-DS218 | 58.32 | 6.13 |
| Kylo-17-DS133 | 53.94 | 6.81 | Kylo-17-DS227 | 64.5 | 7.4 |
| Kylo-17-DS135 | 61.53 | 2.29 | Kylo-17-DS241 | 64.27 | 6.26 |
| Kylo-17-DS136 | 25.97 | 10.05 | Kylo-17-DS142 | 63.81 | 2.19 |
| Kylo-17-DS137 | 34.47 | 8.54 | Kylo-17-DS177 | 63.8 | 4.08 |
| Kylo-17-DS140 | 62.16 | 0.89 | Kylo-17-DS263 | 63.57 | 7.91 |
| Kylo-17-DS25 | 80.2 | 4.5 | Kylo-17-DS143 | 63.56 | 2.98 |
| Kylo-17-DS29 | 63.93 | 14.36 | Kylo-17-DS195 | 63.53 | 3.47 |
| Kylo-17-DS30 | 65.64 | 1.3 | Kylo-17-DS182 | 63.5 | 2.54 |
| Kylo-17-DS31 | 64 | 8.75 | Kylo-17-DS230 | 63.39 | 3.52 |
| Kylo-17-DS32 | 57.53 | 2.74 | Kylo-17-DS264 | 63.22 | 0.25 |
| Kylo-17-DS33 | 46.87 | 14.31 | Kylo-17-DS228 | 62.94 | 3.05 |
| Kylo-17-DS38 | 76.83 | 4.3 | Kylo-17-DS204 | 62.84 | 2.3 |
| Kylo-17-DS39 | 70.34 | 3.16 | Kylo-17-DS242 | 62.82 | 9.91 |
| Kylo-17-DS43 | 59.6 | 4.09 | Kylo-17-DS231 | 62.78 | 6.23 |
| Kylo-17-DS44 | 58.65 | 6.79 | Kylo-17-DS216 | 62.53 | 3.23 |
| Kylo-17-DS186 | 83.78 | 1.91 | Kylo-17-DS205 | 61.55 | 2.56 |
| Kylo-17-DS223 | 83.66 | 1.49 | Kylo-17-DS159 | 61.41 | 2.44 |
| Kylo-17-DS149 | 83.34 | 1.9 | Kylo-17-DS180 | 61.25 | 2.42 |
| Kylo-17-DS134 | 82.7 | 2.48 | Kylo-17-DS72 | 61.09 | 6.75 |
| Kylo-17-DS209 | 82.61 | 3.18 | Kylo-17-DS167 | 60.88 | 6.56 |
| Kylo-17-DS145 | 82.49 | 1.67 | Kylo-17-DS259 | 60.76 | 4.07 |
| Kylo-17-DS37 | 82.33 | 4.88 | Kylo-17-DS56 | 60.25 | 1.21 |
| Kylo-17-DS77 | 81.26 | 2.68 | Kylo-17-DS239 | 60.21 | 9.68 |
| Kylo-17-DS148 | 81.09 | 1.92 | Kylo-17-DS254 | 60.17 | 8.08 |
| Kylo-17-DS202 | 80.3 | 3.27 | Kylo-17-DS188 | 58.81 | 8.85 |
| Kylo-17-DS217 | 80.06 | 0.59 | Kylo-17-DS215 | 58.48 | 4.91 |
| Kylo-17-DS64 | 79.86 | 0.73 | Kylo-17-DS168 | 58.41 | 4.84 |
| Kylo-17-DS152 | 79.29 | 1.46 | Kylo-17-DS240 | 51.14 | 3.19 |
| Kylo-17-DS104 | 78.09 | 1.67 | Kylo-17-DS245 | 50.87 | 5.81 |
| Kylo-17-DS95 | 78.01 | 2.07 | Kylo-17-DS214 | 57.65 | 6.44 |
| Kylo-17-DS84 | 77.97 | 4.96 | Kylo-17-DS251 | 57.29 | 3.53 |
| Kylo-17-DS171 | 77.89 | 2.77 | Kylo-17-DS243 | 57.17 | 6.78 |
| Kylo-17-DS82 | 77.42 | 0.77 | Kylo-17-DS225 | 56.68 | 7.69 |
| Kylo-17-DS63 | 77.35 | 7.84 | Kylo-17-DS179 | 56.54 | 4.83 |
| Kylo-17-DS221 | 77.15 | 4.27 | Kylo-17-DS62 | 56.52 | 6.45 |
| Kylo-17-DS131 | 77.02 | 2.56 | Kylo-17-DS255 | 29.42 | 3.26 |
| Kylo-17-DS86 | 76.96 | 2.43 | Kylo-17-DS53 | 53.04 | 5.17 |
| Kylo-17-DS199 | 76.53 | 2.35 | Kylo-17-DS198 | 54.63 | 10.23 |
| Kylo-17-DS36 | 76.52 | 2.5 | Kylo-17-DS200 | 53.93 | 4.23 |
| Kylo-17-DS60 | 76.51 | 2.24 | Kylo-17-DS189 | 53.52 | 1.07 |
| Kylo-17-DS266 | 62.17 | 7.4 | Kylo-17-DS229 | 52.84 | 3.55 |
| Kylo-17-DS41 | 76.29 | 6.15 | Kylo-17-DS187 | 51.77 | 4.64 |
| Kylo-17-DS40 | 76.18 | 4.94 | Kylo-17-DS196 | 50.99 | 7.08 |
| Kylo-17-DS141 | 76.1 | 1.4 | Kylo-17-DS190 | 49.84 | 0.79 |
| Kylo-17-DS194 | 75.76 | 4.38 | Kylo-17-DS250 | 49.38 | 6.61 |
| Kylo-17-DS35 | 75.67 | 6.04 | Kylo-17-DS172 | 48.61 | 4.33 |
| Kylo-17-DS181 | 75.51 | 0.89 | Kylo-17-DS192 | 48.46 | 1.36 |
| Kylo-17-DS146 | 75.42 | 3.27 | Kylo-17-DS220 | 47.81 | 3.97 |
| Kylo-17-DS201 | 75.42 | 3.78 | Kylo-17-DS197 | 47.4 | 8.73 |
| Kylo-17-DS160 | 75.12 | 2.17 | Kylo-17-DS184 | 47.33 | 2.68 |
| Kylo-17-DS128 | 75.11 | 5.83 | Kylo-17-DS213 | 46.64 | 5.49 |
| Kylo-17-DS224 | 75.04 | 3.29 | Kylo-17-DS191 | 46.33 | 1.63 |
| Kylo-17-DS58 | 74.8 | 2.8 | Kylo-17-DS175 | 45.69 | 6.55 |
| Kylo-17-DS85 | 74.73 | 1.26 | Kylo-17-DS237 | 45.66 | 10.5 |
| Kylo-17-DS69 | 74.4 | 5.49 | Kylo-17-DS161 | 65.33 | 0.77 |
| Kylo-17-DS116 | 74.04 | 4.47 | Kylo-17-DS253 | 45.14 | 6.99 |
| Kylo-17-DS138 | 73.86 | 3.75 | Kylo-17-DS244 | 52.38 | 6 |
| Kylo-17-DS139 | 73.81 | 2.16 | Kylo-17-DS211 | 40.34 | 6.68 |
| Kylo-17-DS78 | 73.76 | 3.49 | Kylo-17-DS154 | 39.39 | 4.2 |
| Kylo-17-DS55 | 73.73 | 2.63 | Kylo-17-DS246 | 38.63 | 2.7 |
| Kylo-17-DS158 | 73.38 | 5.53 | Kylo-17-DS169 | 38.23 | 2.02 |
| Kylo-17-DS67 | 73.37 | 1.52 | Kylo-17-DS238 | 37.91 | 1.21 |
| Kylo-17-DS151 | 73.19 | 1.2 | Kylo-17-DS247 | 37.24 | 2.87 |
| Kylo-17-DS176 | 73.09 | 1.12 | Kylo-17-DS163 | 65.16 | 1.69 |
| Kylo-17-DS178 | 72.97 | 2.32 | Kylo-17-DS174 | 34.57 | 0.8 |
| Kylo-17-DS219 | 72.82 | 3.54 | Kylo-17-DS165 | 43.97 | 4.24 |
| Kylo-17-DS222 | 72.74 | 6.88 | Kylo-17-DS262 | 32.74 | 8.76 |
| Kylo-17-DS203 | 72.58 | 5 | Kylo-17-DS233 | 31.17 | 6.89 |
| Kylo-17-DS144 | 72.38 | 2.49 | Kylo-17-DS212 | 30.72 | 4.3 |
| Kylo-17-DS147 | 72.19 | 3.55 | Kylo-17-DS185 | 30.55 | 8.79 |
| Kylo-17-DS164 | 72.17 | 1.85 | Kylo-17-DS166 | 30.02 | 1.58 |
| Kylo-17-DS110 | 72.07 | 1.99 | Kylo-17-DS248 | 29.02 | 3.39 |
| Kylo-17-DS258 | 72.06 | 4.61 | Kylo-17-DS173 | 28.88 | 0.8 |
| Kylo-17-DS249 | 71.68 | 3.64 | Kylo-17-DS193 | 25.72 | 9.46 |
| Kylo-17-DS267 | 71.55 | 2.46 | Kylo-17-DS268 | 24.44 | 5.63 |
| Kylo-17-DS156 | 71.44 | 0.6 | Kylo-17-DS162 | 64.35 | 5.5 |
| Kylo-17-DS208 | 71.39 | 4.47 | Kylo-17-DS236 | 17.16 | 7.46 |
| Kylo-17-DS108 | 71.13 | 5.24 | Kylo-17-DS232 | 16.85 | 1.17 |
| Kylo-17-DS49 | 70.53 | 1.31 | Kylo-17-DS235 | 10.79 | 9.06 |
| | | | Kylo-17-DS234 | 20.91 | 3.2 |

**Table 15-3 Inhibitory effect of 0.015 nM CFB RNA inhibitor in Hep3B cells**

| **siRNA inhibitor (0.015 nM)** | **Inhibition rate %** | **SD** |
|---|---|---|
| Kylo-17-DS96 | 46.75 | 17.41 |
| Kylo-17-DS87 | 29.59 | 7.15 |
| Kylo-17-DS83 | 32.3 | 10.6 |
| Kylo-17-DS80 | 50.19 | 3.96 |
| Kylo-17-DS72 | 68.23 | 5.27 |
| Kylo-17-DS65 | 74.74 | 2.34 |
| Kylo-17-DS64 | 79.59 | 3.9 |
| Kylo-17-DS62 | 57.7 | 3 |
| Kylo-17-DS59 | 64.11 | 5.93 |
| Kylo-17-DS56 | 58.95 | 4.19 |
| Kylo-17-DS53 | 59.33 | 4.47 |
| Kylo-17-DS42 | 73.11 | 4.04 |
| Kylo-17-DS39 | 67.47 | 2.9 |
| Kylo-17-DS38 | 78.74 | 3.09 |
| Kylo-17-DS36 | 61.01 | 13.62 |
| Kylo-17-DS30 | 58.32 | 6.51 |
| Kylo-17-DS117 | 46.44 | 4.41 |
| Kylo-17-DS112 | 55.13 | 11.21 |
| Kylo-17-DS111 | 48.57 | 1.28 |
| Kylo-17-DS105 | 60.79 | 9.61 |

**Table 15-4 Inhibitory effect of 0.1 CFB RNA inhibitor in Hep3B cells**

| **siRNA inhibitor 0.1 nM** | Inhibition rate (%) | **SD** |
|---|---|---|
| Kylo-17-DS104 | 76.94 | 1.51 |
| Kylo-17-DS108 | 56.00 | 3.35 |
| Kylo-17-DS109 | 71.63 | 1.60 |
| Kylo-17-DS110 | 76.39 | 0.79 |
| Kylo-17-DS113 | 22.55 | 8.80 |
| Kylo-17-DS114 | 78.36 | 0.66 |
| Kylo-17-DS116 | 80.46 | 2.12 |
| Kylo-17-DS118 | 86.47 | 3.31 |
| Kylo-17-DS128 | 68.98 | 4.41 |
| Kylo-17-DS131 | 80.69 | 1.14 |
| Kylo-17-DS134 | 75.91 | 2.81 |
| Kylo-17-DS138 | 83.03 | 2.32 |
| Kylo-17-DS139 | 71.29 | 3.28 |
| Kylo-17-DS141 | 52.14 | 5.76 |
| Kylo-17-DS144 | 83.31 | 0.62 |
| Kylo-17-DS145 | 50.50 | 6.73 |
| Kylo-17-DS146 | 76.72 | 1.21 |
| Kylo-17-DS147 | 77.62 | 3.01 |
| Kylo-17-DS148 | 89.63 | 0.34 |
| Kylo-17-DS149 | 85.49 | 0.96 |
| Kylo-17-DS150 | 68.49 | 3.12 |
| Kylo-17-DS151 | 83.80 | 2.45 |
| Kylo-17-DS153 | 66.28 | 4.87 |
| Kylo-17-DS156 | 66.99 | 4.54 |
| Kylo-17-DS157 | 87.37 | 0.63 |
| Kylo-17-DS158 | 78.79 | 3.99 |
| Kylo-17-DS160 | 81.67 | 2.32 |
| Kylo-17-DS164 | 78.45 | 3.43 |
| Kylo-17-DS171 | 85.28 | 2.23 |
| Kylo-17-DS176 | 83.65 | 0.57 |
| Kylo-17-DS178 | 83.55 | 1.06 |
| Kylo-17-DS181 | 85.10 | 1.84 |
| Kylo-17-DS183 | 84.71 | 0.90 |
| Kylo-17-DS186 | 87.07 | 0.70 |
| Kylo-17-DS194 | 83.98 | 2.47 |
| Kylo-17-DS199 | 90.24 | 0.97 |
| Kylo-17-DS201 | 79.07 | 2.45 |
| Kylo-17-DS202 | 88.67 | 2.96 |
| Kylo-17-DS203 | 81.04 | 0.75 |
| Kylo-17-DS208 | 81.64 | 1.21 |
| Kylo-17-DS209 | 89.47 | 0.76 |
| Kylo-17-DS217 | 83.09 | 1.85 |
| Kylo-17-DS219 | 83.47 | 0.37 |
| Kylo-17-DS221 | 82.28 | 0.43 |
| Kylo-17-DS222 | 81.32 | 0.57 |
| Kylo-17-DS223 | 86.41 | 1.32 |
| Kylo-17-DS224 | 70.13 | 3.20 |
| Kylo-17-DS249 | 85.13 | 0.84 |
| Kylo-17-DS258 | 74.17 | 0.77 |
| Kylo-17-DS265 | 77.13 | 0.64 |
| Kylo-17-DS266 | 88.16 | 0.83 |
| Kylo-17-DS267 | 83.37 | 1.92 |
| Kylo-17-DS34 | 82.50 | 1.57 |
| Kylo-17-DS35 | 74.84 | 2.70 |
| Kylo-17-DS37 | 85.15 | 2.02 |
| Kylo-17-DS40 | 83.71 | 2.36 |
| Kylo-17-DS41 | 74.35 | 2.53 |
| Kylo-17-DS49 | 76.83 | 2.45 |
| Kylo-17-DS55 | 83.28 | 3.70 |
| Kylo-17-DS58 | 84.44 | 2.08 |
| Kylo-17-DS60 | 88.93 | 2.73 |
| Kylo-17-DS63 | 88.35 | 0.37 |
| Kylo-17-DS67 | 83.08 | 1.27 |
| Kylo-17-DS69 | 85.00 | 3.36 |
| Kylo-17-DS75 | 75.65 | 1.01 |
| Kylo-17-DS77 | 84.05 | 2.21 |
| Kylo-17-DS78 | 88.53 | 0.60 |
| Kylo-17-DS82 | 87.23 | 1.32 |
| Kylo-17-DS84 | 86.72 | 1.96 |
| Kylo-17-DS85 | 86.86 | 1.00 |
| Kylo-17-DS86 | 80.73 | 1.23 |
| Kylo-17-DS95 | 85.68 | 0.86 |

**Table 15-5 Inhibitory effect of 1nM C5 RNA inhibitor in Huh7 cells**

| siRNA inhibitor (1 nM) | Inhibition rate % | SD | siRNA inhibitor 1nM | Inhibition rate % | SD |
|---|---|---|---|---|---|
| Kylo-19-DS35 3 | 90.2 | 0.8 | Kylo-19-DS683 | 45.9 | 0.6 |
| Kylo-19-DS35 4 | 91.9 | 0.4 | Kylo-19-DS684 | 42.6 | 3.6 |
| Kylo-19-DS61 9 | 84 | 3.2 | Kylo-19-DS683 | 75.4 | 1.1 |
| Kylo-19-DS62 0 | 37 | 4.8 | Kylo-19-DS684 | 45.6 | 3.3 |
| Kylo-19-DS62 1 | 81.6 | 2.4 | Kylo-19-DS685 | 74.7 | 2.1 |
| Kylo-19-DS62 2 | 76.7 | 2.9 | Kylo-19-DS686 | 84.1 | 1.2 |
| Kylo-19-DS62 3 | 79.6 | 0.4 | Kylo-19-DS688 | 75.5 | 1.3 |
| Kylo-19-DS62 4 | 53.4 | 6.7 | Kylo-19-DS689 | 84.8 | 0.9 |
| Kylo-19-DS62 5 | 52 | 10.1 | Kylo-19-DS690 | 73 | 3.8 |
| Kylo-19-DS62 6 | 75.7 | 5.3 | Kylo-19-DS691 | 56.9 | 2.8 |
| Kylo-19-DS62 7 | 50.1 | 5.8 | Kylo-19-DS692 | 76.2 | 1 |
| Kylo-19-DS62 8 | 49.6 | 1 | Kylo-19-DS694 | 68.1 | 2.2 |
| Kylo-19-DS62 9 | 70.4 | 3.1 | Kylo-19-DS696 | 76.3 | 1.3 |
| Kylo-19-DS63 0 | 78.2 | 2 | Kylo-19-DS697 | 69 | 6 |
| Kylo-19-DS63 1 | 35 | 2.9 | Kylo-19-DS698 | 66.8 | 2.7 |
| Kylo-19-DS63 2 | 88 | 1.5 | Kylo-19-DS699 | 6.2 | 5.7 |
| Kylo-19-DS63 3 | 0 | 7.3 | Kylo-19-DS700 | 45.5 | 3 |
| Kylo-19-DS63 4 | 50.8 | 1.4 | Kylo-19-DS701 | 29.5 | 4.7 |
| Kylo-19-DS63 5 | 90.3 | 1.6 | Kylo-19-DS702 | 84.4 | 1.4 |
| Kylo-19-DS63 6 | 83.8 | 0.9 | Kylo-19-DS703 | 80 | 1.4 |
| Kylo-19-DS63 7 | 90.6 | 1.1 | Kylo-19-DS704 | 77 | 2.9 |
| Kylo-19-DS63 8 | 77.4 | 0.9 | Kylo-19-DS707 | 81.4 | 1.1 |
| Kylo-19-DS64 0 | 64.9 | 14.5 | Kylo-19-DS710 | 91.7 | 1 |
| Kylo-19-DS64 1 | 90.7 | 0.8 | Kylo-19-DS711 | 96.1 | 0.1 |
| Kylo-19-DS64 2 | 83.8 | 1.5 | Kylo-19-DS712 | 94.8 | 0.3 |
| Kylo-19-DS64 3 | 89.2 | 1.72 | Kylo-19-DS713 | 61 | 2.6 |
| Kylo-19-DS64 4 | 32.9 | 4 | Kylo-19-DS821 | 90.4 | 0.5 |
| Kylo-19-DS64 5 | 63.7 | 5.9 | Kylo-19-DS714 | 87.7 | 0.5 |
| Kylo-19-DS64 6 | 49.8 | 5.6 | Kylo-19-DS715 | 91.4 | 0.6 |
| Kylo-19-DS64 7 | 0.9 | 4 | Kylo-19-DS716 | 70.4 | 1.1 |
| Kylo-19-DS64 8 | 0 | 10.7 | Kylo-19-DS717 | 74.6 | 0.7 |
| Kylo-19-DS64 9 | 12.4 | 5.5 | Kylo-19-DS718 | 83.5 | 1.4 |
| Kylo-19-DS65 0 | 74.3 | 3.1 | Kylo-19-DS719 | 85.9 | 1 |
| Kylo-19-DS65 1 | 52 | 2.7 | Kylo-19-DS720 | 88 | 0.7 |
| Kylo-19-DS65 2 | 65.3 | 1.9 | Kylo-19-DS721 | 85.1 | 1.5 |
| Kylo-19-DS65 3 | 0 | 7 | Kylo-19-DS722 | 51.1 | 2.6 |
| Kylo-19-DS65 4 | 87.4 | 1 | Kylo-19-DS723 | 80.6 | 1.2 |
| Kylo-19-DS65 5 | 26.5 | 7.7 | Kylo-19-DS822 | 92.6 | 1.1 |
| Kylo-19-DS65 6 | 0 | 9.3 | Kylo-19-DS724 | 85.4 | 1.3 |
| Kylo-19-DS65 7 | 70.4 | 2.5 | Kylo-19-DS725 | 91.2 | 1.6 |
| Kylo-19-DS65 8 | 57.9 | 3.4 | Kylo-19-DS726 | 95.9 | 0.3 |
| Kylo-19-DS65 9 | 14.9 | 3.8 | Kylo-19-DS727 | 82.7 | 0.6 |
| Kylo-19-DS66 0 | 60.2 | 0.7 | Kylo-19-DS728 | 80.1 | 0.9 |
| Kylo-19-DS66 2 | 70.5 | 2.7 | Kylo-19-DS729 | 84.7 | 2.6 |
| Kylo-19-DS66 3 | 76.5 | 2.3 | Kylo-19-DS730 | 60.4 | 3.3 |
| Kylo-19-DS66 4 | 75.5 | 1.3 | Kylo-19-DS731 | 71.5 | 4.1 |
| Kylo-19-DS66 5 | 0 | 9.8 | Kylo-19-DS732 | 58 | 1.7 |
| Kylo-19-DS66 7 | 84.8 | 1 | Kylo-19-DS733 | 0 | 14.8 |
| Kylo-19-DS66 8 | 74.4 | 2.2 | Kylo-19-DS734 | 60.5 | 1.3 |
| Kylo-19-DS66 9 | 88.9 | 0.5 | Kylo-19-DS735 | 61.9 | 4.3 |
| Kylo-19-DS67 0 | 59.6 | 1.7 | Kylo-19-DS736 | 0 | 8.3 |
| Kylo-19-DS67 1 | 0 | 12.8 | Kylo-19-DS737 | 70.3 | 2.1 |
| Kylo-19-DS67 2 | 16.1 | 7 | Kylo-19-DS738 | 75.3 | 0.4 |
| Kylo-19-DS67 3 | 37.6 | 4.6 | Kylo-19-DS740 | 20.2 | 7.2 |
| Kylo-19-DS67 4 | 80.8 | 0.4 | Kylo-19-DS741 | 51.6 | 1.6 |
| Kylo-19-DS67 5 | 62.3 | 3 | Kylo-19-DS742 | 0 | 11.1 |
| Kylo-19-DS67 6 | 84.5 | 0.8 | Kylo-19-DS744 | 58.3 | 10.7 |
| Kylo-19-DS67 7 | 84.4 | 0.4 | Kylo-19-DS745 | 9.6 | 3.1 |
| Kylo-19-DS67 8 | 72.2 | 2.3 | Kylo-19-DS746 | 44.5 | 12.9 |
| Kylo-19-DS67 9 | 73.7 | 0.8 | Kylo-19-DS747 | 66.2 | 3.3 |
| Kylo-19-DS68 0 | 83.1 | 1.1 | Kylo-19-DS748 | 0 | 2.2 |
| Kylo-19-DS68 1 | 51.2 | 5.5 | Kylo-19-DS749 | 0 | 6.8 |
| Kylo-19-DS68 2 | 71.1 | 2.6 | | | |

**Table 15-6 Inhibitory effect of 1 and 0.1 nM C5 RNA inhibitor in Huh7 cells**

| siRNA inhibitor | Inhibition rate (%) | | SD | |
|---|---|---|---|---|
| | 1nM | 0.1nM | 1nM | 0.1nM |
| Kylo-19-DS639 | 90.7 | 79.7 | 1.3 | 4.7 |
| Kylo-19-DS689 | 83.1 | 74.3 | 1.7 | 4.7 |
| Kylo-19-DS695 | 88.5 | 80.4 | 1.7 | 1.7 |
| Kylo-19-DS707 | 88.3 | 82.6 | 1.8 | 0.8 |
| Kylo-19-DS708 | 93.2 | 81.6 | 1.2 | 1.7 |
| Kylo-19-DS710 | 81.3 | 75.2 | 0.3 | 5 |
| Kylo-19-DS711 | 90.8 | 78.4 | 1.2 | 2.7 |

**Table 15-7 Inhibitory effect of 0.01 nM C5 RNA inhibitor in Hep3B cells**

| siRNA inhibitor (0.01 nM) | Inhibition rate % | SD |
|---|---|---|
| Kylo-19-DS313 | 74.79 | 4.5 |
| Kylo-19-DS315 | 48.2 | 3.33 |
| Kylo-19-DS316 | 26.76 | 4 |
| Kylo-19-DS317 | 53.81 | 12.64 |
| Kylo-19-DS318 | 64.25 | 6.68 |
| Kylo-19-DS320 | 54.19 | 3.96 |
| Kylo-19-DS321 | 69.57 | 4.17 |
| Kylo-19-DS322 | 57.69 | 7.8 |
| Kylo-19-DS323 | 68.91 | 1.23 |
| Kylo-19-DS324 | 43.7 | 1.19 |
| Kylo-19-DS325 | 65.32 | 4.31 |
| Kylo-19-DS326 | 67.95 | 3.61 |
| Kylo-19-DS327 | 46.76 | 11.09 |
| Kylo-19-DS328 | 59.69 | 3.06 |
| Kylo-19-DS329 | 70.01 | 3.5 |
| Kylo-19-DS330 | 70.11 | 5.74 |
| Kylo-19-DS331 | 68.07 | 2.79 |
| Kylo-19-DS332 | 73.14 | 2.12 |
| Kylo-19-DS333 | 61.93 | 2.86 |
| Kylo-19-DS334 | 75.03 | 8.16 |
| Kylo-19-DS335 | 63.3 | 6.05 |
| Kylo-19-DS336 | 38.83 | 9.19 |
| Kylo-19-DS337 | 63.27 | 3.09 |
| Kylo-19-DS338 | 66.29 | 2.32 |
| Kylo-19-DS339 | 63.88 | 4.3 |
| Kylo-19-DS340 | 70.37 | 1 |
| Kylo-19-DS341 | 79.4 | 3.73 |
| Kylo-19-DS342 | 52.93 | 16.93 |
| Kylo-19-DS343 | 49.34 | 5.44 |
| Kylo-19-DS344 | 42.65 | 17.33 |
| Kylo-19-DS345 | 10.86 | 4.87 |
| Kylo-19-DS346 | 65.28 | 3.92 |
| Kylo-19-DS347 | 60.42 | 10.97 |
| Kylo-19-DS348 | 42.9 | 16.73 |
| Kylo-19-DS349 | 45.23 | 8.64 |
| Kylo-19-DS350 | 64.55 | 8.08 |
| Kylo-19-DS351 | 0 | 23.45 |
| Kylo-19-DS352 | 0 | 22.55 |
| Kylo-19-DS353 | 71.78 | 1.53 |
| Kylo-19-DS354 | 77.72 | 5.55 |
| Kylo-19-DS355 | 71.22 | 8.87 |
| Kylo-19-DS356 | 68.63 | 12.82 |
| Kylo-19-DS357 | 66.46 | 5.22 |

**Table 15-8 Inhibitory effect of 0.01 nM C5 RNA inhibitor in Hep3B cells**

| siRNA inhibitor (0.01 nM) | Inhibition rate % | SD | siRNA inhibitor (0.01 nM) | Inhibition rate % | SD |
|---|---|---|---|---|---|
| Kylo-19-DS367 | 70.15 | 1.33 | Kylo-19-DS498 | 58.14 | 7.43 |
| Kylo-19-DS368 | 67.66 | 0.93 | Kylo-19-DS499 | 74.04 | 3.05 |
| Kylo-19-DS369 | 48.38 | 5.52 | Kylo-19-DS500 | 66.27 | 4.06 |
| Kylo-19-DS370 | 76.58 | 4.32 | Kylo-19-DS501 | 32.3 | 7.92 |
| Kylo-19-DS371 | 75.65 | 3.75 | Kylo-19-DS502 | 68.49 | 4.03 |
| Kylo-19-DS372 | 46.88 | 2.92 | Kylo-19-DS503 | 45.78 | 3.76 |
| Kylo-19-DS373 | 53.68 | 17.42 | Kylo-19-DS504 | 55.55 | 2.96 |
| Kylo-19-DS374 | 79.94 | 2.93 | Kylo-19-DS505 | 54.24 | 4.62 |
| Kylo-19-DS375 | 66.19 | 2.8 | Kylo-19-DS506 | 0 | 13.66 |
| Kylo-19-DS376 | 31.49 | 7.79 | Kylo-19-DS507 | 30.34 | 7.63 |
| Kylo-19-DS377 | 66.78 | 1.74 | Kylo-19-DS508 | 32.76 | 8.27 |
| Kylo-19-DS378 | 47.05 | 17 | Kylo-19-DS509 | 42.5 | 1.03 |
| Kylo-19-DS379 | 45.58 | 9.83 | Kylo-19-DS510 | 49.82 | 1.32 |
| Kylo-19-DS380 | 31.93 | 14.97 | Kylo-19-DS511 | 31.64 | 7.33 |
| Kylo-19-DS381 | 51.99 | 8.17 | Kylo-19-DS512 | 13.45 | 3.45 |
| Kylo-19-DS382 | 48.99 | 1.31 | Kylo-19-DS513 | 42.79 | 12.16 |
| Kylo-19-DS383 | 28.2 | 9.66 | Kylo-19-DS514 | 39.82 | 5.87 |
| Kylo-19-DS384 | 20.3 | 0.6 | Kylo-19-DS515 | 48.39 | 5.64 |
| Kylo-19-DS385 | 19.82 | 6.8 | Kylo-19-DS516 | 44.79 | 5.73 |
| Kylo-19-DS386 | 20.14 | 4.83 | Kylo-19-DS517 | 1.79 | 18.83 |
| Kylo-19-DS387 | 80.09 | 2.18 | Kylo-19-DS518 | 50.86 | 12.5 |
| Kylo-19-DS388 | 65.1 | 1.2 | Kylo-19-DS519 | 42.06 | 4.1 |
| Kylo-19-DS389 | 43.43 | 7.74 | Kylo-19-DS520 | 34.72 | 5.63 |
| Kylo-19-DS390 | 23.4 | 4.1 | Kylo-19-DS521 | 64.77 | 12.31 |
| Kylo-19-DS391 | 52.38 | 5.89 | Kylo-19-DS522 | 14.53 | 1.91 |
| Kylo-19-DS392 | 66.05 | 4.38 | Kylo-19-DS523 | 14.62 | 9.09 |
| Kylo-19-DS393 | 70.54 | 4.69 | Kylo-19-DS524 | 24.17 | 1.56 |
| Kylo-19-DS394 | 50.74 | 3.09 | Kylo-19-DS525 | 40.51 | 9.53 |
| Kylo-19-DS395 | 40.05 | 4.57 | Kylo-19-DS526 | 0 | 3.28 |
| Kylo-19-DS396 | 40.79 | 8.27 | Kylo-19-DS527 | 42.78 | 5.17 |
| Kylo-19-DS397 | 58.44 | 3.51 | Kylo-19-DS528 | 59.7 | 3.46 |
| Kylo-19-DS398 | 53.34 | 3.19 | Kylo-19-DS529 | 64.07 | 6.56 |
| Kylo-19-DS399 | 79.15 | 0.87 | Kylo-19-DS530 | 62.45 | 0.16 |
| Kylo-19-DS400 | 81.37 | 2.98 | Kylo-19-DS531 | 70.41 | 0.92 |
| Kylo-19-DS401 | 73.02 | 2.35 | Kylo-19-DS532 | 30.46 | 5.76 |
| Kylo-19-DS402 | 82.26 | 2.33 | Kylo-19-DS533 | 51.31 | 2.94 |
| Kylo-19-DS403 | 59.05 | 3.05 | Kylo-19-DS534 | 39.47 | 8.23 |
| Kylo-19-DS404 | 61.49 | 2.11 | Kylo-19-DS535 | 69.66 | 1.76 |
| Kylo-19-DS405 | 48.96 | 4.96 | Kylo-19-DS536 | 70.11 | 4.18 |
| Kylo-19-DS406 | 57.87 | 6.44 | Kylo-19-DS537 | 75.9 | 1.36 |
| Kylo-19-DS407 | 60.22 | 2.55 | Kylo-19-DS538 | 42.99 | 9.87 |
| Kylo-19-DS408 | 63.36 | 2.85 | Kylo-19-DS539 | 62.96 | 3.54 |
| Kylo-19-DS409 | 37.1 | 6.35 | Kylo-19-DS540 | 44.82 | 8.91 |
| Kylo-19-DS410 | 67.79 | 2.58 | Kylo-19-DS541 | 46.91 | 6.64 |
| Kylo-19-DS411 | 69.49 | 6.07 | Kylo-19-DS542 | 17.04 | 10.38 |
| Kylo-19-DS412 | 44.02 | 8.6 | Kylo-19-DS543 | 24.88 | 9.15 |
| Kylo-19-DS413 | 68.16 | 1.73 | Kylo-19-DS544 | 57.32 | 3.05 |
| Kylo-19-DS414 | 60.84 | 4.53 | Kylo-19-DS545 | 35.21 | 5.78 |
| Kylo-19-DS415 | 65.39 | 5.06 | Kylo-19-DS546 | 37.39 | 9.7 |
| Kylo-19-DS416 | 57.92 | 11.09 | Kylo-19-DS547 | 39.26 | 8 |
| Kylo-19-DS417 | 71.09 | 9.52 | Kylo-19-DS548 | 69.88 | 4.15 |
| Kylo-19-DS418 | 15.88 | 8.43 | Kylo-19-DS549 | 75.07 | 2.31 |
| Kylo-19-DS419 | 64.9 | 4.01 | Kylo-19-DS550 | 59.18 | 3.76 |
| Kylo-19-DS420 | 0 | 2.82 | Kylo-19-DS551 | 28.5 | 11.88 |
| Kylo-19-DS421 | 11.38 | 5.54 | Kylo-19-DS552 | 36.91 | 6.92 |
| Kylo-19-DS422 | 27.33 | 7.31 | Kylo-19-DS553 | 60.3 | 2.19 |
| Kylo-19-DS423 | 3.9 | 10.94 | Kylo-19-DS554 | 48.52 | 4.42 |
| Kylo-19-DS424 | 2.12 | 9.33 | Kylo-19-DS555 | 51.15 | 3.66 |
| Kylo-19-DS425 | 27.39 | 5.75 | Kylo-19-DS556 | 31.84 | 11.34 |
| Kylo-19-DS426 | 31.07 | 9.48 | Kylo-19-DS557 | 43.62 | 4.69 |
| Kylo-19-DS427 | 31.49 | 9.7 | Kylo-19-DS558 | 59.62 | 4.24 |
| Kylo-19-DS428 | 46.53 | 3.53 | Kylo-19-DS559 | 67.4 | 4.7 |
| Kylo-19-DS429 | 14.88 | 6.38 | Kylo-19-DS560 | 59.61 | 3.94 |
| Kylo-19-DS430 | 63.98 | 1.01 | Kylo-19-DS561 | 64.73 | 2.14 |
| Kylo-19-DS431 | 15.04 | 13.18 | Kylo-19-DS562 | 75.46 | 3.73 |
| Kylo-19-DS432 | 24.18 | 6.79 | Kylo-19-DS563 | 25.18 | 5.67 |
| Kylo-19-DS433 | 56.67 | 2.43 | Kylo-19-DS564 | 40.76 | 2.23 |
| Kylo-19-DS434 | 54.07 | 5.32 | Kylo-19-DS565 | 40.5 | 8.84 |
| Kylo-19-DS435 | 38.68 | 9.57 | Kylo-19-DS566 | 27.47 | 6.05 |
| Kylo-19-DS436 | 49.44 | 2.97 | Kylo-19-DS567 | 40.08 | 1.71 |
| Kylo-19-DS437 | 39.5 | 2.3 | Kylo-19-DS568 | 17.57 | 4.73 |
| Kylo-19-DS438 | 32.35 | 2.09 | Kylo-19-DS569 | 75.15 | 4.31 |
| Kylo-19-DS439 | 36.01 | 4.25 | Kylo-19-DS570 | 81.29 | 1.5 |
| Kylo-19-DS440 | 43.99 | 15.77 | Kylo-19-DS571 | 51.34 | 1.91 |
| Kylo-19-DS441 | 21.43 | 7.08 | Kylo-19-DS572 | 8.74 | 5.45 |
| Kylo-19-DS442 | 60.17 | 1.23 | Kylo-19-DS573 | 50.22 | 5.21 |
| Kylo-19-DS443 | 54.38 | 0.79 | Kylo-19-DS574 | 44.65 | 8.77 |
| Kylo-19-DS444 | 48.72 | 2.86 | Kylo-19-DS575 | 71.33 | 3.36 |
| Kylo-19-DS445 | 15.38 | 7.33 | Kylo-19-DS576 | 29.11 | 6.48 |
| Kylo-19-DS446 | 51.66 | 1.76 | Kylo-19-DS577 | 15.59 | 4.74 |
| Kylo-19-DS447 | 38.58 | 7 | Kylo-19-DS578 | 41.25 | 2.04 |
| Kylo-19-DS448 | 47.4 | 2.49 | Kylo-19-DS579 | 51.03 | 6.8 |
| Kylo-19-DS449 | 50.8 | 6.61 | Kylo-19-DS580 | 64.52 | 1.08 |
| Kylo-19-DS450 | 78.04 | 1.54 | Kylo-19-DS581 | 46.93 | 6.21 |
| Kylo-19-DS451 | 34.43 | 6.34 | Kylo-19-DS582 | 43.85 | 9.01 |
| Kylo-19-DS452 | 38.37 | 8.04 | Kylo-19-DS583 | 49.52 | 4.12 |
| Kylo-19-DS453 | 77.92 | 1.55 | Kylo-19-DS584 | 18.82 | 15.17 |
| Kylo-19-DS454 | 44.99 | 10.85 | Kylo-19-DS585 | 69.03 | 3.63 |
| Kylo-19-DS455 | 65.62 | 0.78 | Kylo-19-DS586 | 50.12 | 9.41 |
| Kylo-19-DS456 | 74.26 | 6.58 | Kylo-19-DS587 | 46.5 | 4.64 |
| Kylo-19-DS457 | 62.76 | 4.38 | Kylo-19-DS588 | 36.33 | 2.86 |
| Kylo-19-DS458 | 76.72 | 0.73 | Kylo-19-DS589 | 52.94 | 6.79 |
| Kylo-19-DS459 | 76.71 | 2.31 | Kylo-19-DS590 | 41.4 | 2.14 |
| Kylo-19-DS460 | 75.46 | 7.39 | Kylo-19-DS591 | 48.38 | 3.02 |
| Kylo-19-DS461 | 76.35 | 5.22 | Kylo-19-DS592 | 49.34 | 5.82 |
| Kylo-19-DS462 | 70.1 | 3.42 | Kylo-19-DS593 | 25.62 | 11.57 |
| Kylo-19-DS463 | 82.9 | 1.38 | Kylo-19-DS594 | 41.28 | 8.82 |
| Kylo-19-DS464 | 64.11 | 9.5 | Kylo-19-DS595 | 46.4 | 3.76 |
| Kylo-19-DS465 | 53 | 7.32 | Kylo-19-DS596 | 7.15 | 19.62 |
| Kylo-19-DS466 | 76.03 | 6.23 | Kylo-19-DS597 | 65.67 | 3.49 |
| Kylo-19-DS467 | 58.68 | 16.26 | Kylo-19-DS598 | 56.48 | 1.52 |
| Kylo-19-DS468 | 45.32 | 1.85 | Kylo-19-DS599 | 48.21 | 4.93 |
| Kylo-19-DS469 | 38.44 | 8.95 | Kylo-19-DS600 | 68.57 | 2.73 |
| Kylo-19-DS470 | 71.66 | 9.02 | Kylo-19-DS601 | 26.36 | 3.41 |
| Kylo-19-DS471 | 68.45 | 2.73 | Kylo-19-DS602 | 70.58 | 0.89 |
| Kylo-19-DS472 | 68.1 | 4.31 | Kylo-19-DS603 | 66.84 | 1.23 |
| Kylo-19-DS473 | 29.24 | 5.73 | Kylo-19-DS604 | 69.14 | 2.41 |
| Kylo-19-DS474 | 29.22 | 2.17 | Kylo-19-DS605 | 58.51 | 4.3 |
| Kylo-19-DS475 | 71.08 | 3.19 | Kylo-19-DS606 | 46.5 | 7.35 |
| Kylo-19-DS476 | 63.53 | 2.1 | Kylo-19-DS607 | 61.76 | 3.85 |
| Kylo-19-DS477 | 67.38 | 5.44 | Kylo-19-DS608 | 57.49 | 1.92 |
| Kylo-19-DS478 | 66.47 | 3.26 | Kylo-19-DS609 | 56.48 | 0.87 |
| Kylo-19-DS479 | 76.51 | 2.78 | Kylo-19-DS610 | 69.28 | 2.96 |
| Kylo-19-DS480 | 63.56 | 10.19 | Kylo-19-DS611 | 69.04 | 2.63 |
| Kylo-19-DS481 | 58.52 | 4.34 | Kylo-19-DS612 | 69.35 | 2.78 |
| Kylo-19-DS482 | 57.23 | 17.52 | Kylo-19-DS613 | 61.89 | 4.09 |
| Kylo-19-DS483 | 62.11 | 7.47 | Kylo-19-DS614 | 62.2 | 2.68 |
| Kylo-19-DS484 | 23.3 | 4.29 | Kylo-19-DS615 | 57.44 | 4.78 |
| Kylo-19-DS485 | 42.62 | 3.66 | Kylo-19-DS616 | 62.78 | 2.74 |
| Kylo-19-DS486 | 46.82 | 7.78 | Kylo-19-DS617 | 57.03 | 0.77 |
| Kylo-19-DS487 | 37.59 | 15.36 | Kylo-19-DS618 | 57.45 | 1.19 |
| Kylo-19-DS488 | 78.41 | 4.44 | Kylo-19-DS314 | 74.69 | 6.8 |
| Kylo-19-DS489 | 41.54 | 25.27 | Kylo-19-DS358 | 19.48 | 11.43 |
| Kylo-19-DS490 | 44.85 | 7.12 | Kylo-19-DS359 | 41.62 | 2.66 |
| Kylo-19-DS491 | 5.74 | 11.17 | Kylo-19-DS360 | 40.11 | 3.38 |
| Kylo-19-DS492 | 52.41 | 1.35 | Kylo-19-DS361 | 35.32 | 2.77 |
| Kylo-19-DS493 | 38.44 | 12.89 | Kylo-19-DS362 | 79.23 | 2.65 |
| Kylo-19-DS494 | 74.09 | 5.5 | Kylo-19-DS363 | 80.86 | 0.41 |
| Kylo-19-DS495 | 26.85 | 10.18 | Kylo-19-DS364 | 70.25 | 5.18 |
| Kylo-19-DS496 | 53.94 | 8.33 | Kylo-19-DS365 | 68.42 | 2.13 |
| Kylo-19-DS497 | 61.59 | 2.92 | Kylo-19-DS366 | 61.65 | 5.26 |

### Example 2-2 In Vitro Assay of RNA Inhibitors for Inhibiting C3 Gene Expression

The RNA inhibitors used in this example are shown in Table 1-3. Plasmid DNA (C3_PSICHECK(TM)-2) was transfected into Hep3B and Huh7 cells using Fugene HD. The transfected cells were seeded into 96-well plates at a density of 10,000 cells per well, with 100 µL of culture medium per well. The cells were cultured overnight in an incubator at 37 °C with 5% CO₂. Then, the RNA inhibitors were prepared with PBS into nanolipid-encapsulated RNA inhibitor sample solutions of corresponding concentrations. RNAiMAX/Opti-MEM was added to each well at the corresponding position, and the diluted RNA inhibitor sample solution of corresponding concentration was added into the wells, mixed well and incubated. The incubated mixture was uniformly mixed with DMEM containing 10% FBS. The culture medium in each well was aspirated, and then fresh culture medium containing the sample was added. After addition, the cells were cultured in an incubator at 37 °C with 5% CO₂, and the final test concentration of the sample was 0.01 nM.

The cells were taken out from the incubator, the supernatant was discarded, fresh culture medium and detection reagent were added, and the mixture was shaken in the dark. After sufficient cell lysis, the sample was transferred to an opaque white plate to detect the firefly luminescence signal; Dual-Glo^{®}Stop & Glo^{®} detection reagent was added to each well, and the mixture was shaken in the dark to detect the Renilla luciferase signal. The ratio of the signal of the primary reporter gene to the signal of the internal reference reporter gene in each well was calculated. The test results obtained are shown in Table 15-9 below.

**Table 15-9 Inhibitory Effect of 0.01 nM RNA Inhibitors on C3 mRNA in Hep3B Cells**

| Duplex Code | Inhibitio n Rate % | SD% | Duplex Code | Inhibition Rate % | SD% |
|---|---|---|---|---|---|
| Kylo-27-DS341 | 90.1 | 0.26 | Kylo-27-DS501 | 50.6 | 4.55 |
| Kylo-27-DS359 | 89.29 | 0.23 | Kylo-27-DS542 | 50.55 | 5.01 |
| Kylo-27-DS358 | 86.34 | 2.64 | Kylo-27-DS418 | 50.11 | 13.12 |
| Kylo-27-DS361 | 85.95 | 1.53 | Kylo-27-DS484 | 50.08 | 4.4 |
| Kylo-27-DS351 | 85.89 | 2.29 | Kylo-27-DS387 | 49.69 | 6.6 |
| Kylo-27-DS564 | 85.62 | 0.87 | Kylo-27-DS357 | 49.41 | 8.21 |
| Kylo-27-DS368 | 85.5 | 0.94 | Kylo-27-DS552 | 49.36 | 2.91 |
| Kylo-27-DS365 | 85.35 | 3.32 | Kylo-27-DS576 | 49.1 | 7.66 |
| Kylo-27-DS374 | 83.9 | 1.61 | Kylo-27-DS557 | 49.02 | 16.37 |
| Kylo-27-DS376 | 83.37 | 2.07 | Kylo-27-DS379 | 48.94 | 4.56 |
| Kylo-27-DS560 | 83.31 | 5.24 | Kylo-27-DS575 | 48.91 | 8.77 |
| Kylo-27-DS367 | 82.64 | 1.93 | Kylo-27-DS354 | 48.68 | 6.54 |
| Kylo-27-DS417 | 81.95 | 1.89 | Kylo-27-DS442 | 47.61 | 7.82 |
| Kylo-27-DS565 | 81.36 | 0.93 | Kylo-27-DS517 | 47.3 | 7.02 |
| Kylo-27-DS503 | 81.21 | 2.07 | Kylo-27-DS472 | 47.14 | 9.87 |
| Kylo-27-DS375 | 79.59 | 4.03 | Kylo-27-DS371 | 46.9 | 1.05 |
| Kylo-27-DS520 | 79.53 | 3.06 | Kylo-27-DS485 | 46.88 | 1.72 |
| Kylo-27-DS355 | 78.59 | 3.38 | Kylo-27-DS468 | 46.36 | 11.24 |
| Kylo-27-DS366 | 78.36 | 0.47 | Kylo-27-DS391 | 46.33 | 8.89 |
| Kylo-27-DS563 | 78.35 | 4.43 | Kylo-27-DS521 | 45.42 | 9.27 |
| Kylo-27-DS364 | 77.93 | 1.86 | Kylo-27-DS413 | 45.34 | 9.02 |
| Kylo-27-DS352 | 76.9 | 1.52 | Kylo-27-DS429 | 44.96 | 1.94 |
| Kylo-27-DS473 | 76.65 | 4.3 | Kylo-27-DS581 | 44.71 | 10.37 |
| Kylo-27-DS497 | 76.38 | 3.8 | Kylo-27-DS405 | 44.55 | 5.73 |
| Kylo-27-DS362 | 76.03 | 1.69 | Kylo-27-DS479 | 44.5 | 4.41 |
| Kylo-27-DS443 | 75.99 | 1.53 | Kylo-27-DS547 | 44.43 | 6.32 |
| Kylo-27-DS411 | 75.6 | 3.31 | Kylo-27-DS385 | 44.23 | 12.08 |
| Kylo-27-DS577 | 75.07 | 2.11 | Kylo-27-DS447 | 44.09 | 13.45 |
| Kylo-27-DS492 | 74.98 | 7.44 | Kylo-27-DS573 | 44.07 | 2.59 |
| Kylo-27-DS421 | 74.78 | 3.31 | Kylo-27-DS496 | 43.4 | 16.2 |
| Kylo-27-DS449 | 74.47 | 4.14 | Kylo-27-DS502 | 43.38 | 9.39 |
| Kylo-27-DS377 | 74.22 | 3.47 | Kylo-27-DS384 | 43.29 | 12.15 |
| Kylo-27-DS505 | 73.59 | 3.91 | Kylo-27-DS549 | 43.12 | 9.9 |
| Kylo-27-DS471 | 73.57 | 10.58 | Kylo-27-DS467 | 43.05 | 9.9 |
| Kylo-27-DS482 | 73.56 | 6.41 | Kylo-27-DS489 | 43 | 4.04 |
| Kylo-27-DS474 | 73.42 | 4.8 | Kylo-27-DS537 | 42.93 | 5.62 |
| Kylo-27-DS469 | 72.64 | 7.46 | Kylo-27-DS450 | 42.84 | 8.33 |
| Kylo-27-DS369 | 71.94 | 2.16 | Kylo-27-DS425 | 42.68 | 0.97 |
| Kylo-27-DS584 | 71.69 | 4.04 | Kylo-27-DS529 | 42.62 | 12.09 |
| Kylo-27-DS466 | 71.63 | 0.92 | Kylo-27-DS463 | 42.38 | 1.12 |
| Kylo-27-DS554 | 71.61 | 0.25 | Kylo-27-DS432 | 41.93 | 13.61 |
| Kylo-27-DS545 | 71.44 | 5.25 | Kylo-27-DS580 | 41.63 | 5.17 |
| Kylo-27-DS465 | 69.99 | 4.85 | Kylo-27-DS389 | 41.32 | 10.7 |
| Kylo-27-DS373 | 69.48 | 0.98 | Kylo-27-DS458 | 40.44 | 4.07 |
| Kylo-27-DS483 | 69.38 | 4.8 | Kylo-27-DS556 | 40.11 | 10.67 |
| Kylo-27-DS441 | 69.28 | 5.32 | Kylo-27-DS507 | 39.92 | 5.1 |
| Kylo-27-DS403 | 68.85 | 6.59 | Kylo-27-DS380 | 39.81 | 9.97 |
| Kylo-27-DS445 | 68.77 | 5.19 | Kylo-27-DS506 | 39.52 | 8.1 |
| Kylo-27-DS455 | 68.39 | 3.06 | Kylo-27-DS534 | 39.28 | 2.69 |
| Kylo-27-DS561 | 67.89 | 3.74 | Kylo-27-DS571 | 39.19 | 4.38 |
| Kylo-27-DS527 | 67.69 | 2.48 | Kylo-27-DS427 | 38.86 | 8.1 |
| Kylo-27-DS388 | 67.47 | 2.35 | Kylo-27-DS433 | 38.27 | 11.43 |
| Kylo-27-DS460 | 67.42 | 3.83 | Kylo-27-DS541 | 37.9 | 1.83 |
| Kylo-27-DS524 | 67.28 | 3.19 | Kylo-27-DS444 | 37.56 | 5.62 |
| Kylo-27-DS546 | 67.26 | 6.73 | Kylo-27-DS453 | 37.18 | 5.81 |
| Kylo-27-DS583 | 66.97 | 5.01 | Kylo-27-DS356 | 36.89 | 4.67 |
| Kylo-27-DS536 | 66.63 | 2.9 | Kylo-27-DS511 | 36.86 | 1.95 |
| Kylo-27-DS470 | 66.55 | 8.15 | Kylo-27-DS430 | 36.8 | 3.88 |
| Kylo-27-DS353 | 66.35 | 7.94 | Kylo-27-DS540 | 36.44 | 7.66 |
| Kylo-27-DS567 | 65.4 | 3.62 | Kylo-27-DS510 | 35.48 | 5.28 |
| Kylo-27-DS579 | 65.04 | 1.14 | Kylo-27-DS437 | 35.11 | 14.57 |
| Kylo-27-DS488 | 64.78 | 1.94 | Kylo-27-DS422 | 34.85 | 5.61 |
| Kylo-27-DS360 | 64.69 | 3.16 | Kylo-27-DS532 | 34.78 | 8.95 |
| Kylo-27-DS464 | 64.67 | 2.68 | Kylo-27-DS512 | 34.58 | 4.89 |
| Kylo-27-DS526 | 64.51 | 2.91 | Kylo-27-DS423 | 34.29 | 8.2 |
| Kylo-27-DS533 | 64.36 | 0.72 | Kylo-27-DS424 | 33.84 | 2.09 |
| Kylo-27-DS516 | 64.16 | 14.21 | Kylo-27-DS582 | 33.75 | 8.08 |
| Kylo-27-DS404 | 64.07 | 4.16 | Kylo-27-DS414 | 33.26 | 10.2 |
| Kylo-27-DS528 | 63.79 | 1.11 | Kylo-27-DS439 | 33.23 | 13.55 |
| Kylo-27-DS487 | 63.35 | 2.27 | Kylo-27-DS410 | 33.07 | 12.82 |
| Kylo-27-DS551 | 63.16 | 3.94 | Kylo-27-DS426 | 32.89 | 5.66 |
| Kylo-27-DS454 | 63.14 | 3.42 | Kylo-27-DS513 | 32.85 | 9.22 |
| Kylo-27-DS478 | 63.02 | 4.44 | Kylo-27-DS522 | 32.26 | 2.94 |
| Kylo-27-DS461 | 62.67 | 6.99 | Kylo-27-DS477 | 31.53 | 7.75 |
| Kylo-27-DS498 | 62.61 | 7.45 | Kylo-27-DS370 | 31.08 | 6.44 |
| Kylo-27-DS490 | 62.6 | 5.96 | Kylo-27-DS390 | 30.73 | 13.26 |
| Kylo-27-DS570 | 62.26 | 18.24 | Kylo-27-DS395 | 30.71 | 11.95 |
| Kylo-27-DS480 | 62.23 | 8.95 | Kylo-27-DS382 | 29.35 | 10.74 |
| Kylo-27-DS456 | 62.18 | 6.23 | Kylo-27-DS462 | 28.25 | 9.23 |
| Kylo-27-DS481 | 62.04 | 7.48 | Kylo-27-DS531 | 26.83 | 5.04 |
| Kylo-27-DS459 | 61.72 | 2.26 | Kylo-27-DS412 | 25.96 | 8.97 |
| Kylo-27-DS574 | 61.27 | 6.84 | Kylo-27-DS523 | 25.9 | 12.08 |
| Kylo-27-DS419 | 61.19 | 1.92 | Kylo-27-DS436 | 25.77 | 5.25 |
| Kylo-27-DS434 | 61.04 | 7.28 | Kylo-27-DS407 | 24.81 | 10.73 |
| Kylo-27-DS543 | 60.52 | 6.69 | Kylo-27-DS396 | 23.4 | 14.34 |
| Kylo-27-DS491 | 60.32 | 9.64 | Kylo-27-DS446 | 23.14 | 5.39 |
| Kylo-27-DS558 | 60.25 | 6.49 | Kylo-27-DS448 | 23.1 | 4.9 |
| Kylo-27-DS452 | 60.05 | 1.74 | Kylo-27-DS508 | 22.02 | 1.4 |
| Kylo-27-DS550 | 59.28 | 3.05 | Kylo-27-DS438 | 21.29 | 12.99 |
| Kylo-27-DS499 | 59.18 | 2.4 | Kylo-27-DS539 | 20.98 | 12.57 |
| Kylo-27-DS562 | 58.92 | 6.47 | Kylo-27-DS486 | 20.32 | 5 |
| Kylo-27-DS530 | 58.4 | 1.45 | Kylo-27-DS514 | 20.27 | 11.05 |
| Kylo-27-DS548 | 58.37 | 5.26 | Kylo-27-DS397 | 20.05 | 2.53 |
| Kylo-27-DS578 | 57.8 | 3.47 | Kylo-27-DS509 | 19.4 | 4.63 |
| Kylo-27-DS572 | 57.31 | 4.33 | Kylo-27-DS585 | 19.22 | 24.41 |
| Kylo-27-DS420 | 57.26 | 3.28 | Kylo-27-DS451 | 19.02 | 6.34 |
| Kylo-27-DS500 | 56.79 | 8.86 | Kylo-27-DS569 | 17.58 | 10.55 |
| Kylo-27-DS494 | 56.15 | 11.19 | Kylo-27-DS515 | 17.46 | 6.43 |
| Kylo-27-DS493 | 56.13 | 11.49 | Kylo-27-DS415 | 16.53 | 6.58 |
| Kylo-27-DS555 | 55.89 | 5.74 | Kylo-27-DS476 | 16.45 | 3.02 |
| Kylo-27-DS525 | 55.27 | 3.9 | Kylo-27-DS398 | 16.19 | 6.02 |
| Kylo-27-DS409 | 54.8 | 7.94 | Kylo-27-DS495 | 15.37 | 9.39 |
| Kylo-27-DS457 | 54.02 | 10.45 | Kylo-27-DS431 | 14.97 | 3.16 |
| Kylo-27-DS538 | 53.97 | 6.54 | Kylo-27-DS381 | 14.52 | 12.55 |
| Kylo-27-DS566 | 53.94 | 12.18 | Kylo-27-DS402 | 14.29 | 7.99 |
| Kylo-27-DS435 | 53.69 | 6.41 | Kylo-27-DS399 | 14.28 | 2.12 |
| Kylo-27-DS559 | 53.58 | 7.43 | Kylo-27-DS428 | 13.56 | 4.82 |
| Kylo-27-DS378 | 53.33 | 3.21 | Kylo-27-DS386 | 13.02 | 14.36 |
| Kylo-27-DS440 | 53.14 | 11.43 | Kylo-27-DS401 | 12.8 | 4.93 |
| Kylo-27-DS535 | 52.9 | 6.84 | Kylo-27-DS383 | 7.77 | 4.9 |
| Kylo-27-DS416 | 52.59 | 5.91 | Kylo-27-DS394 | 4.29 | 1.71 |
| Kylo-27-DS504 | 52.54 | 9.27 | Kylo-27-DS400 | 2.09 | 4.44 |
| Kylo-27-DS553 | 52.44 | 4.09 | Kylo-27-DS392 | 1.52 | 12.05 |
| Kylo-27-DS372 | 52.12 | 2.91 | Kylo-27-DS393 | -12 | 10.56 |
| Kylo-27-DS363 | 52.06 | 3.05 | Kylo-27-DS586 | -12.73 | 3.82 |
| Kylo-27-DS475 | 51.81 | 4.99 | Kylo-27-DS587 | 48.09 | 2.21 |
| Kylo-27-DS544 | 51.53 | 8.47 | Kylo-27-DS588 | 62.09 | 5.63 |
| Kylo-27-DS518 | 51.35 | 3.15 | Kylo-27-DS589 | 39.28 | 1.66 |
| Kylo-27-DS408 | 51.15 | 10.64 | Kylo-27-DS590 | -23.79 | 11.97 |
| Kylo-27-DS519 | 51.02 | 12.71 | Kylo-27-DS591 | -19.04 | 1.13 |
| Kylo-27-DS568 | 50.78 | 0.75 | Kylo-27-DS240 | 78.59 | 3.43 |
| Kylo-27-DS406 | 50.62 | 9.52 | | | |

Based on the primary sequence screening results, some sequences were selected for modification and carrier conjugation for further inhibitor screening.

### Example 3 Synthesis of Carrier Structures

When the 3'-end of the sense strand or antisense strand of the RNA inhibitor of the present application is conjugated with the carrier structure 3'MVIP, the solid support of 3'MVIP serves as the starting monomer for solid-phase synthesis. When the 5'-end of the sense strand or antisense strand of the RNA inhibitor of the present application is conjugated with the carrier structure 5'MVIP, the 5'-MVIP phosphoramidite monomer serves as the last monomer for solid-phase synthesis.

The general formula of the solid support for 3'MVIP is as follows:

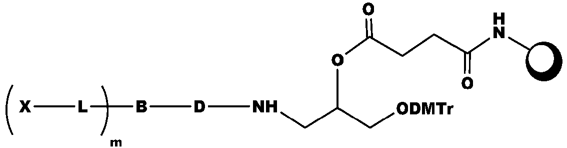

When m is 1-4, the linker B moiety in the general formula is branched 1 to 4 times, respectively, to obtain the corresponding solid support of 3'MVIP.

The general formula of the 5'MVIP phosphoramidite monomer is as follows:

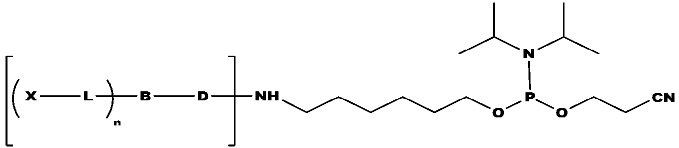

When n is 1-4, the linker B moiety in the general formula is branched 1 to 4 times, respectively, to obtain the corresponding 5'MVIP phosphoramidite monomer.

Only several chemical synthesis processes of 3'MVIP solid supports and 5'MVIP phosphoramidite monomers are exemplified below. With reference to the method described in the examples, those skilled in the art can easily synthesize the remaining 3'MVIP solid supports and 5'MVIP phosphoramidites involved in the present invention. The synthetic process is described as follows:

### 3.1 Synthesis of 3'MVIP Solid Support

### 3.1.1 Synthesis of 3'MVIP09 Solid Support

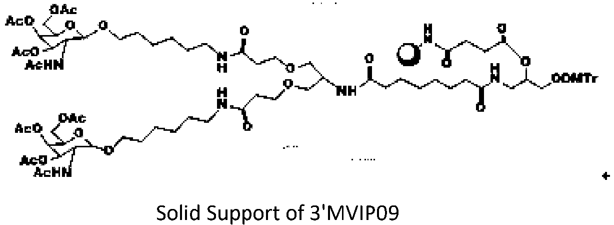

### Description of Synthesis process :

### 3.1.1.1 Synthesis of ERC-01-c1

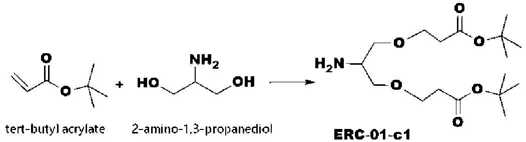

Weigh 2-amino-1,3-propanediol (5.0 g, 54.9 mmol), add 50 mL of DMSO and 5 mL of sodium hydroxide solution (1 g/mL), cool to 0°C, add tert-butyl acrylate (20 mL, 137.8 mmol) dropwise, complete the addition in 2 hours, react at room temperature for 48 h, add petroleum ether (100 mL), wash with saturated brine twice, and dry the organic layer. Purify by column chromatography (eluent: ethyl acetate: petroleum ether = 25%-75%), add 0.05% triethylamine to the column, obtain 6.2 g of colorless oil.

### 3.1.1.2 Synthesis of ERC-01-c2

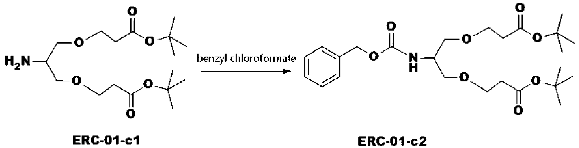

Weigh ERC-01-c1 (6.2 g, 17.9 mmol), add 50 mL of dichloromethane and 23 mL of sodium carbonate solution (25%), add benzyl chloroformate (8.2 mL, 57.4 mmol) dropwise at room temperature, complete the addition in 2 hours, react overnight at room temperature, wash with saturated brine three times, dry over anhydrous sodium sulfate, evaporate the solvent to dryness, and purify by column chromatography (ethyl acetate: petroleum ether = 5%-30%) to obtain 4.0 g of oil.

### 3.1.1.3 Synthesis of ERC-01-c3

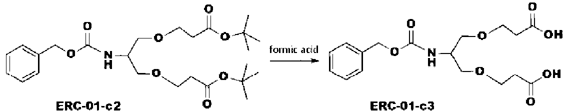

Take ERC-01-c2 (4.0 g, 8.3 mmol), add 12 mL of formic acid, react overnight at room temperature, and evaporate the solvent under reduced pressure to obtain 2.8 g of product.

### 3.1.1.4 Synthesis of ERCd-01-c1

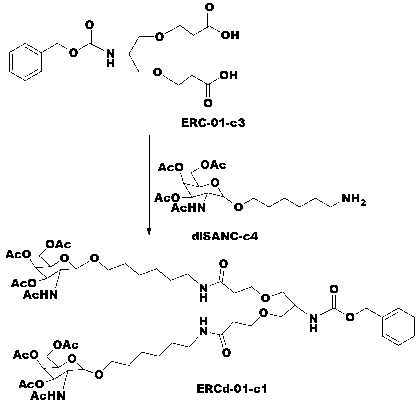

Add compound ERC-01-c3 (1.11 g, 3.00 mmol) and dISANC-c4 (3.6 g, 8.04 mmol) into a reaction vessel, then add HOBt (2.24 g) and HBTU (3.36 g), then slowly add DIEA (4.16 mL). Stir the reaction mixture at room temperature, then add water, extract the aqueous layer with dichloromethane, combine the organic layers, wash successively with saturated sodium bicarbonate solution (2×60 mL) and saturated brine (60 mL), dry over anhydrous sodium sulfate, evaporate to dryness under reduced pressure, and purify by silica gel column chromatography (3-15% MeOH in DCM) to obtain 3.24 g of pale yellow solid.

### 3.1.1.5 Synthesis of ERCd-01-c2

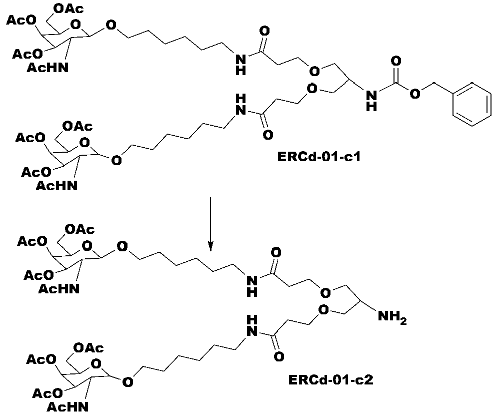

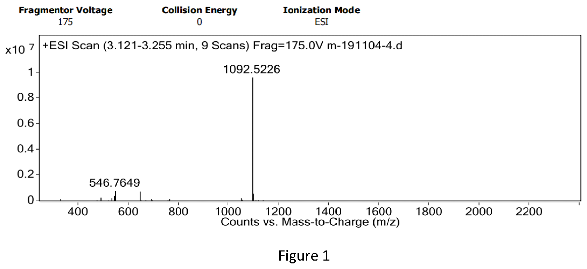

Dissolve ERCd-01-c1 (3.24 g, 2.6 mmol) in methanol (60 mL), add 10% palladium on carbon (0.3 g) and acetic acid (2.0 mL). Hydrogenate at atmospheric pressure overnight. Filter the reaction mixture through celite, and evaporate the filtrate under reduced pressure to obtain 2.9 g of ERCd-01-c2 oil. Its high-resolution mass spectrum is shown in Figure 1.

### 3.1.1.6 Synthesis of 3'MVIP09-c1

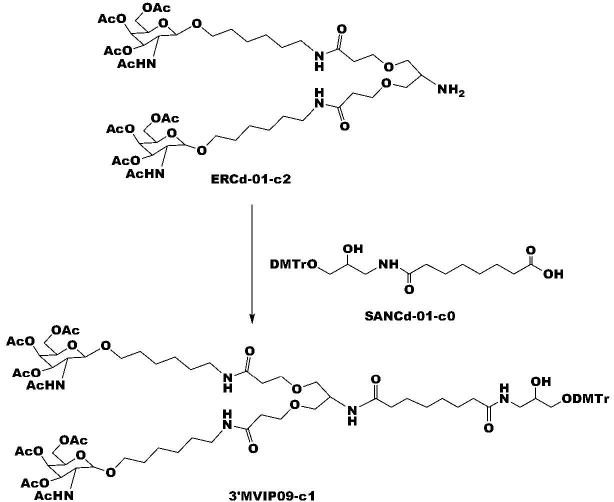

Add SANCd-01-c0 (0.824 g, 1.5 mmol) and ERCd-01-c2 (1.09 g, 1.0 mmol) to a reaction flask in sequence, then add 10 mL of DCM, stir to dissolve, then add TBTU (0.963 g) and DIPEA (0.517 g) in sequence, react overnight, add water, extract with DCM, wash the organic phase with saturated brine, dry, filter, concentrate, and finally purify by silica gel column to obtain 1.3 g of product.

### 3.1.1.7 Synthesis of 3'MVIP09-c2

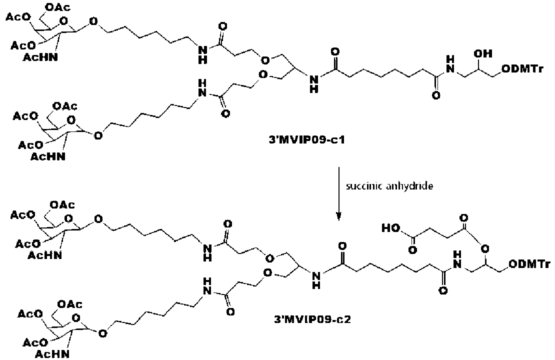

Add 3'MVIP09-c1 (1.62 g, 1 µmol) and 10 mL of DCM to a reaction flask in sequence, stir to dissolve at room temperature, then add DMAP (0.366 g) and succinic anhydride (0.2 g, 3 µmol) in sequence, stir at room temperature for reaction, monitor by TLC, concentrate to remove DCM after qualified reaction, add water, extract with DCM, wash the organic phase with saturated brine, dry the organic phase over anhydrous sodium sulfate, filter, concentrate, and finally purify by silica gel column to obtain 1.55 g of product.

### 3.1.1.8 Synthesis of 3'MVIP09 Solid Support

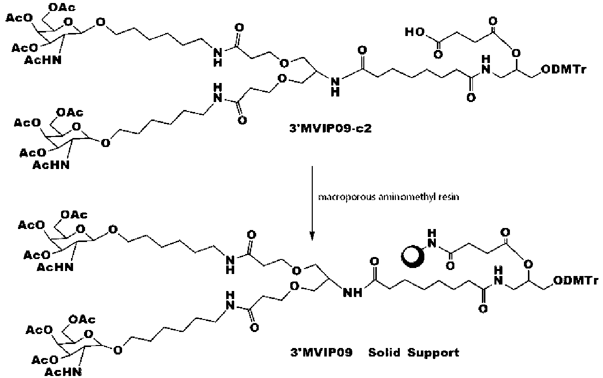

Add 3'MVIP09-c2 (0.86 g, 0.5 µmol) and 10 mL of DMF to a reaction flask in sequence to dissolve, then add HBTU (0.19 g), DIPEA (0.194 g) and macroporous aminomethyl resin (2.0 g) in sequence, shake for 24 h, filter, wash the resin with 10% methanol/DCM, then cap with 25% acetic acid/pyridine, substitution degree 150 µmol/g.

### 3.1.2 Synthesis of 3'MVIP17 Solid Support

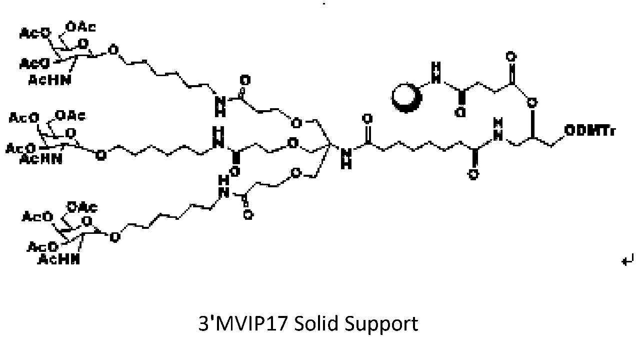

### 3.1.2.1 Synthesis of SANC-01-c1

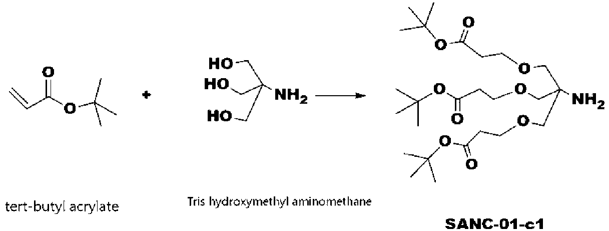

The synthetic procedure refers to the synthesis of ERC-01-c1 in 3.1.1.1.

### 3.1.2.2 Synthesis of SANC-01-c2

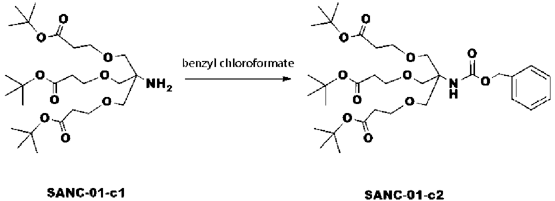

The synthetic procedure refers to the synthesis of ERC-01-c2 in 3.1.1.2.

### 3.1.2.3 Synthesis of SANC-01-c3

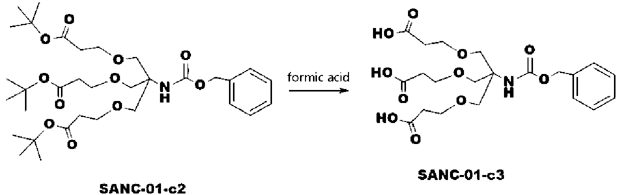

The synthetic procedure refers to the synthesis of ERC-01-c3 in 3.1.1.3.

### 3.1.2.4 Synthesis of SANCd-01-c1

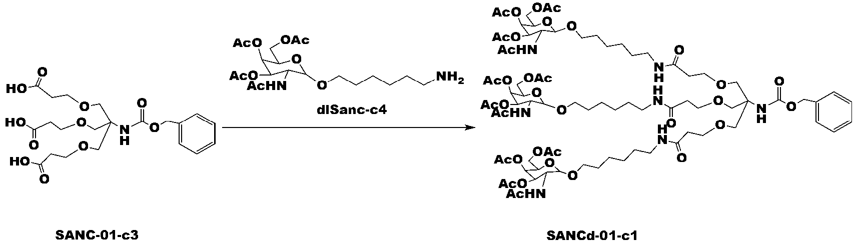

The synthetic procedure refers to the synthesis of ERCd-01-c1 in 3.1.1.4.

### 3.1.2.5 Synthesis of SANCd-01-c2

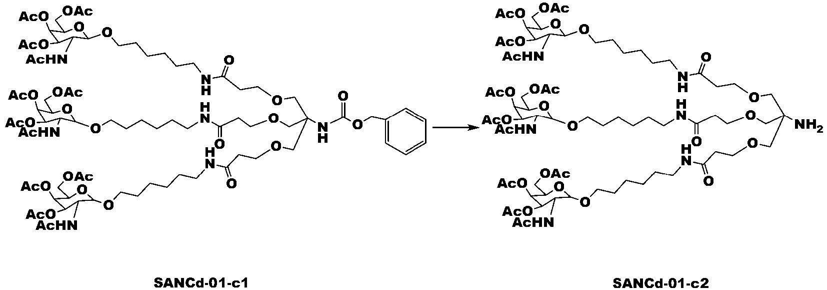

The synthetic procedure refers to the synthesis of ERCd-01-c2 in 3.1.1.5.

### 3.1.2.6 Synthesis of 3'MVIP17-c1

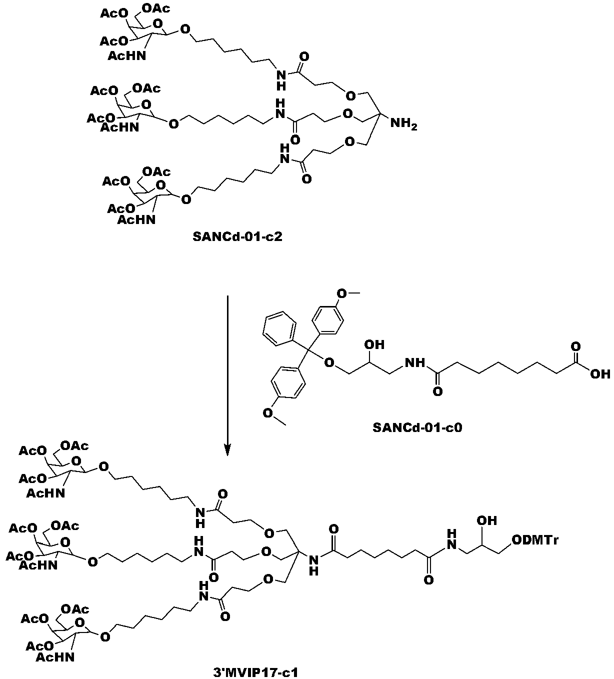

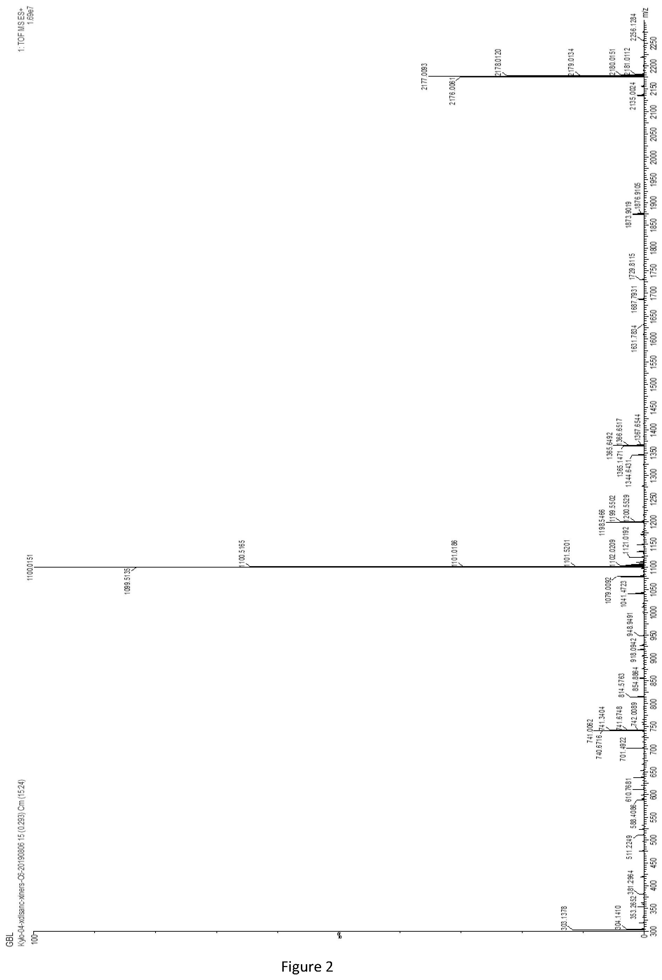

The synthetic procedure refers to the synthesis of 3'MVIP09-c1 in 3.1.1.6. The high-resolution mass spectrum of the synthesized 3'MVIP17-c1 is shown in Figure 2.

### 3.1.2.7 Synthesis of 3'MVIP17-c2

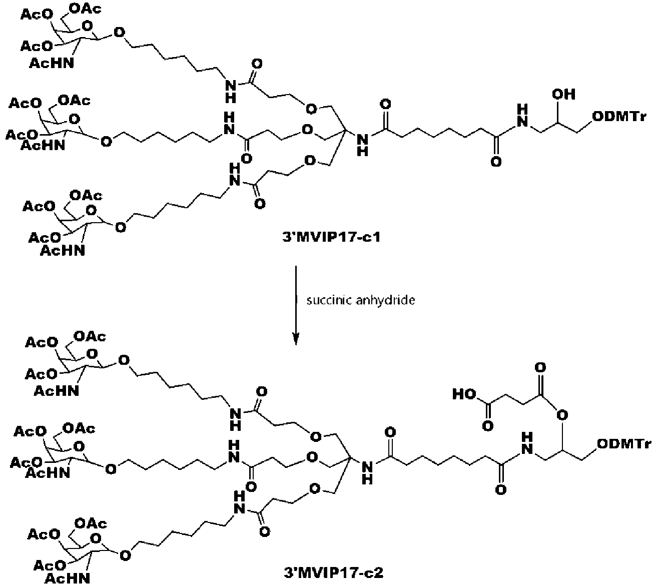

The synthetic procedure refers to the synthesis of 3'MVIP09-c2 in 3.1.1.7.

### 3.1.2.8 Synthesis of 3'MVIP17 Solid Support

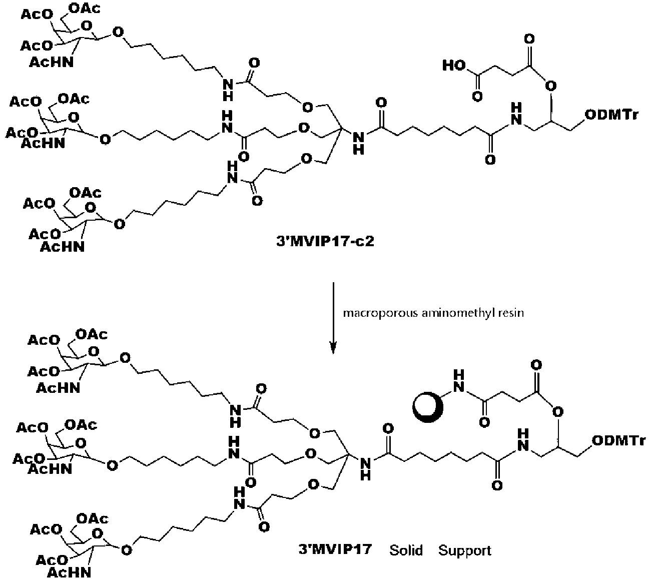

The synthetic procedure refers to the synthesis of 3'MVIP09 Solid Support in 3.1.1.8.

### 3.1.3 Synthesis of 3'MVIP01 Solid Support

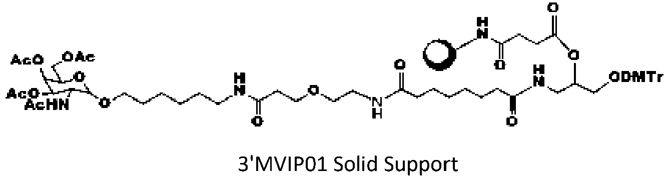

### Description of Synthesis process :

### 3.1.3.1 Synthesis of 3'MVIP01-c1

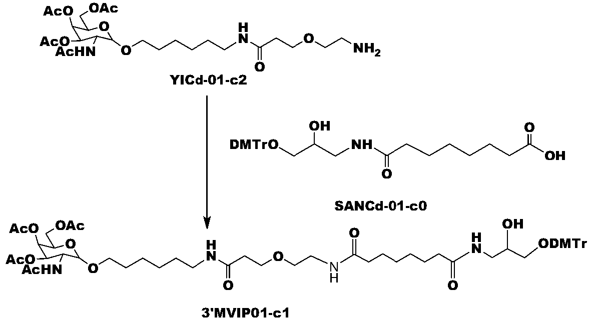

The synthetic procedure refers to the synthesis of 3'MVIP09-c1 in 3.1.1.6.

### 3.1.3.2 Synthesis of 3'MVIP01-c2

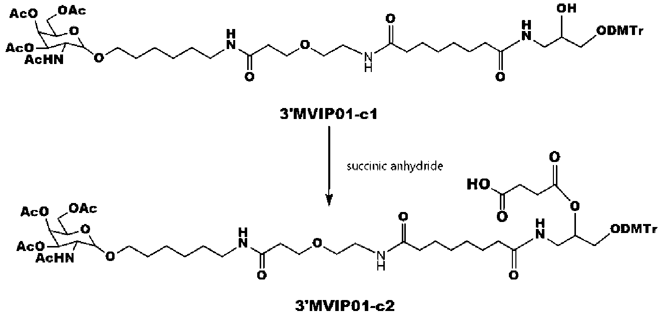

The synthetic procedure refers to the synthesis of 3'MVIP09-c2 in 3.1.1.7.

### 3.1.3.3 Synthesis of 3'MVIP01 Solid Support

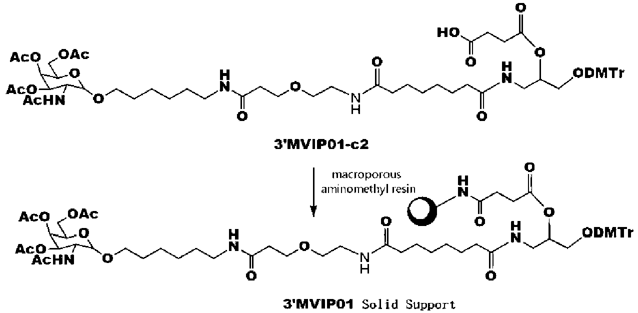

The synthetic procedure refers to the synthesis of 3'MVIP09 Solid Support in 3.1.1.8.

### 3.2 Synthesis of 5'MVIP Phosphoramidite Monomer

### 3.2.1 Synthesis of 5'MVIP09 Phosphoramidite Monomer

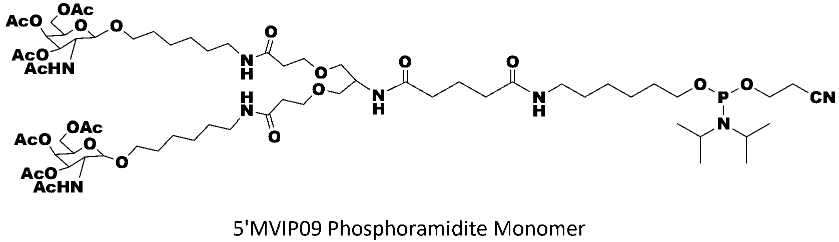

### 3.2.1.1 Synthesis of 5'MVIP09-ERCd-PFP-c1

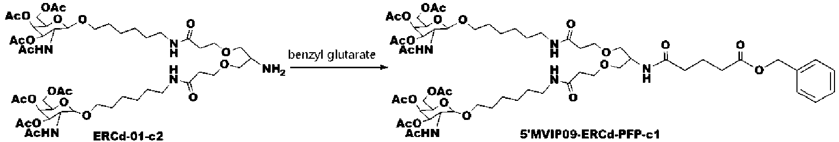

Weigh ERCd-01-c2 (2.18 g, 2.0 mmol) and dissolve in DMF (50 mL), add benzyl glutarate (0.53 g, 2.4 mmol), DIPEA (0.78 g) and TBTU (0.84 g), stir overnight at room temperature, quench with water (50 mL), extract with DCM (30 mL×3), wash with 10% citric acid (50 mL×3), saturated sodium bicarbonate (50 mL) and pyridine (100 mL), dry over anhydrous sodium sulfate, filter, rotary evaporate, and purify by column to obtain 5'MVIP09-ERCd-PFP-c1 (2.15 g).

### 3.2.1.2 Synthesis of 5'MVIP09-ERCd-PFP-c2

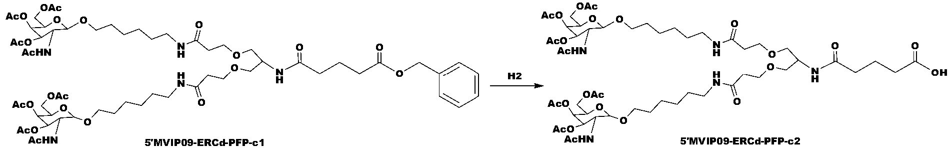

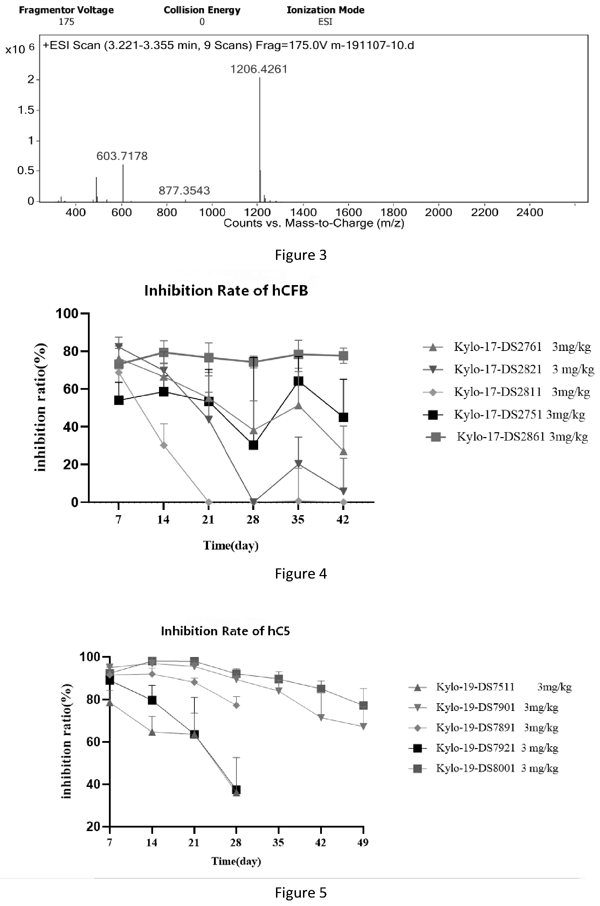

Weigh 5'MVIP09-ERCd-PFP-c1 (2.15 g, 1.66 mmol) and 10% palladium on carbon (0.21 g), add methanol (50 mL), stir at room temperature for hydrogenation overnight. After the reaction, filter off palladium on carbon through celite, and rotary evaporate to obtain crude 5'MVIP09-ERCd-PFP-c2 (1.9 g). Its high-resolution mass spectrum is shown in Figure 3.

### 3.2.1.3 Synthesis of 5'MVIP09-ERCd-PFP

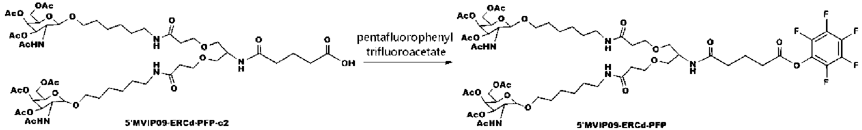

Weigh crude 5'MVIP09-ERCd-PFP-c2 (1.9 g, 1.58 mmol) and dissolve in DCM (60 mL), add DIPEA (1.33 g), cool, add pentafluorophenyl trifluoroacetate (2.21 g, 7.9 mmol), stir at room temperature for 2 h, rotary evaporate, redissolve in DCM (60 mL), wash with saturated sodium bicarbonate (30 mL×3), 10% citric acid (30 mL×1), saturated brine (50 mL×1), dry over anhydrous sodium sulfate, filter, rotary evaporate to obtain crude 5'MVIP09-ERCd-PFP (2.35 g), which is used directly in the next step without purification after pumping dry.

### 3.2.1.4 Synthesis of 5'MVIP09 Phosphoramidite Monomer-c1

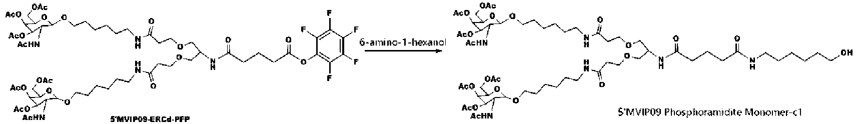

Dissolve crude 5'MVIP09-ERCd-PFP (2.35 g, 1.58 mmol) in DCM (60 mL), add DIPEA (0.82 g, 6.32 mmol) and 6-amino-1-hexanol (0.37 g, 3.16 mmol), stir at room temperature overnight. Add 10% citric acid (30 mL), extract with DCM (30 mL×3), wash with saturated brine (50 mL), dry over anhydrous sodium sulfate, filter, rotary evaporate, and purify by column to obtain 5'MVIP09 monomer-c1 (1.73 g).

### 3.2.1.5 Synthesis of 5'MVIP09 Phosphoramidite Monomer

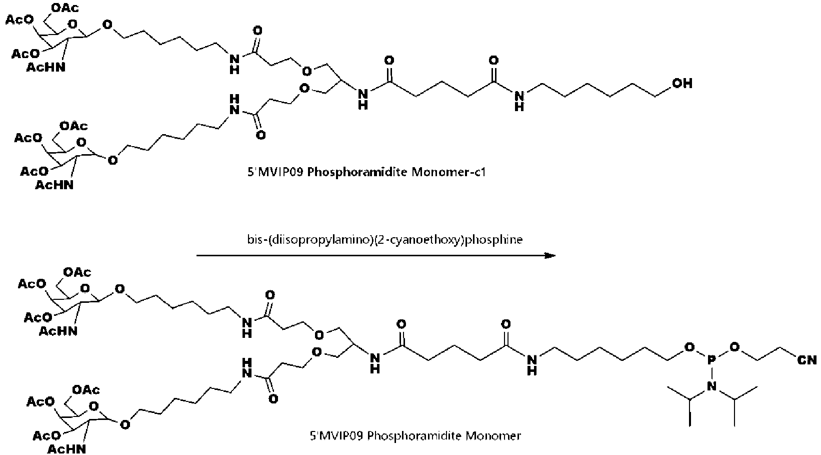

Weigh 5'MVIP09 Phosphoramidite Monomer-c1 (1.3 g, 1.0 mmol) and dissolve in acetonitrile (30 mL), add diisopropylamine triazole (0.22 g), add bis-(diisopropylamino)(2-cyanoethoxy)phosphine (0.36 g, 1.2 mmol) dropwise in ice bath, react at room temperature for 4 h, monitor by HPLC, concentrate and purify by column after qualified reaction to obtain 5'MVIP09 monomer (1.2 g).

### 3.2.2 Synthesis of 5'MVIP01 Phosphoramidite Monomer

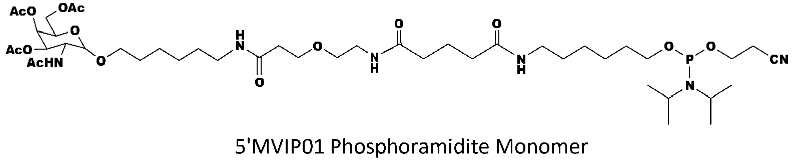

Weigh YICd-01-c2 (1.12 g, 2.0 mmol), and refer to 3.2.1.1.~3.2.1.5 for the remaining operations.

### Example 4 Synthesis of RNA Inhibitors Conjugated with Carriers

Synthesis description of antisense strand conjugated with carrier (3'MVIP09 conjugation): Purge the reagent bottle with argon for at least 2 min. Add phosphoramidite monomer or and acetonitrile to the reagent bottle in sequence, tighten the cap, shake until the solid is completely dissolved visually, then add 3A molecular sieve, and let stand for more than 8 h for later use. Purge the reagent bottle with argon for at least 2 min. Add xanthane hydride and dry pyridine to the reagent bottle in sequence, tighten the cap, shake until the solid is completely dissolved visually, and store temporarily for later use. Confirm the following operations are performed at room temperature of 20^{~}30°C: Weigh 3'MVIP Solid Support, add it to the reagent bottle, then add acetonitrile, shake to mix evenly, transfer to the synthesis column, and rinse the residual part in the reagent bottle into the synthesis column with acetonitrile. After rinsing, fill the synthesis column with acetonitrile and record the amount of acetonitrile used. Install and fix the synthesis column according to the instrument operation.

Connect the prepared monomer solution, CAP A, CAP B, oxidizing agent, sulfurizing reagent, activator, capping reagent and acetonitrile to the corresponding pipelines of AKTA PILOT100, ensuring that the pipelines are inserted into the bottom of the reagent bottles.

After setting the synthesis method and preparing all instrument operations, click run to start synthesis. Observe and record the peak area of each detritylation online. Supplement the deprotection reagent according to the actual usage during synthesis.

After synthesis, purge the synthesis column with argon for ≥2 h, and unload the synthesis column according to the operating procedures. Transfer the solid support in the synthesis column to a reaction flask, add methylamine aqueous solution and ammonia water, place the reaction flask in a shaker at 35°C for 2-3 hours. Filter the solution into a round-bottom flask, wash the residual solid with 50% ethanol aqueous solution, filter again and combine with the previous filtrate. Connect the round-bottom flask to a rotary evaporator, set the water temperature to 50°C and evaporate until no distillate is obtained.Add ethanol to the round-bottom flask, mix evenly, evaporate again until no distillate is obtained, and repeat the operation until white powder appears at the bottom of the flask. Prepare the obtained white powder into a solution, purify it with a reverse chromatography column, and sample to detect OD260 and purity. Aliquot the purified antisense strand solution into vials, lyophilize for later use, and store the product sealed in a -20°C refrigerator.

The synthesis operation of the sense strand conjugated with carrier (5'MVIP09 conjugation) is the same as that of the antisense strand, wherein the Solid Support for column packing is Universal carrier. Add DIPEA to the obtained intermediate to prepare a solution, add 5'MVIP phosphoramidite monomer, mix evenly, place the reaction flask in a shaker at 35°C for 2-3 hours.

### Description of annealing process for synthesis of RNA inhibitor:

Take the obtained sense strand and antisense strand, mix them in a 1:1 equimolar ratio in a reaction flask, heat in a water bath at 95°C for 5 minutes, turn off the water bath power supply, and let it cool naturally to below 40°C. Add 3M sodium acetate aqueous solution to the double-strand solution, mix evenly, then add an appropriate volume of anhydrous ethanol, mix evenly, and place the reaction solution in a -20°C refrigerator for 45 min. Pre-cool a refrigerated high-speed centrifuge at 4°C, put the double-strand solution into the centrifuge after reaching the temperature, and start the centrifuge. Take out the centrifuged double-strand solution, discard the supernatant, add ultrapure water to completely dissolve the solid, sample to detect OD260 and purity to obtain the RNA inhibitors in Table 14. Aliquot the purified finished product solution into vials, lyophilize for later use, and store the product sealed in a -20°C refrigerator.

Only the synthesis of RNA inhibitors conjugated with 5'MVIP09/3'MVIP09 is exemplified above. All RNA inhibitors described in the present invention but not listed are applicable to this rule.With reference to the method described in this example, those skilled in the art can easily synthesize the remaining RNA inhibitors involved in the present invention.

### Example 5 Screening Assay of CFB Inhibitors and C5 Inhibitors in PHH Cells

The RNA inhibitors used in this example are selected from Table 1-7, and the inhibitory effects of RNA inhibitors are verified by free uptake of PHH cells.

Verification method: On Day 0, plate PHH cells and transfect the compounds into the cells using Lipofectamine^{®} RNAiMAX. 48h after transfection, extract total RNA using Eastep^{®} Super Total RNA Extraction Kit (Promega-LS1040), synthesize cDNA using One-Step gDNA Removal, and perform qPCR detection using PerfectStart^{®} Green qPCR SuperMix(+Dye I).

Data analysis: The mRNA expression level of the target gene in the sample is calculated according to the Ct value of each sample using the ΔΔCt relative quantification method. The relative expression level of the target gene is expressed as 2-(-ΔΔCT). The calculation formula is as follows: ΔCT = average Ct value of target gene - average Ct value of internal reference gene; ΔΔCT = ΔCT (dosing group) - ΔCT (control group); relative mRNA expression level of target gene = 2-(-ΔΔCT); inhibition rate of target gene = (1 - value of sample / mean value of RNAiMAX Control) × 100%. The results are expressed as mean = SD of three replicate wells. The inhibitory effects of RNA inhibitors on complement system mRNA in PHH cells at concentrations of 500 nM, 100 nM, 10 nM, 1 nM, 0.02 nM, 10 nM, 1 nM and 0.1 nM were investigated. The test results of the inhibitory effects of CFB RNA inhibitors in PHH cells are shown in Table 16-1.

**Table 16-1 Inhibitory effects of CFB RNA inhibitors in PHH cells**

| **siRNA Inhibitor** | **1 nM Inhibition Rate (%)** | **0.02 nM Inhibition Rate (%)** | **1 nM-SD** | **0.02 nM-SD** |
|---|---|---|---|---|
| Kylo-17-DS2731 | 80.55 | 32.09 | 0.45 | 12.28 |
| Kylo-17-DS2741 | 23.50 | 13.28 | 5.02 | 6.44 |
| Kylo-17-DS2751 | 80.73 | 39.91 | 4.33 | 4.95 |
| Kylo-17-DS2761 | 86.84 | 68.37 | 1.69 | 4.39 |
| Kylo-17-DS8301 | 73.14 | 21.00 | 1.84 | 12.25 |
| Kylo-17-DS270 | 74.24 | 22.99 | 2.11 | 7.40 |
| Kylo-17-DS8311 | 69.82 | 30.79 | 3.79 | 9.16 |
| Kylo-17-DS8321 | 72.31 | 9.57 | 2.76 | 10.42 |
| Kylo-17-DS2771 | 71.42 | 5.02 | 3.78 | 3.75 |
| Kylo-17-DS2781 | 68.93 | 16.89 | 1.16 | 3.48 |
| Kylo-17-DS2791 | 62.41 | 32.98 | 3.86 | 7.34 |
| Kylo-17-DS2801 | 63.70 | 36.48 | 1.92 | 3.77 |
| Kylo-17-DS2811 | 79.32 | 49.09 | 0.77 | 5.24 |
| Kylo-17-DS2821 | 81.38 | 45.89 | 1.49 | 3.41 |
| Kylo-17-DS2831 | 75.76 | 29.47 | 1.74 | 8.73 |
| Kylo-17-DS2841 | 59.71 | 14.98 | 3.61 | 14.76 |
| Kylo-17-DS2851 | 83.41 | 33.63 | 0.99 | 10.84 |
| Kylo-17-DS2861 | 83.61 | 34.76 | 5.07 | 2.95 |
| Kylo-17-DS2871 | 80.08 | 24.80 | 1.45 | 7.02 |

### Example 6 In Vitro Assay of Modified Sequence RNA Inhibitors of CFB, C5 and C3 for Inhibiting Complement System Gene Expression

The RNA inhibitors used in this example are selected from Table 7.

Example 6-1 The RNA inhibitors were prepared by the method described in Example 4.

With reference to the test method of Example 2, the inhibitory effects of RNA inhibitors on CFB mRNA, C5 mRNA and C3 mRNA in Hep3B cells at concentrations of 1 nM and 0.01 nM were investigated.

The test results are shown in Tables 17-1 to 17-5.

**Table 17-1 Inhibitory effects of RNA inhibitors on C5 mRNA in Hep3B cells**

| **siRNA Inhibitor** | **Inhibitor %** | | **SD** | |
|---|---|---|---|---|
| | **1 nM** | **0.01 nM** | **1 nM** | **0.01 nM** |
| Kylo-19-DS7501 | 64.65 | 31.88 | 11.13 | 3.88 |
| Kylo-19-DS7511 | 84.74 | 77.50 | 2.11 | 3.20 |
| Kylo-19-DS7521 | 86.33 | 42.01 | 5.18 | 10.27 |
| Kylo-19-DS7531 | 79.64 | 37.62 | 4.28 | 8.53 |
| Kylo-19-DS7541 | 80.61 | 36.62 | 3.64 | 4.17 |

**Table 17-2 Inhibitory effects of RNA inhibitors on C5 mRNA in Hep3B cells**

| siRNA Inhibitor 1nM | **Inhibition** Rate (%) | SD |
|---|---|---|
| Kylo-19-DS8021 | 79.85 | 2.18 |
| Kylo-19-DS7921 | 88.89 | 1.51 |
| Kylo-19-DS8011 | 81.06 | 1.93 |
| Kylo-19-DS7891 | 89.21 | 1.06 |
| Kylo-19-DS7911 | 82.27 | 3.36 |
| Kylo-19-DS7951 | 85.29 | 4.38 |
| Kylo-19-DS7901 | 86.68 | 1.34 |
| Kylo-19-DS8001 | 91.33 | 2.63 |
| Kylo-19-DS7931 | 89.31 | 6.05 |
| Kylo-19-DS7961 | 83.08 | 8.65 |
| Kylo-19-DS7991 | 70.41 | 2.87 |
| Kylo-19-DS7971 | 79.58 | 1.85 |
| Kylo-19-DS7981 | 73.71 | 3.58 |
| Kylo-19-DS7941 | 72.70 | 4.20 |
| Kylo-19-DS8091 | 80.53 | 3.49 |
| Kylo-19-DS8081 | 83.25 | 1.44 |
| Kylo-19-DS8061 | 83.27 | 1.88 |
| Kylo-19-DS8041 | 85.10 | 2.17 |
| Kylo-19-DS8101 | 91.38 | 1.78 |
| Kylo-19-DS8071 | 75.20 | 2.52 |
| Kylo-19-DS8051 | 91.16 | 1.37 |
| Kylo-19-DS8031 | 81.29 | 2.06 |

**Table 17-3 Inhibitory effects of RNA inhibitors on C5 mRNA in Hep3B cells**

| **siRNA Inhibitor** | **1nM Inhibition Rate (%)** | **0.01 nM Inhibition Rate (%)** | **1 nM-SD** | **0.01 nM-SD** |
|---|---|---|---|---|
| Kylo-19-DS7511 | 93.13 | 84.01 | 0.87 | 1.74 |
| Kylo-19-DS7871 | 89.67 | 78.18 | 0.50 | 0.21 |
| Kylo-19-DS7881 | 95.77 | 80.46 | 0.16 | 0.80 |
| Kylo-19-DS8001 | 94.71 | 79.01 | 0.01 | 4.87 |
| Kylo-19-DS7891 | 87.56 | 72.30 | 1.29 | 3.34 |
| Kylo-19-DS7921 | 92.21 | 76.32 | 0.66 | 1.62 |
| Kylo-19-DS7901 | 87.03 | 76.56 | 1.30 | 1.94 |

**Table 17-4 Inhibitory effects of RNA inhibitors on C3 mRNA in Hep3B cells in vitro**

| siRNA sample | 0.01 nM Inhibition Rate % | 0.001 nM Inhibition Rate % | 0.01 nM-SD | 0.001 nM-SD |
|---|---|---|---|---|
| Kylo-27-DS8201 | 76.60 | 43.85 | 3.53 | 6.66 |
| Kylo-27-DS8771 | 75.81 | 38.90 | 4.41 | 5.26 |
| Kylo-27-DS8141 | 71.70 | 37.92 | 2.73 | 3.18 |
| Kylo-27-DS8711 | 36.85 | 28.42 | 11.42 | 9.78 |
| Kylo-27-DS8661 | 13.01 | 24.94 | 9.46 | 4.98 |
| Kylo-27-DS8611 | 49.07 | 24.88 | 2.21 | 1.19 |
| Kylo-27-DS8521 | 34.43 | 17.27 | 7.27 | 6.86 |

### Example 6-2 Assay for Determination of EC50 of RNA Inhibitors for Inhibiting C3 Gene In Vitro

The RNA inhibitors used in this example are selected from Table 7. The inhibitory effects of RNA inhibitors Kylo-27-DS8201, Kylo-27-DS8771 and Kylo-27-DS8141 on C3 mRNA in Hep3B cells at different concentrations were investigated. Three inhibition rate data were tested for each concentration, and the average value was calculated. The inhibition rate-concentration curve was plotted based on the experimental data, and the corresponding EC50, EC75, EC85 and EC95 values were calculated. The test results obtained are shown in Table 18-1 and Table 18-2.

**Table 18-1 Inhibitory effects of RNA inhibitors on C3 mRNA in Hep3B cells**

| Concentration(nM) | Kylo-27-DS8201 | Kylo-27-DS8771 | Kylo-27-DS8141 |
|---|---|---|---|
| 1.0000 | 92.74 | 91.69 | 90.12 |
| 0.1000 | 90.05 | 91.11 | 89.22 |
| 0.0333 | 88.72 | 85.45 | 85.93 |
| 0.0111 | 82.23 | 82.03 | 80.69 |
| 0.0037 | 70.54 | 66.22 | 69.53 |
| 0.0012 | 49.22 | 43.46 | 49.72 |
| 0.0004 | 33.50 | 35.52 | 34.93 |
| 0.0001 | 31.58 | 22.54 | 19.58 |

**Table 18-2 EC values of RNA inhibitors for C3 mRNA inhibition in Hep3B cells**

| siRNA Sample | Kylo-27-DS8201 | Kylo-27-DS8771 | Kylo-27-DS8141 |
|---|---|---|---|
| Abs EC50(nM) | 0.0013 | 0.0015 | 0.0011 |
| Abs EC75(nM) | 0.0052 | 0.0070 | 0.0062 |
| AbsEC85(nM) | 0.0134 | 0.0204 | 0.0246 |
| Abs EC95(nM) | >1 | >1 | >1 |

### Example 7 Evaluation of In Vivo Activity of RNA Inhibitors in Mice

The RNA inhibitors used in this example are selected from Table 7, and the inhibitory effects are verified in humanized CFB mice.

### Example 7-1 Inhibitory effect of Kylo-17 inhibitor on protein level in humanized CFB mice

After adaptive feeding of test animals, they were randomly divided into saline group, Kylo-17-DS2761, Kylo-17-DS2821, Kylo-17-DS2811, Kylo-17-DS2751 and Kylo-17-DS2861 groups according to the hCFB protein content in plasma on Day -1, with 5 mice in each group. A single subcutaneous injection was administered at a dose of 3 mg/kg, a volume of 5 mL/kg, and a concentration of 0.6 mg/mL. The day of administration was recorded as Day 0. Animals were individually identified by ear tags, and cages were identified by hanging cage cards.

Detection time: About 200 µL of blood was collected from the inner canthus on Day -1 before administration, and Day 7, 14, 21, 28, 35 and 42 after administration. Whole blood samples were incubated in a 37°C water bath for 1 h before centrifugation, centrifuged at 3000 r for 10 min, and the supernatant was collected as fresh serum samples for hCFB protein detection.

Detection method of hCFB protein level: detected by ELISA kit (single well); serum samples were freshly detected after centrifugation of whole blood samples.

Test data are expressed as mean ± standard deviation (Mean±SD), converted to the effect of inhibitors on hCFB protein inhibition rate in mice (%, Mean±SD). T-test was used for statistical analysis, P<0.05 indicated statistical significance. The experimental results are shown in Table 19 and Figure 4.

**Table 19 Effect of hCFB administration on the inhibition rate of hCFB protein in mouse serum**

| Inhibito r | D7 Inhibition Rate (%) | D14 Inhibition Rate (%) | D21 Inhibition Rate (%) | D28 Inhibition Rate (%) | D35 Inhibition Rate (%) | D42 Inhibition Rate (%) |
|---|---|---|---|---|---|---|
| Kylo-17-DS2761 | 76.1±5.41 *** | 66.5±7.41* ** | 55.2±11.79 ** | 38.2±15.54 * | 51.4±17.82 * | 27±13.4 |
| Kylo-17-DS2821 | 82.2±5.24 *** | 69.7±9.99* ** | 43.7±14.49 ** | -2.1±38.09 | 20.2±14.34 | 5.7±17.58 |
| Kylo-17-DS2811 | 68.7±1.81 *** | 30.2±11.37 * | -15.7±28.14 | -26.6±11.4 | 0.7±17.29 | -20.6±38.5 4 |
| Kylo-17-DS2751 | 54±9.7** | 58.5±8.42* ** | 53.4±16.89 ** | 30.2±46.55 | 64.2±13.04 ** | 45±20.14 |
| Kylo-17-DS2861 | 73.1±9.74 *** | 79.4±6.12* ** | 76.6±7.81* ** | 74.3±3.18* * | 78.4±7.48* ** | 77.6±4.08* ** |

| | | | | | | |
|---|---|---|---|---|---|---|
| Note: Compared with the saline group, "*" indicates P ≤ 0.05, "**" indicates P ≤ 0.01, and "***" indicates P ≤ 0.001. | | | | | | |

### Example 7-2 Inhibitory Effect of Kylo-19 Inhibitor on Protein Level in Humanized C5 Mice

After adaptive feeding, animals were randomized into saline group, Kylo-19-DS7511, Kylo-19-DS7901, Kylo-19-DS7891, Kylo-19-DS7921 and Kylo-19-DS8001 groups (n=5 per group) based on the hC5 protein levels in serum on Day-3, with five mice in each group. The drug was administered via single subcutaneous injection at a dose of 3 mg/kg, a volume of 5 mL/kg, and a concentration of 0.6 mg/mL. The administration day was designated as Day 0. Animal identification was performed using ear tags for individual marking, while the housing cages were identified through hanging tags.

Detection time: Approximately 200 µL of blood was collected from the inner canthus on Day -3 before administration, and on Day 7, Day 14, Day 21, Day 28, Day 35, Day 42 and Day 49 after administration.Whole blood samples were incubated in a water bath at 37°C for 1 h before centrifugation, centrifuged at 3000 r for 10 minutes, and the supernatant was collected to obtain fresh serum samples for hC5 protein detection.

The experimental data are expressed as mean ± standard deviation (Mean ± SD), and converted into the effect of the inhibitor on the inhibition rate of hC5 protein in mice (%, Mean ± SD).Data statistical analysis was performed by T-test, and P<0.05 was considered statistically significant.The experimental results are shown in Table 20 and Figure 5:

**Table 20 Effect of Administration on Inhibition Rate of hC5 Protein in Serum of hC5 Mice (%, Mean ± SD)**

| Inhibitor | D7 Inhibitio n Rate (%) | D14 Inhibitio n Rate (%) | D21 Inhibition Rate (%) | D28 Inhibition Rate (%) | D35 Inhibitio n Rate (%) | D42 Inhibition Rate (%) | D49 Inhibitio n Rate (%) |
|---|---|---|---|---|---|---|---|
| Kylo-19-D S7511 | 68.3±8.4 3*** | 59.5±8.4 9*** | 63.3±10. 02*** | 18±21.07 ** | N/A | N/A | N/A |
| Kylo-19-D S7901 | 92.5±2.5 8*** | 96.4±3.3 6*** | 95.5±2.8 9*** | 86.5±3.5 5*** | 76.2±7.8 3*** | 57.1±18. 34*** | 54±13.0 7*** |
| Kylo-19-D S7891 | 87.3±2.4 1*** | 90.7±3.2 *** | 88.1±1.7 9*** | 70.9±5.3 1*** | N/A | N/A | N/A |
| Kylo-19-D | 83.7±3.3 | 76.7±8.0 | 63.4±17. | 19.8±19. | N/A | N/A | N/A |
| Kylo-19-D | 88.5±4.3 | 97.8±1.6 | 97.9±1.8 | 89.8±3.0 | 84.5±5.1 | 77.6±5.6 | 68±11.2 |

| | | | | | | | |
|---|---|---|---|---|---|---|---|
| Note: Compared with the normal control group, "*" indicates P ≤ 0.05, "**" indicates P ≤ 0.01, and "***" indicates P ≤ 0.001. | | | | | | | |

### Example 7-3 Inhibitory Effect of Kylo-19 Inhibitor on Protein Level in Humanized C5 Mice

After adaptive feeding of the test animals, the mice were randomly divided into saline group, Kylo-19-DS7871 group and Kylo-19-DS7881 group according to the hC5 protein content in serum on Day -3, with 5 mice in each group.The drug was administered by a single subcutaneous injection at a dose of 3 mg/kg, a volume of 5 mL/kg, and a concentration of 0.6 mg/mL respectively. The day of administration was recorded as Day 0.Individual animals were identified by ear tags, and the feeding cages were identified by hanging cage cards.

Detection time: Approximately 200 µL of blood was collected from the inner canthus on Day -3 before administration, and on Day 7, Day 14, Day 21, Day 28, Day 35, Day 42 and Day 49 after administration.Whole blood samples were incubated in a water bath at 37°C for 1 h before centrifugation, centrifuged at 3000 r for 10 minutes, and the supernatant was collected to obtain fresh serum samples for hC5 protein detection.

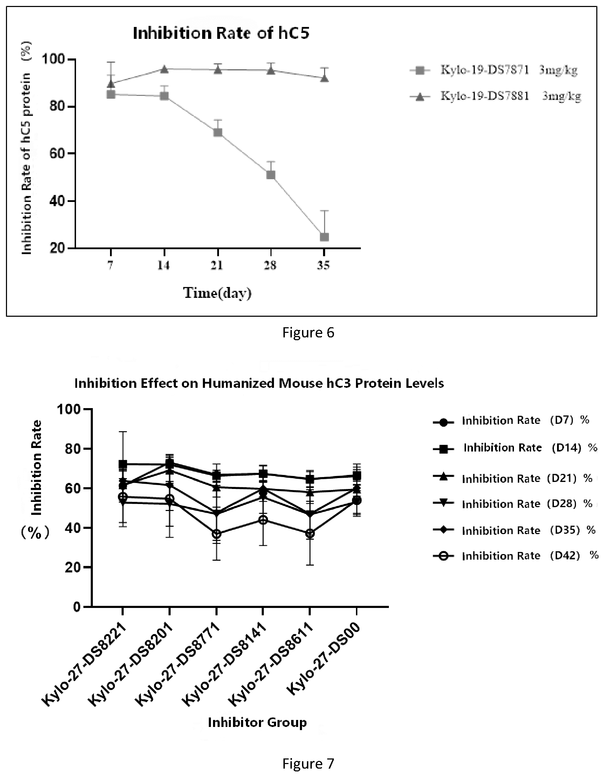

The experimental data are expressed as mean ± standard deviation (Mean ± SD), and converted into the effect of the inhibitor on the inhibition rate of hC5 protein in mice (%, Mean ± SD).Data statistical analysis was performed by t-test, and P<0.05 was considered statistically significant.The experimental results are shown in Table 21 and Figure 6:

**Table 21 Effect of Administration on Inhibition Rate of hC5 Protein in Serum of hC5 Mice**

| (%, Mean ± SD) | | | | | |
|---|---|---|---|---|---|
| Inhibitor | D7 Inhibition Rate (%) | D14 Inhibition Rate (%) | D21 Inhibition Rate (%) | D28 Inhibition Rate (%) | D35 Inhibition Rate (%) |
| Kylo-19-DS787 1 | 85.1±8.27** * | 84.4±4.27** * | 69±5.34*** | 51.2±5.55** | 24.9±11.22* |
| Kylo-19-DS788 | 89.6±9.27** | 96±0.99*** | 95.7±2.39** | 95.4±3.07** | 92.1±4.38** |

| | | | | | |
|---|---|---|---|---|---|
| Note: Compared with the normal control group, "*" indicates P ≤0.05, "**" indicates P ≤0.01, "***" indicates P ≤0.001 | | | | | |

Inhibitors with significant inhibitory effects were selected and optimized for cynomolgus monkey experiments.

### Example 8 Evaluation of In Vivo Activity of RNA Inhibitors in Cynomolgus Monkeys

### Example 8-1 Verification Experiment of In Vivo Pharmacodynamic Activity of CFB Inhibitor in NHP Cynomolgus Monkeys

The RNA inhibitors in this example are selected from Table 7, and the inhibitory effect on the expression level of serum CFB protein and the evaluation of the effect on CFB mRNA level of the positive control group and Kylo-17-DS2911 inhibitor in NHP cynomolgus monkeys were verified in cynomolgus monkeys. The inhibitor selected for the positive control group is: Sense strand: AsAsGAGAfAGfUfCfGUUUCAUUCAU-L96, Antisense strand: AsfUsGAAfUGfAfAACGAfCUfUCUCUUsGsU.

After adaptive feeding of healthy cynomolgus monkeys, blood was collected on Day -1 to detect the content of CFB protein in serum. The animals were grouped according to the content of CFB protein into normal control group, positive control group and Kylo-17-DS2911 group. The administration dose was 6 mg/kg, with 2 animals (1 male and 1 female) in the normal control group, and 3 animals (2 males and 1 female) in each of the positive control group and G3 group. Single subcutaneous injection (injection at the back of the neck) was administered at a volume of 1 mL/kg, and the day of administration was recorded as Day 0. Blood was collected intravenously once a week on Day -2 before administration, and on Day 7, Day 14, Day 21, Day 28 after administration until the end of the test (Day 91) to detect the level of CFB protein in serum of NHP cynomolgus monkeys.

Detection Method of CFB Protein Level: Detected by ELISA kit (single well); serum samples were freshly tested after preparation from centrifuged whole blood samples. Detection Time: Blood was collected on Day -1 before administration, and on Day 7, Day 14, Day 21, Day 28 after administration, and intravenously once a week until the end of the test. About 2 mL of blood was collected each time. The whole blood sample was allowed to stand at room temperature for 1 h before centrifugation, centrifuged at 3000 r for 10 minutes, and the supernatant was collected to obtain fresh serum samples for detection of CFB protein.

Detection Method of CFB mRNA Level in Liver Tissue: Liver biopsy of cynomolgus monkeys was performed, and the mRNA level of the target in liver was determined by probe-based qPCR.

The experimental data are expressed as Mean ± Standard Deviation (Mean±SD), and data plotting and analysis were performed using GraphPad Prism 8.3 analysis software. Data statistical analysis was performed by T-test, and P<0.05 indicates statistical significance.

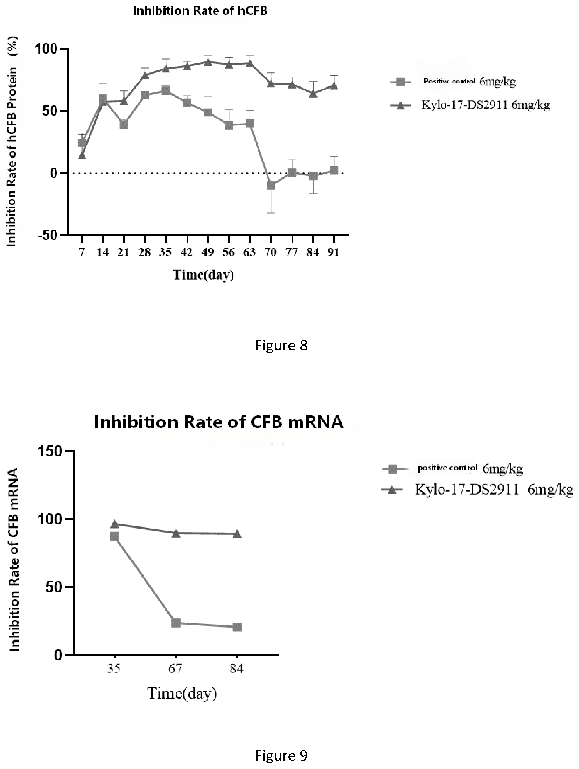

The experimental results of CFB protein level are shown in Table 23 and Figure 8; the experimental results of CFB mRNA level are shown in Table 24 and Figure 9.

**Table 23 Effect of CFB RNA Inhibitor on CFB Protein Inhibition Rate in NHP Cynomolgus Monkeys (%, Mean±SD)**

| Group | D7 | D14 | D21 | D28 | D35 | D42 |
|---|---|---|---|---|---|---|
| Positiv e contro l | 24.5±7.9 1* | 59.8±3.22* ** | 39±4.01*** | 62.68±4.17* ** | 66.17±4.36 ** | 56.61±5.71* * |
| Kylo-1 7-DS2 | 14.7±16. 59 | 57.4±14.73 ** | 58.1±8.03* ** | 78.8±6.06** * | 84.21±7.92 ** | 86.31±3.67* ** |

**Continuation of Table 23**

| Group | D49 | D56 | D63 | D70 | D77 | D84 | D91 |
|---|---|---|---|---|---|---|---|
| Positive control | 48.84±1 3.02* | 38.65 ±12.5 6 | 39.84±10. 83* | -9.75±22. 16 | 0.55±10.86 | -2.22±14. 04 | 2.34±11 .39 |
| Kylo-17 | 89.52±5. | 87.64 | 88.48±6.1 | 72.18±8.4 | 71.38±5.83 | 64.23±9. | 70.51±8 |

**Table 24 Effect of CFB RNA Inhibitor on CFB mRNA Level in Liver Tissue of Cynomolgus Monkeys**

| Group | Relative Expression Level of CFB mRNA | | | Inhibition Rate of CFB mRNA | | |
|---|---|---|---|---|---|---|
| | D35 | D67 | D84 | D35 | D67 | D84 |
| Normal control group | 1 | 1.08±0.58 | 1.07±0.56 | - | - | - |
| Positive control | 0.127 | 0.765±0.12 | 0.794±0.04 | 87.3 | 23.46±11.7 1 | 20.57±4.2 |
| Kylo-17-DS2 | 0.035 | 0.103±0.03* | 0.108±0.02* | 96.5 | 89.66±2.75 | 89.17±1.6 |

It can be seen from the experimental results in Table 23 that:In terms of CFB protein level: the inhibition rate increased rapidly on Day 28 after administration, and the CFB protein level could be continuously inhibited from Day 28 to Day 63 (inhibition rates ranged from 78.8% to 89.52%, P<0.05 or P<0.01 or P<0.001), and the inhibition rates ranged from 64.23% to 72.18% from Day 70 to Day 91. The inhibition rate of Kylo-17-DS2911 surpassed that of the positive control from Day 21, showing a significant inhibitory effect, and the inhibitory effect was stable as shown by the inhibition rates from Day 21 to Day 91. It can be seen from the comparison of inhibition rates between Kylo-17-DS2911 and the positive control from Day 70 to Day 91 that the inhibitor of the present invention has a more stable inhibitory effect.

It can be seen from the experimental results in Table 24 that:In terms of CFB mRNA level in liver tissue: the CFB mRNA level in liver tissue of Kylo-17-DS2911 decreased significantly on Day 35 after administration, with an inhibition rate of 96.5%, and it could still stably and efficiently inhibit CFB mRNA expression on Day 67 and Day 84, with inhibition rates of 89.66% and 89.17% respectively. Compared with the positive control, it has a more significant CFB mRNA inhibition level, and the inhibition is more stable and durable.

### Example 8-2 Effect of C5 Inhibitor on Serum C5 Protein Content and CH50 Activity in NHP Cynomolgus Monkeys

The RNA inhibitors in this example are selected from Table 7, and the effects of the positive control group (Sense strand: AsAsfGCfAAfGAfUfAfUUfUUUfAUfAAUA-L96, Antisense strand: UsfAsfUUfAUAfAAfAAUAfUCfUUfGCUUsUsUdTdT) and Kylo-19-DS7881 inhibitor on serum C5 protein content and CH50 activity in NHP cynomolgus monkeys were verified in cynomolgus monkeys.

After adaptive feeding of healthy cynomolgus monkeys, blood was collected on Day -2 to detect the content of C5 protein in serum. The animals were grouped according to the content of C5 protein into normal control group G1, positive control group G2 and Kylo-19-DS7881 group G3. The administration dose was 6 mg/kg, with 2 animals (1 male and 1 female) in the normal control group, and 3 animals (2 females and 1 male) in each of G2 and G3 groups. Single subcutaneous injection (injection at the back of the neck) was administered at a volume of 1 mL/kg, and the day of administration was recorded as Day 0. Blood was collected intravenously once a week on Day -2 before administration, and on Day 7, Day 14, Day 21, Day 28 after administration until the end of the test (Day 91) to detect the level of C5 protein in serum of NHP cynomolgus monkeys.

Blood was collected intravenously once a week on Day -1 before administration, and on Day 7, Day 14, Day 21, Day 28 after administration until the end of the test. About 2 mL of blood was collected each time. The whole blood sample was allowed to stand at room temperature for 1 h before centrifugation, centrifuged at 3000 r for 10 minutes, and the supernatant was collected to obtain fresh serum samples for detection of C5 protein. Detection Method of C5 Protein Level: Detected by ELISA kit (single well); serum samples were freshly tested after preparation from centrifuged whole blood samples.

The CH50 activity in serum samples was detected on Day -2 before administration, and on Day 14, Day 28, Day 42, Day 49, Day 56, Day 63, Day 70, Day 77 after administration. Detection Method of CH50 Activity: Detected by ELISA kit (single well); serum samples were obtained after centrifugation of whole blood samples.

The experimental data are expressed as Mean ± Standard Deviation (Mean±SD), and data plotting and analysis were performed using GraphPad Prism 8.3 analysis software. Data statistical analysis was performed by t-test, and P<0.05 indicates statistical significance.

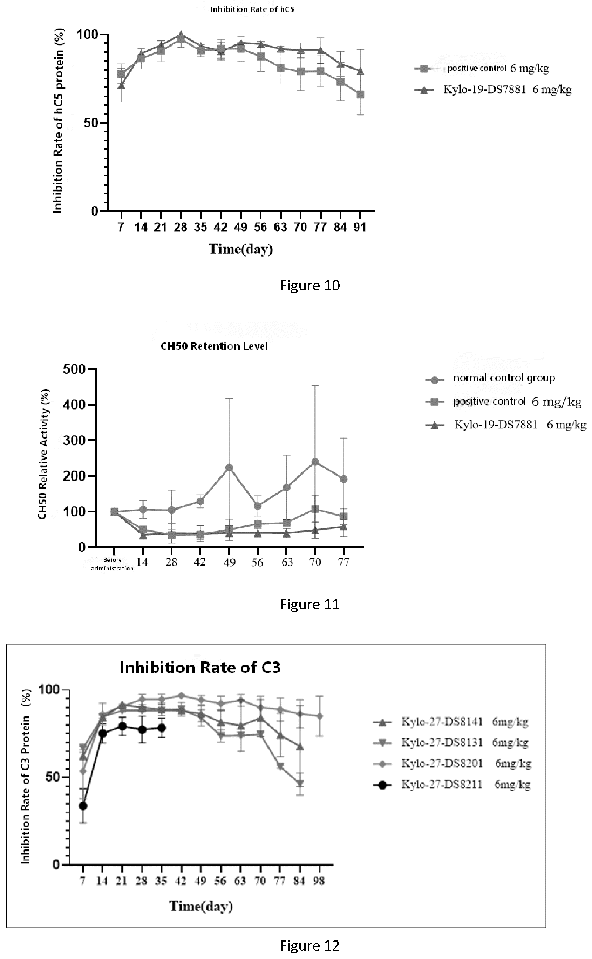

The results of C5 protein inhibition rate are shown in Table 24 and Figure 10; the experimental results of CH50 activity level are shown in Table 25 and Figure 11:

**Table 24 Effect of C5 RNA Inhibitor on C5 Protein Inhibition Rate in NHP Cynomolgus Monkeys (%, Mean±SD)**

| Group | D7 | D14 | D21 | D28 | D35 | D42 |
|---|---|---|---|---|---|---|
| Positiv e contro I | 77.8±5.62* ** | 86.4±5.81* ** | 90.7±6.16* ** | 97.4±4.57* ** | 90.8±3.41* ** | 91.9±5.28* ** |
| Kylo-1 | 71.4±9.49* | 89.2±3.08* | 94±2.5*** | 100±0*** | 93.5±1.22* | 90.5±4.89* |

| | | | | | | |
|---|---|---|---|---|---|---|
| Note: Compared with the normal control group, "*" indicates P ≤0.05, "**" indicates P ≤0.01, "***" indicates P ≤0.001 | | | | | | |

### Continuation of Table 24

| Group | D49 | D56 | D63 | D70 | D77 | D84 | D91 |
|---|---|---|---|---|---|---|---|
| Positive control | 91.9±7 .05** | 87.5±8. 26** | 81.2±9.2 1** | 79±10.6 6* | 79.2±8. 82** | 73.4±10 .75** | 66.2±1 1.67* |
| Kylo-19-D | 95.3±0 | 94.5±1. | 91.9±1.5 | 91±4.24 | 91±7.2* | 83.4±7. | 79.4±1 |

| | | | | | | | |
|---|---|---|---|---|---|---|---|
| Note: Compared with the normal control group, "*" indicates P ≤0.05, "**" indicates P ≤0.01, "***" indicates P ≤0.001 | | | | | | | |

**Table 25 Effect of C5 RNA Inhibitor on CH50 Activity Retention Level in NHP Cynomolgus Monkeys (Mean±SD)**

| Group | D14 | D28 | D42 | D49 |
|---|---|---|---|---|
| Normal saline | 107±25.11 | 105.4±55.79 | 129.9±18.73 | 224±194.81 |
| Positive control | 50.2±1.65* | 34.7±6.23 | 35.5±13.16** | 50.1±29.22 |
| Kylo-19-D | 35±10.57* | 39.8±27.86 | 38.8±22.23* | 40.5±20.84 |

### Continuation of Table 25

| Group | D56 | D63 | D70 | D77 |
|---|---|---|---|---|
| Normal saline | 116.8±28.4 | 168.1±90.22 | 240.2±214.92 | 191.9±114.58 |
| Positive control | 66.4±13.79 | 69.7±5.82 | 108.2±37.45 | 87±22.28 |
| Kylo-19-DS | 40.8±13.58* | 40.3±12.47 | 48.7±23.57 | 58.4±27.43 |

It can be seen from Table 24 and Figure 10 that the serum C5 protein level of Kylo-19-DS7881 of the present invention decreased significantly on Day 7 after administration, with an inhibition rate of 71.4%, and further decreased on Day 14. The expression of serum C5 protein level was continuously and stably inhibited from Day 14 to Day 77 (inhibition rates ranged from 89.2% to 100%, P<0.05 or P<0.01 or P<0.001). The protein level rebounded slightly on Day 84 after administration, and the inhibition rates on Day 84 and Day 91 were 83.4% and 79.4% respectively. Compared with the positive control, its inhibitory ability was significantly better after Day 56, and it was more stable and had a longer duration of inhibitory effect than the positive control.

It can be seen from Table 25 and Figure 11 that the serum CH50 activity of Kylo-19-DS7881 of the present invention decreased significantly on Day 14 after administration, with a CH50 activity retention level of 35%. The serum CH50 activity was continuously and stably reduced from Day 28 to Day 63 (retention levels ranged from 38.8% to 40.3%, P<0.05). The CH50 activity rebounded slightly on Day 70 after administration, and the CH50 activity retention levels on Day 70 and Day 77 were 48.7% and 58.4% respectively. Compared with the positive control, it has a better effect on reducing CH50 activity and a longer duration of the reducing effect.

### Example 8-3 Effect of C3 Inhibitor on Serum C3 Protein Content in NHP Cynomolgus Monkeys

The RNA inhibitors in this example are selected from Table 7, and the inhibitory effect of Kylo-27-DS8141 and Kylo-27-DS8201 inhibitors on the expression level of serum C3 protein in NHP cynomolgus monkeys was evaluated in cynomolgus monkeys.

After adaptive feeding of healthy cynomolgus monkeys, blood was collected on Day -1 to detect the content of C3 protein in serum. The animals were grouped according to the content of C3 protein into normal control group, Kylo-27-DS8141 group, Kylo-27-DS8131 group, Kylo-27-DS8201 group and Kylo-27-DS8211 group. The administration dose was 6 mg/kg, with 3 animals (2 males and 1 female) in each group. Single subcutaneous injection (injection at the back of the neck) was administered at a volume of 1 mL/kg, and the day of administration was recorded as Day 0.

Blood was collected intravenously once a week on Day -1 before administration, and on Day 7, Day 14, Day 21, Day 28, Day 35, Day 42 after administration until the end of the test. About 2 mL of blood was collected each time. The whole blood sample was allowed to stand at room temperature for 1 h before centrifugation, centrifuged at 3000 r for 10 minutes, and the supernatant was collected to obtain fresh serum samples for detection of C3 protein.

Detection Method of C3 Protein Level: Detected by ELISA kit (single well); serum samples were freshly tested after preparation from centrifuged whole blood samples.

The experimental data are expressed as Mean ± Standard Deviation (Mean±SD), and data plotting and analysis were performed using GraphPad Prism 8.3 analysis software. Data statistical analysis was performed by t-test, and P<0.05 indicates statistical significance. The experimental results are shown in Table 26 and Figure 12.

**Table 26 Effect of C3 RNA Inhibitor on C3 Protein Inhibition Rate in NHP Cynomolgus Monkeys (%, Mean±SD)**

| Group | D7 | D14 | D21 | D28 | D35 | D42 |
|---|---|---|---|---|---|---|
| Kylo-2 7-DS81 41 | 62.3±2.18* * | 84.3±0.44** * | 91.6±0.34* | 90±0.52* | 88.9±3.65* * | 88.2±0.95* * |
| Kylo-2 7-DS81 31 | 66.8±0.91* * | 84.7±0.92** * | 88.4±0.71* | 88.4±0.71 * | 88.3±0.3** | 89±3.87** |
| Kylo-2 | 53.6±15.5 | 86.2±6.3** | 90.7±0.63* | 94.7±2.88 | 94.7±2.94* | 96.8±0.37* |
| Kylo-2 | 33.9±9.76* | 75.2±5.51** | 79.3±5.15* | 77.4±7.6* | 78.4±5.5* | N/A |

### Continuation of Table 26

| Group | D49 | D56 | D63 | D70 | D77 | D84 | D98 |
|---|---|---|---|---|---|---|---|
| Kylo-2 7-DS8 141 | 86.5±4.55 ** | 81.6±7.1 8* | 79.6±14.6 1* | 84±10.58 * | 74.3±12.4 4* | 67.9±23. 19 | N/A |
| Kylo-2 7-DS8 131 | 84.1±4.61 ** | 73.8±3.5 1* | 74±0.34* * | 74.6±1.07 ** | 56.2±0.77 ** | 46.3±6.3 5 | N/A |
| Kylo-2 | 94.3±2.5* | 92.3±4.1 | 94.1±3.22 | 90±6.32* | 88.9±6.66 | 86.4±8.0 | 85.1±11.3 |
| Kylo-2 | N/A | N/A | N/A | N/A | N/A | N/A | N/A |

| | | | | | | | |
|---|---|---|---|---|---|---|---|
| Note: Compared with the normal control group, "*" indicates P ≤0.05, "**" indicates P ≤0.01, "***" indicates P ≤0.001 | | | | | | | |

It can be seen from Table 26 and Figure 12 that Kylo-27-DS8141 and Kylo-27-DS8201 have significant and durable C3 protein inhibition rates. Kylo-27-DS8141 continuously and stably inhibits the expression of serum C3 protein level from Day 14 to Day 70 (inhibition rates ranged from 79.6% to 91.6%, P<0.05 or P<0.01), and the inhibition rates can still be maintained at 74.3% and 67.9% on Day 77 and Day 84 with good durability. Kylo-27-DS8201 continuously and stably inhibits the expression of serum C3 protein level from Day 14 to Day 98, with an inhibition rate of 96.8% on Day 42 and still maintaining 85.1% on Day 98 with good durability.

## Claims

1. An RNA inhibitor inhibiting the gene expression of the complement system or the pharmaceutically acceptable salt thereof, **characterized in that** the RNA inhibitor is formed by base pairing of a sense strand and an antisense strand with a chain length of 15-30, the chain length is preferably 19-23, wherein the antisense strand comprises a region complementary to the mRNA encoding the complement system, and wherein the complementary region comprises at least 15 consecutive nucleotides differing by 0, 1, 2 or 3 nucleotides from any antisense strand in any one of Table 1-1, Table 1-2 or Table 1-3.

2. The RNA inhibitor or the pharmaceutically acceptable salt thereof according to Claim 1, **characterized in that** the RNA inhibitor is an RNA inhibitor inhibiting the expression of the CFB gene, wherein the antisense strand comprises a region complementary to a target sequence, and the target sequence is 5' gucuagucaacuuaauugaga 3' SEQ ID NO: 25, starting at position 1157 of NM_001710.5, and there is at least 85% base complementarity between the sense strand and the antisense strand.

3. The RNA inhibitor or the pharmaceutically acceptable salt thereof according to Claim 1, **characterized in that** the RNA inhibitor is an RNA inhibitor inhibiting the expression of the C5 gene, wherein the antisense strand comprises a region complementary to a target sequence, and the target sequence is 5' uugucccaguauucuauguuu 3' SEQ ID NO: 826, starting at position 3073 of NM_001735.3, and there is at least 85% base complementarity between the sense strand and the antisense strand.

4. The RNA inhibitor or the pharmaceutically acceptable salt thereof according to Claim 1, **characterized in that** the RNA inhibitor is an RNA inhibitor inhibiting the expression of the C3 gene, wherein the antisense strand comprises a region complementary to a target sequence, and the target sequences are 5' gguguugacagauacaucu 3' SEQ ID NO: 1952, starting at position 4329 of NM_000064.4; 5' ggagccuacagagaaauucua 3' SEQ ID NO: 2039, starting at position 771 of NM_000064.4; the target sequence is 5' agaaauucuacuacaucuaua 3' SEQ ID NO: 2048, starting at position 782 of NM_000064.4; the target sequence is 5' gcugaggagaauugcuucaua 3' SEQ ID NO:2240, starting at position 4603 of NM_000064.4; the target sequence is 5' ggagaauugcuucauacaaaa 3' SEQ ID NO: 2245, starting at position 4608 of NM_000064.4; and there is at least 85% base complementarity between the sense strand and the antisense strand.

5. The RNA inhibitor or the pharmaceutically acceptable salt thereof according to Claim 2, **characterized in that** the antisense strand is selected from the following sequence:
| | |
|---|---|
| 5' ucucaauuaaguugacuagacac 3' | SEQ ID NO: 293; |
or a sequence having at least 15 consecutive nucleotides identical to the sequence of SEQ ID NO:293, or a sequence differing by one, two or three nucleotides from the sequence of SEQ ID NO:293,
wherein g= guanosine, a = adenosine, u = uridine, c = cytidine, t = thymidine deoxyribonucleotide.

6. The RNA inhibitor or the pharmaceutically acceptable salt thereof according to Claim 2, **characterized in that** the sense strand is selected from the following sequence:
| | |
|---|---|
| 5' gucuagucaacuuaauugaga 3' | SEQ ID NO: 25; |
or a sequence having at least 15 consecutive nucleotides identical to the sequence of SEQ ID NO:25, or a sequence differing by one, two or three nucleotides from the sequence of SEQ ID NO:25,
wherein g = guanosine, a = adenosine, u = uridine, c = cytidine.

7. The RNA inhibitor or the pharmaceutically acceptable salt thereof according to Claim 3, **characterized in that** the antisense strand is selected from the following sequence:
| | |
|---|---|
| 5' aaacauagaauacugggacaacg 3' | SEQ ID NO: 1278; |
or a sequence having at least 15 consecutive nucleotides identical to the sequence of SEQ ID NO:1278, or a sequence differing by one, two or three nucleotides from the sequence of SEQ ID NO:1278,
wherein g = guanosine, a = adenosine, u = uridine, c = cytidine, t = thymidine deoxyribonucleotide.

8. The RNA inhibitor or the pharmaceutically acceptable salt thereof according to Claim 3, **characterized in that** the sense strand is selected from the following sequence:
| | |
|---|---|
| 5' uugucccaguauucuauguuu 3' | SEQ ID NO: 826; |
or a sequence having at least 15 consecutive nucleotides identical to the sequence of SEQ ID NO:826, or a sequence differing by one, two or three nucleotides from the sequence of SEQ ID NO:826,
wherein g = guanosine, a = adenosine, u = uridine, c = cytidine.

9. The RNA inhibitor or the pharmaceutically acceptable salt thereof according to Claim 4, **characterized in that** the antisense strand is selected from the following sequences:
| | |
|---|---|
| 5' agauguaucugucaacaccau 3' | SEQ ID NO. 2543; |
| 5' uagaauuucucuguaggcuccac 3' | SEQ ID NO. 2630; |
| 5' uauagauguaguagaauuucucu 3' | 3'SEQ ID NO. 2639; |
| 5' uaugaagcaauucuccucagcac 3' | SEQ ID NO. 2831; |
| 5' uuuuguaugaagcaauucuccuc 3' | SEQ ID NO. 2590; |
or a sequence having at least 15 consecutive nucleotides identical to any of the above sequences, or a sequence differing by one, two or three nucleotides from any of the above sequences,
wherein g = guanosine, a = adenosine, u = uridine, c = cytidine, t = thymidine deoxyribonucleotide.

10. The RNA inhibitor or the pharmaceutically acceptable salt thereof according to Claim 4, **characterized in that** the sense strand is selected from the following sequences:
| | |
|---|---|
| 5' gguguugacagauacaucu 3' | SEQ ID NO. 1952; |
| 5' ggagccuacagagaaauucua 3' | SEQ ID NO. 2039; |
| 5' agaaauucuacuacaucuaua 3' | SEQ ID NO. 2048; |
| 5' gcugaggagaauugcuucaua 3' | SEQ ID NO. 2240; |
| 5' ggagaauugcuucauacaaaa 3' | SEQ ID NO. 2245; |
or a sequence having at least 15 consecutive nucleotides identical to any of the above sequences, or a sequence differing by one, two or three nucleotides from any of the above sequences,
wherein g = guanosine, a = adenosine, u = uridine, c = cytidine.

11. The RNA inhibitor or the pharmaceutically acceptable salt thereof according to Claim 2, **characterized in that** the sense strand is SEQ ID NO:25 or a sequence having at least 15 consecutive nucleotides identical thereto, or a sequence differing by one, two or three nucleotides therefrom; and the antisense strand is SEQ ID NO:293 or a sequence having at least 15 consecutive nucleotides identical thereto, or a sequence differing by one, two or three nucleotides therefrom:
| | |
|---|---|
| Sense strand: 5' gucuagucaacuuaauugaga 3' | SEQ ID NO: 25; |
| Antisense strand: 5' ucucaauuaaguugacuagacac 3' | SEQ ID NO: 293; |
wherein g = guanosine, a = adenosine, u = uridine, c = cytidine, t = thymidine deoxyribonucleotide.

12. The RNA inhibitor or the pharmaceutically acceptable salt thereof according to Claim 3, **characterized in that** the sense strand is SEQ ID NO:826 or a sequence having at least 15 consecutive nucleotides identical thereto, or a sequence differing by one, two or three nucleotides therefrom; and the antisense strand is SEQ ID NO:1278 or a sequence having at least 15 consecutive nucleotides identical thereto, or a sequence differing by one, two or three nucleotides therefrom:
| | |
|---|---|
| Sense strand: 5' uugucccaguauucuauguuu 3' | SEQ ID NO: 826; |
| Antisense strand: 5' aaacauagaauacugggacaacg 3' | SEQ ID NO: 1278; |
wherein g = guanosine, a = adenosine, u = uridine, c = cytidine, t = thymidine deoxyribonucleotide.

13. The RNA inhibitor or the pharmaceutically acceptable salt thereof according to Claim 4, **characterized in that** the sense strand is SEQ ID NO:2048 or a sequence having at least 15 consecutive nucleotides identical thereto, or a sequence differing by one, two or three nucleotides therefrom; and the antisense strand is SEQ ID NO:2639 or a sequence having at least 15 consecutive nucleotides identical thereto, or a sequence differing by one, two or three nucleotides therefrom:
| | |
|---|---|
| Sense strand: 5' agaaauucuacuacaucuaua 3' | SEQ ID NO: 2048; |
| Antisense strand: 5' uauagauguaguagaauuucucu 3 | SEQ ID NO: 2639; |
wherein g = guanosine, a = adenosine, u = uridine, c = cytidine, t = thymidine deoxyribonucleotide.

14. The RNA inhibitor or the pharmaceutically acceptable salt thereof according to Claim 4, **characterized in that** the sense strand is SEQ ID NO:2639 or a sequence having at least 15 consecutive nucleotides identical thereto, or a sequence differing by one, two or three nucleotides therefrom; and the antisense strand is SEQ ID NO:2831 or a sequence having at least 15 consecutive nucleotides identical thereto, or a sequence differing by one, two or three nucleotides therefrom:
| | |
|---|---|
| Sense strand: 5' gcugaggagaauugcuucaua 3' | SEQ ID NO: 2240; |
| Antisense strand: 5' uaugaagcaauucuccucagcac 3' | SEQ ID NO: 2831; |
wherein g = guanosine, a = adenosine, u = uridine, c = cytidine, t = thymidine deoxyribonucleotide.

15. The RNA inhibitor or the pharmaceutically acceptable salt thereof according to any one of Claim 1, **characterized in that** at least one nucleotide of the RNA inhibitor is modified.

16. The RNA inhibitor or the pharmaceutically acceptable salt thereof according to Claim 15, **characterized in that** the modification comprises: one or a combination of 2'-fluoro modification, 2'-methoxy modification, phosphorothioate modification, invAb modification, glycerol nucleotide, 3'-terminal deoxythymidine (dT) nucleotide, locked nucleotide, unlocked nucleotide, conformationally restricted nucleotide, constrained ethyl nucleotide, 2'-amino modified nucleotide, 2'-O-allyl modified nucleotide, 2'-C-alkyl modified nucleotide, 2'-hydroxy modified nucleotide, 2'-methoxyethyl modified nucleotide, 2'-O-alkyl modified nucleotide, 2'-phosphate modification or 2-O-(N-methylacetamide) modification, morpholino nucleotide, phosphoramidate, abasic nucleotide, abasic deoxynucleotide, nucleotide containing non-natural base, tetrahydropyran modified nucleotide, 1,5-anhydrohexitol modified nucleotide, cyclohexenyl modified nucleotide, methylphosphonate modification, 5'-phosphate modification, 5'-phosphate analog modification, thermolabile nucleotide, and nucleotide analog.

17. The RNA inhibitor or the pharmaceutically acceptable salt thereof according to Claim 16, **characterized in that** the RNA inhibitor is an RNA inhibitor inhibiting the expression of the CFB gene, and the antisense strand is selected from the following sequence:
| | |
|---|---|
| 5' UsdCsUCdAATUAAGTUfGACUAGACsAsC 3' | SEQ ID NO: 588; |
wherein G = 2'-O-methylguanosine, A = 2'-O-methyladenosine, U = 2'-O-methyluridine, C = 2'-O-methylcytidine; Gs = 2'-O-methyl-3'-phosphorothioguanosine, As = 2'-O-methyl-3'-phosphorothioadenosine, Cs = 2'-O-methyl-3'-phosphorothiocytidine; fG = 2'-fluoroguanosine, fA = 2'-fluoroadenosine, fU = 2'-fluorouridine, fC = 2'-fluorocytidine; fGs = 2'-fluoro-3'-phosphorothioguanosine, T = 2'-deoxythymidine, dA = 2'-deoxyadenosine, dG = 2'-deoxyguanosine.

18. The RNA inhibitor or the pharmaceutically acceptable salt thereof according to Claim 16, **characterized in that** the RNA inhibitor is an RNA inhibitor inhibiting the expression of the C5 gene, and the antisense strand is selected from the following sequence:
| | |
|---|---|
| 5' AsdAsACdAUdAGAAUdACfUGGGACAAsCsG 3' | SEQ ID NO: 1598; |
wherein G = 2'-O-methylguanosine, A = 2'-O-methyladenosine, U = 2'-O-methyluridine, C = 2'-O-methylcytidine; Gs = 2'-O-methyl-3'-phosphorothioguanosine, As = 2'-O-methyl-3'-phosphorothioadenosine, Cs = 2'-O-methyl-3'-phosphorothiocytidine; fG = 2'-fluoroguanosine, fA = 2'-fluoroadenosine, fU = 2'-fluorouridine, fC = 2'-fluorocytidine; fGs = 2'-fluoro-3'-phosphorothioguanosine, T = 2'-deoxythymidine, dA = 2'-deoxyadenosine, dG = 2'-deoxyguanosine.

19. The RNA inhibitor or the pharmaceutically acceptable salt thereof according to Claim 16, **characterized in that** the RNA inhibitor is an RNA inhibitor inhibiting the expression of the C3 gene, and the antisense strand is selected from the following sequences:
| | |
|---|---|
| 5' UsdAsUAdGATGUAGTAfGAAUUUCUsCsU 3' | SEQ ID NO: 1665; |
| 5' UsfAsUGAfAGfCAAUUCfUCfCUCAGCsAsC 3' | SEQ ID NO: 1671; |
wherein G = 2'-O-methylguanosine, A = 2'-O-methyladenosine, U = 2'-O-methyluridine, C = 2'-O-methylcytidine; Gs = 2'-O-methyl-3'-phosphorothioguanosine, As = 2'-O-methyl-3'-phosphorothioadenosine, Us = 2'-O-methyl-3'-phosphorothiouridine, Cs = 2'-O-methyl-3'-phosphorothiocytidine; fG = 2'-fluoroguanosine, fA = 2'-fluoroadenosine, fU = 2'-fluorouridine, fC = 2'-fluorocytidine; fGs = 2'-fluoro-3'-phosphorothioguanosine, fAs = 2'-fluoro-3'-phosphorothioadenosine, fUs = 2'-fluoro-3'-phosphorothiouridine, fCs = 2'-fluoro-3'-phosphorothiocytidine, T = 2'-deoxythymidine, dG = 2'-deoxyguanosine, dAs = 2'-deoxy-3'-phosphorothioadenosine.

20. The RNA inhibitor or the pharmaceutically acceptable salt thereof according to Claim 16, **characterized in that** the RNA inhibitor is an RNA inhibitor inhibiting the expression of the CFB gene, and the sense strand is selected from the following sequence:
| | |
|---|---|
| 5' GsUsCUAGfUCfAfAfCUUAAUUGAsGsA 3' | SEQ ID NO: 559; |
wherein G = 2'-O-methylguanosine, A = 2'-O-methyladenosine, U = 2'-O-methyluridine, C = 2'-O-methylcytidine; Gs = 2'-O-methyl-3'-phosphorothioguanosine, As = 2'-O-methyl-3'-phosphorothioadenosine, Us = 2'-O-methyl-3'-phosphorothiouridine, Cs = 2'-O-methyl-3'-phosphorothiocytidine; fG = 2'-fluoroguanosine, fA = 2'-fluoroadenosine, fU = 2'-fluorouridine, fC = 2'-fluorocytidine.

21. The RNA inhibitor or the pharmaceutically acceptable salt thereof according to Claim 16, **characterized in that** the RNA inhibitor is an RNA inhibitor inhibiting the expression of the C5 gene, and the sense strand is selected from the following sequence:
| | |
|---|---|
| 5' UsUsGUCCfCAfGfUfAUUCUAUGUsUsU 3' | SEQ ID NO: 1537; |
wherein G = 2'-O-methylguanosine, A = 2'-O-methyladenosine, U = 2'-O-methyluridine, C = 2'-O-methylcytidine; Gs = 2'-O-methyl-3'-phosphorothioguanosine, As = 2'-O-methyl-3'-phosphorothioadenosine, Us = 2'-O-methyl-3'-phosphorothiouridine, Cs = 2'-O-methyl-3'-phosphorothiocytidine; fG = 2'-fluoroguanosine, fA = 2'-fluoroadenosine, fU = 2'-fluorouridine, fC = 2'-fluorocytidine.

22. The RNA inhibitor or the pharmaceutically acceptable salt thereof according to Claim 16, **characterized in that** the RNA inhibitor is an RNA inhibitor inhibiting the expression of the C3 gene, and the sense strand is selected from the following sequences:
| | |
|---|---|
| 5' AsGsAAAUfUCfUfAfCUACAUCUAsUsA 3' | SEQ ID NO: 1645; |
| 5' GsCsUGAGfGAfGfAfAUUGCUUCAsUsA 3' | SEQ ID NO: 1651; |
wherein G = 2'-O-methylguanosine, A = 2'-O-methyladenosine, U = 2'-O-methyluridine, C = 2'-O-methylcytidine; Gs = 2'-O-methyl-3'-phosphorothioguanosine, As = 2'-O-methyl-3'-phosphorothioadenosine, Us = 2'-O-methyl-3'-phosphorothiouridine, Cs = 2'-O-methyl-3'-phosphorothiocytidine; fG = 2'-fluoroguanosine, fA = 2'-fluoroadenosine, fU = 2'-fluorouridine, fC = 2'-fluorocytidine.

23. The RNA inhibitor or the pharmaceutically acceptable salt thereof according to Claim 16, **characterized in that** the RNA inhibitor is an RNA inhibitor inhibiting the expression of the CFB gene, the sense strand is SEQ ID NO:559 or a sequence differing by one, two or three nucleotides therefrom, and the antisense strand is SEQ ID NO:588 or a sequence differing by one, two or three nucleotides therefrom:
| | | | |
|---|---|---|---|
| Sense strand: | 5' GsUsCUAGfUCfAfAfCUUAAUUGAsGsA 3' | SEQ ID NO: | 559; |
| Antisense strand: | 5' UsdCsUCdAATUAAGTUfGACUAGACsAsC 3' | SEQ ID NO: | 588; |
wherein G = 2'-O-methylguanosine, A = 2'-O-methyladenosine, U = 2'-O-methyluridine, C = 2'-O-methylcytidine; Gs = 2'-O-methyl-3'-phosphorothioguanosine, As = 2'-O-methyl-3'-phosphorothioadenosine, Us = 2'-O-methyl-3'-phosphorothiouridine, Cs = 2'-O-methyl-3'-phosphorothiocytidine; fG = 2'-fluoroguanosine, fA = 2'-fluoroadenosine, fU = 2'-fluorouridine, fC = 2'-fluorocytidine; fGs = 2'-fluoro-3'-phosphorothioguanosine, fAs = 2'-fluoro-3'-phosphorothioadenosine, fUs = 2'-fluoro-3'-phosphorothiouridine, fCs = 2'-fluoro-3'-phosphorothiocytidine, T = 2'-deoxythymidine, Ts = 2'-deoxy-3'-phosphorothiothymidine, dA = 2'-deoxyadenosine.

24. The RNA inhibitor or the pharmaceutically acceptable salt thereof according to Claim 16, **characterized in that** the RNA inhibitor is an RNA inhibitor inhibiting the expression of the C5 gene, the sense strand is SEQ ID NO:1537 or a sequence differing by one, two or three nucleotides therefrom, and the antisense strand is SEQ ID NO:1598 or a sequence differing by one, two or three nucleotides therefrom:
| | | | |
|---|---|---|---|
| Sense strand: | 5' UsUsGUCCfCAfGfUfAUUCUAUGUsUsU 3' | SEQ ID NO: | 1537; |
| Antisense strand: | 5' AsdAsACdAUdAGAAUdACfUGGGACAAsCsG 3' | SEQ ID NO: | 1598; |
wherein G = 2'-O-methylguanosine, A = 2'-O-methyladenosine, U = 2'-O-methyluridine, C = 2'-O-methylcytidine; Gs = 2'-O-methyl-3'-phosphorothioguanosine, As = 2'-O-methyl-3'-phosphorothioadenosine, Us = 2'-O-methyl-3'-phosphorothiouridine, Cs = 2'-O-methyl-3'-phosphorothiocytidine; fG = 2'-fluoroguanosine, fA = 2'-fluoroadenosine, fU = 2'-fluorouridine, fC = 2'-fluorocytidine; fGs = 2'-fluoro-3'-phosphorothioguanosine, fAs = 2'-fluoro-3'-phosphorothioadenosine, fUs = 2'-fluoro-3'-phosphorothiouridine, fCs = 2'-fluoro-3'-phosphorothiocytidine, T = 2'-deoxythymidine, Ts = 2'-deoxy-3'-phosphorothiothymidine, dA = 2'-deoxyadenosine.

25. The RNA inhibitor or the pharmaceutically acceptable salt thereof according to Claim 16, **characterized in that** the RNA inhibitor is an RNA inhibitor inhibiting the expression of the C3 gene, the sense strand is SEQ ID NO:1651 or a sequence differing by one, two or three nucleotides therefrom, and the antisense strand is SEQ ID NO:1671 or a sequence differing by one, two or three nucleotides therefrom:
| | | | |
|---|---|---|---|
| Sense strand: | 5' AsGsAAAUfUCfUfAfCUACAUCUAsUsA 3' | SEQ ID NO: | 1645; |
| Antisense strand: | 5' UsdAsUAdGATGUAGTAfGAAUUUCUsCsU 3' | SEQ ID NO: | 1665; |
alternatively, the sense strand is SEQ ID NO:1645 or a sequence differing by one, two or three nucleotides therefrom, and the antisense strand is SEQ ID NO:1665 or a sequence differing by one, two or three nucleotides therefrom:
| | | | |
|---|---|---|---|
| Sense strand: | 5' GsCsUGAGfGAfGfAfAUUGCUUCAsUsA 3' | SEQ ID NO: | 1651; |
| Antisense strand: | 5' UsfAsUGAfAGfCAAUUCfUCfCUCAGCsAsC 3' | SEQ ID NO: | 1671; |
wherein G = 2'-O-methylguanosine, A = 2'-O-methyladenosine, U = 2'-O-methyluridine, C = 2'-O-methylcytidine; Gs = 2'-O-methyl-3'-phosphorothioguanosine, As = 2'-O-methyl-3'-phosphorothioadenosine, Us = 2'-O-methyl-3'-phosphorothiouridine, Cs = 2'-O-methyl-3'-phosphorothiocytidine; fG = 2'-fluoroguanosine, fA = 2'-fluoroadenosine, fU = 2'-fluorouridine, fC = 2'-fluorocytidine; fGs = 2'-fluoro-3'-phosphorothioguanosine, fAs = 2'-fluoro-3'-phosphorothioadenosine, fUs = 2'-fluoro-3'-phosphorothiouridine, fCs = 2'-fluoro-3'-phosphorothiocytidine, T = 2'-deoxythymidine, dG = 2'-deoxyguanosine, dAs = 2'-deoxy-3'-phosphorothioadenosine.

26. The RNA inhibitor or the pharmaceutically acceptable salt thereof according to any one of Claims 1-25, **characterized in that** the RNA inhibitor further contains a carrier structure 5'MVIP and 3'MVIP, and the structure of the RNA inhibitor is as shown in Formula la, Ib or Ic: 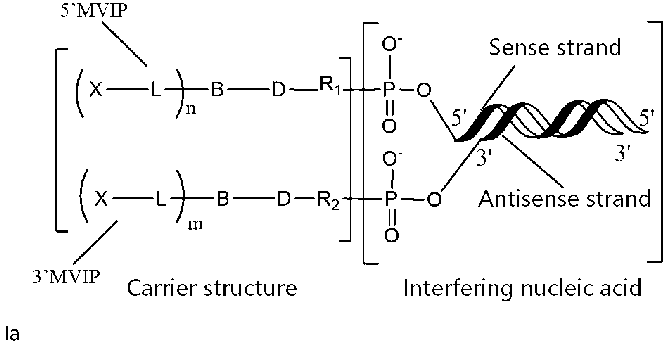 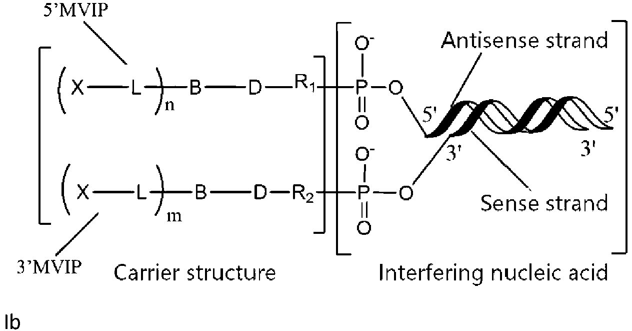 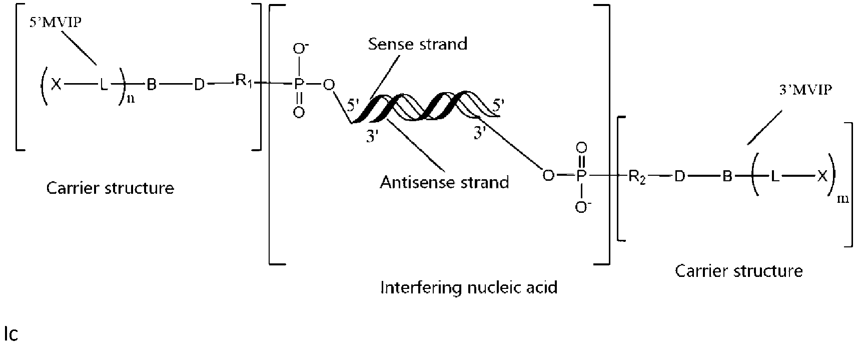 Wherein,
the 5'MVIP is composed of a junction point R₁, a linking chain D, a linker B, a branched chain L and a liver-targeting specific ligand X, and is connected to the 5'-end of the sense strand or the 5'-end of the antisense strand through the junction point R₁, and its structure is as shown in General Formula I:
(X-L)ₙ-B-D-R₁- I
the 3'MVIP is composed of a junction point R₂, a linking chain D, a linker B, a branched chain L and a liver-targeting specific ligand X, and is connected to the 3'-end of the sense strand or the 3'-end of the antisense strand through the junction point R₂, and its structure is as shown in General Formula II:
(X-L)ₘ-B-D-R₂- II
Wherein,
n and m are each independently any integer of 0-4, each independently preferably an integer of 1-3, and n+m is an integer of 2-6, preferably n+m=2, 3 or 4, more preferably 4;
the junction point R₁ is a heterocyclic or carbocyclic structure containing N, S or O as shown below: 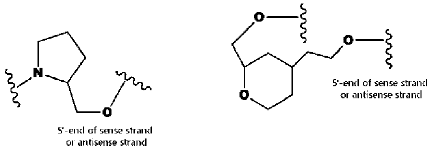 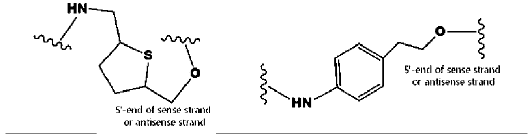
alternatively, the R₁ is -NH(CH₂)ₓCH₂O-, wherein x is any integer of 3-12, preferably any integer of 4-6;
the junction point R₂ is a heterocyclic or carbocyclic structure containing N, S or O as shown below: 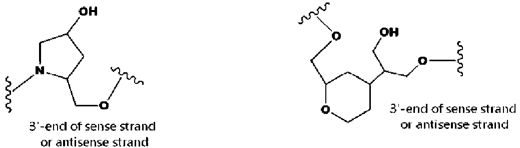 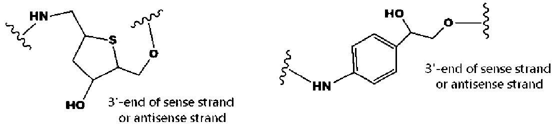
alternatively, the junction point R₂ is -NH(CH₂)ₓ₁CH(OH)(CH₂)ₓ₂CH₂O-, wherein x1 is any integer of 1-4, x2 is any integer of 0-4;
the liver-targeting specific ligand X is the same or different within each of 5'MVIP and 3'MVIP or between 5'MVIP and 3'MVIP, and is selected from monosaccharides and derivatives thereof, preferably N-acetylgalactosamine and derivatives thereof, more preferably selected from the following structures: 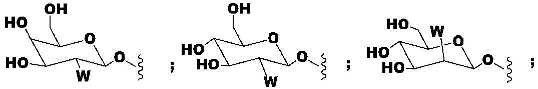 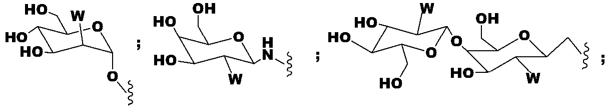 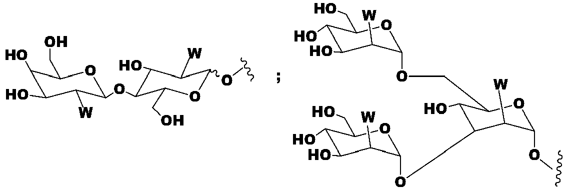
Wherein, W is selected from one or two of -OH, -NHCOOH and -NHCO(CH₂)_{q}CH₃, wherein q is an integer of 0-4;
the branched chain L is the same or different within each of 5'MVIP and 3'MVIP or between 5'MVIP and 3'MVIP, and is selected from one or more of the following structures: 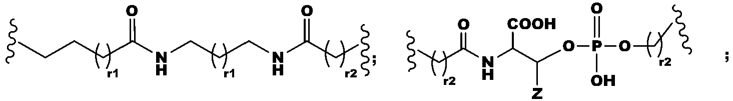 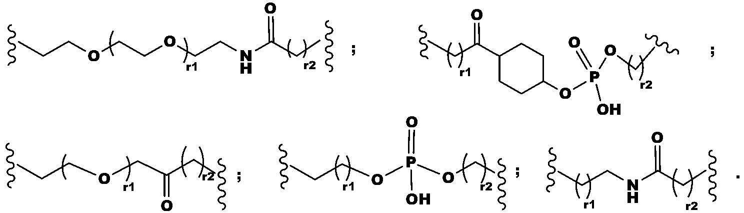 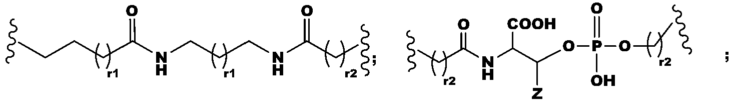 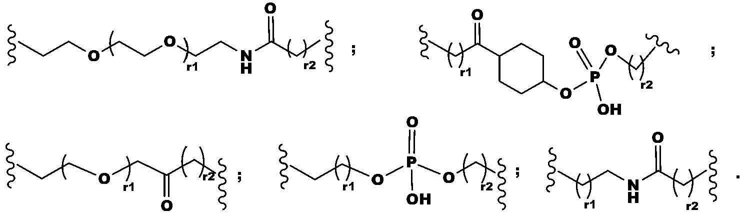
Wherein, r1 is any integer of 1-12, r2 is any integer of 0-20, Z is H, alkyl or amido group, and the alkyl is such as C₁-C₅ alkyl;
the linker B is the same or different within each of 5'MVIP and 3'MVIP or between 5'MVIP and 3'MVIP, and is selected from the following structures: 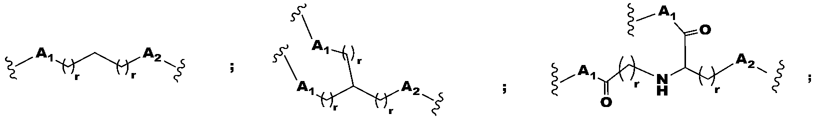 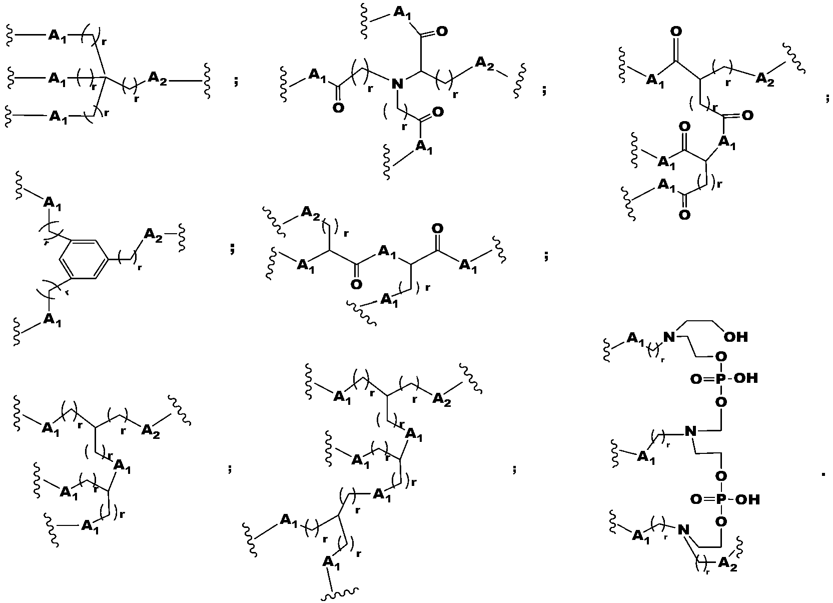
Wherein, A₁ and A₂ are each independently C, O, S, -NH-, carbonyl, amido, phosphoryl or thiophosphoryl, r is any integer of 0-4;
the linking chain D is the same or different within each of 5'MVIP and 3'MVIP or between 5'MVIP and 3'MVIP, and is selected from the following structures: 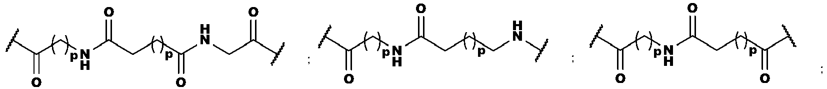 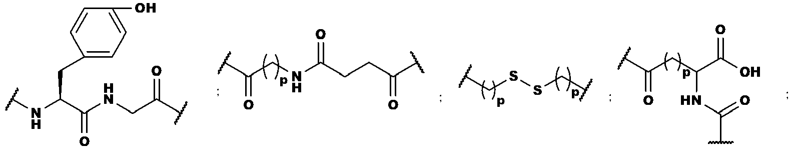 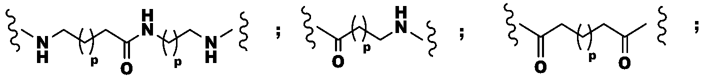 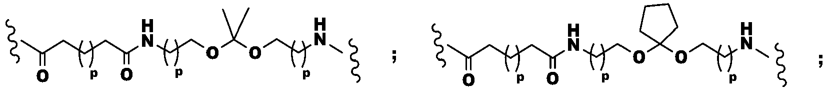 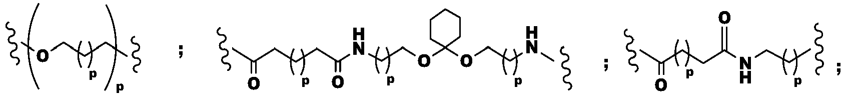 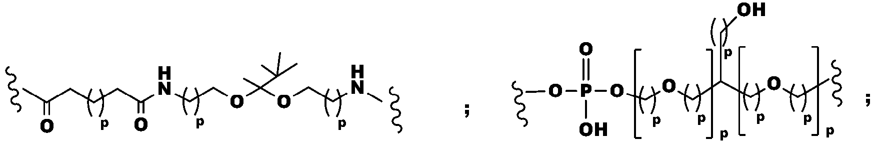 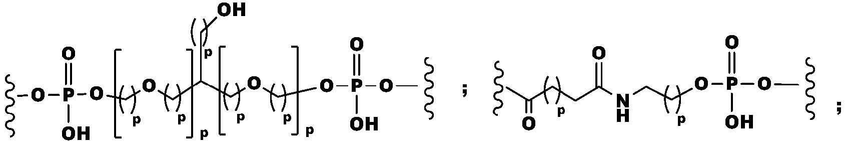 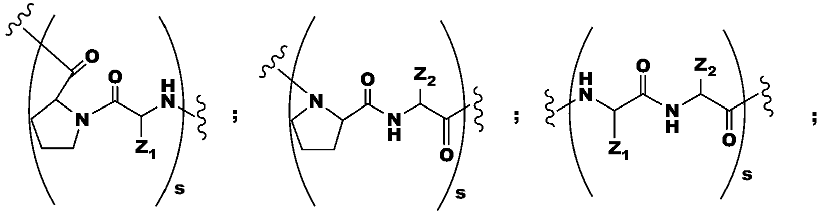 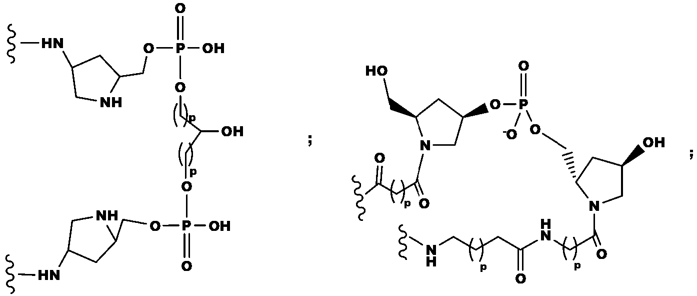 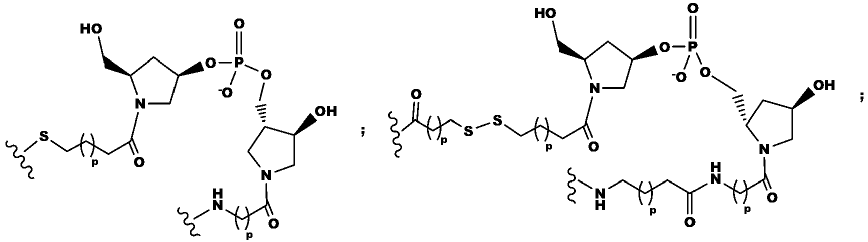 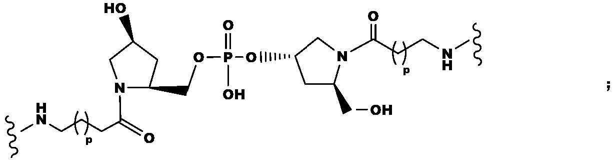 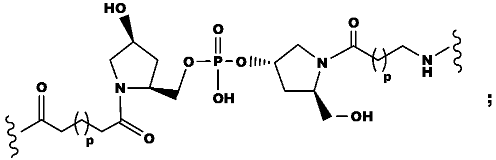 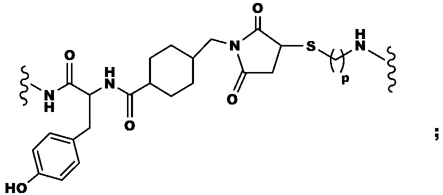
Wherein, each p is independently any integer of 1-20; s is any integer of 2-13; Z₁ and Z₂ are the same or different substituent groups.

27. The RNA inhibitor or the pharmaceutically acceptable salt thereof according to Claim 26, **characterized in that** the 5'MVIP is 5'MVIP01 or 5'MVIP09 as shown below, and the 3'MVIP is 3'MVIP01, 3'MVIP09 or 3'MVIP17 as shown below:

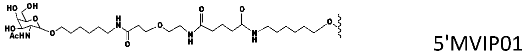

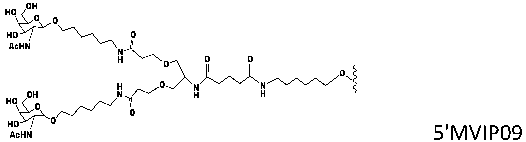

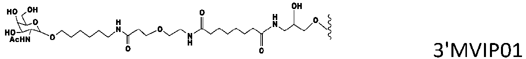

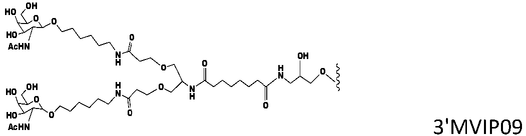

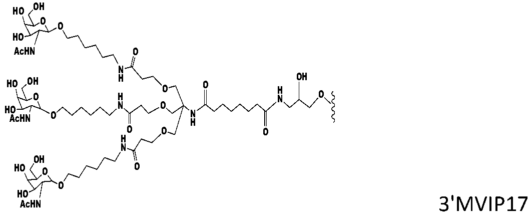

28. The RNA inhibitor or the pharmaceutically acceptable salt thereof according to Claim 27, **characterized in that** the combination of 5'MVIP of the sense strand and 3'MVIP of the antisense strand is 5'MVIP01/3'MVIP01, 5'MVIP01/3'MVIP17 or 5'MVIP09/3'MVIP09, or the combination of 5'MVIP of the sense strand and 3'MVIP of the sense strand is 5'MVIP01/3'MVIP09 or 5'MVIP09/3'MVIP01.

29. The CFB RNA inhibitor or the pharmaceutically acceptable salt thereof according to Claim 1, **characterized in that** the RNA inhibitor is selected from Kylo-17-DS2911.

30. The C5 RNA inhibitor or the pharmaceutically acceptable salt thereof according to Claim 1, **characterized in that** the RNA inhibitor is selected from Kylo-19-DS7881.

31. The C3 RNA inhibitor or the pharmaceutically acceptable salt thereof according to Claim 1, **characterized in that** the RNA inhibitor is selected from Kylo-27-DS8201, Kylo-27-DS8141.

32. Use of the RNA inhibitor or the pharmaceutically acceptable salt thereof according to any one of Claims 1-31 in the preparation of a medicament for the treatment and/or prevention of diseases associated with elevated levels of the complement system, **characterized in that** the diseases include but are not limited to lipid metabolism disorders.

33. A pharmaceutical composition, **characterized in that** the pharmaceutical composition comprises the RNA inhibitor according to any one of Claims 1-31 or another therapeutic agent for the treatment or prevention of complement system-related diseases.

34. A pharmaceutical composition, **characterized in that** the pharmaceutical composition comprises the RNA inhibitor according to any one of Claims 1-31 or a pharmaceutically acceptable salt thereof and pharmaceutically acceptable excipients, and the dosage form is oral preparation, intravenous injection, or subcutaneous or intramuscular injection, preferably subcutaneous injection.
